# EUROPEAN PATENT APPLICATION

(11) **EP 4 647 075 A2**
(43) Date of publication of application: **12.11.2025**
(21) Application number: 25205463.0
(22) Date of filing: 14.08.2021
(51) Int. Cl.: A61K 31/519

(54) **NON-HYDRATED KETONE INHIBITORS OF NAV1.7 FOR THE TREATMENT OF PAIN**

(30) Priority: 14.08.2020 US 202063066012 P
(62) Divisional of application: 21766757.5
(71) Applicant: SiteOne Therapeutics, Inc., South San Francisco, CA 94080 (US)
(72) Inventor: PAJOUHESH, Hassan, San Francisco, 94080 (US); MULCAHY, John, San Francisco, 94080 (US); MONTELEONE, Dennis, San Francisco, 94080 (US); ZHOU, Xiang, San Francisco, 94080 (US)
(74) Representative: Petty, Catrin Helen

(57) **Abstract**

Provided herein are compounds, pharmaceutical compositions comprising the compounds, methods of preparing the compounds, and methods of using the compounds and compositions in treating conditions associated with voltage-gated sodium channel function where the compounds are 11,13-modified, non-hydrated ketone saxitoxins.

## Description

### CROSS-REFERENCE TO RELATED APPLICATIONS

This application claims priority to and the benefit of U.S. Provisional Patent Application No. 63/066,012 filed August 14, 2020, the entirety of which is herein incorporated for all purposes.

### FIELD

Provided herein are compounds, methods of making the compounds, pharmaceutical compositions comprising the compounds, and methods of using the compounds and compositions in treating conditions associated with voltage-gated sodium channel function, for example pain and conditions associated with pain. The compounds are 11,13-modified, non-hydrated ketone saxitoxins. Also provided herein are methods of treating pain in a mammal comprising administering a therapeutically or prophylactically effective amount of a 11,13-modified, non-hydrated ketone saxitoxin or composition to a mammal. In some or any embodiments, the mammal is a human.

### BACKGROUND

Voltage-gated sodium channels are large integral membrane protein complexes present in neurons and excitable tissues where they contribute to processes such as membrane excitability and muscle contraction (Ogata et al., Jpn. J. Pharmacol. (2002) 88(4) 365-77). They have been identified as a primary target for the treatment of pain. Genes encoding for nine distinct mammalian isoforms of Na_{V} channels (Na_{V} isoforms 1.1-1.9) have been sequenced. Variation in the gating properties of different Na_{V} isoforms, cellular distributions, and expression levels influence the physiology of nerve cell conduction. A mounting body of evidence suggests that individual Nav isoforms Nav 1.3, 1.7, 1.8 and 1.9 are disproportionately involved in pain signaling and nociception, and that an isoform-specific inhibitor of Na_{V} could provide pain relief without the accompanying undesirable effects of a non-specific Na_{V} antagonist or an opioid drug (Momin et al., Curr Opin Neurobiol. 18(4): 383-8, 2008; Rush et al., J. Physiol. 579(Pt 1): 1-14, 2007).

A human genetic disorder resulting in a loss of function mutation in Nav 1.7 has been correlated with congenital insensitivity to pain (Cox et al., Nature. (2006) 444(7121) 894-898). The design of a drug which selectively inhibits Na_{V} 1.7 over the other Na_{V} channels is therefore desirable. Such a drug design is challenging given the high structural homology (75-96%) of the mammalian Na_{V} isoforms.

There exists a need for compounds which treat pain and conditions associated with voltage-gated sodium channel function, particularly which selectively inhibit Nav 1.7 over other Nav isoforms.

### SUMMARY

Provided herein are compounds, methods of making the compounds, pharmaceutical compositions comprising the compounds, and methods of using the compounds and compositions for the treatment of conditions modulated by voltage-gated sodium channels, in some or any embodiments, in the treatment of pain. The compounds are 11,13-modified, non-hydrated ketone saxitoxins. Also provided herein are methods of treating pain and/or conditions modulated by voltage-gated sodium channels in a mammal comprising administering a therapeutically or prophylactically effective amount of a 11,13-modified, non-hydrated ketone saxitoxin or composition to a mammal. In some or any embodiments, the mammal is a human. Also provided herein are compounds which have improved stability.

Saxitoxin has the chemical structure provided below with selected atom numbering used herein:

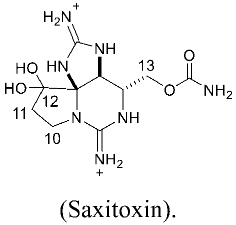

Compounds containing a hydrated ketone functional group at the C12 position (*i.e.* >C(OH)₂) can epimerize at the C11 position under certain conditions leading to a mixture of C11R and C11S diastereomers. For example, it has been shown that saxitoxinethanoic acid, a modified saxitoxin substituted with ethanoic acid at the C11 position, epimerizes at the C11 position and exists as a mixture of C11R and C11S diastereomers when synthesized (Wang et al., Angew Chem Int Ed Engl. 12(55): 11600-11603, 2016) and when isolated as a natural product (Arakawa et al., Toxicon 33(12): 1577-1584, 1995). Similarly, gonyautoxin, which is saxitoxin substituted with a sulfate at C11, has been shown to undergo epimerization when synthesized (Mulcahy et al., J. Am. Chem. Soc. 130(38): 12630-12631, 2008) and when isolated (Shimizu et al., J. Am. Chem. Soc. 98(17): 5414-5416, 1976). In contrast, compounds described herein are C12 mono-hydroxy analogues that do not epimerize at C11.

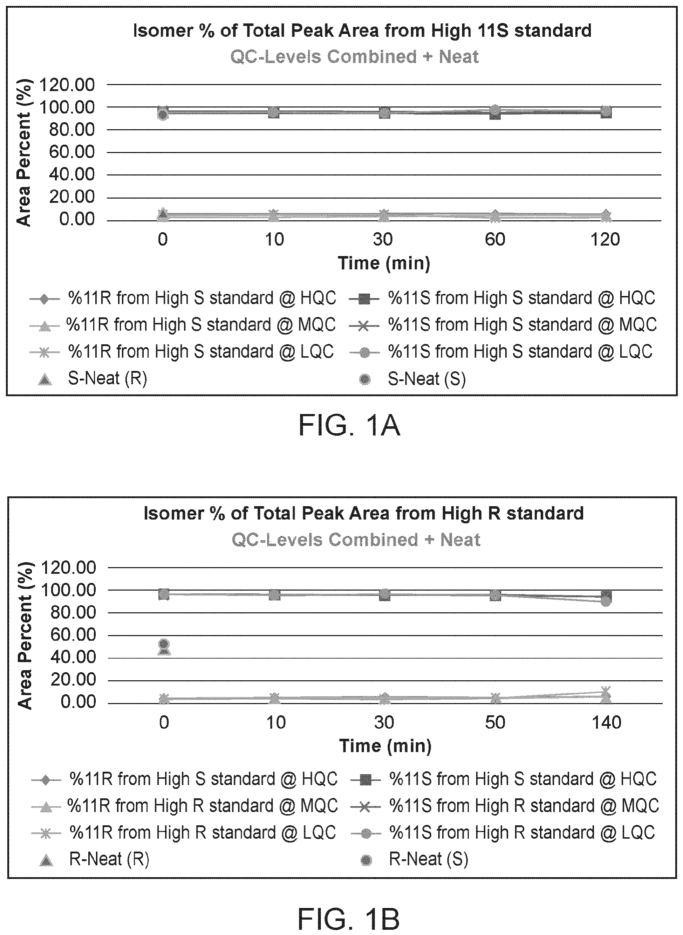

For example, the epimerization of a select hydrated ketone compound ("Comparative Compound") is described in Example 8. The ratio of the C11S and C11R diastereomer upon addition to human, monkey, and rat whole blood:buffer was determined for two solutions of the comparative saxitoxin compound. The first solution was high in the C11S diastereomer and the second solution was high in the C11R diastereomer. As described in Example 8 and shown in FIGS. 1A - 3B, upon addition to blood:buffer, the diastereomers from both the high C11S solution and the high C11R solution underwent epimerization and the equilibrium favored the C11S diastereomer. The equilibrium that favored the C11S diastereomer was relatively constant across all species.

Equilibriums that favor one diastereomer over the other can make it challenging to isolate, test, and administer the diastereomer which is unfavored in the equilibrium. In certain instances, it may be difficult to determine if the activity resides in the C11S or C11R diastereomer. The Comparative Compound of Example 8 exists as an equilibrium mixture which favors the C11S diastereomer, and it is difficult to determine which diastereomer might be more potent when tested. In contrast, compounds described herein are C12 mono-hydroxy analogues that do not epimerize at C11, and in fact, it has been surprisingly discovered that the activity of the compounds described herein primarily resides in the C11R diastereomer. C11R diastereomer activity could not have been predicted in advance, and may even be difficult to determine by testing. This is because of the epimerization of similar hydrated ketone compounds with C11S and C11R diastereomers since these diastereomers exist in equilibrium and one diastereomer, the C11S diastereomer, may be favored over the other. In addition, a particular advantage of the Compounds of Formula (I) are that they can be prepared as a particular isomer at C11, *i.e.* the most potent isomer.

Removal of the hydrated ketone functional group may improve stability in buffer and in certain biological matrices (e.g. plasma, hepatocytes, etc.). The compounds described herein are stable under many conditions. As described in Example 3, a salt form of a select compound of Formula (I) was subjected to forced degradation studies to determine its stability. Virtually no degradation was observed under thermal, photolytic, or acidic stress, and only 5% degradation was observed after 24 hours at ambient conditions under basic conditions. Further, under azobisisobutyronitrile (AIBN)-based oxidation, only 5% degradation was observed and under 4,4'-azobis(4-cyanovaleric acid) (ACVA)-based oxidation, no degradation was observed. The compound was also stable under bench top, freeze-thaw, and long-term stability studies (34 days) in rat and monkey plasma (Example 4). Further, as described in Example 5, two compounds of Formula (I) were stable for up to two hours in cynomolgus monkey plasma at room temperature and 4 °C. Therefore, in certain embodiments, the compounds of Formula (I), substituted with a C12 mono-hydroxyl group, represent stable saxitoxin analogs.

In another aspect, provided herein is a Compound of Formula (I): 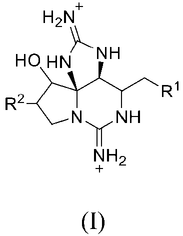 wherein
R¹ is -NR^{3a}R³, -NR⁴C(O)R^{4a}, -CH₂NR⁴C(O)R^{4a}, heteroaryl, -CH₂NH₂, -CH₂CH₂NH₂, -CH₂NH₃⁺, -CH₂CH₂NH₃⁺, -NR⁵S(O)₂R^{5a}, -CH₂NR⁵S(O)₂R^{5a}, -NR⁶C(O)NR^{6a}R^{6b}, -CH₂NR⁶C(O)NR^{6a}R^{6b}, -NR⁸C(O)OR^{8a}, or -CH₂NR⁸C(O)OR^{8a};
R² is -NR⁷C(O)R^{7a}, -NR⁷S(O)₂R^{7a}, or -NR⁷C(O)OR^{7a};
R³ is hydrogen or C₁₋₆alkyl;
R^{3a} is hydrogen or C₁₋₆alkyl;
R⁴ is hydrogen or C₁₋₆alkyl;
R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
one pair of two R^{4b} groups are present on one carbon, the second pair of two R^{4b} are present on another carbon, and each pair of R^{4b} are taken together to form an oxo group;
R⁵ is hydrogen or C₁₋₆alkyl;
R^{5a} is heterocycloalkyl, amino, C₁₋₆alkylamino, or di- C₁₋₆alkylamino;
R⁶ is hydrogen or C₁₋₆alkyl;
R^{6a} is hydrogen, or C₁₋₆alkyl;
R^{6b} is hydrogen, C₁₋₆alkyl, C₁₋₆alkoxy or cycloalkyl;
R⁸ is hydrogen or C₁₋₆alkyl;
R^{8a} is hydrogen, C₁₋₆alkyl, cycloalkyl, or phenyl;
R⁷ is hydrogen or C₁₋₆alkyl;
R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; or
a pharmaceutically acceptable salt, hydrate, solvate, stereoisomer, tautomer, mixture, or combination thereof.

In another aspect, provided herein is a Compound of Formula (P-I): 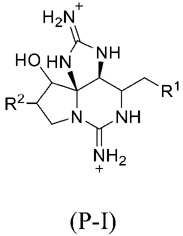 wherein
R¹ is -NR^{3a}R³, -NR⁴C(O)R^{4a}, -CH₂NR⁴C(O)R^{4a}, heteroaryl, -CH₂NH₂, -CH₂CH₂NH₂, -CH₂NH₃⁺, -CH₂CH₂NH₃⁺, -NR⁵S(O)₂R^{5a}, -CH₂NR₅S(O)₂R^{5a}, -NR⁶C(O)NR^{6a}R^{6b}, -CH₂NR⁶C(O)N^{R6a}R^{6b}, -NR⁸C(O)OR^{8a}, or -CH₂NR⁸C(O)OR^{8a};
R² is -NR⁷C(O)R^{7a}, -NR⁷S(O)₂R^{7a}, or -NR⁷C(O)OR^{7a};
R³ is hydrogen or C₁₋₆alkyl;
R^{3a} is hydrogen or C₁₋₆alkyl;
R⁴ is hydrogen or C₁₋₆alkyl;
R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl; cycloalkylalkyl; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
each R^{4b}, when present, is independently C₁₋₆alkyl, hydroxy, halo, halo-C₁₋₆alkyl, C₁₋₆alkoxy, cyano, or phenyl;
R⁵ is hydrogen or C₁₋₆alkyl;
R^{5a} is heterocycloalkyl, amino, C₁₋₆alkylamino, or di- C₁₋₆alkylamino;
R⁶ is hydrogen or C₁₋₆alkyl;
R^{6a} is hydrogen, or C₁₋₆alkyl;
R^{6b} is hydrogen, C₁₋₆alkyl, C₁₋₆alkoxy or cycloalkyl;
R⁸ is hydrogen or C₁₋₆alkyl;
R^{8a} is hydrogen, C₁₋₆alkyl, cycloalkyl, or phenyl;
R⁷ is hydrogen or C₁₋₆alkyl;
R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; or
a pharmaceutically acceptable salt, hydrate, solvate, stereoisomer, tautomer, mixture, or combination thereof.

In another aspect, provided herein are pharmaceutical compositions, single unit dosage forms, and kits suitable for use in treating pain and/or conditions modulated by voltage-gated sodium channels which comprise a therapeutically or prophylactically effective amount of a compound provided herein, *e.g.,* of some or any of the embodiments of Formula (I), (P-I), (P-Ia), (P-Ib-1), (P-Ib-2), (P-Ic-1), (P-Ic-2), (P-Id-1), and (P-Id-2) and compounds 1-46,49-52, and 53-70.

In an aspect, a method of treatment of pain and/or conditions modulated by voltage-gated sodium channels is provided comprising administering to an individual in need thereof a therapeutically or prophylactically effective amount of a 11,13-modified, non-hydrated ketone saxitoxin described herein, *e.g.,* of some or any of the embodiments of Formula (I), (P-I), (P-Ia), (P-Ib-1), (P-Ib-2), (P-Ic-1), (P-Ic-2), (P-Id-1), and (P-Id-2) and compounds 1-46, 49-52, and 53-70, or administering to an individual in need thereof a pharmaceutical composition comprising a therapeutically or prophylactically effective amount of a 11,13-modified, non-hydrated ketone saxitoxin described herein, *e.g.,* of some or any of the embodiments of Formula (I), (P-I), (P-Ia), (P-Ib-1), (P-Ib-2), (P-Ic-1), (P-Ic-2), (P-Id-1), and (P-Id-2) and compounds 1-46, 49-52, and 53-70 and a pharmaceutically acceptable carrier.

In a further aspect, provided herein is a compound of Formula (X) 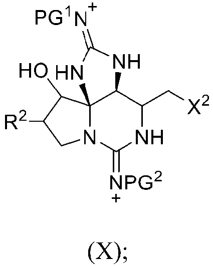 or a salt thereof,
wherein
PG¹ is a nitrogen-protecting group;
PG² is a nitrogen-protecting group;
X² is halo (preferably Br, I), CN, or N₃;
R² is -NR⁷C(O)R^{7a}, -NR⁷S(O)₂R^{7a}, or -NR⁷C(O)OR^{7a};
R⁷ is hydrogen or C₁₋₆alkyl;
R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl.

In an additional aspect, provided herein is a method of preparing a compound of Formula (I) comprising
a) deprotecting and reducing a compound of Formula (X-1) 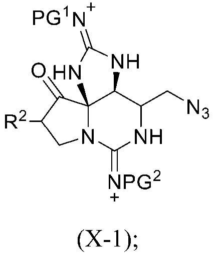 to obtain a compound of Formula (X-2): 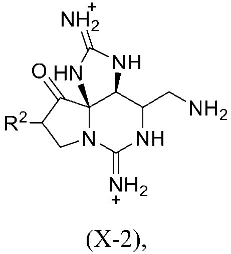 or a salt thereof;
b) reducing the ketone of Formula (X-2) to obtain a compound of Formula (X-3): 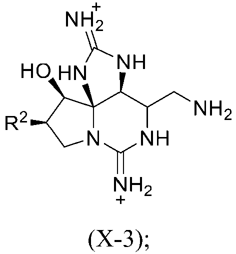 or a salt thereof;
c) coupling the compound of Formula (X-3) with a compound of Formula X⁴-LG to obtain a compound of Formula (I); wherein
   PG¹ is a nitrogen-protecting group;
   PG² is a nitrogen-protecting group;
   X⁴ is -C(O)R^{4a}, -S(O)₂R^{5a}, -C(O)NR^{6a}R^{6b}, or -C(O)OR^{8a};
   LG is a leaving group selected from the group consisting of halo, 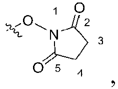 and 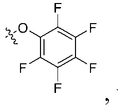 where 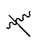 represents the point of attachment of LG to X⁴;
   R² is -NR⁷C(O)R^{7a}, -NR⁷S(O)₂R^{7a}, or -NR⁷C(O)OR^{7a};
   R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
   each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   one pair of two R^{4b} groups are present on one carbon, the second pair of two R^{4b} are present on another carbon, and each pair of R^{4b} are taken together to form an oxo group;
   R^{5a} is heterocycloalkyl, amino, C₁₋₆alkylamino, or di- C₁₋₆alkylamino;
   R^{6a} is hydrogen, or C₁₋₆alkyl;
   R^{6b} is hydrogen, C₁₋₆alkyl, C₁₋₆alkoxy or cycloalkyl;
   R^{8a} is hydrogen, C₁₋₆alkyl, cycloalkyl or phenyl;
   R⁷ is hydrogen or C₁₋₆alkyl;
   R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
   each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; to yield a compound of Formula I; and
d) optionally isolating the compound of Formula I.

In a further aspect, provided herein is a method of preparing a compound of Formula (I) comprising
a) reducing the ketone in a compound of Formula (X-1) 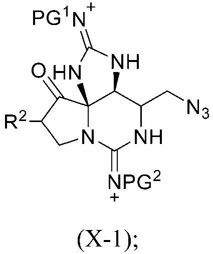 to obtain a compound of Formula (X-2B): 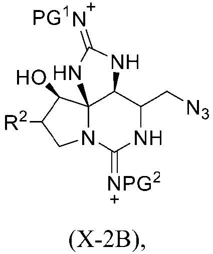 or a salt thereof;
b) deprotecting and reducing the compound of Formula (X-2B) to obtain a compound of Formula (X-3) 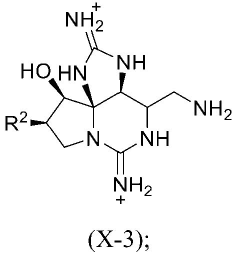 or a salt thereof;
c) coupling the compound of Formula (X-3) with a compound of Formula X⁴-LG to obtain a compound of Formula I; wherein
   PG¹ is a nitrogen-protecting group;
   PG² is a nitrogen-protecting group;
   X⁴ is -C(O)R^{4a}, -S(O)₂R^{5a}, -C(O)NR^{6a}R^{6b}, or -C(O)OR^{8a;}
   LG is a leaving group selected from the group consisting of halo, 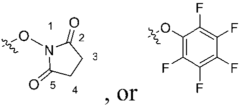 , where 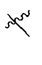 represents the point of attachment of LG to X⁴;
   R² is -NR⁷C(O)R^{7a}, -NR⁷S(O)₂R^{7a}, or -NR⁷C(O)OR^{7a};
   R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
   each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   one pair of two R^{4b} groups are present on one carbon, the second pair of two R^{4b} are present on another carbon, and each pair of R^{4b} are taken together to form an oxo group;
   R^{5a} is heterocycloalkyl, amino, C₁₋₆alkylamino, or di- C₁₋₆alkylamino;
   R^{6a} is hydrogen or C₁₋₆alkyl;
   R^{6b} is hydrogen, C₁₋₆alkyl, C₁₋₆alkoxy, or cycloalkyl;
   R^{8a} is hydrogen, C₁₋₆alkyl, cycloalkyl, or phenyl;
   R⁷ is hydrogen or C₁₋₆alkyl;
   R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
   each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; to yield a compound of Formula I; and
d) optionally isolating the compound of Formula I.

### BRIEF DESCRIPTION OF FIGURES

FIG. 1A is a graph of the 11S and 11R diasteromers percentage of total peak area from the high 11S standard in monkey blood:buffer as described in Example 8. The percentage of 11S and 11R from the high 11S standard is shown for the high quality control (HQC), medium quality control (MQC), and low quality control (LQC). The percentage of 11S and 11R diastereomers is also shown for the high 11S neat material (S-neat (R) and S-neat (S)). The x-axis is time measured in minutes and the y-axis is area measured in percent.
FIG. 1B is a graph of the 11S and 11R diastereomers percentage of total peak area from the high 11R standard in monkey blood:buffer as described in Example 8. The percentage of 11S and 11R from the high 11R standard is shown for the high quality control (HQC), medium quality control (MQC), and low quality control (LQC). The percentage of 11S and 11R diastereomers is also shown for the high 11R neat material (R-neat (R) and R-neat (S)). The x-axis is time measured in minutes and the y-axis is area measured in percent. As discussed in Example 5, both the high 11S material (as shown in FIG. 1A) and the high 11R material come to a relatively constant ratio of diastereomers upon addition to blood.

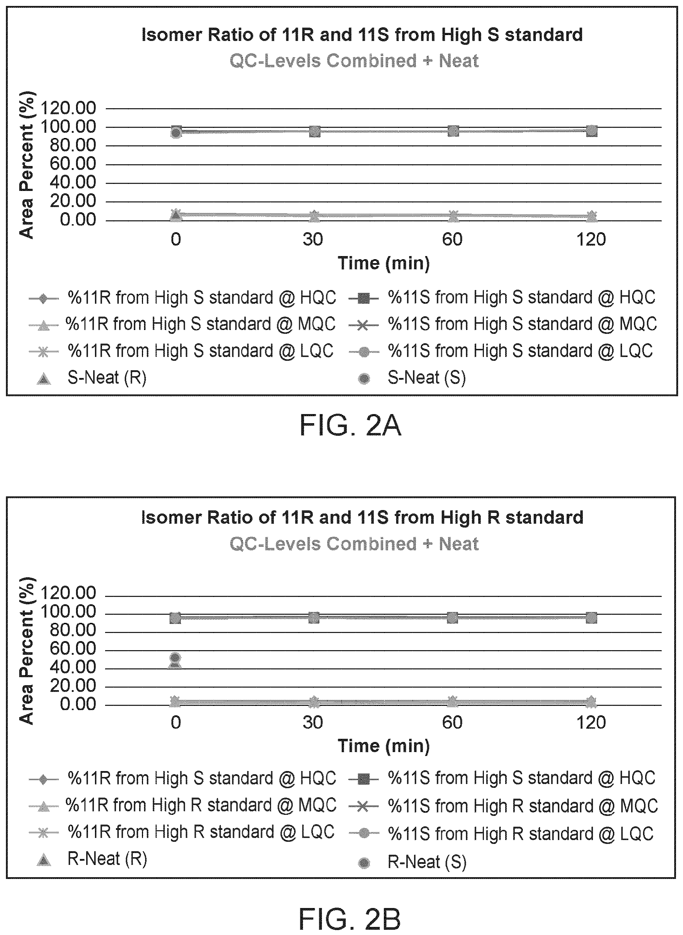

FIG. 2A is a graph of the 11S and 11R diastereomers percentage of total peak area from the high 11S standard in human blood:buffer as described in Example 8. The percentage of 11S and 11R from the high 11S standard is shown for the high quality control (HQC), medium quality control (MQC), and low quality control (LQC). The percentage of 11S and 11R diastereomers is also shown for the high 11S neat material (S-neat (R) and S-neat (S)). The x-axis is time measured in minutes and the y-axis is area measured in percent.
FIG. 2B is a graph of the 11S and 11R diastereomers percentage of total peak area from the high 11R standard in human blood:buffer as described in Example 8. The percentage of 11S and 11R from the high 11R standard is shown for the high quality control (HQC), medium quality control (MQC), and low quality control (LQC). The percentage of 11S and 11R diastereomers is also shown for the high 11R neat material (R-neat (R) and R-neat (S)). The x-axis is time measured in minutes and the y-axis is area measured in percent. As discussed in Example 5, both the high 11S material (as shown in FIG. 2A) and the high 11R material come to a relatively constant ratio of diastereomers upon addition to blood.

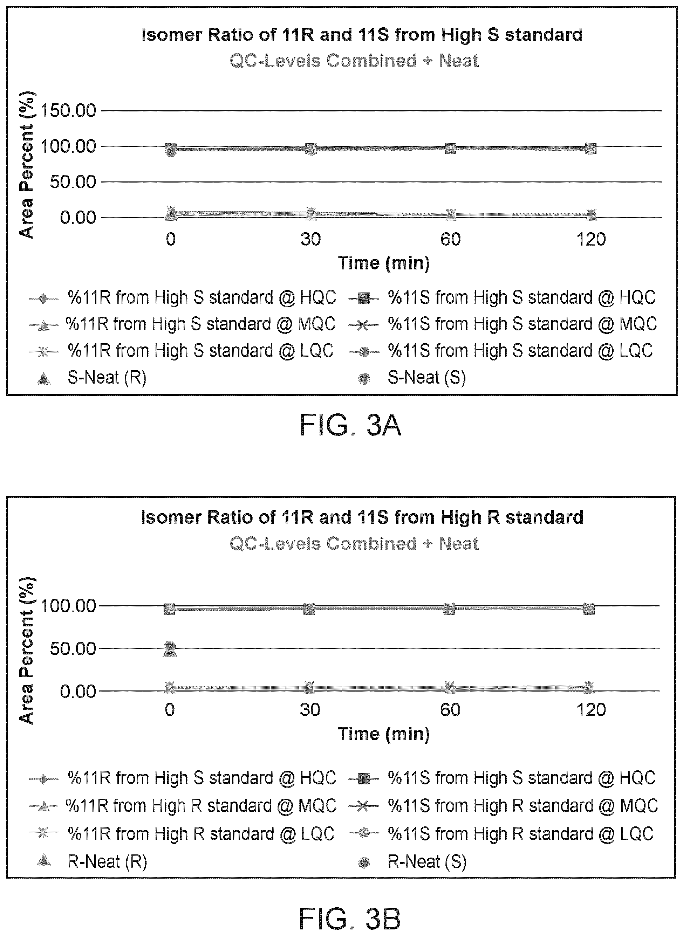

FIG. 3A is a graph of the 11S and 11R diastereomers percentage of total peak area from the high 11S standard in rat blood:buffer as described in Example 8. The percentage of 11S and 11R from the high 11S standard is shown for the high quality control (HQC), medium quality control (MQC), and low quality control (LQC). The percentage of 11S and 11R diastereomers is also shown for the high 11S neat material (S-neat (R) and S-neat (S)). The x-axis is time measured in minutes and the y-axis is area measured in percent.
FIG. 3B is a graph of the 11S and 11R diastereomers percentage of total peak area from the high 11R standard in rat blood:buffer as described in Example 8. The percentage of 11S and 11R from the high 11R standard is shown for the high quality control (HQC), medium quality control (MQC), and low quality control (LQC). The percentage of 11S and 11R diastereomers is also shown for the high 11R neat material (R-neat (R) and R-neat (S)). The x-axis is time measured in minutes and the y-axis is area measured in percent. As discussed in Example 5, both the high 11S material (as shown in FIG. 3A) and the high 11R material come to a relatively constant ratio of diastereomers upon addition to blood.

### DETAILED DESCRIPTION

Provided herein are compounds, methods of making the compounds, pharmaceutical compositions comprising the compounds, and methods of using the compounds and compositions in the treatment of pain and/or conditions modulated by voltage-gated sodium channels. The compounds are 11,13-modified, non-hydrated ketone saxitoxins. Also provided herein are methods of treating pain in a mammal comprising administering a therapeutically or prophylactically effective amount of a 11,13-modified, non-hydrated ketone saxitoxin or composition to a mammal. In an embodiment, the mammal is a human.

### Definitions

When referring to the compounds provided herein, the following terms have the following meanings unless indicated otherwise. Unless defined otherwise, all technical and scientific terms used herein have the same meaning as is commonly understood by one of ordinary skill in the art. In the event that there is a plurality of definitions for a term herein, those in this section prevail unless stated otherwise. Unless specified otherwise, where a term is defined as being substituted, the groups in the list of substituents are themselves unsubstituted. For example, a substituted alkyl group can be substituted, for example, with a cycloalkyl group, and the cycloalkyl group is not further substituted unless specified otherwise.

Reference to "about" a value or parameter herein includes (and describes) variations that are directed to that value or parameter per se. For example, description referring to "about X" includes description of "X". As used herein, and unless otherwise specified, the terms "about" and "approximately," when used in connection with temperatures, doses, amounts, or weight percent of ingredients of a composition or a dosage form, mean a dose, amount, or weight percent that is recognized by those of ordinary skill in the art to provide a pharmacological effect equivalent to that obtained from the specified dose, amount, or weight percent. Specifically, the terms "about" and "approximately," when used in this context, contemplate a dose, amount, or weight percent within 15%, within 10%, within 5%, within 4%, within 3%, within 2%, within 1%, or within 0.5% of the specified dose, amount, or weight percent.

The terms "a" or "an," as used in herein means one or more, unless context clearly dictates otherwise.

The term "alkyl," as used herein, unless otherwise specified, refers to a saturated straight or branched hydrocarbon. In some or any embodiments, the alkyl group is a primary, secondary, or tertiary hydrocarbon. In some or any embodiments, the alkyl group includes one to ten carbon atoms, *i.e.*, C₁ to C₁₀ alkyl. In some or any embodiments, the alkyl is a C₁₋₆alkyl. In some or any embodiments, the alkyl group is selected from the group consisting of methyl, ethyl, propyl, isopropyl, butyl, isobutyl, secbutyl, t-butyl, pentyl, isopentyl, neopentyl, hexyl, isohexyl, 3-methylpentyl, 2,2-dimethylbutyl, and 2,3-dimethylbutyl.

The term "alkoxy" and "alkyloxy" as used herein, and unless otherwise specified, refer to the group -OR' where R' is alkyl. Alkoxy and alkyloxy groups include, in some or any embodiments, methoxy, ethoxy, n-propoxy, isopropoxy, n-butoxy, tert-butoxy, sec-butoxy, n-pentoxy, n-hexyloxy, 1,2-dimethylbutoxy, and the like. In some embodiments, alkoxy is C₁₋₆alkoxy.

The term "alkylthio" as used herein, and unless otherwise specified, refers to the group -SR' where R' is C₁₋₁₀alkyl. In some or any embodiments, alkylthio is C₁₋₆alkylthio. In some or any embodiments, alkylthio is methylthio.

The term "alkylsulfinyl" as used herein, and unless otherwise specified, refers to the group -S(O)R' where R' is C₁₋₁₀alkyl. In some or any embodiments, the alkylsulfinyl is C₁₋₆alkylsulfinyl.

The term "alkylsulfonyl" as used herein, and unless otherwise specified, refers to the group -S(O)₂R' where R' is C₁₋₁₀alkyl. In some or any embodiments, the alkylsulfonyl is C₁₋₆alkylsulfonyl.

The term "amino" means -NH₂.

The term "alkylamino," as used herein, and unless otherwise specified, refers to the group -NHR' where R' is C₁₋₁₀alkyl, as defined herein. In some or any embodiments, the alkylamino is C₁₋₆alkylamino.

The term "dialkylamino," as used herein, and unless otherwise specified, refers to the group -NR'R' where each R' is independently C₁₋₁₀alkyl, as defined herein. In some or any embodiments, the dialkylamino is di-C₁₋₆alkylamino.

The term "aminoalkyl," as used herein, and unless otherwise specified, refers to an alkyl group, as defined herein, which is substituted with one or more amino groups. In some or any embodiments, the aminoalkyl is an alkyl group substituted with one -NH₂ group (e.g., -R'(NH₂) where R' is -C₁₋₁₀alkyl, as defined herein). In some or any embodiments, the aminoalkyl is an alkyl group substituted with two -NH₂ groups. In some embodiments, "aminoalkyl" is aminoC₁₋₆alkyl

An "alkylaminoalkyl," as used herein, and unless otherwise specified, refers to an alkyl group, as defined herein, which is substituted with one or more alkylamino groups as defined herein. In some embodiments, an "alkylaminoalkyl" is C₁₋₆alkylaminoC₁₋₆alkyl. In some embodiments, each alkyl in alkylaminoalkyl is independently selected.

A "dialkylaminoalkyl" as used herein, and unless otherwise specified, refers to an alkyl group, as defined herein, which is substituted with one or more dialkylamino groups as defined herein. In some embodiments, "dialkylaminoalkyl" is di-C₁₋₆alkylaminoC₁₋₆alkyl. In some embodiments, each alkyl in dialkylaminoalkyl is independently selected.

The term "aryl," as used herein, and unless otherwise specified, refers to a monovalent C₆- C₁₅ carbocyclic ring system which comprises at least one aromatic ring wherein the aryl ring system is mono, di, or tricyclic. The aryl may be attached to the main structure through any of its rings, *i.e.* any aromatic or nonaromatic ring. In some or any embodiments, the aryl group may be a bridged (where chemically feasible) or non-bridged, spirocyclic (where chemically feasible) or not spirocyclic, and/or fused or not fused multicyclic group. In some or any embodiments, aryl is phenyl, naphthyl, bicyclo[4.2.0]octa-1,3,5-trienyl, indanyl, fluorenyl, 6,7,8,9-tetrahydro-5H-benzo[7]annulenyl, 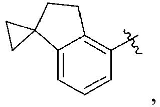 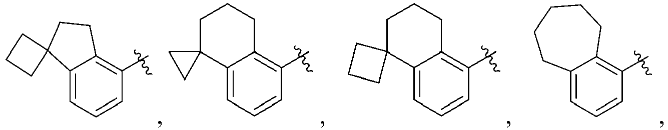 or tetrahydronaphthyl. When aryl is substituted, it can be substituted on any ring, *i.e.* on any aromatic or nonaromatic ring comprised by aryl. In some or any embodiments, aryl is phenyl, naphthyl, tetrahydronaphthyl, fluorenyl, 6,7,8,9-tetrahydro-5H-benzo[7]annulenyl, or indanyl; each of which is optionally substituted with 1, 2, 3, or 4 groups as defined throughout the specification, including in some or any embodiments with group(s) independently selected from amino, hydroxy, halo, halo- C₁₋₆alkoxy, C₁₋₆alkoxy, C₁₋₆alkyl, halo-C₁₋₆alkyl, C₁₋₆alkylthio, C₁₋₆alkylsulfonyl, and phenyl.

The term "aryl-C₁₋₆alkyl," as used herein, and unless otherwise specified, refers to a C₁₋₆alkyl group, as used herein, substituted with one or two aryl groups, as used herein.

The term "biphenyl," as used herein, unless otherwise specified, refers to a phenyl group substituted with a second phenyl group. When the biphenyl is "optionally substituted," the optional substituent(s) can be substituted on either of the phenyl rings.

The term "aryloxy," as used herein, and unless otherwise specified, refers to an - OR group where R is aryl, as defined herein. In some embodiments, "aryloxy" is phenyloxy or phenoxy.

The term "arylalkyl" and "aralkyl," as used herein, and unless otherwise specified, refer to an alkyl group substituted with one or two aryl groups, as defined herein, where the alkyl group is the point of attachment to the remainder of the molecule. In some embodiments, aralkyl is phenylmethyl, phenyleth-1-yl, phenyleth-2-yl, diphenylmethyl, 2,2-diphenylethyl, 3,3-diphenylpropyl, or 3-phenylpropyl; each of which is optionally substituted on the ring with 1, 2, 3, or 4 groups as defined throughout the specification. In some embodiments, arylalkyl is C₁₋₆alkyl substituted with one phenyl group. In some embodiments, arylalkyl is benzyl.

The term "C₃-C₈-cycloalkyl," as used herein, refers to a monovalent, saturated, monocyclic hydrocarbon. In some or any embodiments, the cycloalkyl group includes three to six carbon atoms, *i.e.*, C₃ to C₆ cycloalkyl. In some or any embodiments, the cycloalkyl has 3, 4, or 5 (C₃₋₅); 3 or 4 (C₃₋₄); 3 (C₃); 4 (C₄); or 5 (C₅) carbon atoms. In some or any embodiments, the cycloalkyl group is cyclopropyl, cyclobutyl, cyclopentyl, or cyclohexyl. In some or any embodiments, the cycloalkyl group is cyclopropyl, cyclobutyl, or cyclopentyl. In some or any embodiments, the cycloalkyl group is cyclopropyl. In some or any embodiments, the cycloalkyl group is cyclobutyl. In some or any embodiments, the cycloalkyl group is cyclopentyl.

The term "cycloalkylalkyl" refers to an alkyl as defined herein, which is substituted by one or more than one cycloalkyl groups as defined herein. In some embodiments, "cycloalkylalkyl" is C₃₋₈cycloalkylC₁₋₆alkyl. In some embodiments, "cycloalkylalkyl" is alkyl substituted with one cycloalkyl. In some embodiments, cycloalkylalkyl is cyclopropylmethyl.

The term "cycloalkylene," as used herein, and unless otherwise specified, refers to a divalent, saturated, monocyclic hydrocarbon. In some or any embodiments, the cycloalkylene group includes three to six carbon atoms, *i.e.*, C₃ to C₆ cycloalkylene. In some or any embodiments, the cycloalkylene has 3, 4, or 5 (C₃₋₅); 3 or 4 (C₃₋₄); 3 (C₃); 4 (C₄); or 5 (C₅) carbon atoms. In some or any embodiments, the cycloalkylene group is cycloprop-diyl, cyclobut-diyl, cyclopent-diyl, or cyclohex-diyl. In some or any embodiments, the cycloalkylene group is cycloprop-diyl, cyclobut-diyl, or cyclopent-diyl. In some or any embodiments, the cycloalkylene group is cycloprop-diyl. In some or any embodiments, the cycloalkylene group is cyclobut-diyl. In some or any embodiments, the cycloalkylene group is cyclopent-diyl.

The term "haloalkyl," as used herein, and unless otherwise specified, refers to an alkyl group substituted with 1, 2, 3, 4, or 5 halo groups. In some or any embodiments, the haloalkyl is a halo-C₁₋₆alkyl. In some or any embodiments, the haloalkyl is -CF₃, -CH₂F, -CHF₂, or -CH₂CF₃.

The term "haloalkylthio," as used herein, and unless otherwise specified, refers to an -SR group where R is halo-C₁₋₁₀alkyl as defined herein. In some or any embodiments, the haloalkylthio is a halo-C₁₋₆alkylthio.

The term "haloalkoxy," as used herein, and unless otherwise specified, refers to an -OR group where R is halo-C₁₋₁₀alkyl as defined herein. In some or any embodiments, the haloalkoxy is a halo-C₁₋₆alkoxy.

The term "haloalkylsulfinyl," as used herein, and unless otherwise specified, refers to an -S(O)R group where R is halo-C₁₋₁₀alkyl as defined herein. In some or any embodiments, the haloalkylsulfinyl is a halo-C₁₋₆alkylsulfinyl.

The term "haloalkylsulfonyl," as used herein, and unless otherwise specified, refers to an -S(O)₂R group where R is halo-C₁₋₁₀alkyl as defined herein. In some or any embodiments, the haloalkylsulfonyl is a halo-C₁₋₆alkylsulfonyl.

The terms "halogen" and "halo," as used herein, and unless otherwise specified, are synonymous and refer to chloro, bromo, fluoro or iodo.

The term "heteroaralkyl," as used herein, and unless otherwise specified, refers to an alkyl group, as used herein, substituted with one or two heteroaryl groups, as used herein.

The term "heteroaryl," as used herein, and unless otherwise specified, refers to a monocyclic aromatic ring system or multicyclic aromatic ring system wherein one or more (in some or any embodiments, 1, 2, 3, or 4) of the ring atoms is a heteroatom independently selected from O, S(O)₀₋₂, NH, and N, and the remaining ring atoms are carbon atoms, and where the ring may be optionally substituted as described herein. The heteroaryl group is bonded to the rest of the molecule through any atom in the ring system, valency rules permitting. In certain embodiments, each ring of a heteroaryl group can contain one or two O atoms, one or two S atoms, and/or one to four N atoms, or a combination thereof, provided that the total number of heteroatoms in each ring is four or less and each ring contains at least one carbon atom. In certain embodiments, the heteroaryl has from 5 to 20, from 5 to 15, or from 5 to 10 ring atoms. When heteroaryl is substituted, it can be substituted on any ring. In some embodiments, heteroaryl includes, but is not limited to furanyl, pyrrolyl, pyrazolyl, imidazolyl, triazolyl, tetrazolyl, thienyl, pyridinyl, pyrimidinyl, pyridazinyl, pyrazinyl, indolyl, quinolinyl, isoquinolinyl, oxazolyl, isoxazolyl, thienopyridinyl, thienopyrimidinyl, azaindolinyl and the like. In some embodiments, a heteroaryl is 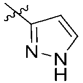 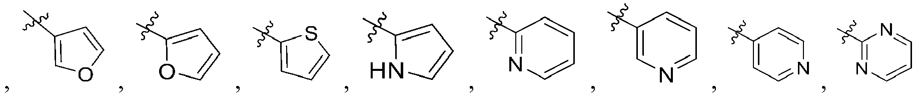 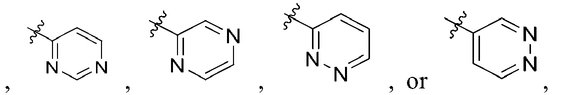 wherein 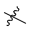 indicates the point of attachment of the heteroaryl to the rest of the molecule. In some embodiments, the heteroaryl when substituted one or more hydroxy can be named or drawn as either the keto or enol tautomer. For example, 2,4(1*H*,3*H*)-dioxo-pyrimidinyl, 2,4-dihydroxy-pyrimidinyl; 2(1*H*)-oxo-4-hydroxypyrimidinyl, 4-hydroxy-2(3H)-oxo-pyrimidinyl, and 2-hydroxy-4(3*H*)-oxo-pyrimidinyl are within the scope of heteroaryl when substituted with two hydroxy.

In certain embodiments, monocyclic heteroaryl groups include, but are not limited to, furanyl, imidazolyl, isothiazolyl, isoxazolyl, oxadiazolyl, oxadiazolyl, oxazolyl, pyrazinyl, pyrazolyl, pyridazinyl, pyridyl, pyrimidinyl, pyrrolyl, thiadiazolyl, thiazolyl, thienyl, tetrazolyl, triazinyl and triazolyl. In certain embodiments, bicyclic heteroaryl groups include, but are not limited to, benzofuranyl, benzimidazolyl, benzoisoxazolyl, benzopyranyl, benzothiadiazolyl, benzothiazolyl, benzothienyl, benzotriazolyl, benzoxazolyl, furopyridyl, imidazopyridinyl, imidazothiazolyl, indolizinyl, indolyl, indazolyl, isobenzofuranyl, isobenzothienyl, isoindolyl, isoquinolinyl, isothiazolyl, naphthyridinyl, oxazolopyridinyl, phthalazinyl, pteridinyl, purinyl, pyridopyridyl, pyrrolopyridyl, quinolinyl, quinoxalinyl, quinazolinyl, thiadiazolopyrimidyl, and thienopyridyl. In certain embodiments, tricyclic heteroaryl groups include, but are not limited to, acridinyl, benzindolyl, carbazolyl, dibenzofuranyl, perimidinyl, phenanthrolinyl, phenanthridinyl, and phenazinyl. In some or any embodiments, heteroaryl is indolyl, furanyl, pyridinyl, pyrimidinyl, imidazolyl, or pyrazolyl; each of which is optionally substituted with 1, 2, 3, or 4 groups as defined throughout the specification, including in some embodiments with group(s) independently selected from C₁₋₆alkyl, hydroxy, halo, halo-C₁₋₆alkyl, C₁₋₆alkoxy, cyano, or phenyl.

The term "heterocyclic," as used herein, and unless otherwise specified, refers to a monovalent monocyclic non-aromatic ring system and/or a multicyclic ring system that contains at least one non-aromatic ring; wherein one or more (in some or any embodiments, 1, 2, 3, or 4) of the non-aromatic monocyclic ring atoms is a heteroatom independently selected from O, S(O)₀₋₂, and N, and the remaining ring atoms are carbon atoms; and wherein one or more (in some or any embodiments, 1, 2, 3, or 4) of any of the ring atoms in the multicyclic ring system is a heteroatom(s) independently selected from O, S(O)₀₋₂, and N, and the remaining ring atoms are carbon. The term "heterocyclic" does not include fully aromatic ring(s), *i.e.* does not include imidazole, pyrimidine, pyridine, and the like. In some or any embodiments, the heterocyclic ring comprises one or two heteroatom(s) which are independently selected from nitrogen and oxygen. In some or any embodiments, the heterocyclic ring comprises one or two heteroatom(s) which are oxygen. In some or any embodiments, the heterocyclic ring comprises one or two heteroatom(s) which are nitrogen (where the nitrogen is substituted as described in any aspect or embodiment described herein). In some or any embodiments, heterocyclic is multicyclic and comprises one heteroatom in a non-aromatic ring, or comprises one heteroatom in an aromatic ring, or comprises two heteroatoms in an aromatic ring, or comprises two heteroatoms where one is in an aromatic ring and the other is in a non-aromatic ring. In some or any embodiments, the heterocyclic group has from 3 to 20, 3 to 15, 3 to 10, 3 to 8, 4 to 7, or 5 to 6 ring atoms. In some or any embodiments, the heterocyclic is a monocyclic, bicyclic, tricyclic, or tetracyclic ring system. In some or any embodiments, the heterocyclic group may be a bridged or non-bridged, spirocyclic or not spirocyclic, and/or fused or not fused multicyclic group. One or more of the nitrogen and sulfur atoms may be optionally oxidized, one or more of the nitrogen atoms may be optionally quaternized, one or more of the carbon atoms may be optionally replaced with 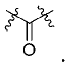 Some rings may be partially or fully saturated, or aromatic provided that heterocyclic is not fully aromatic. The monocyclic and multicyclic heterocyclic rings may be attached to the main structure at any heteroatom or carbon atom which results in a stable compound. The multicyclic heterocyclic may be attached to the main structure through any of its rings, including any aromatic or nonaromatic ring, regardless of whether the ring contains a heteroatom. In some or any embodiments, heterocyclic is "heterocycloalkyl" which is 1) a saturated or partially unsaturated (but not aromatic) monovalent monocyclic group which contains at least one ring heteroatom, as described herein, or 2) a saturated or partially unsaturated (but not aromatic) monovalent bi- or tri-cyclic group in which at least one ring contains at least one heteroatom as described herein. When heterocyclic and heterocycloalkyl are substituted, they can be substituted on any ring, i.e. on any aromatic or nonaromatic ring comprised by heterocyclic and heterocycloalkyl. In some or any embodiments, such heterocyclic includes, but are not limited to, azepinyl, benzodioxanyl, benzodioxolyl, 3,4-dihydro-2H-benzo[b][1,4]oxazinyl, 3,4-dihydro-2H-benzo[b][1,4]dioxepinyl, 1,3-dihydroisobenzofuranyl, benzofuranonyl, benzopyranonyl, benzopyranyl, dihydrobenzofuranyl, 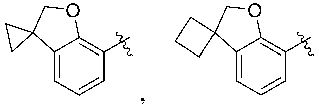 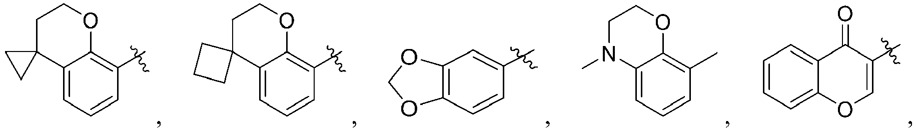 benzotetrahydrothienyl, 2,2-dioxo-1,3-dihydrobenzo[c]thienyl, benzothiopyranyl, benzoxazinyl, β-carbolinyl, chromanyl, chromonyl, cinnolinyl, coumarinyl, decahydroquinolinyl, decahydroisoquinolinyl, dihydrobenzisothiazinyl, dihydrobenzisoxazinyl, dihydrofuryl, dihydroisoindolyl, dihydropyranyl, dihydropyrazolyl, dihydropyrazinyl, dihydropyridinyl, dihydropyrimidinyl, dihydropyrrolyl, dioxolanyl, 1,4-dithianyl, furanonyl, imidazolidinyl, 2,4-dioxo-imidazolidinyl, imidazolinyl, indolinyl, 2-oxo-indolinyl, isobenzotetrahydrofuranyl, isobenzotetrahydrothienyl, isochromanyl, isocoumarinyl, isoindolinyl, 1-oxo-isoindolinyl, 1,3-dioxo-isoindolinyl, isothiazolidinyl, isoxazolidinyl, 3-oxo-isoxazolidinyl, morpholinyl, 3,5-dioxo-morpholinyl, octahydroindolyl, octahydroisoindolyl, 1-oxo-octahydroisoindolyl, 1,3-dioxo-hexahydroisoindolyl, oxazolidinonyl, oxazolidinyl, oxiranyl, piperazinyl, 2,6-dioxo-piperazinyl, piperidinyl, 2,6-dioxo-piperidinyl, 4-piperidonyl, pyrazolidinyl, pyrazolinyl, pyrrolidinyl, pyrrolinyl, 2-oxopyrrolidinyl, 2,5-dioxopyrrolidinyl, quinuclidinyl, tetrahydrofuryl, tetrahydroisoquinolinyl, tetrahydropyranyl, tetrahydrothienyl, thiamorpholinyl, thiomorpholinyl, 3,5-dioxo-thiomorpholinyl, thiazolidinyl, 2,4-dioxo-thiazolidinyl, tetrahydroquinolinyl, phenothiazinyl, phenoxazinyl, xanthenyl, and 1,3,5-trithianyl. In some or any embodiments, heterocyclic is benzo-1,4-dioxanyl, benzodioxolyl, indolinyl, 2-oxo-indolinyl, pyrrolidinyl, piperidinyl, 2,3-dihydrobenzofuranyl, or decahydroquinolinyl; each of which is optionally substituted with 1, 2, 3, or 4 groups as defined throughout the specification, including in some or any embodiments with group(s) independently selected from halo, alkyl, and phenyl. In some embodiments, heterocycloalkyl is pyrrolidinyl. In some embodiments, heterocycloalkyl is an N-linked heterocycloalkyl.

The term "heterocyclicalkyl," as used herein, and unless otherwise specified, refers to an alkyl group, as used herein, substituted with one or two heterocyclic groups, as used herein.

The term "hydroxyalkyloxy," as used herein, and unless otherwise specified, refers to a group in which the alkyl group in the alkyloxy group, as defined herein, is substituted with one or more hydroxyl groups. In some or any embodiments, the hydroxyalkyloxy group is selected from hydroxymethyloxy, 2-hydroxyethyloxy, 3-hydroxypropyloxy, and 2-hydroxypropyloxy. In some or any embodiments, hydroxyalkyloxy is hydroxyC₁₋₆alkyloxy.

The term "oxo" as used herein and unless otherwise specified, refers to a keto group (C=O). An oxo group that is a substituent of a nonaromatic carbon results in a conversion of a -CH₂- to -C=O. An oxo group that is a substituent of an aromatic carbon results in a conversion of -CH- to -C=O. When a substituent is oxo, then two hydrogens on the atom are replaced. When an oxo group substitutes aromatic moieties, the corresponding partially unsaturated ring replaces the aromatic ring. For example, a pyridyl group substituted by an oxo group is a pyridone. The person of ordinary skill in the art will appreciate that in some embodiments that such a group, e.g. pyridone and 2,4(1*H*,3*H*)-dioxo-pyrimidinyl, can exist in its tautomeric form, e.g. hydroxypyridine and 2,4-dihydroxypyrimidinyl, respectively.

The term "protecting group," as used herein, and unless otherwise specified, refers to a group that is added to an oxygen, nitrogen or phosphorus atom to prevent its further reaction or for other purposes. A wide variety of oxygen and nitrogen protecting groups are known to those skilled in the art of organic synthesis. (See for example those described in Greene, et al., Protective Groups in Organic Synthesis, John Wiley and Sons, Fourth Edition, 2006, hereby incorporated by reference.) In some or any embodiments, a "nitrogen-protecting group" (e.g. for PG¹ and PG²) is 9-fluorenylmethyloxycarbonyl (Fmoc), *tert*-butoxycarbonyl (Boc), benzyloxycarbonyl (CBz), acetyl, trichloroacetyl, trifluoroacetyl, -C(O)OCH₂CCl₃ (Troc), p-methoxyphenyl, benzyl, p-methoxybenzyl, p-methoxybenzylcarbonyl, triphenylmethyl, benzylidenyl, 2,2,2-trichloroethoxysulfonyl (Tces), p-methoxybenzenesulfonyl (Mbs) or p-toluenesulfonyl (tosyl). In some or any embodiments, an oxygen-protecting group (e.g. for X¹) is methoxymethyl (MOM), ethoxyethyl, methoxyethoxymethyl, tetrahydrofuranyl, tetrahydropyranyl, methyl, *tert-*butyl, allyl, benzyl, trimethylsilyl, triethylsilyl, triisopropylsilyl, *tert*-butyldimethylsilyl, *tert*-butyldiphenylsilyl, acetyl, pivalyl, benzoyl, dimethoxytrityl, trityl, methoxytrityl, p-methoxybenzyl, or methylthiomethyl.

The term "pharmaceutically acceptable salt," as used herein, and unless otherwise specified, refers to any salt of a compound provided herein which retains its biological properties and which is not toxic or otherwise desirable for pharmaceutical use. Such salts may be derived from a variety of organic and inorganic counter-ions well known in the art. Such salts include, but are not limited to: (1) acid addition salts formed with organic or inorganic acids such as hydrochloric, hydrobromic, sulfuric, nitric, phosphoric, sulfamic, acetic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, *tert*-butylacetic, lauryl sulfuric, gluconic, benzoic, glutamic, hydroxynaphthoic, salicylic, stearic, cyclohexylsulfamic, quinic, muconic acid and the like acids; and (2) base addition salts formed when an acidic proton present in the parent compound either (a) is replaced by a metal ion, *e.g.,* an alkali metal ion, an alkaline earth ion or an aluminum ion, or alkali metal or alkaline earth metal hydroxides, such as sodium, potassium, calcium, magnesium, aluminum, lithium, zinc, and barium hydroxide, ammonia or (b) coordinates with an organic base, such as aliphatic, alicyclic, or aromatic organic amines, such as ammonia, methylamine, dimethylamine, diethylamine, picoline, ethanolamine, diethanolamine, triethanolamine, ethylenediamine, lysine, arginine, ornithine, choline, N,N'-dibenzylethylene-diamine, chloroprocaine, diethanolamine, procaine, N-benzylphenethylamine, *N*-methylglucamine piperazine, tris(hydroxymethyl)-aminomethane, tetramethylammonium hydroxide, and the like.

Pharmaceutically acceptable salts further include, in some or any embodiments, and without limitation, sodium, potassium, calcium, magnesium, ammonium, tetraalkylammonium salts and the like. When the compound contains a basic functionality, salts of non-toxic organic or inorganic acids, such as hydrohalides, *e.g*. hydrochloride and hydrobromide, sulfate, phosphate, sulfamate, nitrate, acetate, trifluoroacetate, trichloroacetate, propionate, hexanoate, cyclopentylpropionate, glycolate, glutarate, pyruvate, lactate, malonate, succinate, sorbate, ascorbate, malate, maleate, fumarate, tartarate, citrate, benzoate, 3-(4-hydroxybenzoyl)benzoate, picrate, cinnamate, mandelate, phthalate, laurate, methanesulfonate (mesylate), ethanesulfonate, 1,2-ethane-disulfonate, 2-hydroxyethanesulfonate, benzenesulfonate (besylate), 4-chlorobenzenesulfonate, 2-naphthalenesulfonate, 4-toluenesulfonate, camphorate, camphorsulfonate, 4-methylbicyclo[2.2.2]-oct-2-ene-1-carboxylate, glucoheptonate, 3-phenylpropionate, trimethylacetate, *tert*-butylacetate, lauryl sulfate, gluconate, benzoate, glutamate, hydroxynaphthoate, salicylate, stearate, cyclohexylsulfamate, quinate, muconate and the like.

The term "substantially free of" or "substantially in the absence of" stereoisomers with respect to a composition refers to a composition that includes at least 85 or 90% by weight, in some or any embodiments 95%, 98 %, 99% or 100% by weight, of a designated stereoisomer of a compound in the composition. In some or any embodiments, in the methods and compounds provided herein, the compounds are substantially free of stereoisomers.

Similarly, the term "isolated" with respect to a composition refers to a composition that includes at least 85, 90%, 95%, 98%, 99% to 100% by weight, of a specified compound, the remainder comprising other chemical species or stereoisomers.

The term "solvate," as used herein, and unless otherwise specified, refers to a compound provided herein or a salt thereof, that further includes a stoichiometric or non-stoichiometric amount of solvent bound by non-covalent intermolecular forces. Where the solvent is water, the solvate is a hydrate.

The term "isotopic composition," as used herein, and unless otherwise specified, refers to the amount of each isotope present for a given atom, and "natural isotopic composition" refers to the naturally occurring isotopic composition or abundance for a given atom. Atoms containing their natural isotopic composition may also be referred to herein as "non-enriched" atoms. Unless otherwise designated, the atoms of the compounds recited herein are meant to represent any stable isotope of that atom. For example, unless otherwise stated, when a position is designated specifically as "H" or "hydrogen," the position is understood to have hydrogen at its natural isotopic composition.

The term "isotopic enrichment," as used herein, and unless otherwise specified, refers to the percentage of incorporation of an amount of a specific isotope at a given atom in a molecule in the place of that atom's natural isotopic abundance. In some or any embodiments, deuterium enrichment of 1% at a given position means that 1% of the molecules in a given sample contain deuterium at the specified position. Because the naturally occurring distribution of deuterium is about 0.0156%, deuterium enrichment at any position in a compound synthesized using non-enriched starting materials is about 0.0156%. The isotopic enrichment of the compounds provided herein can be determined using conventional analytical methods known to one of ordinary skill in the art, including mass spectrometry and nuclear magnetic resonance spectroscopy.

The term "isotopically enriched," as used herein, and unless otherwise specified, refers to an atom having an isotopic composition other than the natural isotopic composition of that atom. "Isotopically enriched" may also refer to a compound containing at least one atom having an isotopic composition other than the natural isotopic composition of that atom.

As used herein, the term "local anesthetic" means a drug which provides local numbness or pain relief. In some or any embodiments, local anesthetic includes aminoacylanilide compounds (in some or any embodiments, lidocaine, prilocaine, bupivacaine, ropivacaine, and mepivacaine) and related local anesthetic compounds having various substituents on the ring system or amine nitrogen; aminoalkyl benzoate compounds (in some or any embodiments, procaine, chloroprocaine, propoxycaine, hexylcaine, tetracaine, cyclomethycaine, benoxinate, butacaine, and proparacaine) and related local anesthetic compounds; cocaine; amino carbonate compounds (in some or any embodiments, diperodon); N-phenylamidine compounds (in some or any embodiments, phenacaine); N-aminoalkyl amide compounds (in some or any embodiments, dibucaine); aminoketone compounds (in some or any embodiments, falicaine and dyclonine); and amino ether compounds (in some or any embodiments, pramoxine and dimethisoquien).

As used herein, "alkyl," "cycloalkyl," "aryl," "alkoxy," "heterocycloalkyl," and "heterocyclic" groups optionally comprise deuterium at one or more positions where hydrogen atoms are present, and wherein the deuterium composition of the atom or atoms is other than the natural isotopic composition.

Also as used herein, "alkyl," "cycloalkyl," "aryl," "alkoxy," "heterocycloalkyl," "heterocyclic" groups optionally comprise carbon-13 at an amount other than the natural isotopic composition.

As used herein, and unless otherwise specified, the term "IC₅₀" refers to an amount, concentration or dosage of a particular test compound that achieves a 50% inhibition of a maximal response in an assay that measures such response.

As used herein, the terms "subject" and "patient" are used interchangeably. The terms "subject" and "subjects" refer to an animal, such as a mammal including a non-primate (*e.g.*, a cow, pig, horse, cat, dog, rat, and mouse) and a primate (*e.g.*, a monkey such as a cynomolgous monkey, a chimpanzee and a human), and in some or any embodiments, a human. In some or any embodiments, the subject is a farm animal (*e.g*., a horse, a cow, a pig, etc.) or a pet (*e.g.*, a dog or a cat). In some or any embodiments, the subject is a human.

As used herein, the terms "therapeutic agent" and "therapeutic agents" refer to any agent(s) which can be used in the treatment or prevention of a disorder or one or more symptoms thereof. In some or any embodiments, the term "therapeutic agent" includes a compound provided herein. In some or any embodiments, a therapeutic agent is an agent which is known to be useful for, or has been or is currently being used for the treatment or prevention of a disorder or one or more symptoms thereof.

"Therapeutically effective amount" refers to an amount of a compound or composition that, when administered to a subject for treating a condition, is sufficient to effect such treatment for the condition. A "therapeutically effective amount" can vary depending on, *inter alia*, the compound, the condition and its severity, and the age, weight, *etc.*, of the subject to be treated.

"Treating" or "treatment" of any condition or disorder refers, in some or any embodiments, to ameliorating a condition or disorder that exists in a subject, including prophylactically. In another embodiment, "treating" or "treatment" includes ameliorating at least one physical parameter, which may be indiscernible by the subject. In yet another embodiment, "treating" or "treatment" includes modulating the condition or disorder, either physically (*e.g.*, stabilization of a discernible symptom) or physiologically (*e.g.*, stabilization of a physical parameter) or both. In yet another embodiment, "treating" or "treatment" includes delaying the onset of the condition or disorder. In yet another embodiment, "treating" or "treatment" includes the reduction or elimination of either the condition (*e.g*. pain) or one or more symptoms (*e.g.* pain) of the condition (*e.g.* sciatica), or to retard the progression of the condition (*e.g.* pain) or of one or more symptoms (*e.g.* pain) of the condition (*e.g.* sciatica), or to reduce the severity of the condition (*e.g.* pain) or of one or more symptoms (*e.g.* pain) of the condition (*e.g.* sciatica). In yet another embodiment, "treating" or "treatment" includes administering a compound described herein prophylactically.

As used herein, the terms "prophylactic agent" and "prophylactic agents" refer to any agent(s) which can be used in the prevention of a condition or one or more symptoms thereof and/or which prevents or impedes the onset, development, progression and/or severity of a condition. In some or any embodiments, the term "prophylactic agent" includes a compound provided herein. In some or any other embodiments, the term "prophylactic agent" does not refer a compound provided herein. In some or any embodiments, the agent is administered prophylactically, for example before surgery to prevent or impede the onset, duration, progression and/or severity of pain (*e.g.*, post surgical pain).

As used herein, the phrase "prophylactically effective amount" refers to the amount of a therapy (*e.g*., prophylactic agent) which is sufficient to result in the prevention or reduction of the development, recurrence or onset of one or more symptoms associated with a condition, or to enhance or improve the prophylactic effect(s) of another therapy (*e.g*., another prophylactic agent).

### Compounds

Provided herein are compounds that can modulate the activity of voltage-gated ion channels (*e.g*., voltage-gated sodium channels). The 11,13-modified, non-hydrated ketone saxitoxins can be formed as described herein and used for the treatment of conditions associated with voltage-gated sodium channel function. In some or any embodiments, the condition associated with voltage-gated sodium channel function is pain or a condition associated with pain. In some or any embodiments, the condition associated with voltage-gated sodium channel function is a condition associated with pain. In some or any embodiments, the condition associated with voltage-gated sodium channel function is pain, itch, cough, epilepsy, Parkinson's disease, a mood disorder, psychosis, amyotrophic lateral sclerosis, glaucoma, ischemia, spasticity disorders and obsessive compulsive disorder. In some or any embodiments, the condition associated with voltage-gated sodium channel function is pain (in some embodiments, subacute or chronic pain). In some embodiments, the pain associated with voltage-gated sodium channel function includes pain and/or discomfort associated with dry eye syndrome, pain associated with (acute) corneal injuries or abrasions, acute ocular pain, chronic ocular pain, pain associated with corneal infections, pain associated with Parkinson's disease, pain associated with ALS, and pain associated with surgery (in some embodiments, ocular surgery).

The aspects and embodiments described herein include the recited compounds as well as a pharmaceutically acceptable salt, hydrate, solvate, stereoisomer, tautomer, mixture, or combination thereof.

Included herein, if chemically possible, are all stereoisomers of the compounds, including diastereomers and enantiomers. Also included are mixtures of possible stereoisomers in any ratio, including, but not limited to, racemic mixtures. Unless stereochemistry is explicitly indicated in a structure at a particular atom, the structure is intended to embrace all possible stereoisomers of the compound depicted. If stereochemistry is explicitly indicated for one portion or portions of a molecule, but not for another portion or portions of a molecule, the structure is intended to embrace all possible stereoisomers for the portion or portions where stereochemistry is not explicitly indicated.

It will be apparent that certain structures recite specific stereochemistry at particular atoms.

Certain multicyclic structures provided herein are drawn with one or more floating substituents. Unless provided otherwise or otherwise clear from the context, the substituent(s) may be present on any atom of the multicyclic ring, where chemically feasible and valency rules permitting. For example, in the structure: 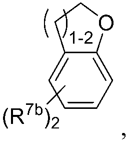 the R^{7b} substituents can be on the benzo portion of the bicyclic ring or the dihydrofuranyl portion of the bicyclic ring.

In some or any embodiments, provided herein is a compound of Formula (P-I) wherein
R¹ is -NR^{3a}R³ or -NR⁴C(O)R^{4a};
R² is -NR⁷C(O)R^{7a};
R³ is hydrogen or C₁₋₆alkyl;
R^{3a} is hydrogen or C₁₋₆alkyl;
R⁴ is hydrogen or C₁₋₆alkyl;
R^{4a} is heteroaryl optionally substituted with 1, 2, or 3 R^{4b} groups;
each R^{4b}, when present, is independently C₁₋₆alkyl, hydroxy, halo, halo-C₁₋₆alkyl, C₁₋₆alkoxy, or cyano;
R⁷ is hydrogen;
R^{7a} is aryl optionally substituted with 1, 2, 3, or 4 R^{7b}; or heterocyclic optionally substituted with 1, 2, 3, or 4 R^{7b}; and
each R^{7b}, when present, is independently halo, C₁₋₆alkyl, halo-C₁₋₆alkyl, hydroxy, C₁₋₆alkoxy, or halo-C₁₋₆alkoxy; or
a pharmaceutically acceptable salt, hydrate, solvate, stereoisomer, tautomer, mixture, or combination thereof.

In some or any embodiments, provided herein is a compound of Formula (I) wherein
R¹ is -NR^{3a}R³ or -NR⁴C(O)R^{4a};
R² is -NR⁷C(O)R^{7a};
R³ is hydrogen or C₁₋₆alkyl;
R^{3a} is hydrogen or C₁₋₆alkyl;
R⁴ is hydrogen or C₁₋₆alkyl;
R^{4a} is cycloalkyl optionally substituted with 1, 2, 3, or 4 R^{4b} groups; heteroaryl optionally substituted with 1, 2, or 3 R^{4b} groups; heteroaralkyl where the heteroaryl portion is optionally substituted with 1, 2, 3, or 4 R^{4b} groups; phenyl optionally substituted with 1, 2, 3, or 4 R^{4b} groups; or heterocyclic optionally substituted with 1, 2, 3, or 4 R^{4b} groups;
each of the 1, 2, 3, or 4 R^{4b}, when present, is independently C₁₋₆alkyl, hydroxy, hydroxyC₁₋₆alkyloxy, halo, halo-C₁₋₆alkyl, C₁₋₆alkoxy, cyano, or pyridinyl or
one pair of two R^{4b} groups are present on one carbon, the second pair of two R^{4b} are present on another carbon, and each pair of R^{4b} are taken together to form an oxo group;
R⁷ is hydrogen;
R^{7a} is aryl optionally substituted with 1, 2, 3, or 4 R^{7b}; or heterocyclic optionally substituted with 1, 2, 3, or 4 R^{7b}; and
each R^{7b}, when present, is independently halo, C₁₋₆alkyl, halo-C₁₋₆alkyl, hydroxy, hydroxyalkyloxy, C₁₋₆alkoxy, or halo-C₁₋₆alkoxy, or heteroaryl or 2 R^{4b} groups when on the same carbon are taken together to form an oxo group; or
a pharmaceutically acceptable salt, hydrate, solvate, stereoisomer, tautomer, mixture, or combination thereof.

In some embodiments, each R^{4b}, when present, is independently C₁₋₆alkyl or hydroxy; and each R^{7b}, when present, is independently C₁₋₆alkyl.

In some or any embodiments described above and herein, the compound of Formula (I) or Formula (P-1) is according to Formula (P-Ia): 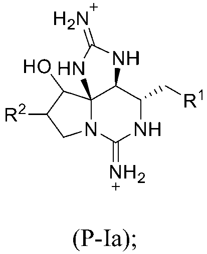 or a pharmaceutically acceptable salt, hydrate, solvate, stereoisomer, tautomer, mixture, or combination thereof, where R¹ and R² are as defined in the Summary or as defined in some or any embodiments described herein.

In some or any embodiments, the compound of Formula (I) or Formula (P-1) is according to Formula (P-Ib-1) or (P-Ib-2): 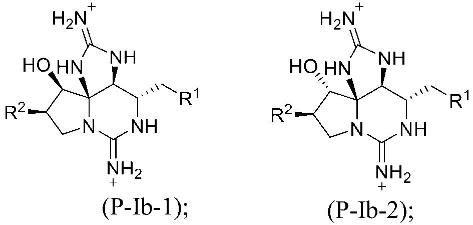 or a pharmaceutically acceptable salt, hydrate, solvate, stereoisomer, tautomer, mixture, or combination thereof, where R¹ and R² are as defined in the Summary or as defined in some or any embodiments described herein. In some embodiments, the compound of Formula (I) or Formula (P-1) is according to Formula (P-Ib-1); or a pharmaceutically acceptable salt, hydrate, solvate, stereoisomer, tautomer, mixture, or combination thereof. In some embodiments, the compound of Formula (I) or Formula (P-1) is according to Formula (P-Ib-2); or a pharmaceutically acceptable salt, hydrate, solvate, stereoisomer, tautomer, mixture, or combination thereof.

In some or any embodiments, the compound of Formula I or Formula (P-1) is according to Formula (P-Ic-1) or (P-Ic-2): 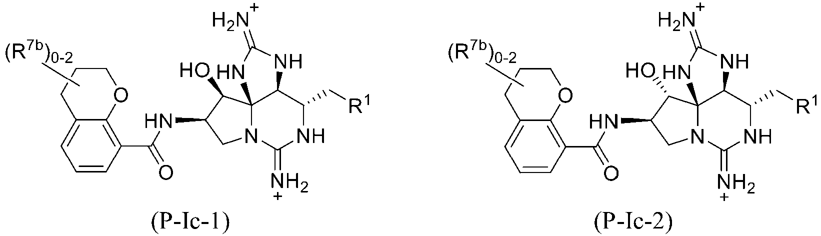 wherein R^{7b}, when present, and R¹ are as defined in Formula (I) in the Summary or as defined in some or any embodiments described herein. In some or any embodiments of Formula (P-Ic-1) and (P-Ic-2), each R^{7b}, when present, is independently C₁₋₃alkyl or halo, and R¹ is as defined in Formula (I) in the Summary or as defined in some or any embodiments described herein. In some embodiments, the compound of Formula (I) or Formula (P-1) is according to Formula (P-Ic-1); or a pharmaceutically acceptable salt, hydrate, solvate, stereoisomer, tautomer, mixture, or combination thereof. In some embodiments, the compound of Formula (I) or Formula (P-1) is according to Formula (P-Ic-2); or a pharmaceutically acceptable salt, hydrate, solvate, stereoisomer, tautomer, mixture, or combination thereof.

In some or any embodiments, the compound of Formula I or Formula (P-1) is according to Formula (P-Id-1) and (P-Id-2): 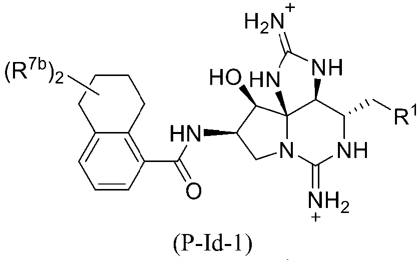 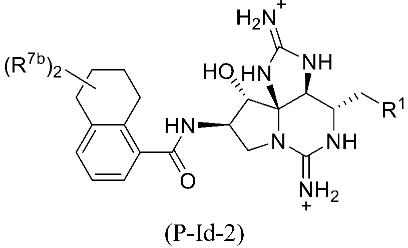 wherein R^{7b}, when present, and R¹ are as defined in Formula (I) in the Summary or as defined in some or any embodiments described herein. In some or any embodiments of Formula (P-Id-1) and (P-Id-2), each R^{7b}, when present, is independently C₁₋₃alkyl or halo, and R¹ is as defined in Formula (I) in the Summary or as defined in some or any embodiments described herein. In some or any embodiments of Formula (P-Id-1) and (P-Id-2), each R^{7b}, when present, is hydrogen, and R¹ is as defined in Formula (I) in the Summary or as defined in some or any embodiments described herein. In some embodiments, the compound of Formula (I) or Formula (P-1) is according to Formula (P-Id-1); or a pharmaceutically acceptable salt, hydrate, solvate, stereoisomer, tautomer, mixture, or combination thereof. In some embodiments, the compound of Formula (I) or Formula (P-1) is according to Formula (P-Id-2); or a pharmaceutically acceptable salt, hydrate, solvate, stereoisomer, tautomer, mixture, or combination thereof.

In some or any embodiments, the compound of Formula (I), (P-I), (P-Ia), (P-Ib-1), (P-Ib-2), (P-Ic-1), (P-Ic-2), (P-Id-1), and (P-Id-2) is that where R¹ is -NR⁴C(O)R^{4a}, heteroaryl,
-CH₂NH₂, -CH₂CH₂NH₂, -CH₂NH₃⁺, -CH₂CH₂NH₃⁺, -NR⁵S(O)₂R^{5a}
-NR⁶C(O)NR^{6a}R^{6b}, or-NR⁸C(O)OR^{9a}; and all other groups are as defined in the Summary or as defined in some or any embodiments described herein. In some or any embodiments, the compound of Formula (I), (P-I), (P-Ia), (P-Ib-1), (P-Ib-2), (P-Ic-1), (P-Ic-2), (P-Id-1), and (P-Id-2) is that where R¹ is -NR^{3a}R³ or -NR⁴C(O)R^{4a}; and all other groups are as defined in the Summary or as defined in some or any embodiments described herein. In some or any embodiments, the compound of Formula (I), P-I), (P-Ia), (P-Ib-1), (P-Ib-2), (P-Ic-1), (P-Ic-2), (P-Id-1), and (P-Id-2) is that where R¹ is -NH₂ or -NHC(O)R^{4a}; and all other groups are as defined in the Summary or as defined in some or any embodiments described herein. In some or any embodiments, the compound of Formula (I), (P-I) (P-Ia), (P-Ib-1), (P-Ib-2), (P-Ic-1), (P-Ic-2), (P-Id-1), and (P-Id-2) is that where R¹ is
-NH₂ or -NHC(O)R^{4a}; and R^{4a} is a 5- or 6-membered heteroaryl ring optionally substituted with 1, 2, 3, or 4 R^{4b} groups; and all other groups are as defined in Formula (I) in the Summary or as defined in some or any embodiments described herein. In some or any embodiments, the compound of Formula (I), (P-I), (P-Ia), (P-Ib-1), (P-Ib-2), (P-Ic-1), (P-Ic-2), (P-Id-1), and (P-Id-2) is that where R¹ is -NH₂ or -NHC(O)R^{4a}; and R^{4a} is a 6-membered heteroaryl ring optionally substituted with 1, 2, 3, or 4 R^{4b} groups; and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R¹ is -NH₂ or -NHC(O)R^{4a}; and R^{4a} is 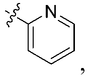 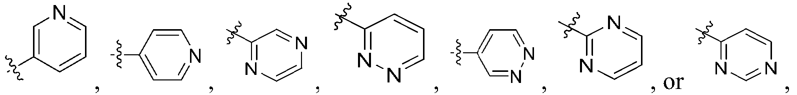 wherein 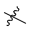 indicates the point of attachment the rest of the molecule and the R^{4a} ring is optionally substituted with 1, 2, or 3 R^{4b} groups; and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R¹ is -NH₂ or -NHC(O)R^{4a}; and R^{4a} is 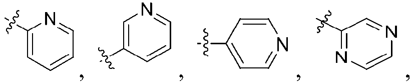 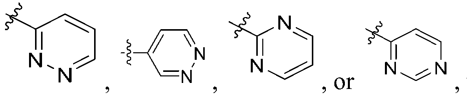 wherein 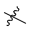 indicates the point of attachment the rest of the molecule and the R^{4a} ring is optionally substituted with 1, 2, or 3 R^{4b} groups where each R^{4b} group, when present, is independently alkyl or hydroxy; and all other groups are as defined in Formula (I) in the Summary or as defined in some or any embodiments described herein. In some embodiments of Formula (I), (P-I), (P-Ia), (P-Ib-1), (P-Ib-2), (P-Ic-1), (P-Ic-2), (P-Id-1), and (P-Id-2), R¹ is -NH₂ or -NHC(O)R^{4a}; and R^{4a} is pyridinyl, methyl-pyridinyl, hydroxy-pyridinyl or a tautomer thereof, pyrimidinyl, methyl-pyrimidinyl, hydroxy-pyrimidinyl or a tautomer thereof, dihydroxy-pyrimidinyl or a tautomer thereof, 2,4(1*H*,3*H*)-dioxo-pyrimidin-6-yl or a tautomer thereof, pyridazinyl, methyl-pyridazinyl, hydroxy-pyridazinyl or a tautomer thereof, pyrazinyl, hydroxy-pyrazinyl or a tautomer thereof, or methyl-pyrazinyl; and all other groups are as defined in Formula (I) in the Summary or as defined in some or any embodiments described herein.

In some or any embodiments of Formula (I), (P-I), (P-Ia), (P-Ib-1), (P-Ib-2), (P-Ic-1), (P-Ic-2), (P-Id-1), and (P-Id-2), R¹ is -NR^{3a}R³; and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R¹ is -NR^{3a}R³ where one of R^{3a} and R³ is hydrogen and the other is C₁₋₆alkyl, or both are hydrogen; and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R¹ is -NH₂; and all other groups are as defined in Formula (I) in the Summary or as defined in some or any embodiments described herein.

In some or any embodiments of Formula (I), (P-I), (P-Ia), (P-Ib-1), (P-Ib-2), (P-Ic-1), (P-Ic-2), (P-Id-1), and (P-Id-2), R⁴ is hydrogen, and all other groups are as defined in Formula (I) in the Summary or as defined in some or any embodiments described herein.

In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁴C(O)R^{4a}, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁴C(O)R^{4a}, R⁴ is hydrogen, and all other groups are as defined in Formula (I) in the Summary or as defined in some or any embodiments described herein.

In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁴C(O)R^{4a}, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁴C(O)R^{4a}, R⁴ is hydrogen, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is hydrogen and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is C₁₋₆alkyl and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is halo-C₁₋₆alkyl and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is amino-C₁₋₆alkyl; C₁₋₆alkylaminoC₁₋₆alkyl; or di-C₁₋₆alkylaminoC₁₋₆alkyl and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is C₁₋₆alkoxyC₁₋₆alkyl and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is cycloalkyl and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is cycloalkylalkyl and all other groups are as defined in Formula (I) in the Summary or as defined in some or any embodiments described herein.

In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁴C(O)R^{4a}, R^{4a} is heteroaryl optionally substituted with 1, 2, 3, or 4 R^{4b} groups, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁴C(O)R^{4a}, R⁴ is hydrogen, R^{4a} is heteroaryl optionally substituted with 1, 2, 3, or 4 R^{4b} groups, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is unsubstituted heteroaryl and all other groups are as defined in Formula (I) in the Summary or as defined in some or any embodiments described herein, including in any embodiments described in this paragraph. In some or any embodiments, the compound of Formula (I), (PI), (P-Ia), (P-Ib-1), (P-Ib-2), (P-Ic-1), (P-Ic-2), (P-Id-1), and (P-Id-2) is that where R¹ is - NH₂ or -NHC(O)R^{4a}; and R^{4a} is a 5- or 6-membered heteroaryl ring optionally substituted with 1, 2, 3, or 4 R^{4b} groups; and all other groups are as defined in Formula (I) in the Summary or as defined in some or any embodiments described herein, including in any embodiments described in this paragraph. In some or any embodiments, the compound of Formula (I), (P-I), (P-Ia), (P-Ib-1), (P-Ib-2), (P-Ic-1), (P-Ic-2), (P-Id-1), and (P-Id-2) is that where R¹ is -NH₂ or -NHC(O)R^{4a}; and R^{4a} is a 6-membered heteroaryl ring optionally substituted with 1, 2, 3, or 4 R^{4b} groups; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), R¹ is -NH₂ or - NHC(O)R^{4a}; and R^{4a} is 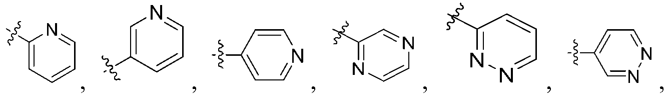 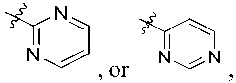 wherein 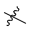 indicates the point of attachment the rest of the molecule and the R^{4a} ring is optionally substituted with 1, 2, or 3 R^{4b} groups; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), R¹ is -NH₂ or -NHC(O)R^{4a}; and R^{4a} is 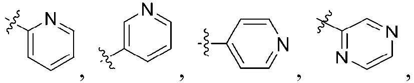 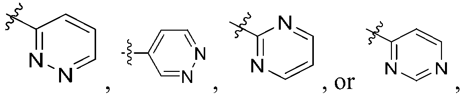 wherein 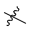 indicates the point of attachment the rest of the molecule and the R^{4a} ring is optionally substituted with 1, 2, or 3 R^{4b} groups where each R^{4b} group, when present, is independently alkyl or hydroxy; and all other groups are as defined in Formula (I) in the Summary or as defined in some or any embodiments described herein, including in any embodiments described in this paragraph. In some or any embodiments of Formula (I), (P-Ia), (P-Ib-1), (P-Ib-2), (P-Ic-1), (P-Ic-2), (P-Id-1), and (P-Id-2), R¹ is -NH₂ or -NHC(O)R^{4a}; and R^{4a} is 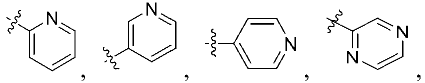 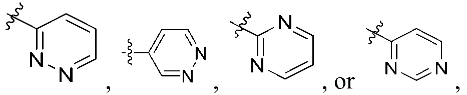 wherein indicates the point of attachment the rest of the molecule and the R^{4a} ring is optionally substituted with 1, 2, or 3 R^{4b} groups where each R^{4b} group, when present, is hydroxyalkyloxy; and all other groups are as defined in Formula (I) in the Summary or as defined in some or any embodiments described herein, including in any embodiments described in this paragraph. In some embodiments of Formula (I), (P-I), (P-Ia), (P-Ib-1), (P-Ib-2), (P-Ic-1), (P-Ic-2), (P-Id-1), and (P-Id-2), R¹ is -NH₂ or - NHC(O)R^{4a}; and R^{4a} is pyridinyl, methyl-pyridinyl, hydroxy-pyridinyl or a tautomer thereof, pyrimidinyl, methyl-pyrimidinyl, hydroxy-pyrimidinyl or a tautomer thereof, 2,4(1*H*,3*H*)-dioxo-pyrimidin-6-yl or a tautomer thereof, pyridazinyl, methyl-pyridazinyl, hydroxy-pyridazinyl or a tautomer thereof, pyrazinyl, hydroxy-pyrazinyl or a tautomer thereof, or methyl-pyrazinyl; and all other groups are as defined in Formula (I) in the Summary or as defined in some or any embodiments described herein, including in any embodiments described in this paragraph.

In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁴C(O)R^{4a}, R^{4a} is heteroaralkyl optionally substituted with 1, 2, 3, or 4 R^{4b} groups, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁴C(O)R^{4a}, R⁴ is hydrogen, R^{4a} is heteroaralkyl optionally substituted with 1, 2, 3, or 4 R^{4b} groups, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is unsubstituted heteroaralkyl and all other groups are as defined in Formula (I) in the Summary or as defined in some or any embodiments described herein, including in any embodiments described in this paragraph. In some or any embodiments, the compound of Formula (I), (PI), (P-Ia), (P-Ib-1), (P-Ib-2), (P-Ic-1), (P-Ic-2), (P-Id-1), and (P-Id-2) is that where R¹ is - NHC(O)R^{4a}; and R^{4a} is heteroaralkyl and the heteroaryl portion is a 5- or 6-membered heteroaryl ring optionally substituted with 1, 2, 3, or 4 R^{4b} groups; and all other groups are as defined in Formula (I) in the Summary or as defined in some or any embodiments described herein, including in any embodiments described in this paragraph. In some or any embodiments, the compound of Formula (I), (P-I), (P-Ia), (P-Ib-1), (P-Ib-2), (P-Ic-1), (P-Ic-2), (P-Id-1), and (P-Id-2) is that where -NHC(O)R^{4a}; and R^{4a} is heteroaralkyl wherein the heteroaryl portion is a 6-membered heteroaryl ring optionally substituted with 1, 2, 3, or 4 R^{4b} groups; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), R¹ is -NHC(O)R^{4a}; and R^{4a} is 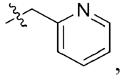 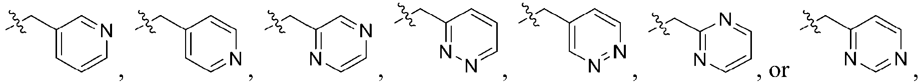 wherein 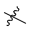 indicates the point of attachment the rest of the molecule and the R^{4a} ring is optionally substituted with 1, 2, or 3 R^{4b} groups; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), R¹ is - NHC(O)R^{4a}; and R^{4a} is 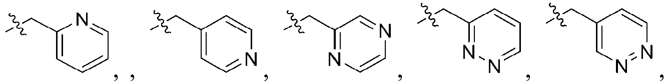 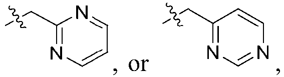 wherein 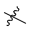 indicates the point of attachment the rest of the molecule and the R^{4a} ring is optionally substituted with 1, 2, or 3 R^{4b} groups where each R^{4b} group, when present, is independently alkyl; and all other groups are as defined in Formula (I) in the Summary or as defined in some or any embodiments described herein, includ ing in any embodiments described in this paragraph. In some embodiments of Formula (I), (PI), (P-Ia), (P-Ib-1), (P-Ib-2), (P-Ic-1), (P-Ic-2), (P-Id-1), and (P-Id-2), R¹ is -NHC(O)R^{4a}; and R^{4a} is heteroaralkyl wherein the heteroaryl portion is pyridinyl, methyl-pyridinyl, hydroxy-pyridinyl or a tautomer thereof, pyrimidinyl, methyl-pyrimidinyl, hydroxy-pyrimidinyl or a tautomer thereof, 2,4(1*H*,3*H*)-dioxo-pyrimidin-6-yl or a tautomer thereof, pyridazinyl, methyl-pyridazinyl, hydroxy-pyridazinyl or a tautomer thereof, pyrazinyl, hydroxy-pyrazinyl or a tautomer thereof, or methyl-pyrazinyl; and all other groups are as defined in Formula (I) in the Summary or as defined in some or any embodiments described herein, including in any embodiments described in this paragraph.

In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁴C(O)R^{4a}, R^{4a} is cycloalkyl, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁴C(O)R^{4a}, R⁴ is hydrogen, R^{4a} is cycloalkyl, and all other groups are as defined in Formula (I) in the Summary or as defined in some or any embodiments described herein. In some or any embodiments, the compound of Formula (I), (P-Ia), (P-Ib-1), (P-Ib-2), (P-Ic-1), (P-Ic-2), (P-Id-1), and (P-Id-2) is that where R¹ is -NHC(O)R^{4a}; and R^{4a} is cycloalkyl optionally substituted with 1, 2, 3, or 4 R^{4b} groups; and all other groups are as defined in Formula (I) in the Summary or as defined in some or any embodiments described herein, including in any embodiments described in this paragraph. In some or any embodiments, the compound of Formula (I), (P-Ia), (P-Ib-1), (P-Ib-2), (P-Ic-1), (P-Ic-2), (P-Id-1), and (P-Id-2) is that where -NHC(O)R^{4a}; and R^{4a} is cycloalkyl optionally substituted with 1 R^{4b} groups; and all other groups are as defined in Formula (I) in the Summary or as defined in some or any embodiments described herein, including in any embodiments described in this paragraph. In some or any embodiments, the compound of Formula (I), (P-Ia), (P-Ib-1), (P-Ib-2), (P-Ic-1), (P-Ic-2), (P-Id-1), and (P-Id-2) is that where -NHC(O)R^{4a}; R^{4a} is cycloalkyl optionally substituted with 1 R^{4b} group; R^{4b} is aryl or heteroaryl; and all other groups are as defined in Formula (I) in the Summary or as defined in some or any embodiments described herein, including in any embodiments described in this paragraph. In some or any embodiments, the compound of Formula (I), (P-Ia), (P-Ib-1), (P-Ib-2), (P-Ic-1), (P-Ic-2), (P-Id-1), and (P-Id-2) is that where -NHC(O)R^{4a}; R^{4a} is cycloalkyl optionally substituted with 1 R^{4b} group; R^{4b} is heteroaryl; and all other groups are as defined in Formula (I) in the Summary or as defined in some or any embodiments described herein, including in any embodiments described in this paragraph. In some or any embodiments, the compound of Formula (I), (P-Ia), (P-Ib-1), (P-Ib-2), (P-Ic-1), (P-Ic-2), (P-Id-1), and (P-Id-2) is that where -NHC(O)R^{4a}; R^{4a} is cycloalkyl optionally substituted with 1 R^{4b} group; R^{4b} is 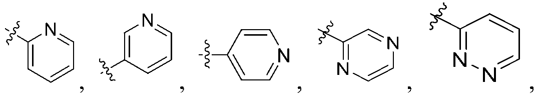 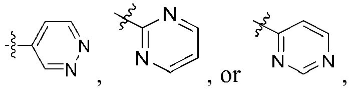 wherein 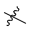 indicates the point of attachment the rest of the molecule; and and all other groups are as defined in Formula (I) in the Summary or as defined in some or any embodiments described herein, including in any embodiments described in this paragraph.

In some or any embodiments of Formula (I), (P-I), (P-Ia), (P-Ib-1), (P-Ib-2), (P-Ic-1), (P-Ic-2), (P-Id-1), and (P-Id-2), one R^{4b} is present; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), one R^{4b} is present and is C₁₋₆alkyl or hydroxy; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), two R^{4b} are present and are independently selected; and all other groups are as defined in Formula (I) in the Summary or as defined in some or any embodiments described herein, including in any embodiments described in this paragraph. In some or any embodiments of Formula (I), (PI), (P-Ia), (P-Ib-1), (P-Ib-2), (P-Ic-1), (P-Ic-2), (P-Id-1), and (P-Id-2), two R^{4b} are present and are independently selected from C₁₋₆alkyl and hydroxy; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), each R^{4b}, when present, is independently C₁₋₆alkyl or hydroxy, and all other groups are as defined in Formula (I) in the Summary or as defined in some or any embodiments described herein, including in any embodiments described in this paragraph.

In some or any embodiments of Formula (I), (P-I), (P-1a), (P-Ib-1), (P-Ib-2), (P-Ic-1), (P-Ic-2), (P-Id-1), and (P-Id-2), R¹ is -NR⁴C(O)R^{4a}, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R¹ is
-NR⁴C(O)R^{4a}, R⁴ is hydrogen, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is heterocyclic optionally substituted with 1, 2, 3, or 4 R^{4b} groups and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is heterocyclic substitued with 1, 2, or 3 halo groups and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is heterocyclicalkyl where the heterocyclic portion is optionally substituted with 1, 2, 3, or 4 R^{4b} groups and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is heterocyclic substitued with 1, 2, or 3 halo groups and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is aryl optionally substituted with 1, 2, 3 or 4 R^{4b} groups and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a} is aralkyl where the aryl portion is optionally substituted with 1, 2, 3, or 4 R^{4b} and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R^{4a}, when aryl or arylalkyl, is phenyl or phenylalkyl, where R^{4a} is optionally substituted with 1, 2, 3, or 4 R^{4b} groups; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), R^{4a} is heterocycloalkyl optionally substituted with 1, 2, 3, or 4 R^{4b} groups and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), one R^{4b} is present; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), one R^{4b} is present and is C₁₋₆alkyl or hydroxy; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), two R^{4b} are present and are independently selected; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), two R^{4b} are present and are independently selected from C₁₋₆alkyl and hydroxy; and all other groups are as defined in Formula (I) in the Summary or as defined in some or any embodiments described herein, including in any embodiments described in this paragraph. In some or any embodiments of Formula (I), (P-I), (P-Ia), (P-Ib-1), (P-Ib-2), (P-Ic-1), (P-Ic-2), (P-Id-1), and (P-Id-2), each R^{4b}, when present, is independently C₁₋₆alkyl or hydroxy, and all other groups are as defined in Formula (I) in the Summary or as defined in some or any embodiments described herein, including in any embodiments described in this paragraph.

In some or any embodiments of Formula (I), (P-I), (P-Ia), (P-Ib-1), (P-Ib-2), (P-Ic-1), (P-Ic-2), (P-Id-1), and (P-Id-2), one R^{4b} is present; and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), one R^{4b} is present and is C₁₋₆alkyl or hydroxy; and all other groups are as defined in Formula (I) in the Summary or as defined in some or any embodiments described herein.

In some or any embodiments of Formula (I), (P-I), (P-Ia), (P-Ib-1), (P-Ib-2), (P-Ic-1), (P-Ic-2), (P-Id-1), and (P-Id-2), two R^{4b} are present and are independently selected; and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), two R^{4b} are present and are independently selected from C₁₋₆alkyl and hydroxy; and all other groups are as defined in Formula (I) in the Summary or as defined in some or any embodiments described herein.

In some or any embodiments of Formula (I), (P-I), (P-Ia), (P-Ib-1), (P-Ib-2), (P-Ic-1), (P-Ic-2), (P-Id-1), and (P-Id-2), each R^{4b}, when present, is independently C₁₋₆alkyl or hydroxy, and all other groups are as defined in Formula (I) in the Summary or as defined in some or any embodiments described herein.

In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁵S(O)₂R^{5a}. In some or any embodiments of Formula (I), (P-I), (P-Ia), (P-Ib-1), (P-Ib-2), (P-Ic-1), (P-Ic-2), (P-Id-1), and (P-Id-2), R⁵ is hydrogen, and all other groups are as defined in Formula (I) in the Summary or as defined in some or any embodiments described herein.

In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁶C(O)NR^{6a}R⁶⁶, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R⁶ is hydrogen and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R⁶ and R^{6a} are hydrogen and all other groups are as defined in Formula (I) in the Summary or as defined in some or any embodiments described herein.

In some or any embodiments of Formula (I), (P-I), (P-Ia), (P-Ib-1), (P-Ib-2), (P-Ic-1), (P-Ic-2), (P-Id-1), and (P-Id-2), R¹ is -NR⁸C(O)OR^{8a}, and all other groups are as defined in Formula (I) in the Summary or as defined in some or any embodiments described herein. In some or any embodiments of Formula (I), (P-I), (P-Ia), (P-Ib-1), (P-Ib-2), (P-Ic-1), (P-Ic-2), (P-Id-1), and (P-Id-2), R⁸ is hydrogen, and all other groups are as defined in Formula (I) in the Summary or as defined in some or any embodiments described herein.

In some or any embodiments of the compound of Formula (I), (P-I), (P-Ia), (P-Ib-1), and (P-Ib-2), R⁷ is hydrogen, and R¹ is -NHC(O)R^{4a}, where R^{4a} is a heteroaryl selected from the group consisting of pyridinyl, pyrimidinyl, pyridazinyl, and pyrazinyl, each of which is optionally substituted with 1, 2, or 3 groups selected from the group consisting of C₁₋₆alkyl, hydroxy, halo, halo-C₁₋₆alkyl, C₁₋₆alkoxy, and cyano, and all other groups are as defined in Formula (I) in the Summary or as defined in some or any embodiments described herein. In some or any embodiments of the compound of Formula (I), (P-I), (P-Ia), (P-Ib-1), and (P-Ib-2), including any embodiments in this paragraph, R^{4a} is a heteroaryl selected from 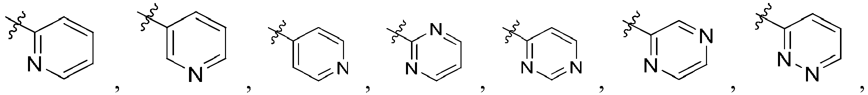 and 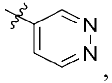 where each R^{4a} is optionally substituted with one or two groups selected from C₁₋₆alkyl, hydroxy, halo, halo-C₁₋₆alkyl, and C₁₋₆alkoxy, and wherein 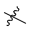 indicates the point of attachment of R^{4a} to the rest of the molecule and all other groups are as defined in Formula (I) in the Summary or as defined in some or any embodiments described herein.

In some or any embodiments of the compound of Formula (I), (P-I), (P-Ia), (P-Ib-1), and (P-Ib-2), R⁷ is hydrogen, and R¹ is
-NH⁴C(O)R^{4a}, where R^{4a} is aryl optionally substituted with 1, 2, or 3 groups selected from the group consisting of C₁₋₆alkyl, hydroxy, halo, halo-C₁₋₆alkyl, C₁₋₆alkoxy, and cyano, and all other groups are as defined in Formula (I) in the Summary or as defined in some or any embodiments described herein.

In some or any embodiments of the compound of Formula (I), (P-I), (P-Ia), (P-Ib-1), and (P-Ib-2), R⁷ is hydrogen, and R¹ is -NHC(O)R^{4a}, where R^{4a} is heterocyclic optionally substituted with 1, 2, or 3 groups selected from the group consisting of C₁₋₆alkyl, hydroxy, halo, halo-C₁₋₆alkyl, C₁₋₆alkoxy, oxo, and cyano, and all other groups are as defined in Formula (I) in the Summary or as defined in some or any embodiments described herein. In some or any embodiments, R^{4a} is 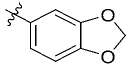 optionally substituted with 1 or 2 groups selected from C₁₋₆alkyl, and halo, and all other groups are as defined in Formula (I) in the Summary or as defined in some or any embodiments described herein. In some or any embodiments, R^{4a} is 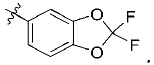 In some or any embodiments of the compound of Formula (I), (P-I), (P-Ia), (P-Ib-1), and (P-Ib-2), including any embodiments in this paragraph, R⁷ is hydrogen, and R^{7a} is a heterocyclic group optionally substituted with 1, 2, 3, or 4 R^{7b}, wherein each R^{7b}, when present, is independently halo, hydroxyl, C₁₋₆alkyl, halo-C₁₋₆alkyl, C₁₋₆alkoxy.

In some or any embodiments of the compound of Formula (I), (P-I), (P-Ia), (P-Ib-1), and (P-Ib-2), R⁷ is hydrogen, and R¹ is -NHC(O)R^{4a}, where R^{4a} is heterocycoalkyl optionally substituted with 1, 2, or 3 groups selected from the group consisting of C₁₋₆alkyl, hydroxy, halo, halo-C₁₋₆alkyl, C₁₋₆alkoxy, oxo, and cyano, and all other groups are as defined in Formula (I) in the Summary or as defined in some or any embodiments described herein.

In some or any embodiments of the compound of Formula (I), (P-I), (P-Ia), (P-Ib-1), and (P-Ib-2), R⁴, R⁵, R⁶ and R⁷ are each hydrogen; and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is heterocyclic optionally substituted with 1, 2, 3, or 4 R^{7b}, and all other groups are as defined in Formula (I) in the Summary or as defined in some or any embodiments described herein.

In some or any embodiments of Formula (I), (P-I), (P-Ia), (P-Ib-1), and (P-Ib-2), the heterocyclic in R^{7a} is benzo-1,4-dioxanyl, benzodioxolyl, 2,3-dihydrobenzofuranyl, chromanyl, 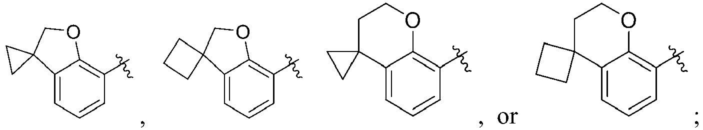 each of which is optionally substituted with 1, 2, or 3 groups independently selected from halo, C₁₋₆alkyl, halo-C₁₋₆alkyl, hydroxy, C₁₋₆alkoxy, halo-C₁₋₆alkoxy, and phenyl, and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is unsubstituted 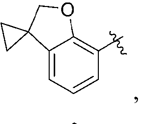 unsubstituted 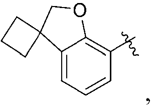 unsubstituted 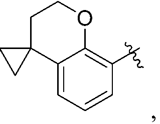 or unsubstituted 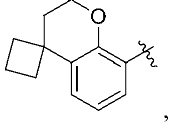 and all other groups are as defined in Formula (I) in the Summary or as defined in some or any embodiments described herein.

In some or any embodiments of Formula (I), (P-I), (P-Ia), (P-Ib-1), and (P-Ib-2), the heterocyclic in R^{7a} comprises one heteroatom which is oxygen and wherein the heterocyclic is optionally substituted with 1, 2, or 3 groups independently selected from halo, C₁₋₆alkyl, halo-C₁₋₆alkyl, hydroxy, C₁₋₆alkoxy, halo-C₁₋₆alkoxy, and phenyl, and all other groups are as defined in Formula (I) in the Summary or as defined in some or any embodiments described herein.

In some or any embodiments of Formula (I), (P-I), (P-Ia), (P-Ib-1), and (P-Ib-2), the heterocyclic in R^{7a} comprises one heteroatom which is oxygen and wherein the heterocyclic is optionally substituted with one gem-di-C₁₋₃alkyl or one gem-dihalo, and all other groups are as defined in Formula (I) in the Summary or as defined in some or any embodiments described herein.

In some or any embodiments of Formula (I), (P-I), (P-Ia), (P-Ib-1), and (P-Ib-2), the heterocyclic in R^{7a} is benzo-1,4-dioxanyl optionally substituted with one gem-di-C₁₋₃alkyl, cyclopropyl, or one gem-dihalo, and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is 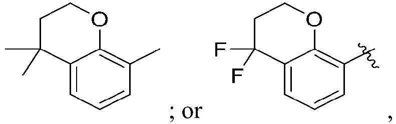 and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is aryl optionally substituted with 1, 2, 3, or 4 R^{7b}, and all other groups are as defined in Formula (I) in the Summary or as defined in some or any embodiments described herein.

In some or any embodiments of Formula (I), (P-I), (P-Ia), (P-Ib-1), and (P-Ib-2), the aryl in R^{7a} is phenyl, naphthyl, tetrahydronaphthyl, fluorenyl, 6,7,8,9-tetrahydro-5H-benzo[7]annulenyl, 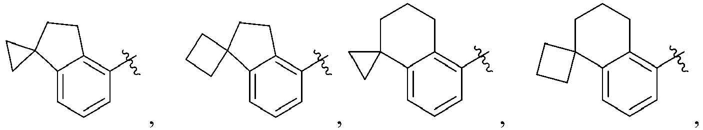 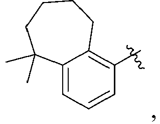 or indanyl; each of which is optionally substituted with 1, 2, or 3 groups independently selected from halo, C₁₋₆alkyl, halo-C₁₋₆alkyl, hydroxy, C₁₋₆alkoxy, halo-C₁₋₆alkoxy, C₁₋₆alkylthio,
C₁₋₆alkylsulfonyl, and amino, and all other groups are as defined in Formula (I) in the Summary or as defined in some or any embodiments described herein.

In some or any embodiments of Formula (I), (P-I), (P-Ia), (P-Ib-1), and (P-Ib-2), the aryl in R^{7a} is tetrahydronaphthyl, 6,7,8,9-tetrahydro-5*H*-benzo[7]annulenyl, or indanyl; each of which is optionally substituted with one gem-di-C₁₋₃alkyl or one gem-di-halo, and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is unsubstituted 6,7,8,9-tetrahydro-5*H*-benzo[7]annulenyl, unsubstituted 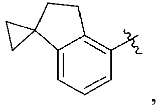 unsubstituted 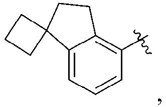 unsubstituted 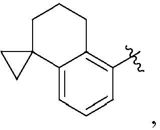 unsubstituted 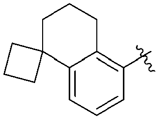 , or unsubstitued

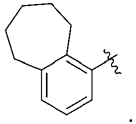

In some or any embodiments of Formula (I), (P-I), (P-Ia), (P-Ib-1), and (P-Ib-2), R^{7a} is 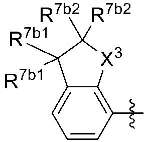 where X³ is -O-, -O-C(R^{7b3})(R^{7b3})-, -C(R^{7b3})(R^{7b3})-O-, -C(R^{7b3})(R^{7b3})-,-C(R^{7b3})(R^{7b3} )-C(R^{7b4})(R^{7b4})-, or
-C(R^{7b3})(R^{7b3})-C(R^{7b4})(R^{7b4})-C(R^{7b5})(R^{7b5})- and each R^{7b1}, R^{7b2}, R^{7b3}, R^{7b4}, and R^{7b5} is independently hydrogen, halo, or C₁₋₃-alkyl, and all other groups are as defined in Formula (I) in the Summary or as defined in some or any embodiments described herein. In some or any embodiments of Formula (I), (P-I), (P-Ia), (P-Ib-1), and (P-Ib-2), a) one R^{7b1} is methyl or ethyl and the other R^{7b1} is hydrogen, or b) the two R^{7b1} are both hydrogen, or c) the two R^{7b1} are both methyl; and each R^{7b2}, R^{7b3}, R^{7b4}, and R^{7b5} are hydrogen, and all other groups are as defined in Formula (I) in the Summary or as defined in some or any embodiments described herein. In some or any embodiments of Formula (I), (P-I), (P-Ia), (P-Ib-1), and (P-Ib-2), X³ is -C(R^{7b3})(R^{7b3})-O-, and all other groups are as defined in Formula (I) in the Summary or as defined in some or any embodiments described herein. In some or any embodiments of Formula (I), (P-I), (P-Ia), (P-Ib-1), and (P-Ib-2), X³ is -C(R^{7b3})(R^{7b3})-C(R^{7b4})(R^{7b4})-, and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is 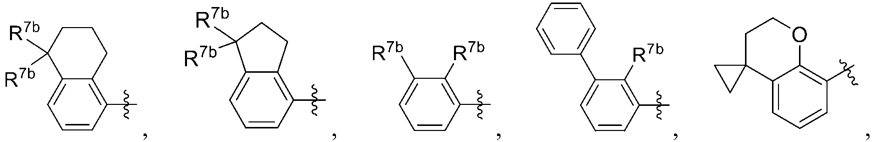 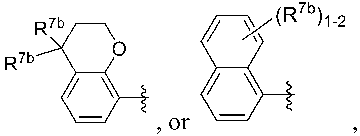 and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is 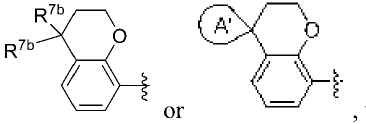 where the A' ring is a C₃₋₅cycloalkyl ring, and all other groups are as defined in Formula (I) in the Summary or as defined in some or any embodiments described herein. In some or any embodiments of Formula (I), (P-I), (P-Ia), (P-Ib-1), and (P-Ib-2), including any of the foregoing embodiments, a) one R^{7b} is methyl or ethyl and the other R^{7b} is hydrogen, b) the two R^{7b} are both hydrogen, c) the two R^{7b} are both methyl, d) the two R^{7b} are both halo, and all other groups are as defined in Formula (I) in the Summary or as defined in some or any embodiments described herein.

In some or any embodiments of Formula (I), (P-I), (P-Ia), (P-Ib-1), and (P-Ib-2), including any of the foregoing embodiments, a) one R^{7b} is methyl or ethyl and the other R^{7b} is hydrogen, b) the two R^{7b} are both hydrogen, c) the two R^{7b} are both methyl, d) the two R^{7b} are both halo, and all other groups are as defined in Formula (I) in the Summary or as defined in some or any embodiments described herein.

In some or any embodiments of Formula (I), (P-I), (P-Ia), (P-Ib-1), and (P-Ib-2), R⁷ is hydrogen; and all other groups are as defined in Formula (I) in the Summary or as defined in some or any embodiments described herein. In some or any embodiments of Formula (I), (P-I), (P-Ia), (P-Ib-1), and (P-Ib-2), R⁷ is C₁₋₆alkyl; and all other groups are as defined in Formula (I) in the Summary or as defined in some or any embodiments described herein. In some or any embodiments of Formula (I), (P-I), (P-Ia), (P-Ib-1), and (P-Ib-2), R⁷ is methyl; and all other groups are as defined in Formula (I) in the Summary or as defined in some or any embodiments described herein. In some or any embodiments of Formula (I), (P-I), (P-Ia), (P-Ib-1), and (P-Ib-2), R⁷ is hydrogen or methyl; and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is 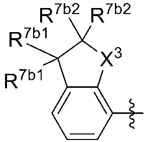 and X³ is -O-, -O-C(R^{7b3})(R^{7b3}), -C(R^{7b3})(R^{7b3})-O-, -C(R^{7b3})(R^{7b3})-,-C(R^{7b3})(R^{7b3})-C(R^{7b4})(R^{7b4})-, or
-C(R^{7b3})(R^{7b3})-C(R^{7b4})(R^{7b4})-C(R^{7b5})(R^{7b5})-; each R^{7b1}, R^{7b2}, R^{7b3}, R^{7b4}, and R^{7b5} is independently hydrogen, halo, or C₁₋₃-alkyl; or the two R^{7b1}, the two R^{7b2}, the two R^{7b3}, the two R^{7b4}, or the two R^{7b5} together with the carbon to which they are attached form a C₃₋₅cycloalkyl ring which has a spirocyclic attachment, and the remaining of R^{7b1}, R^{7b2}, R^{7b3}, R^{7b4}, and R^{7b5} are independently hydrogen, halo, or C₁₋₃-alkyl; and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is 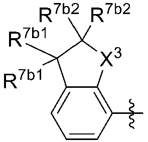 where X³ is -O-; each R^{7b1} and R^{7b2} is independently hydrogen, halo, or C₁₋₃-alkyl; or the two R^{7b1} or the two R^{7b2} together with the carbon to which the two R^{7b1} or the two R^{7b2} are attached form a C₃₋₅cycloalkyl ring (which has a spirocyclic attachment) and the remaining of R^{7b1} and R^{7b2} are hydrogen; and all other groups are as defined in Formula (I) in the Summary or as defined in some or any embodiments described herein. In some or any embodiments of Formula (I), (Ia), (Ib-1), and (Ib-2), R^{7a} is 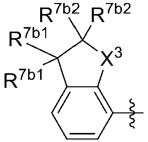 and X³ is -O-C(R^{7b3})(R^{7b3})-; each R^{7b1}, R^{7b2}, and R^{7b3} is independently hydrogen, halo, or C₁₋₃-alkyl; or the two R^{7b1}, the two R^{7b2}, or the two R^{7b3}, together with the carbon to which they are attached form a C₃₋₅cycloalkyl ring (which has a spirocyclic attachment), and the remaining of R^{7b1}, R^{7b2}, and R^{7b3} are independently hydrogen, halo, or C₁₋₃-alkyl; and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is 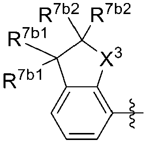 and X³ is -C(R^{7b3})(R^{7b3})-O-; each R^{7b1}, R^{7b2}, and R^{7b3} is independently hydrogen, halo, or C₁₋₃-alkyl; or the two R^{7b1}, the two R^{7b2}, or the two R^{7b3}, together with the carbon to which they are attached form a C₃₋₅cycloalkyl ring (which has a spirocyclic attachment), and the remaining of R^{7b1}, R^{7b2}, and R^{7b3} are independently hydrogen, halo, or C₁₋₃-alkyl; and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is 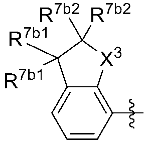 where X³ is -C(R^{7b3})(R^{7b3})-; each R^{7b1}, R^{7b2}, and R^{7b3} is independently hydrogen, halo, or C₁₋₃-alkyl; or the two R^{7b1}, the two R^{7b2}, or the two R^{7b3} together with the carbon to which they are attached form a C₃₋₅cycloalkyl ring (which has a spirocyclic attachment), and the remaining of R^{7b1}, R^{7b2}, and R^{7b3} are hydrogen; and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is 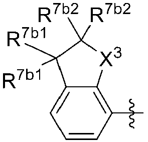 where X³ is -C(R^{7b3})(R^{7b3})-C(R^{7b4})(R^{7b4})- and each R^{7b1}, R^{7b2}, R^{7b3}, and R^{7b4} is independently hydrogen, halo, or C₁₋₃-alkyl; the two R^{7b1}, the two R^{7b2}, the two R^{7b3}, or the two R^{7b4} together with the carbon to which they are attached form a C₃₋₅cycloalkyl ring (which has a spirocyclic attachment), and the remaining of R^{7b1}, R^{7b2}, R^{7b3}, and R^{7b4} are hydrogen; and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is 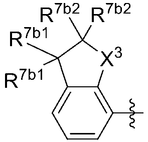 where X³ is -C(R^{7b3})(R^{7b3})-C(R^{7b4})(R^{7b4})-C(R^{7b5})(R^{7b5})-; each R^{7b1}, R^{7b2}, R^{7b3}, R^{7b4}, and R^{7b5} is independently hydrogen, halo, or C₁₋₃-alkyl; or the two R^{7b1}, the two R^{7b2}, the two R^{7b3}, the two R^{7b4}, or the two R^{7b5} together with the carbon to which they are attached form a C₃₋₅cycloalkyl ring (which has a spirocyclic attachment), and the remaining of R^{7b1}, R^{7b2}, R^{7b3}, R^{7b4}, and R^{7b5} are hydrogen; and all other groups are as defined in Formula (I) in the Summary or as defined in some or any embodiments described herein. In some or any embodiments, the R^{7b1} groups are the same, the R^{7b2} groups are the same, the R^{7b3} groups are the same, R^{7b4} groups are the same, and R^{7b5} groups are the same; and all other groups are as defined in Formula (I) in the Summary or as defined in some or any embodiments described herein. In some or any embodiments, each R^{7b1}, R^{7b2}, R^{7b3}, R^{7b4}, and R^{7b5} is hydrogen; and all other groups are as defined in Formula (I) in the Summary or as defined in some or any embodiments described herein. In some or any embodiments, the R^{7b1} groups are the same and are selected from halo (in another embodiment fluoro) and C₁₋₃-alkyl (in another embodiment methyl); and each R^{7b2}, R^{7b3}, R^{7b4}, and R^{7b5} is hydrogen; and all other groups are as defined in Formula (I) in the Summary or as defined in some or any embodiments described herein. In some or any embodiments, the R^{7b2} groups are the same and are selected from halo (in another embodiment fluoro) and C₁₋₃-alkyl (in another embodiment methyl); and each R^{7b1}, R^{7b3}, R^{7b4}, and R^{7b5} is hydrogen; and all other groups are as defined in Formula (I) in the Summary or as defined in some or any embodiments described herein. In some or any embodiments, the R^{7b3} groups are the same and are selected from halo (in another embodiment fluoro) and C₁₋₃-alkyl (in another embodiment methyl); and each R^{7b1}, R^{7b2}, R^{7b4}, and R^{7b5} is hydrogen; and all other groups are as defined in Formula (I) in the Summary or as defined in some or any embodiments described herein. In some or any embodiments, the R^{7b4} groups are the same and are selected from halo (in another embodiment fluoro) and C₁₋₃-alkyl (in another embodiment methyl); and each R^{7b1}, R^{7b2}, R^{7b3}, and R^{7b5} is hydrogen; and all other groups are as defined in Formula (I) in the Summary or as defined in some or any embodiments described herein. In some or any embodiments, the R^{7b5} groups are the same and are selected from halo (in another embodiment fluoro) and C₁₋₃-alkyl (in another embodiment methyl); and each R^{7b1}, R^{7b2}, R^{7b3}, and R^{7b4} is hydrogen; and all other groups are as defined in Formula (I) in the Summary or as defined in some or any embodiments described herein. In some or any embodiments, the C₃₋₅cycloalkyl ring (which has a spirocyclic attachment) is a C₃cycloalkyl; and all other groups are as defined in Formula (I) in the Summary or as defined in some or any embodiments described herein. In some or any embodiments, the C₃₋₅cycloalkyl ring (which has a spirocyclic attachment) is a C₄cycloalkyl; and all other groups are as defined in Formula (I) in the Summary or as defined in some or any embodiments described herein. In some or any embodiments, the C₃₋₅cycloalkyl ring (which has a spirocyclic attachment) is a C₅cycloalkyl; and all other groups are as defined in Formula (I) in the Summary or as defined in some or any embodiments described herein. In some or any embodiments, the two R^{7b1} together with the carbon to which they are attached form a C₃₋₅cycloalkyl ring (which has a spirocyclic attachment); and all other groups are as defined in Formula (I) in the Summary or as defined in some or any embodiments described herein. In some or any embodiments, the two R^{7b1} together with the carbon to which they are attached form a C₃₋₅cycloalkyl ring (which has a spirocyclic attachment); when R^{7b1}, R^{7b2}, R^{7b3}, R^{7b4}, and R^{7b5} are present, each R^{7b1}, R^{7b2}, R^{7b3} R^{7b4}, and R^{7b5} is hydrogen; and all other groups are as defined in Formula (I) in the Summary or as defined in some or any embodiments described herein. In some or any embodiments, a) one R^{7b1} is C₁₋₃alkyl (in some embodiments methyl or ethyl) and the other R^{7b1} is hydrogen, b) the two R^{7b1} are both hydrogen or c) the two R^{7b1} are both C₁₋₃alkyl (in some embodiments, methyl), or d) the two R^{7b1} together with the carbon to which they are attached form a cyclopropylene ring; and when R^{7b2}, R^{7b3}, R^{7b4}, and R^{7b5} are present, each R^{7b2}, R^{7b3}, R^{7b4}, and R^{7b5} is hydrogen; and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is 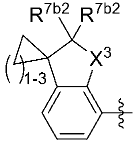 and X³ is -O-,
-C(R^{7b3})(R^{7b3})-, -O-C(R^{7b3})(R^{7b3})-, or -C(R^{7b3})(R^{7b3})-O-; R^{7a} is 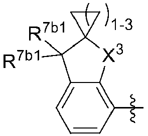 and X³ is -O-, -C(R^{7b3})(R^{7b3})-, -O-C(R^{7b3})(R^{7b3})-, or -C(R^{7b3})(R^{7b3})-O-; or R^{7a} is 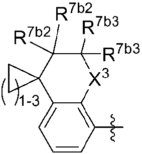 and X³ is -O-, -C(R7b⁴)(R^{7b4})-, -O-C(R^{7b3})(R^{7b3})-, or -C(R^{7b3})(R^{7b3})-O-; and all other groups are as defined in Formula (I) in the Summary or as defined in some or any embodiments described herein. In some or any embodiments, when R^{7b1}, R^{7b2}, R^{7b3}, and R^{7b4} are present, each R^{7b1}, R^{7b2}, R^{7b3}, and R^{7b4} is hydrogen. In some or any embodiments, the spirocyclic ring is cycloprop-di-yl or cyclobut-di-yl. In some or any embodiments, when R^{7b1}, R^{7b2}, R^{7b3}, and R^{7b4} are present, each R^{7b1}, R^{7b2}, R^{7b3}, and R^{7b4} is hydrogen; and the spirocyclic ring is cycloprop-di-yl or cyclobut-di-yl.

In some or any embodiments of Formula (I), (P-I), (P-Ia), (P-Ib-1), and (P-Ib-2), R^{7a} is biphenyl optionally substituted with 1, 2, 3, or 4 R^{7b}; and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is biphenyl optionally substituted with 1 or 2 R^{7b}; and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is biphenyl optionally substituted with 1 or 2 C₁₋₆alkyl; and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is 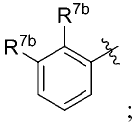 and all other groups are as defined in Formula (I) in the Summary or as defined in some or any embodiments described herein. In some or any embodiments, R^{7a} is 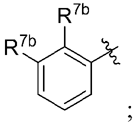 each R^{7b} is independently C₁₋₆alkyl, aryl-C₁₋₆alkyl, aryloxy, or - C(O)(heterocycloalkyl); and all other groups are as defined in Formula (I) in the Summary or as defined in some or any embodiments described herein. In some or any embodiments, R^{7a} is 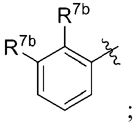 each R^{7b} is independently aryl-C₁₋₆alkyl, aryloxy, or - C(O)(heterocycloalkyl); and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is aryl optionally substituted with 1, 2, 3, or 4 R^{7b}; and all other groups are as defined in Formula (I) in the Summary or as defined in some or any embodiments described herein. In some or any embodiments of Formula (I), (P-I), (P-Ia), (P-Ib-1), and (P-Ib-2), the R^{7a} aryl is phenyl, naphthyl, tetrahydronaphthyl, fluorenyl, 6,7,8,9-tetrahydro-5*H*-benzo[7]annulenyl, 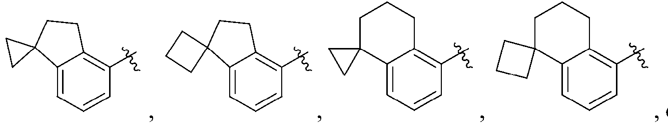 or indanyl; each of which is optionally substituted with 1, 2, 3, or 4 groups independently selected from halo, C₁₋₆alkyl, halo-C₁₋₆alkyl, aryl-C₁₋₆alkyl, hydroxy, C₁₋₆alkoxy, halo-C₁₋₆alkoxy, aryloxy, C₁₋₆alkylthio, C₁₋₆alkylsulfonyl, -C(O)(heterocycloalkyl), and amino; and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is aryl optionally substituted with 1, 2, 3, or 4 R^{7b}; and all other groups are as defined in Formula (I) in the Summary or as defined in some or any embodiments described herein. In some or any embodiments of Formula (I), (P-I), (P-Ia), (P-Ib-1), and (P-Ib-2), the R^{7a} aryl is phenyl, naphthyl, tetrahydronaphthyl, fluorenyl, 6,7,8,9-tetrahydro-5*H*-benzo[7]annulenyl, 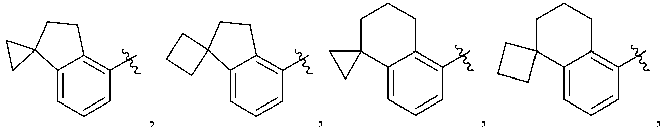 or indanyl; each of which is optionally substituted with 1, 2, 3, or 4 groups independently selected from halo, C₁₋₆alkyl, halo-C₁₋₆alkyl, aryl-C₁₋₆alkyl, hydroxy, C₁₋₆alkoxy, halo-C₁₋₆alkoxy, phenyloxy, C₁₋₆alkylthio, C₁₋₆alkylsulfonyl, and amino; and all other groups are as defined in Formula (I) in the Summary or as defined in some or any embodiments described herein. In some or any embodiments of Formula (I), (P-I), (P-Ia), (P-Ib-1), and (P-Ib-2), the R^{7a} aryl is tetrahydronaphthyl, 6,7,8,9-tetrahydro-5*H*-benzo[7]annulenyl, or indanyl, each of which is optionally substituted with one gem-dialkyl or one gem-dihalo. In some or any embodiments of Formula (I), (P-I), (P-Ia), (P-Ib-1), and (P-Ib-2), the aryl in R^{7a} is tetrahydronaphthyl, 6,7,8,9-tetrahydro-5*H*-benzo[7]annulenyl, or indanyl, each of which is optionally substituted with one gem-dimethyl or one gem-difluoro; and all other groups are as defined in Formula (I) in the Summary or as defined in some or any embodiments described herein. In some or any embodiments of Formula (I), (P-I), (P-Ia), (P-Ib-1), and (P-Ib-2), the R^{7a} aryl comprises a spirocyclic ring; and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is 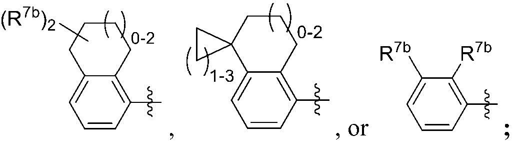 and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is 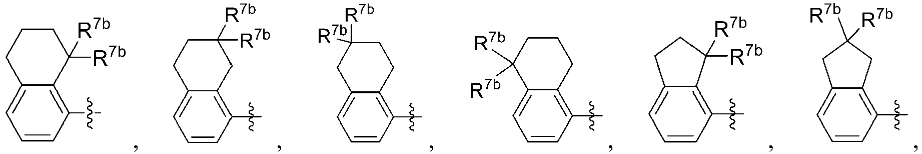 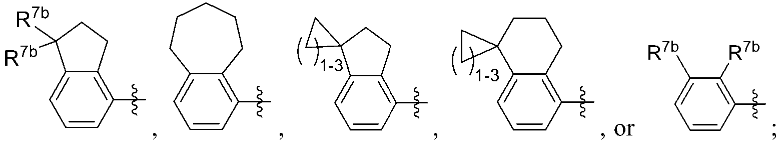 and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is 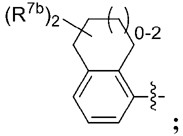 and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is 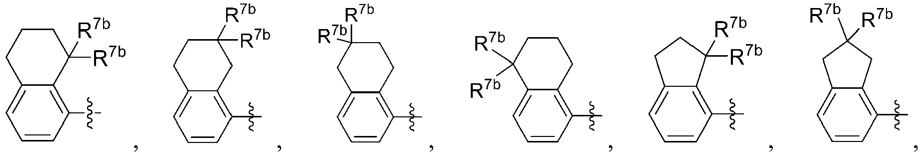 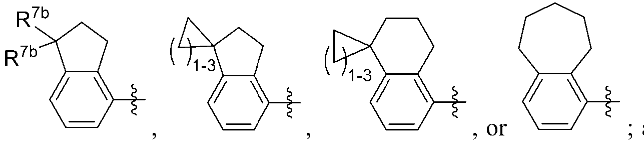 and all other groups are as defined in the Summary or as defined in some or any embodiments described herein. In some or any embodiments, both R^{7b} are hydrogen, both R^{7b} are C₁₋₆alkyl, or both R^{7b} are halo; and all other groups are as defined in Formula (I) in the Summary or as defined in some or any embodiments described herein. In some or any embodiments, both R^{7b} are hydrogen, both R^{7b} are C₁₋₃alkyl, or both R^{7b} are halo; and all other groups are as defined in Formula (I) in the Summary or as defined in some or any embodiments described herein. In some or any embodiments, both R^{7b} are hydrogen, both R^{7b} are methyl, or both R^{7b} are fluoro; and all other groups are as defined in Formula (I) in the Summary or as defined in some or any embodiments described herein. In some or any embodiments, both R^{7b} are hydrogen; and all other groups are as defined in Formula (I) in the Summary or as defined in some or any embodiments described herein. In some or any embodiments, both R^{7b} are methyl; and all other groups are as defined in Formula (I) in the Summary or as defined in some or any embodiments described herein. In some or any embodiments, both R^{7b} are fluoro; and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is heterocyclic optionally substituted with 1, 2, 3, or 4 R^{7b}; and all other groups are as defined in Formula (I) in the Summary or as defined in some or any embodiments described herein. In some or any embodiments of Formula (I), (P-I), (P-Ia), (P-Ib-1), and (P-Ib-2), the heterocyclic in R^{7a} is benzo-1,4-dioxanyl, benzodioxolyl, indolinyl, 2-oxo-indolinyl, pyrrolidinyl, piperidinyl, chromanyl, 2,3-dihydrobenzofuranyl, 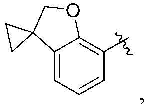 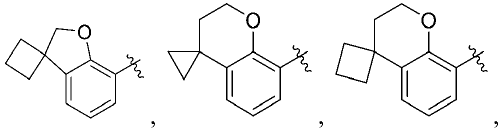 or decahydroquinolinyl; each of which is optionally substituted with 1, 2, or 3 groups independently selected from halo, C₁₋₆alkyl, halo-C₁₋₆alkyl, hydroxy, C₁₋₆alkoxy, halo-C₁₋₆alkoxy, and phenyl; and all other groups are as defined in Formula (I) in the Summary or as defined in some or any embodiments described herein. In some or any embodiments of Formula (I), (P-I), (P-Ia), (P-Ib-1), and (P-Ib-2), the heterocyclic in R^{7a} is benzo-1,4-dioxanyl, benzodioxolyl, chromanyl, 2,3-dihydrobenzofuranyl, 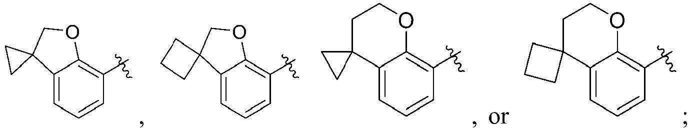 each of which is optionally substituted with 1, 2, or 3 groups independently selected from halo, C₁₋₆alkyl, halo-C₁₋₆alkyl, hydroxy, C₁₋₆alkoxy, halo-C₁₋₆alkoxy, and phenyl; and all other groups are as defined in Formula (I) in the Summary or as defined in some or any embodiments described herein. In some or any embodiments of Formula (I), (P-I), (P-Ia), (P-Ib-1), and (P-Ib-2), the heterocyclic in R^{7a} comprises one heteroatom which is oxygen; and all other groups are as defined in Formula (I) in the Summary or as defined in some or any embodiments described herein. In some or any embodiments of Formula (I), (P-I), (P-Ia), (P-Ib-1), and (P-Ib-2), the R^{7a} heterocyclic is benzo-1,4-dioxanyl, benzodioxolyl, chromanyl, or 2,3-dihydrobenzofuranyl, each of which is optionally substituted with one gem-dialkyl or one gem-dihalo. In some or any embodiments of Formula (I), (P-I), (P-Ia), (P-Ib-1), and (P-Ib-2), the aryl in R^{7a} is benzo-1,4-dioxanyl, benzodioxolyl, chromanyl, or 2,3-dihydrobenzofuranyl, each of which is optionally substituted with one gem-dimethyl or one gem-difluoro; and all other groups are as defined in Formula (I) in the Summary or as defined in some or any embodiments described herein.

In some or any embodiments of Formula (I), (P-I), (P-Ia), (P-Ib-1), and (P-Ib-2), the heterocyclic in R^{7a} unsubstituted benzodioxolyl, unsubstituted 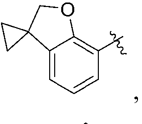 unsubstituted 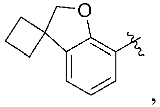 unsubstituted 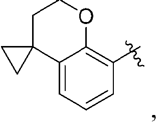 or unsubstituted 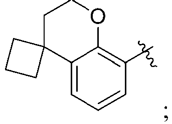 or R^{7a} is chromanyl, 2,3-dihydrobenzofuranyl, where the chromanyl and 2,3-dihydrobenzofuranyl are optionally substituted with 1, 2, or 3 groups independently selected from halo, C₁₋₆alkyl, halo-C₁₋₆alkyl, hydroxy, C₁₋₆alkoxy, halo-C₁₋₆alkoxy, and phenyl; and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is 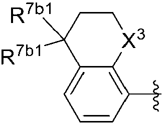 where X³ is CH₂ or O; one R^{7b1} is hydrogen and the other is alkyl (in some embodiments, methyl or ethyl), both R^{7b1} are hydrogen, both R^{7b1} are C₁-C₃alkyl (in some embodiments, methyl), or the two R^{7b1} together with the carbon to which they are attached form cyclopropylene; and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is 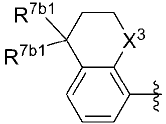 where X³ is CH₂; one R^{7b1} is hydrogen and the other is alkyl (in some embodiments, methyl or ethyl), both R^{7b1} are hydrogen, both R^{7b1} are C₁-C₃alkyl (in some embodiments, methyl), or the two R^{7b1} together with the carbon to which they are attached form cyclopropylene; and all other groups are as defined in Formula (I) in the Summary or as defined in some or any embodiments described herein. In some or any embodiments of Formula (I), (P-I), (P-Ia), (P-Ib-1), and (P-Ib-2), R^{7a} is 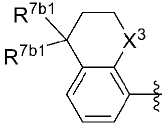 where X³ is O; one R^{7b1} is hydrogen and the other is alkyl (in some embodiments, methyl or ethyl), both R^{7b1} are hydrogen, both R^{7b1} are C₁-C₃alkyl (in some embodiments, methyl), both R^{7b1} are halo (e.g., fluoro), or the two R^{7b1} together with the carbon to which they are attached form cyclopropylene; and all other groups are as defined in Formula (I) in the Summary or as defined in some or any embodiments described herein.

In some or any embodiments of Formula (I), (P-I), (P-Ia), (P-Ib-1), and (P-Ib-2), R^{7a} is 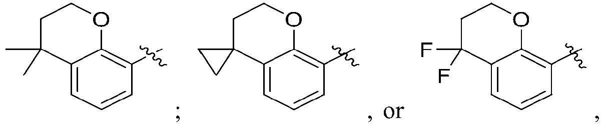 and all other groups are as defined in the Summary or as defined in some or any embodiments described herein.

In some or any embodiments of Formula (I), (P-I), (P-Ia), (P-Ib-1), and (P-Ib-2), R² is -NR⁷C(O)R^{7a}; R^{7a} is 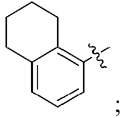 R¹ is -NR⁴C(O)R^{4a}; R^{4a} is heteroaryl optionally substituted with 1, 2, 3, or 4 R^{4b} groups; and all other groups are as defined in the Summary or as defined in some or any embodiments described herein. In some or any embodiments of Formula (I), (P-I), (P-Ia), (P-Ib-1), and (P-Ib-2), R² is -NHC(O)R^{7a}; R^{7a} is 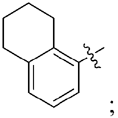 R¹ is -NHC(O)R^{4a}; R^{4a} is heteroaryl optionally substituted with 1, 2, 3, or 4 R^{4b} groups; and all other groups are as defined in the Summary or as defined in some or any embodiments described herein. In some or any embodiments of Formula (I), (P-I), (P-Ia), (P-Ib-1), and (P-Ib-2), R² is -NHC(O)R^{7a}; R^{7a} is 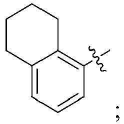 R¹ is -NHC(O)R^{4a}; R^{4a} is heteroaryl substituted with 1, 2, 3, or 4 R^{4b} groups; and all other groups are as defined in the Summary or as defined in some or any embodiments described herein. In some or any embodiments of Formula (I), (P-I), (P-Ia), (P-Ib-1), and (P-Ib-2), R² is -NHC(O)R^{7a}; R^{7a} is 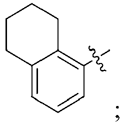 R¹ is -NHC(O)R^{4a}; R^{4a} is independently selected from 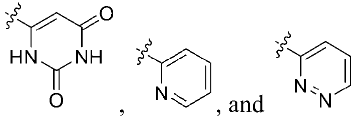 optionally substituted with 1, 2, 3, or 4 R^{4b} groups; and all other groups are as defined in the Summary or as defined in some or any embodiments described herein. In some or any embodiments of Formula (I), (P-I), (P-Ia), (P-Ib-1), and (P-Ib-2), R² is -NHC(O)R^{7a}; R^{7a} is 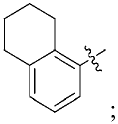 R¹ is -NHC(O)R^{4a}; R^{4a} is independently selected from 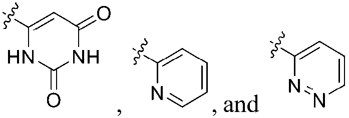 optionally substituted with 1, 2, 3, or 4 R^{4b} groups; R^{4b}, when present, is C₁₋₆alkyl; and all other groups are as defined in the Summary or as defined in some or any embodiments described herein. In some or any embodiments of Formula (I), (P-I), (P-Ia), (P-Ib-1), and (P-Ib-2), R² is
-NHC(O)R^{7a}; R^{7a} is 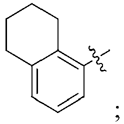 R¹ is -NHC(O)R^{4a}; R^{4a} is independently selected from 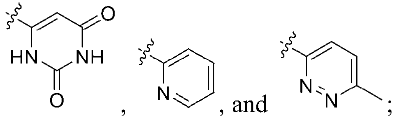 and all other groups are as defined in the Summary or as defined in some or any embodiments described herein.

In some or any embodiments of Formula (I), (P-I), (P-Ia), (P-Ib-1), and (P-Ib-2), the compound is selected from any of Formulas 1-46 and 49-52, or a pharmaceutically acceptable salt, hydrate, solvate, stereoisomer, tautomer, mixture, or combination thereof:

| | |
|---|---|
| **1** | 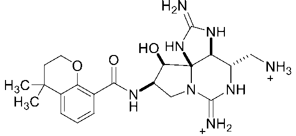 |
| **2** | 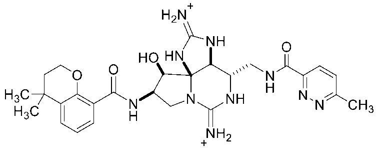 |
| **3** | 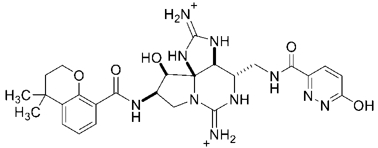 |
| **4** | 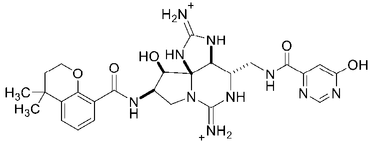 |
| **5** | 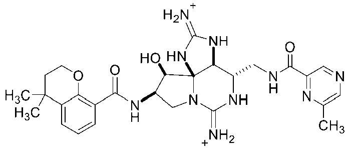 |
| **6** | 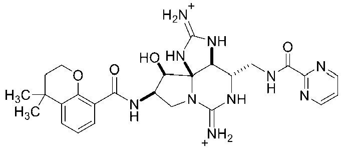 |
| **7** | 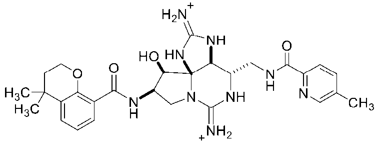 |
| **8** | 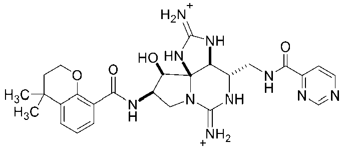 |
| **9** | 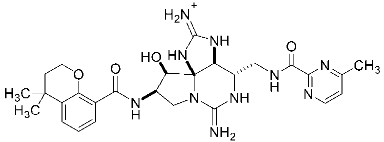 |
| **10** | 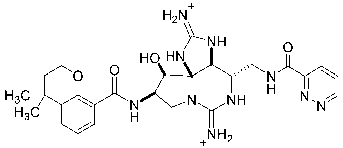 |
| **11** | 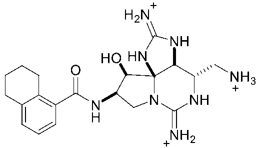 |
| **12** | 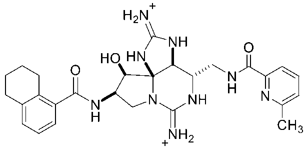 |
| **13** | 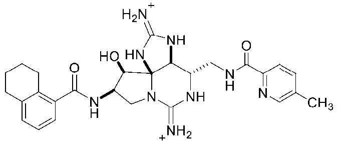 |
| **14** | 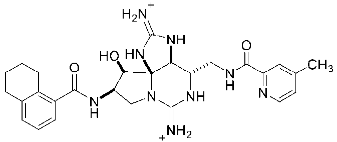 |
| **15** | 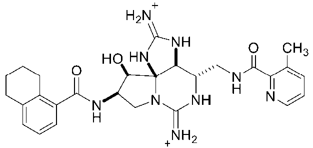 |
| **16** | 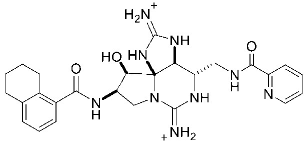 |
| **17** | 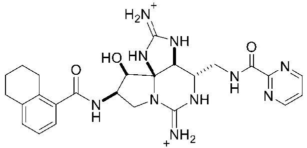 |
| **18** | 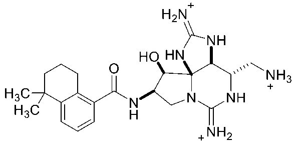 |
| **19** | 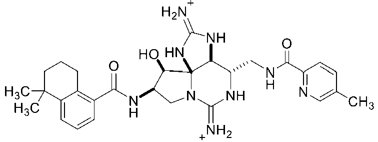 |
| **20** | 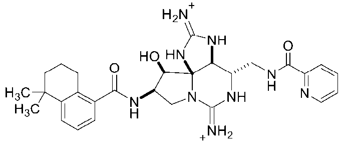 |
| **21** | 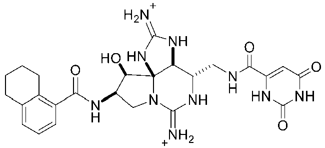 |
| **22** | 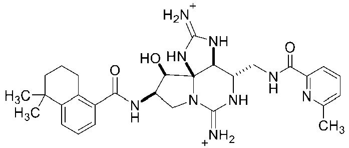 |
| **23** | 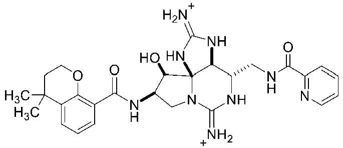 |
| **24** | 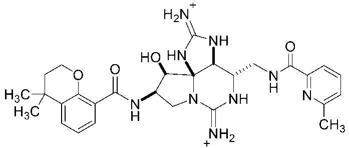 |
| **25** | 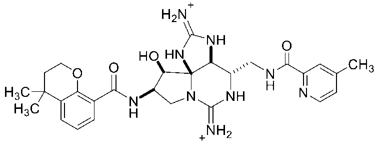 |
| **26** | 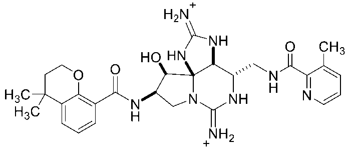 |
| **27** | 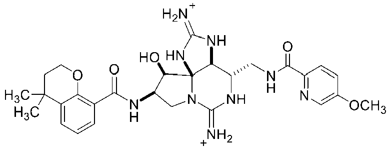 |
| **28** | 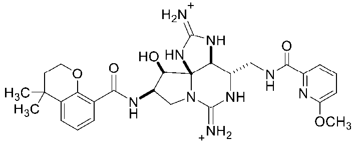 |
| **29** | 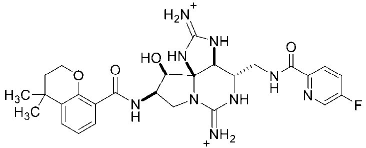 |
| **30** | 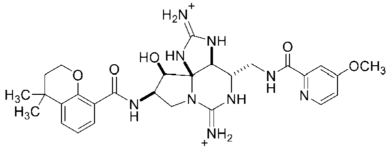 |
| **31** | 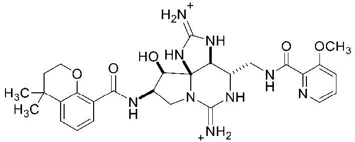 |
| **32** | 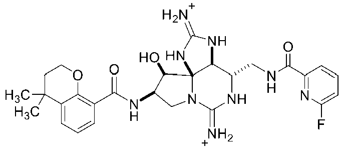 |
| **33** | 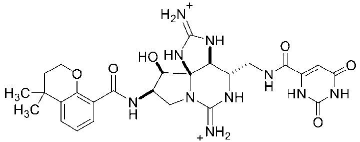 |
| **34** | 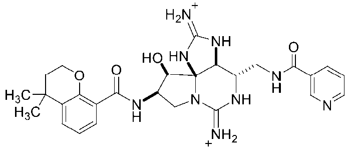 |
| **35** | 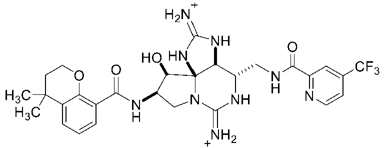 |
| **36** | 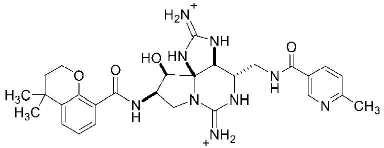 |
| **37** | 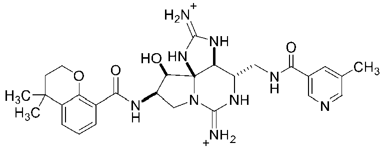 |
| **38** | 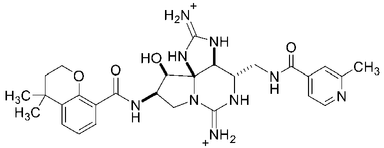 |
| **39** | 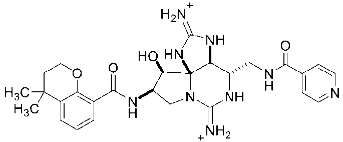 |
| **40** | 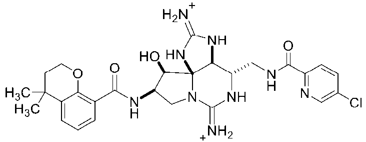 |
| **41** | 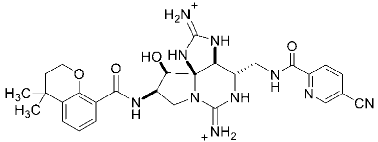 |
| **42** | 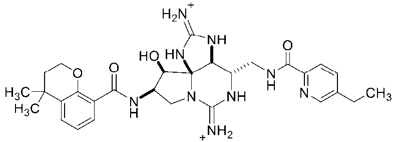 |
| **43** | 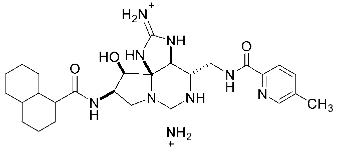 |
| **44** | 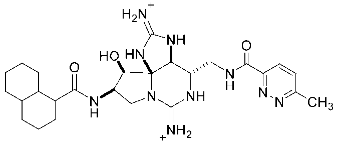 |
| **45** | 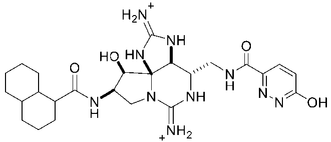 |
| **46** | 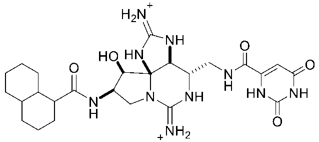 |
| **49** | 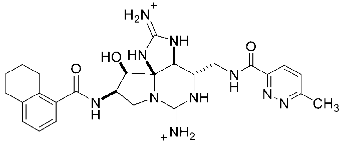 |
| **50** | 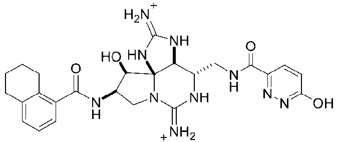 |
| **51** | 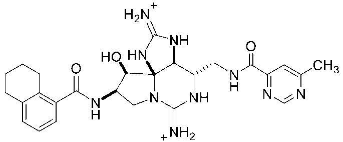 |
| **52** | 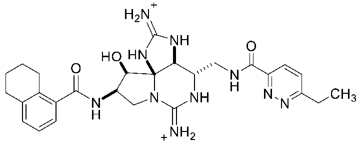 |

In some or any embodiments of Formula (I), (P-I), (P-Ia), (P-Ib-1), and (P-Ib-2), the compound is selected from any of Formulas 53-70, or a pharmaceutically acceptable salt, hydrate, solvate, stereoisomer, tautomer, mixture, or combination thereof:

| | |
|---|---|
| **53** | 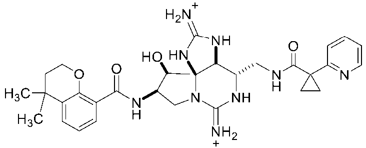 |
| **54** | 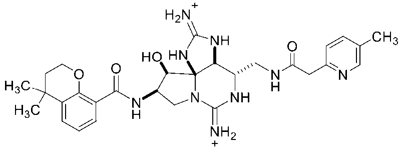 |
| **55** | 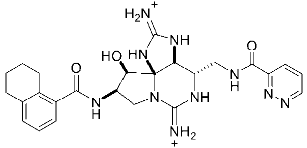 |
| **56** | 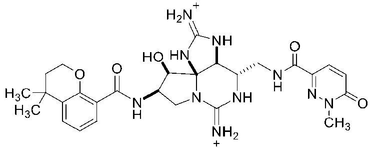 |
| **57** | 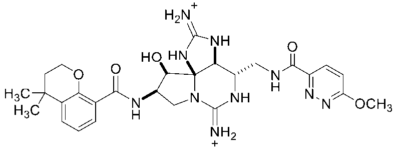 |
| **58** | 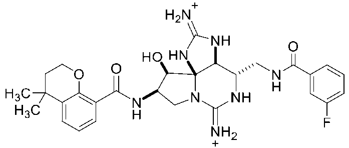 |
| **59** | 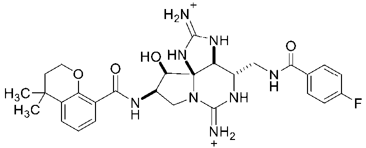 |
| **60** | 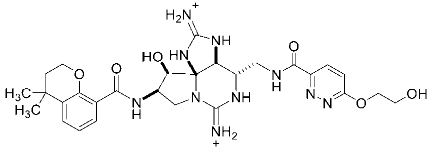 |
| **61** | 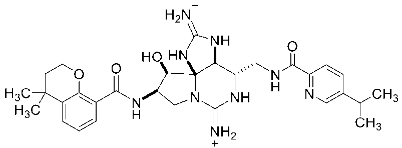 |
| **62** | 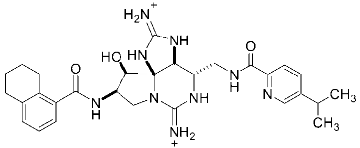 |
| **63** | 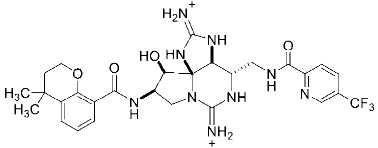 |
| **64** | 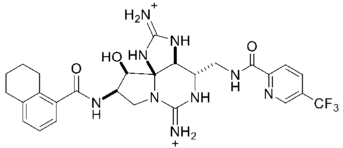 |
| **65** | 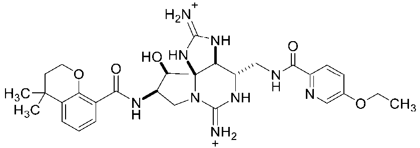 |
| **66** | 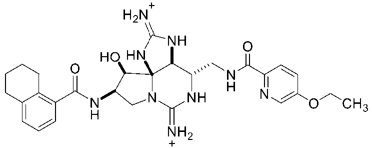 |
| **67** | 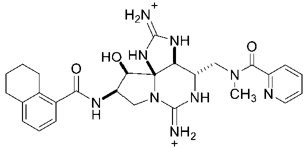 |
| **68** | 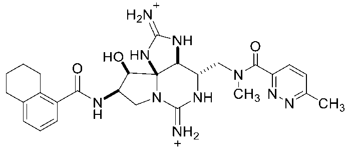 |
| **69** | 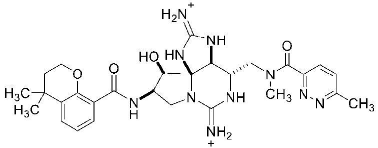 |
| **70** | 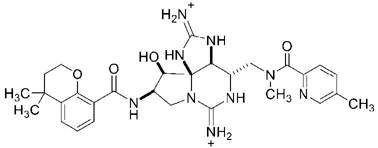 |

In some of any embodiments provided herein is a compound of Formula (X-4) 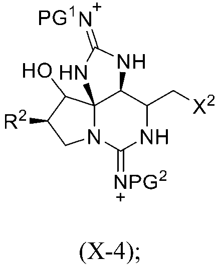 or a salt thereof,
wherein
PG¹ is a nitrogen-protecting group;
PG² is a nitrogen-protecting group;
X² is halo (preferably Br, I), or N₃;
R² is -NR⁷C(O)R^{7a};
R⁷ is hydrogen or C₁₋₆alkyl;
R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl.

In some or any embodiments, provided herein are:
(a) compounds as described herein, *e.g.,* of Formula (I), (P-1), (P-Ia), (P-Ib-1), (P-Ib-2), (P-Ic-1), (P-Ic-2), (P-Id-1), and (P-Id-2) and 1-46, 49-52, and 53-70 and pharmaceutically acceptable salts and compositions thereof;
(b) compounds as described herein, *e.g.,* of Formula (I), (P-1), (P-Ia), (P-Ib-1), (P-Ib-2), (P-Ic-1), (P-Ic-2), (P-Id-1), and (P-Id-2) and 1-46, 49-52, and 53-70 and pharmaceutically acceptable salts and compositions thereof for use in the treatment of pain and/or conditions modulated by voltage-gated sodium channels;
(c) processes for the preparation of compounds as described herein, e.g., of Formula (I), (P-1), (P-Ia), (P-Ib-1), (P-Ib-2), (P-Ic-1), (P-Ic-2), (P-1d-1), and (P-Id-2) and 1-46, 49-52, and 53-70 as described in more detail elsewhere herein;
(d) pharmaceutical formulations comprising a compound as described herein, *e.g.,* of Formula (I), (P-1), (P-Ia), (P-Ib-1), (P-Ib-2), (P-Ic-1), (P-Ic-2), (P-Id-1), and (P-Id-2) and 1-46, 49-52, and 53-70 or a pharmaceutically acceptable salt thereof together with a pharmaceutically acceptable carrier or diluent;
(e) a method for the treatment of a condition associated with voltage-gated sodium channel function in a subject that includes the administration of a therapeutically or prophylactically effective amount of a compound as described herein, *e.g.,* of Formula (I), (P-1), (P-Ia), (P-Ib-1), (P-Ib-2), (P-Ic-1), (P-Ic-2), (P-Id-1), and (P-Id-2) and 1-46, 49-52, and 53-70 or its pharmaceutically acceptable salt or composition;
(f) a method for the treatment of pain in a subject that includes the administration of a therapeutically or prophylactically effective amount of a compound as described herein, *e.g*., of Formula (I), (P-1), (P-Ia), (P-Ib-1), (P-Ib-2), (P-Ic-1), (P-Ic-2), (P-Id-1), and (P-Id-2) and 1-46, 49-52, and 53-70 or its pharmaceutically acceptable salt or composition;
(g) pharmaceutical formulations comprising a compound as described herein, *e.g.,* of Formula (I), (P-1), (P-Ia), (P-Ib-1), (P-Ib-2), (P-Ic-1), (P-Ic-2), (P-Id-1), and (P-Id-2) and 1-46, 49-52, and 53-70 or a pharmaceutically acceptable salt thereof together with one or more other effective agents for treating pain and/or conditions modulated by voltage-gated sodium channels, optionally in a pharmaceutically acceptable carrier or diluent;
(h) a method for the treatment of pain in a subject that includes the administration of a therapeutically or prophylactically effective amount of a compound as described herein, *e.g.,* of Formula (I), (P-1), (P-Ia), (P-Ib-1), (P-Ib-2), (P-Ic-1), (P-Ic-2), (P-Id-1), and (P-Id-2) and 1-46, 49-52, and 53-70 or its pharmaceutically acceptable salt or composition in combination and/or alternation with one or more agent for the treatment of pain and/or conditions modulated by voltage-gated sodium channels; and
(i) a method for the treatment of a condition associated with voltage-gated sodium channel function in a subject that includes the administration of a therapeutically or prophylactically effective amount of a compound as described herein, *e.g*., of Formula (I), (P-1), (P-Ia), (P-Ib-1), (P-Ib-2), (P-Ic-1), (P-Ic-2), (P-Id-1), and (P-Id-2)and 1-46, 49-52, and 53-70 or its pharmaceutically acceptable salt or composition in combination and/or alternation with one or more agent for the treatment of pain.
(j) use of any compound described herein, *e.g.,* of Formula (I), (P-1), (P-Ia), (P-Ib-1), (P-Ib-2), (P-Ic-1), (P-Ic-2), (P-Id-1), and (P-Id-2) and 1-46, 49-52, and 53-70 or a composition comprising any compound described herein, *e.g.*, of Formula (I), (P-1), (P-Ia), (P-Ib-1), (P-Ib-2), (P-Ic-1), (P-Ic-2), (P-Id-1), and (P-Id-2) and 1-46, 49-52, and 53-70 or a pharmaceutically acceptable salt or composition for the treatment of a condition associated with voltage-gated sodium channel function described herein (e.g., pain), optionally in combination and/or alternation with one or more agent for the treatment of pain.

### Optically Active Compounds

It is appreciated that compounds provided herein have several chiral centers and may exist in and be isolated in optically active and racemic forms. It is to be understood that any racemic, optically-active, diastereomeric, tautomeric, or stereoisomeric form, mixture, or combination thereof, of a compound provided herein, which possess the useful properties described herein is within the scope of the invention. It being well known in the art how to prepare optically active forms (in some or any embodiments, by resolution of the racemic form by recrystallization techniques, by synthesis from optically-active starting materials, by chiral synthesis, or by chromatographic separation using a chiral stationary phase).

In some or any embodiments, methods to obtain optically active materials are known in the art, and include at least the following.
i) physical separation of crystals - a technique whereby macroscopic crystals of the individual stereoisomers are manually separated. This technique can be used if crystals of the separate stereoisomers exist, *i.e.,* the material is a conglomerate, and the crystals are visually distinct;
ii) simultaneous crystallization - a technique whereby the individual stereoisomers are separately crystallized from a solution of the racemate, possible only if the latter is a conglomerate in the solid state;
iii) enzymatic resolutions - a technique whereby partial or complete separation of a racemate by virtue of differing rates of reaction for the stereoisomers with an enzyme;
iv) enzymatic asymmetric synthesis - a synthetic technique whereby at least one step of the synthesis uses an enzymatic reaction to obtain an stereoisomerically pure or enriched synthetic precursor of the desired stereoisomer;
v) chemical asymmetric synthesis - a synthetic technique whereby the desired stereoisomer is synthesized from an achiral precursor under conditions that produce asymmetry (*i.e.,* chirality) in the product, which may be achieved using chiral catalysts or chiral auxiliaries;
vi) diastereomer separations - a technique whereby a racemic compound is reacted with an enantiomerically pure reagent (the chiral auxiliary) that converts the individual enantiomers to diastereomers. The resulting diastereomers are then separated by chromatography or crystallization by virtue of their now more distinct structural differences and the chiral auxiliary later removed to obtain the desired enantiomer;
vii) first- and second-order asymmetric transformations - a technique whereby diastereomers from the racemate equilibrate to yield a preponderance in solution of the diastereomer from the desired enantiomer or where preferential crystallization of the diastereomer from the desired enantiomer perturbs the equilibrium such that eventually in principle all the material is converted to the crystalline diastereomer from the desired enantiomer. The desired enantiomer is then released from the diastereomer;
viii) kinetic resolutions - this technique refers to the achievement of partial or complete resolution of a racemate (or of a further resolution of a partially resolved compound) by virtue of unequal reaction rates of the stereoisomers with a chiral, non-racemic reagent or catalyst under kinetic conditions;
ix) stereospecific synthesis from non-racemic precursors - a synthetic technique whereby the desired stereoisomer is obtained from non-chiral starting materials and where the stereochemical integrity is not or is only minimally compromised over the course of the synthesis;
x) chiral liquid chromatography - a technique whereby the stereoisomers of a racemate are separated in a liquid mobile phase by virtue of their differing interactions with a stationary phase. The stationary phase can be made of chiral material or the mobile phase can contain an additional chiral material to provoke the differing interactions;
xi) chiral gas chromatography - a technique whereby the racemate is volatilized and stereoisomers are separated by virtue of their differing interactions in the gaseous mobile phase with a column containing a fixed non-racemic chiral adsorbent phase;
xii) extraction with chiral solvents - a technique whereby the stereoisomers are separated by virtue of preferential dissolution of one stereoisomer into a particular chiral solvent;
xiii) transport across chiral membranes - a technique whereby a racemate is placed in contact with a thin membrane barrier. The barrier typically separates two miscible fluids, one containing the racemate, and a driving force such as concentration or pressure differential causes preferential transport across the membrane barrier. Separation occurs as a result of the non-racemic chiral nature of the membrane which allows only one stereoisomer of the racemate to pass through.

In some or any embodiments, provided is a composition of a 11,13-modified, non-hydrated ketone saxitoxin that comprises a substantially pure designated stereoisomer of the 11,13-modified, non-hydrated ketone saxitoxin. In some or any embodiments, in the methods and compounds, the compounds are substantially free of other stereoisomer. In some or any embodiments, a composition includes a compound that is at least 85%, 90%, 95%, 98%, 99% or 100% by weight, of the 11,13-modified, non-hydrated ketone saxitoxin, the remainder comprising other chemical species or stereoisomers.

### Isotopically Enriched Compounds

Also provided herein are isotopically enriched compounds, including but not limited to isotopically enriched 11,13-modified, non-hydrated ketone saxitoxins.

Isotopic enrichment (in some or any embodiments, deuteration) of pharmaceuticals to improve pharmacokinetics ("PK"), pharmacodynamics ("PD"), and toxicity profiles, has been demonstrated previously with some classes of drugs. See, for example, Lijinsky et. al., Food Cosmet. Toxicol., 20: 393 (1982); Lijinsky et. al., J. Nat. Cancer Inst., 69: 1127 (1982); Mangold et. al., Mutation Res. 308: 33 (1994); Gordon et. al., Drug Metab. Dispos., 15: 589 (1987); Zello et. al., Metabolism, 43: 487 (1994); Gately et. al., J. Nucl. Med., 27: 388 (1986); Wade D, Chem. Biol. Interact. 117: 191 (1999).

Isotopic enrichment of a drug can be used, in some or any embodiments, to (1) reduce or eliminate unwanted metabolites, (2) increase the half-life of the parent drug, (3) decrease the number of doses needed to achieve a desired effect, (4) decrease the amount of a dose necessary to achieve a desired effect, (5) increase the formation of active metabolites, if any are formed, and/or (6) decrees the production of deleterious metabolites in specific tissues and/or create a more effective drug and/or a safer drug for combination therapy, whether the combination therapy is intentional or not.

Replacement of an atom for one of its isotopes often will result in a change in the reaction rate of a chemical reaction. This phenomenon is known as the Kinetic Isotope Effect ("KIE"). For example, if a C-H bond is broken during a rate-determining step in a chemical reaction (i.e. the step with the highest transition state energy), substitution of a deuterium for that hydrogen will cause a decrease in the reaction rate and the process will slow down. This phenomenon is known as the Deuterium Kinetic Isotope Effect ("DKIE"). See, *e.g.,* Foster et al., Adv. Drug Res., vol. 14, pp. 1-36 (1985); Kushner et al., Can. J. Physiol. Pharmacol., vol. 77, pp. 79-88 (1999).

The magnitude of the DKIE can be expressed as the ratio between the rates of a given reaction in which a C-H bond is broken, and the same reaction where deuterium is substituted for hydrogen. The DKIE can range from about 1 (no isotope effect) to very large numbers, such as 50 or more, meaning that the reaction can be fifty, or more, times slower when deuterium is substituted for hydrogen. High DKIE values may be due in part to a phenomenon known as tunneling, which is a consequence of the uncertainty principle. Tunneling is ascribed to the small mass of a hydrogen atom, and occurs because transition states involving a proton can sometimes form in the absence of the required activation energy. Because deuterium has more mass than hydrogen, it statistically has a much lower probability of undergoing this phenomenon.

Tritium ("T") is a radioactive isotope of hydrogen, used in research, fusion reactors, neutron generators and radiopharmaceuticals. Tritium is a hydrogen atom that has 2 neutrons in the nucleus and has an atomic weight close to 3. It occurs naturally in the environment in very low concentrations, most commonly found as T₂O. Tritium decays slowly (half-life = 12.3 years) and emits a low energy beta particle that cannot penetrate the outer layer of human skin. Internal exposure is the main hazard associated with this isotope, yet it must be ingested in large amounts to pose a significant health risk. As compared with deuterium, a lesser amount of tritium must be consumed before it reaches a hazardous level. Substitution of tritium ("T") for hydrogen results in yet a stronger bond than deuterium and gives numerically larger isotope effects. Similarly, substitution of isotopes for other elements, including, but not limited to, ¹³C or ¹⁴C for carbon, ³³S, ³⁴S, or ³⁶S for sulfur, ¹⁵N for nitrogen, and ¹⁷O or ¹⁸O for oxygen, may lead to a similar kinetic isotope effect.

For example, the DKIE was used to decrease the hepatotoxicity of halothane by presumably limiting the production of reactive species such as trifluoroacetyl chloride. However, this method may not be applicable to all drug classes. For example, deuterium incorporation can lead to metabolic switching. The concept of metabolic switching asserts that xenogens, when sequestered by Phase I enzymes, may bind transiently and re-bind in a variety of conformations prior to the chemical reaction (e.g., oxidation). This hypothesis is supported by the relatively vast size of binding pockets in many Phase I enzymes and the promiscuous nature of many metabolic reactions. Metabolic switching can potentially lead to different proportions of known metabolites as well as altogether new metabolites. This new metabolic profile may impart more or less toxicity.

In some embodiments, the compounds described herein may be used as radiopharmaceuticals such as, for example, imaging agents. In one instance, radiopharmaceuticals are positron emission tomography (PET) imaging agents. In such embodiments, substitution of radionuclides (e.g., positron emitting isotopes) for atoms in the compounds allows for the syntheses of radiopharmaceuticals that can function as imaging agents. In some embodiments, radionuclides which can be substituted in the compounds described herein include, and are not limited to, ¹⁸F, ¹¹C, ¹³N, ¹⁵O, ⁷⁶Br, and ¹²⁴I. In some embodiments, the compound is isotopically enriched at one or more atoms, one atom, two atoms, or three atoms. In some embodiments, the compound is administered as an isotopic composition.

The animal body expresses a variety of enzymes for the purpose of eliminating foreign substances, such as therapeutic agents, from its circulation system. In some or any embodiments, such enzymes include the cytochrome P450 enzymes ("CYPs"), esterases, proteases, reductases, dehydrogenases, and monoamine oxidases, to react with and convert these foreign substances to more polar intermediates or metabolites for renal excretion. Some of the most common metabolic reactions of pharmaceutical compounds involve the oxidation of a carbon-hydrogen (C-H) bond to either a carbon-oxygen (C-O) or carbon-carbon (C-C) pi-bond. The resultant metabolites may be stable or unstable under physiological conditions, and can have substantially different pharmacokinetic, pharmacodynamic, and acute and long-term toxicity profiles relative to the parent compounds. For many drugs, such oxidations are rapid. These drugs therefore often require the administration of multiple or high daily doses.

Therefore, isotopic enrichment at certain positions of a compound provided herein will produce a detectable KIE that will affect the pharmacokinetic, pharmacologic, and/or toxicological profiles of a compound provided herein in comparison with a similar compound having a natural isotopic composition.

### Preparation of Compounds

The compounds provided herein can be prepared, isolated or obtained by any method apparent to those of skill in the art. Compounds provided herein can be prepared according to the Exemplary Preparation Schemes provided below. Reaction conditions, steps and reactants not provided in the Exemplary Preparation Schemes would be apparent to, and known by, those skilled in the art.

Additional steps and reagents not provided in the Exemplary Preparation Scheme would be known to those of skill in the art. For example, the compound of formula A (depicted below) where PG¹ is a nitrogen-protecting group, *e.g*. Tces, and PG² is a nitrogen-protecting group, *e.g.* Troc, can be prepared using procedures known to one of ordinary skill in the art (*e.g.* see US2010/0284913. It would be appreciated by one of ordinary skill in the art that an intermediate of formula A-1: 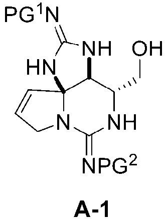 could be prepared using the procedures known by one of ordinary skill in the art or as disclosed in US2010/0284913 (which is herein incorporated by reference in its entirety, particularly the synthetic methods disclosed therein) but replacing L-serine with D-serine. Exemplary methods of preparation are described in detail in the Examples herein. Compound A-1 was converted to compounds described herein as shown in the general schemes A and C and the Examples section.

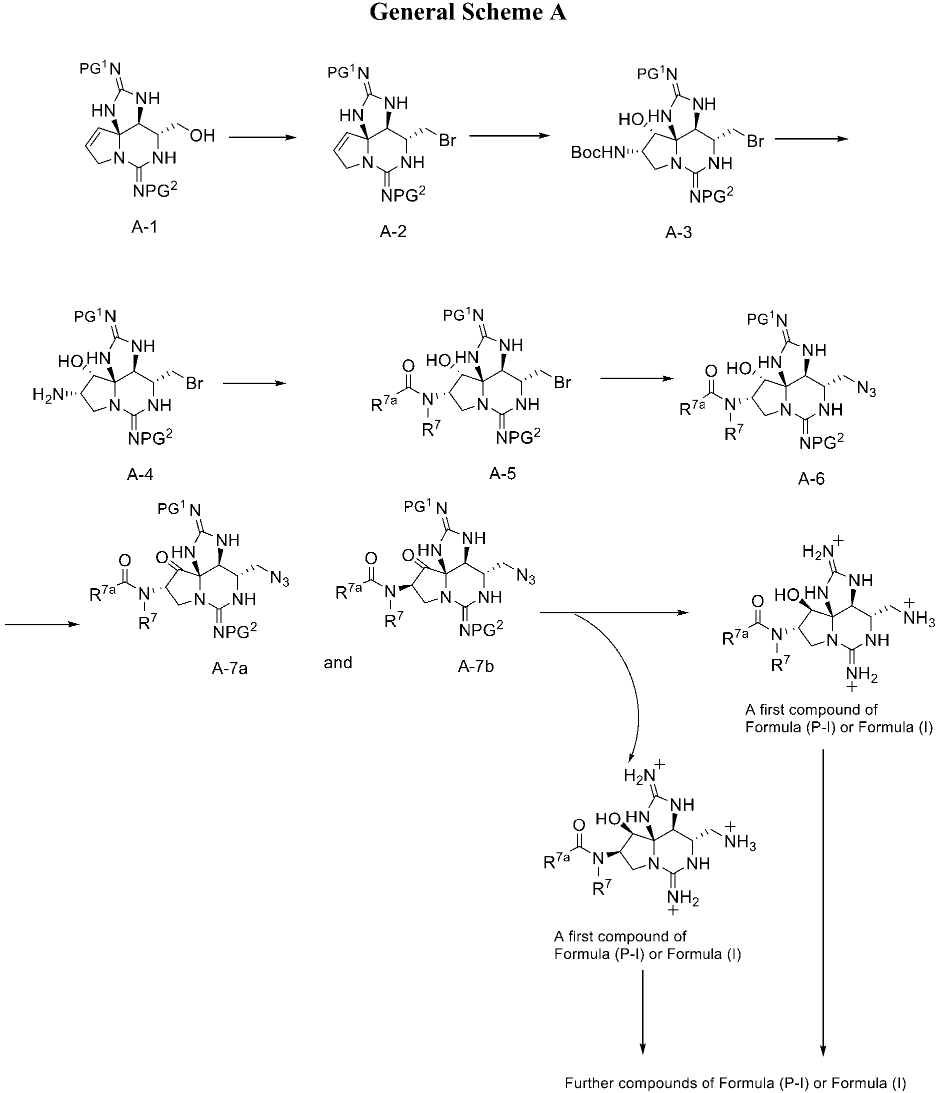

A compound of Formula (P-I) or Formula (I) may be prepared for example by starting with intermediate **A-1.** The hydroxy group in intermediate **A-1** is converted to a bromide using any suitable bromination method known to one of skill in the art (e.g., triphenyl phosphine and carbon tetrabromide are used in the bromination step) to obtain intermediate **A-2** which is optionally purified using column chromatography. An amino hydroxylation of the double bond in intermediate **A-1** is carried out for example, by treating intermediate **A-2** where PG¹ is a nitrogen-protecting group, *e.g.* Tces, and PG² is a nitrogen-protecting group, *e.g.* Troc, with a nitrogen source, *e.g.,* a compound of formula NH(R)PG³ where PG³ is as defined in any of the embodiments herein (*e.g.* PG³ is Boc), and R is, *e.g.,* a chlorobenzoyloxy group, in the presence of OsO₄ in a solvent such as CH₃CN, where the reaction is optionally quenched, for example with NaHCO₃ and where the product is optionally extracted and/or purified by chromatography. The PG³ protecting group is then removed using conditions known to one of ordinary skill in the art, *e.g.* by treating with an acid such as TFA when PG³ is Boc to yield the compound of formula **A-4** or a salt thereof. The product is used in the next step without further purification or is optionally extracted and/or purified by chromatography. The compound of formula **A-4** is then treated with a base such as triethylamine, or diisopropylethylamine and the like, in a solvent such as CH₂Cl₂ with a compound of formula of formula R^{7a}-C(O)OH in the presence of a coupling agent such as HBTU and in the presence of a base such as diisopropylethylamine (DIEA) or treated with a compound of formula R^{7a}-C(O)X (where X is a leaving group such as halo) in the presence of a base such as diisopropylethylamine (DIEA) where the product **A-5** is optionally extracted and/or purified by chromatography. A compound of formula **A-6** is prepared by treating a compound of formula **A-5** with sodium azide in a solvent **(e.g.,** dichloromethane) and optionally purifying the azide product **A-6** by chromatography. The ketone compounds of formula **A-7a** and **A-7b** can be prepared by treating a compound of formula **A-6** with an oxidant, e.g., Dess-Martin periodinane, AZADO and the like with a stoichiometric oxidant such as iodobenzene diacetate, in a solvent such as CH₂Cl₂ and where the product is optionally purified before using in the next step. After the oxidation of **A-6** to **A-7a** and **A-7b,** the C11 position can epimerize (the extent depends on reaction time, workup, etc.). The azides in the compounds of formula **A-7a** and **A-7b** are reduced to amines in the presence of an acid (e.g., trifluoroacetic acid), a palladium catalyst (e.g., PdCl₂), and in the presence of hydrogen to provide a first compound of Formula (P-I) or Formula (I). Once the ketone reduction is performed from **A-7**a and **A-7b** to P-1, the C11 stereochemistry becomes set. Both the C11R and C11S can be obtained and then separated. The protecting groups PG¹ and PG² are also removed in this step. The product (e.g., Compound (1) of Formula (I)) is optionally isolated as a salt that is purified by reverse phase HPLC. Further compounds of Formulas P-I and I can be prepared using methods known to one of ordinary skill in the art, by reacting the first compound of Formulas P-I and I in the above scheme with, for example, one of the following: LG-S(O)₂R^{5a}, LG-C(O)NR^{6a}R^{6b}, and LG-C(O)OR^{8a}.

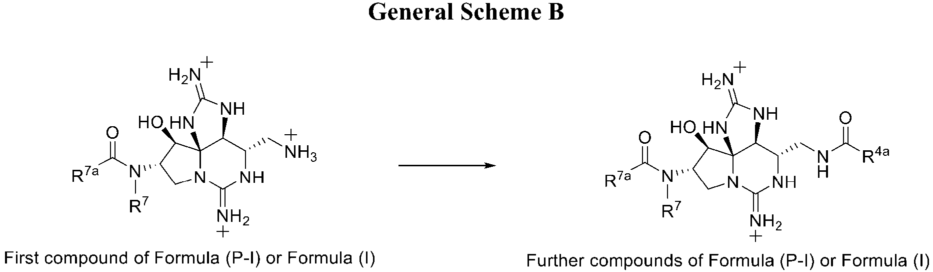

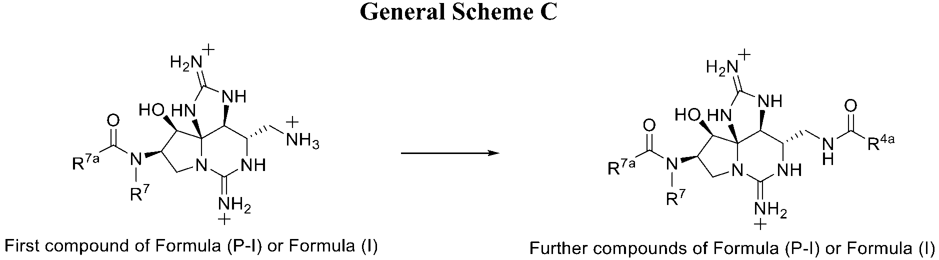

The first compound of Formula (P-1) or Formula (I) in Schemes B and C is further elaborated into other compounds of Formula (P-1) or Formula (I), respectively, by coupling the first compound of Formula (P-I) or (I) with e.g., a suitable activated acid (e.g., 2,5-dioxopyrrolidin-1-yl-activated acid, e.g., 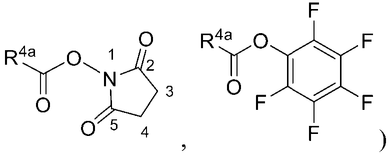 in the presence of a base (e.g., sodium bicarbonate, cesium carbonate). Alternatively, the first compound of Formula (P-I) or (I) is coupled with an acid R¹-COOH in the presence of a coupling agent (e.g., HBTU, CDI) and a base (e.g., diisopropylethylamine), or the first compound of Formula (P-I) or (I)s coupled with an acyl halide (e.g., R¹-C(=O)X, where X is Br, Cl or I) in the presence of a base to obtain other compounds of Formula (P-I) or Formula (I).

In another embodiment, provided herein is a method of preparing a compound of Formula (P-I) comprising
a) in a single step, deprotecting and reducing a compound of Formula (X-1) 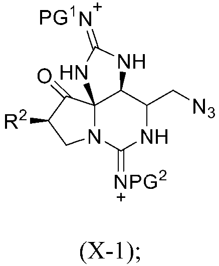 to obtain a compound of Formula (X-2): 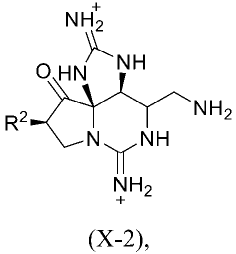 or a salt thereof;
b) reducing the ketone of Formula (X-2) to obtain a compound of Formula (X-3): 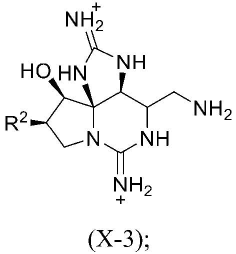 or a salt thereof;
c) coupling the compound of Formula (X-3) with a compound of Formula X⁴-LG to obtain a compound of Formula P-I; wherein
   PG¹ is a nitrogen-protecting group;
   PG² is a nitrogen-protecting group;
   X⁴ is -C(O)R^{4a};
   LG is a leaving group selected from the group consisting of halo, 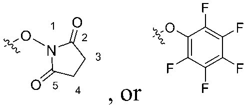 , where 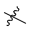 represents the point of attachment of LG to X⁴;
   R² is -NR⁷C(O)R^{7a};
   R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl; cycloalkylalkyl; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
   each R^{4b}, when present, is independently C₁₋₆alkyl, hydroxy, halo, halo-C₁₋₆alkyl, C₁₋₆alkoxy, or cyano;
   R⁷ is hydrogen or C₁₋₆alkyl;
   R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
   each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C_{1**-**6}alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; to yield a compound of Formula P-I; and
d) optionally isolating the compound of Formula P-I.

In a further aspect, provided herein is a method of preparing a compound of Formula (I) comprising
a) reducing the ketone in a compound of Formula (X-1) 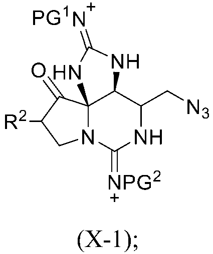 to obtain a compound of Formula (X-2B): 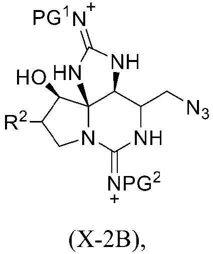 or a salt thereof;
b) deprotecting and reducing the compound of Formula (X-2B) to obtain a compound of Formula (X-3) 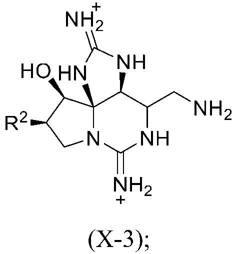 or a salt thereof;
c) coupling the compound of Formula (X-3) with a compound of Formula X⁴-LG to obtain a compound of Formula I; wherein
   PG¹ is a nitrogen-protecting group;
   PG² is a nitrogen-protecting group;
   X⁴ is -C(O)R^{4a}, -S(O)₂R^{5a}, -C(O)NR^{6a}R^{6b}, or -C(O)OR^{8a};
   LG is a leaving group selected from the group consisting of halo, 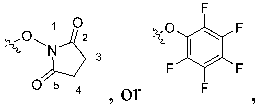 where 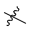 represents the point of attachment of LG to X⁴;
   R² is -NR⁷C(O)R^{7a}, -NR⁷S(O)₂R^{7a}, or -NR⁷C(O)OR^{7a};
   R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
   each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   one pair of two R^{4b} groups are present on one carbon, the second pair of two R^{4b} are present on another carbon, and each pair of R^{4b} are taken together to form an oxo group;
   R^{5a} is heterocycloalkyl, amino, C₁₋₆alkylamino, or di- C₁₋₆alkylamino;
   R^{6a} is hydrogen or C₁₋₆alkyl;
   R^{6b} is hydrogen, C₁₋₆alkyl, C₁₋₆alkoxy, or cycloalkyl;
   R^{8a} is hydrogen, C₁₋₆alkyl, cycloalkyl, or phenyl;
   R⁷ is hydrogen or C₁₋₆alkyl;
   R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
   each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; to yield a compound of Formula I; and
d) optionally isolating the compound of Formula I.

In some and any embodiments of the methods of preparation described herein, provided herein is a method of preparing a compound of Formula (I) comprising
a) converting a compound of Formula (X-1A) to a compound of Formula (X-2A), 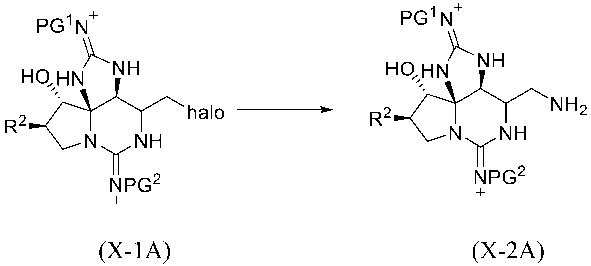
b) oxidizing the hyroxy group of Formula (X-2A) to obtain a ketone of Formula (X-3A): 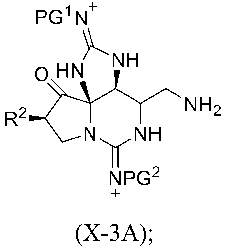 or a salt thereof;
c) deprotecting the compound of Formula (X-3A) to obtain a compound of Formula (X-2): 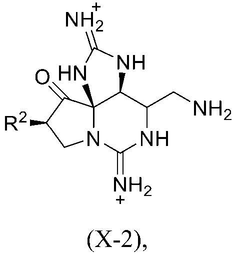 or a salt thereof;
d) reducing the ketone of Formula (X-2) to obtain a compound of Formula (X-3): 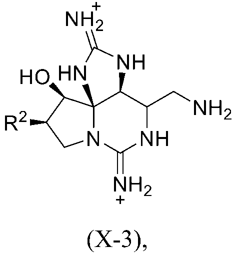
e) coupling the compound of Formula (X-3) with a compound of Formula X⁴-LG to obtain a compound of Formula I; wherein
   halo is Br or I;
   PG¹ is a nitrogen-protecting group;
   PG² is a nitrogen-protecting group;
   X⁴ is -C(=O)R^{4a}, -S(O)₂R^{5a}, -C(O)NR^{6a}R^{6b}, or -C(O)OR^{8a};
   LG is a leaving group selected from the group consisting of halo, 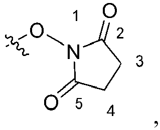 or 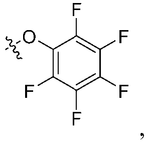 where 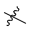 represents the point of attachment of LG to X⁴;
   R² is -NR⁷C(O)R^{7a}, -NR⁷S(O)₂R^{7a}, or -NR⁷C(O)OR^{7a};
   R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
   each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   one pair of two R^{4b} groups are present on one carbon, the second pair of two R^{4b} are present on another carbon, and each pair of R^{4b} are taken together to form an oxo group;
   R^{5a} is heterocycloalkyl, amino, C₁₋₆alkylamino, or di- C₁₋₆alkylamino;
   R^{6a} is hydrogen, or C₁₋₆alkyl;
   R^{6b} is hydrogen, C₁₋₆alkyl, C₁₋₆alkoxy or cycloalkyl;
   R^{8a} is hydrogen, C₁₋₆alkyl, cycloalkyl or phenyl;
   R⁷ is hydrogen or C₁₋₆alkyl;
   R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
   each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; to yield a compound of Formula I; and
f) optionally isolating the compound of Formula I.

In some alternate embodiments of the methods of preparation described herein, provided herein is a method of preparing a compound of Formula (I) comprising
a) converting a compound of Formula (X-1A) to a compound of Formula (X-2A), 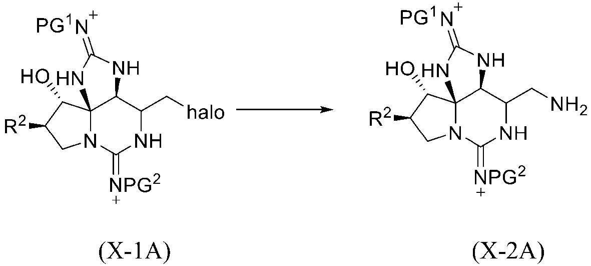
b) oxidizing the hyroxy group of Formula (X-2A) to obtain a ketone of Formula (X-3A): 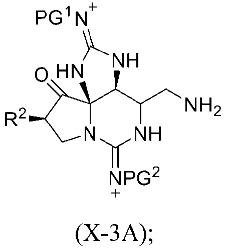 or a salt thereof;
c) reducing the ketone of Formula (X-3A) to obtain a compound of Formula (X-3B): 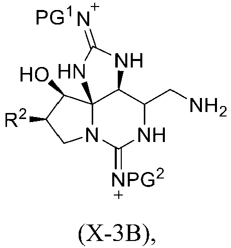 or a salt thereof;
d) deprotecting the compound of Formula (X-3B) to obtain a compound of Formula (X-3): 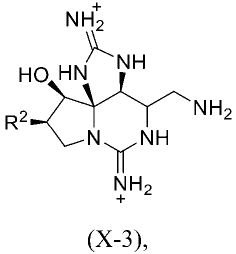 or a salt thereof; and
e) coupling the compound of Formula (X-3) with a compound of Formula X⁴-LG to obtain a compound of Formula I; wherein
   halo is Br or I;
   PG¹ is a nitrogen-protecting group;
   PG² is a nitrogen-protecting group;
   X⁴ is -C(=O)R^{4a}, -S(O)₂R^{5a}, -C(O)NR^{6a}R^{6b}, or -C(O)OR^{8a};
   LG is a leaving group selected from the group consisting of halo, 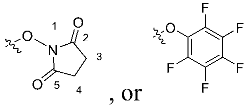 , where 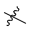 represents the point of attachment of LG to X⁴;
   R² is -NR⁷C(O)R^{7a}, -NR⁷S(O)₂R^{7a}, or -NR⁷C(O)OR^{7a};
   R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
   each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   one pair of two R^{4b} groups are present on one carbon, the second pair of two R^{4b} are present on another carbon, and each pair of R^{4b} are taken together to form an oxo group;
   R^{5a} is heterocycloalkyl, amino, C₁₋₆alkylamino, or di- C₁₋₆alkylamino;
   R^{6a} is hydrogen, or C₁₋₆alkyl;
   R^{6b} is hydrogen, C₁₋₆alkyl, C₁₋₆alkoxy or cycloalkyl;
   R^{8a} is hydrogen, C₁₋₆alkyl, cycloalkyl or phenyl;
   R⁷ is hydrogen or C₁₋₆alkyl;
   R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
   each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; to yield a compound of Formula I; and
f) optionally isolating the compound of Formula I.

In some and any embodiments of the methods of preparation described herein, provided herein is a method of preparing a compound of Formula (P-I) comprising
a) converting a compound of Formula (X-1A) to a compound of Formula (X-2A), 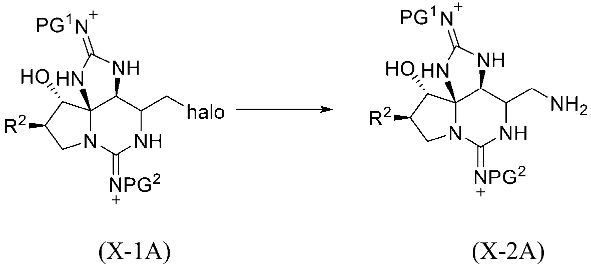
b) oxidizing the hyroxy group of Formula (X-2A) to obtain a ketone of Formula (X-3A): 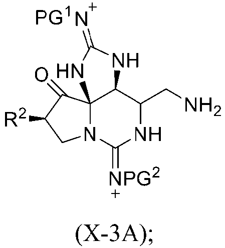 or a salt thereof;
c) deprotecting the compound of Formula (X-3A) to obtain a compound of Formula (X-2): 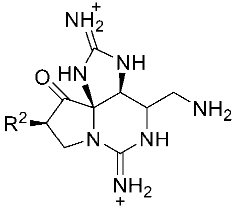 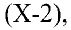 or a salt thereof;
d) reducing the ketone of Formula (X-2) to obtain a compound of Formula (X-3): 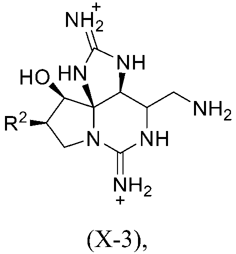
e) coupling the compound of Formula (X-3) with a compound of Formula X⁴-LG to obtain a compound of Formula I; wherein
   halo is Br or I;
   PG¹ is a nitrogen-protecting group;
   PG² is a nitrogen-protecting group;
   X⁴ is -C(=O)R^{4a}, -S(O)₂R^{5a}, -C(O)NR^{6a}R^{6b}, or -C(O)OR^{8a};
   LG is a leaving group selected from the group consisting of halo, 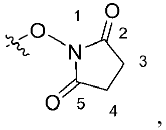 or 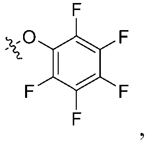 where 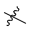 represents the point of attachment of LG to X⁴;
   R² is -NR⁷C(O)R^{7a}, -NR⁷S(O)₂R^{7a}, or -NR⁷C(O)OR^{7a};
   R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl; cycloalkylalkyl; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
   each R^{4b}, when present, is independently C₁₋₆alkyl, hydroxy, halo, halo-C₁₋₆alkyl, C₁₋₆alkoxy, cyano, or phenyl;
   R^{5a} is heterocycloalkyl, amino, C₁₋₆alkylamino, or di- C₁₋₆alkylamino;
   R^{6a} is hydrogen, or C₁₋₆alkyl;
   R^{6b} is hydrogen, C₁₋₆alkyl, C₁₋₆alkoxy or cycloalkyl;
   R^{8a} is hydrogen, C₁₋₆alkyl, cycloalkyl or phenyl;
   R⁷ is hydrogen or C₁₋₆alkyl;
   R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
   each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; to yield a compound of Formula P-I; and
f) optionally isolating the compound of Formula P-I.

In some alternate embodiments of the methods of preparation described herein, provided herein is a method of preparing a compound of Formula (P-I) comprising
a) converting a compound of Formula (X-1A) to a compound of Formula (X-2A), 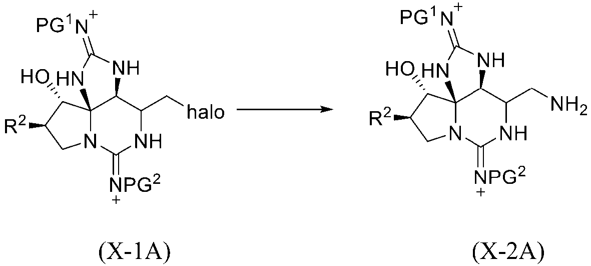
b) oxidizing the hyroxy group of Formula (X-2A) to obtain a ketone of Formula (X-3A): 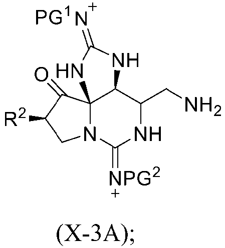 or a salt thereof;
c) reducing the ketone of Formula (X-3A) to obtain a compound of Formula (X-3B): 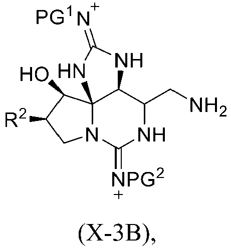 or a salt thereof;
d) deprotecting the compound of Formula (X-3B) to obtain a compound of Formula (X-3): 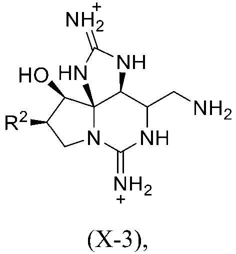 or a salt thereof; and
e) coupling the compound of Formula (X-3) with a compound of Formula X⁴-LG to obtain a compound of Formula I; wherein
   halo is Br or I;
   PG¹ is a nitrogen-protecting group;
   PG² is a nitrogen-protecting group;
   X⁴ is -C(=O)R^{4a}, -S(O)₂R^{5a}, -C(O)NR^{6a}R^{6b}, or -C(O)OR^{8a};
   LG is a leaving group selected from the group consisting of halo, 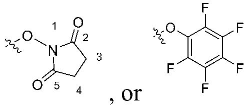 , where 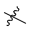 represents the point of attachment of LG to X⁴;
   R² is -NR⁷C(O)R^{7a}, -NR⁷S(O)₂R^{7a}, or -NR⁷C(O)OR^{7a};
   R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl; cycloalkylalkyl; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
   each R^{4b}, when present, is independently C₁₋₆alkyl, hydroxy, halo, halo-C₁₋₆alkyl, C₁₋₆alkoxy, cyano, or phenyl;
   R^{5a} is heterocycloalkyl, amino, C₁₋₆alkylamino, or di- C₁₋₆alkylamino;
   R^{6a} is hydrogen, or C₁₋₆alkyl;
   R^{6b} is hydrogen, C₁₋₆alkyl, C₁₋₆alkoxy or cycloalkyl;
   R^{8a} is hydrogen, C₁₋₆alkyl, cycloalkyl or phenyl;
   R⁷ is hydrogen or C₁₋₆alkyl;
   R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
   each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; to yield a compound of Formula P-I; and
f) optionally isolating the compound of Formula P-I.

One of skill will understand that the order of steps for any process described herein may be changed. For instance, ketone reduction may be carried out prior to deprotection of protecting groups PG¹ and PG². Coupling with a compound of Formula X⁴-LG may be carried out prior to ketone reduction and/or deprotection of protecting groups PG¹ and PG². To illustrate one such instance in which the order of steps may be changed, by way of example only, coupling of the amino group of Formula (X-3A) with a compound of Formula X⁴-LG may be carried out prior to reduction of the ketone of Formula (X-3A). Other variations will be apparent to one of skill in the art and all such variations are contemplated within the scope of embodiments presented herein.

In some and any embodiments of the methods of preparation described herein, a compound of formula (X-1A) is converted to a compound of formula (X-2A) in the presence of ammonia.

In some and any embodiments of the methods of preparation described herein, the compounds of Formula (P-I) or Formula (I) are prepared as described in General Scheme A, General Scheme B, and/or the Examples section. In some or any embodiments, the compound of formula (X-2) is coupled to a compound of formula X⁴-LG using any methods of coupling known in the art and/or methods described herein.

### Pharmaceutical Compositions and Methods of Administration

The compounds provided herein can be formulated into pharmaceutical compositions using methods available in the art and those disclosed herein. Any of the compounds disclosed herein can be provided in the appropriate pharmaceutical composition and be administered by a suitable route of administration. Provided herein are pharmaceutical compositions comprising a compound of Formula (I), (P-1), (P-Ia), (P-Ib), (P-Ic), or (P-Id), as described herein in some and any embodiments, and a pharmaceutically acceptable carrier. In some embodiments, the composition is an oral or injectable composition. In some of such embodiments, the injectable composition is a subcutaneously injectable composition.

The methods provided herein encompass administering pharmaceutical compositions containing at least one compound as described herein, including a compound of Formula (I), (P-1), (P-Ia), (P-Ib), (P-Ic), or (P-Id) and 1-46, 49-52, and 53-70 if appropriate in a salt form, either used alone or in the form of a combination with one or more compatible and pharmaceutically acceptable carriers, such as diluents or adjuvants, or with another agent for the treatment of pain and/or conditions modulated by voltage-gated sodium channels.

In some or any embodiments, the second agent can be formulated or packaged with the compound provided herein. Of course, the second agent will only be formulated with the compound provided herein when, according to the judgment of those of skill in the art, such co-formulation should not interfere with the activity of either agent or the method of administration. In some or any embodiments, the compound provided herein and the second agent are formulated separately. They can be packaged together, or packaged separately, for the convenience of the practitioner of skill in the art.

In clinical practice the active agents provided herein may be administered by any conventional route, in particular orally, parenterally, rectally or by inhalation (e.g. in the form of aerosols). In some or any embodiments, the compound provided herein is administered orally.

Use may be made, as solid compositions for oral administration, of tablets, pills, hard gelatin capsules, powders or granules. In these compositions, the active product is mixed with one or more inert diluents or adjuvants, such as sucrose, lactose or starch.

These compositions can comprise substances other than diluents, for example a lubricant, such as magnesium stearate, or a coating intended for controlled release.

Use may be made, as liquid compositions for oral administration, of solutions which are pharmaceutically acceptable, suspensions, emulsions, syrups and elixirs containing inert diluents, such as water or liquid paraffin. These compositions can also comprise substances other than diluents, in some or any embodiments, wetting, sweetening or flavoring products.

The compositions for parenteral administration can be emulsions or sterile solutions. Use may be made, as solvent or vehicle, of propylene glycol, a polyethylene glycol, vegetable oils, in particular olive oil, or injectable organic esters, in some or any embodiments, ethyl oleate. These compositions can also contain adjuvants, in particular wetting, isotonizing, emulsifying, dispersing and stabilizing agents. Sterilization can be carried out in several ways, in some or any embodiments, using a bacteriological filter, by radiation or by heating. They can also be prepared in the form of sterile solid compositions which can be dissolved at the time of use in sterile water or any other injectable sterile medium.

The compositions for rectal administration are suppositories or rectal capsules which contain, in addition to the active principle, excipients such as cocoa butter, semisynthetic glycerides or polyethylene glycols.

The compositions can also be aerosols. For use in the form of liquid aerosols, the compositions can be stable sterile solutions or solid compositions dissolved at the time of use in apyrogenic sterile water, in saline or any other pharmaceutically acceptable vehicle. For use in the form of dry aerosols intended to be directly inhaled, the active principle is finely divided and combined with a water-soluble solid diluent or vehicle, in some or any embodiments, dextran, mannitol or lactose.

In some or any embodiments, a composition provided herein is a pharmaceutical composition or a single unit dosage form. Pharmaceutical compositions and single unit dosage forms provided herein comprise a prophylactically or therapeutically effective amount of one or more prophylactic or therapeutic agents (*e.g.,* a compound provided herein, or other prophylactic or therapeutic agent), and a typically one or more pharmaceutically acceptable carriers or excipients. In a specific embodiment and in this context, the term "pharmaceutically acceptable" means approved by a regulatory agency of the Federal or a state government or listed in the U.S. Pharmacopeia or other generally recognized pharmacopeia for use in animals, and more particularly in humans. The term "carrier" includes a diluent, adjuvant (*e.g.,* Freund's adjuvant (complete and incomplete)), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water can be used as a carrier when the pharmaceutical composition is administered intravenously. Saline solutions and aqueous dextrose and glycerol solutions can also be employed as liquid carriers, particularly for injectable solutions. Examples of suitable pharmaceutical carriers are described in Remington: The Science and Practice of Pharmacy; Pharmaceutical Press; 22 edition (September 15, 2012).

Typical pharmaceutical compositions and dosage forms comprise one or more excipients. Suitable excipients are well-known to those skilled in the art of pharmacy, and in some or any embodiments, suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Whether a particular excipient is suitable for incorporation into a pharmaceutical composition or dosage form depends on a variety of factors well known in the art including, but not limited to, the way in which the dosage form will be administered to a subject and the specific active ingredients in the dosage form. The composition or single unit dosage form, if desired, can also contain minor amounts of wetting or emulsifying agents, or pH buffering agents.

Lactose free compositions provided herein can comprise excipients that are well known in the art and are listed, in some or any embodiments, in the U.S. Pharmacopeia (USP 36-NF 31 S2). In general, lactose free compositions comprise an active ingredient, a binder/filler, and a lubricant in pharmaceutically compatible and pharmaceutically acceptable amounts. Exemplary lactose free dosage forms comprise an active ingredient, microcrystalline cellulose, pre gelatinized starch, and magnesium stearate.

Further encompassed herein are anhydrous pharmaceutical compositions and dosage forms comprising active ingredients, since water can facilitate the degradation of some compounds. For example, the addition of water (*e.g.,* 5%) is widely accepted in the pharmaceutical arts as a means of simulating long term storage in order to determine characteristics such as shelf life or the stability of formulations over time. See, *e.g.,* Jens T. Carstensen, Drug Stability: Principles & Practice, 2d. Ed., Marcel Dekker, New York, 1995, pp. 379 80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms provided herein can be prepared using anhydrous or low moisture containing ingredients and low moisture or low humidity conditions. Pharmaceutical compositions and dosage forms that comprise lactose and at least one active ingredient that comprises a primary or secondary amine can be anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions can be packaged using materials known to prevent exposure to water such that they can be included in suitable formulary kits. In some or any embodiments, suitable packaging include, but are not limited to, hermetically sealed foils, plastics, unit dose containers (*e.g.,* vials), blister packs, and strip packs.

Further provided are pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

The pharmaceutical compositions and single unit dosage forms can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Such compositions and dosage forms will contain a prophylactically or therapeutically effective amount of a prophylactic or therapeutic agent, in some or any embodiments, in purified form, together with a suitable amount of carrier so as to provide the form for proper administration to the subject. The formulation should suit the mode of administration. In a some or any embodiment, the pharmaceutical compositions or single unit dosage forms are sterile and in suitable form for administration to a subject, in some or any embodiments, an animal subject, such as a mammalian subject, in some or any embodiments, a human subject.

A pharmaceutical composition is formulated to be compatible with its intended route of administration. In some or any embodiments, routes of administration include, but are not limited to, parenteral, e.g., intrathecal, epidural, local or regional for peripheral nerve block, intravenous, intradermal, subcutaneous, intramuscular, subcutaneous, oral, buccal, sublingual, inhalation, intranasal, transdermal, topical (including administration to the eye, and in some embodiments to the cornea), transmucosal, intra-tumoral, intra-synovial, and rectal administration. In a specific embodiment, the composition is formulated in accordance with routine procedures as a pharmaceutical composition adapted for intravenous, subcutaneous, intramuscular, oral, intranasal, or topical (including administration to the eye, and in some embodiments to the cornea) administration to human beings. In a specific embodiment, a pharmaceutical composition is formulated in accordance with routine procedures for subcutaneous administration to human beings. Typically, compositions for intravenous administration are solutions in sterile isotonic aqueous buffer. Where necessary, the composition may also include a solubilizing agent and a local anesthetic such as lignocamne to ease pain at the site of the injection.

In some or any embodiments, dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (*e.g.,* nasal sprays or inhalers); gels; liquid dosage forms suitable for oral or mucosal administration to a subject, including suspensions (*e.g.*, aqueous or non-aqueous liquid suspensions, oil in water emulsions, or a water in oil liquid emulsions), solutions, and elixirs; liquid dosage forms suitable for parenteral administration to a subject; and sterile solids (*e.g.,* crystalline or amorphous solids) that can be reconstituted to provide liquid dosage forms suitable for parenteral administration to a subject.

The composition, shape, and type of dosage forms provided herein will typically vary depending on their use. In some or any embodiments, a dosage form used in the initial treatment of pain may contain larger amounts of one or more of the active ingredients it comprises than a dosage form used in the maintenance treatment of the same infection. Similarly, a parenteral dosage form may contain smaller amounts of one or more of the active ingredients it comprises than an oral dosage form used to treat the same disease or disorder. These and other ways in which specific dosage forms encompassed herein will vary from one another will be readily apparent to those skilled in the art. See, *e.g.,* Remington: The Science and Practice of Pharmacy; Pharmaceutical Press; 22 edition (September 15, 2012).

Generally, the ingredients of compositions are supplied either separately or mixed together in unit dosage form, in some or any embodiments, as a dry lyophilized powder or water free concentrate in a hermetically sealed container such as an ampoule or sachet indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Typical dosage forms comprise a compound provided herein, or a pharmaceutically acceptable salt, solvate or hydrate thereof lie within the range of from about 0.1 mg to about 1000 mg per day, given as a single once-a-day dose in the morning or as divided doses throughout the day taken with food. Particular dosage forms can have about 0.1, 0.2, 0.3, 0.4, 0.5, 1.0, 2.0, 2.5, 5.0, 10.0, 15.0, 20.0, 25.0, 50.0, 100, 200, 250, 500 or 1000 mg of the active compound.

### Oral Dosage Forms

Pharmaceutical compositions that are suitable for oral administration can be presented as discrete dosage forms, such as, but are not limited to, tablets (*e.g.,* chewable tablets), caplets, capsules, and liquids (*e.g.,* flavored syrups). Such dosage forms contain predetermined amounts of active ingredients, and may be prepared by methods of pharmacy well known to those skilled in the art. See generally, Remington: The Science and Practice of Pharmacy; Pharmaceutical Press; 22 edition (September 15, 2012).

In some or any embodiments, the oral dosage forms are solid and prepared under anhydrous conditions with anhydrous ingredients, as described in detail herein. However, the scope of the compositions provided herein extends beyond anhydrous, solid oral dosage forms. As such, further forms are described herein.

Typical oral dosage forms are prepared by combining the active ingredient(s) in an intimate admixture with at least one excipient according to conventional pharmaceutical compounding techniques. Excipients can take a wide variety of forms depending on the form of preparation desired for administration. In some or any embodiments, excipients suitable for use in oral liquid or aerosol dosage forms include, but are not limited to, water, glycols, oils, alcohols, flavoring agents, preservatives, and coloring agents. In some or any embodiments, excipients suitable for use in solid oral dosage forms (*e.g.,* powders, tablets, capsules, and caplets) include, but are not limited to, starches, sugars, micro crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or non-aqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

In some or any embodiments, a tablet can be prepared by compression or molding. Compressed tablets can be prepared by compressing in a suitable machine the active ingredients in a free flowing form such as powder or granules, optionally mixed with an excipient. Molded tablets can be made by molding in a suitable machine a mixture of the powdered compound moistened with an inert liquid diluent.

In some or any embodiments, excipients that can be used in oral dosage forms include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g.,* ethyl cellulose, cellulose acetate, carboxymethyl cellulose calcium, sodium carboxymethyl cellulose), polyvinyl pyrrolidone, methyl cellulose, pre gelatinized starch, hydroxypropyl methyl cellulose, (*e.g.,* Nos. 2208, 2906, 2910), microcrystalline cellulose, and mixtures thereof.

In some or any embodiments, fillers suitable for use in the pharmaceutical compositions and dosage forms disclosed herein include, but are not limited to, talc, calcium carbonate (*e.g.,* granules or powder), microcrystalline cellulose, powdered cellulose, dextrates, kaolin, mannitol, silicic acid, sorbitol, starch, pre gelatinized starch, and mixtures thereof. The binder or filler in pharmaceutical compositions is typically present in from about 50 to about 99 weight percent of the pharmaceutical composition or dosage form.

In some or any embodiments, suitable forms of microcrystalline cellulose include, but are not limited to, the materials sold as AVICEL PH 101, AVICEL PH 103 AVICEL RC 581, AVICEL PH 105 (available from FMC Corporation, American Viscose Division, Avicel Sales, Marcus Hook, PA), and mixtures thereof. A specific binder is a mixture of microcrystalline cellulose and sodium carboxymethyl cellulose sold as AVICEL RC 581. Suitable anhydrous or low moisture excipients or additives include AVICEL PH 103^{™} and Starch 1500 LM.

Disintegrants are used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms. The amount of disintegrant used varies based upon the type of formulation, and is readily discernible to those of ordinary skill in the art. Typical pharmaceutical compositions comprise from about 0.5 to about 15 weight percent of disintegrant, specifically from about 1 to about 5 weight percent of disintegrant.

Disintegrants that can be used in pharmaceutical compositions and dosage forms include, but are not limited to, agar, alginic acid, calcium carbonate, microcrystalline cellulose, croscarmellose sodium, crospovidone, polacrilin potassium, sodium starch glycolate, potato or tapioca starch, pre gelatinized starch, other starches, clays, other algins, other celluloses, gums, and mixtures thereof.

Lubricants that can be used in pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g.,* peanut oil, cottonseed oil, sunflower oil, sesame oil, olive oil, corn oil, and soybean oil), zinc stearate, ethyl oleate, ethyl laureate, agar, and mixtures thereof. Additional lubricants include, in some or any embodiments, a syloid silica gel (AEROSIL 200, manufactured by W.R. Grace Co. of Baltimore, MD), a coagulated aerosol of synthetic silica (marketed by Degussa Co. of Plano, TX), CAB O SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

### Delayed Release Dosage Forms

Active ingredients such as the compounds provided herein can be administered by controlled release means or by delivery devices that are well known to those of ordinary skill in the art. In some or any embodiments, but are not limited to, those described in U.S. Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358; and 6,699,500; each of which is incorporated herein by reference in its entirety. Such dosage forms can be used to provide slow or controlled release of one or more active ingredients using, in some or any embodiments, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled release formulations known to those of ordinary skill in the art, including those described herein, can be readily selected for use with the active ingredients provided herein. Thus encompassed herein are unit dosage forms suitable for oral administration such as, but not limited to, tablets, capsules, gel caps, and caplets that are adapted for controlled release.

All controlled release pharmaceutical products have a common goal of improving drug therapy over that achieved by their non-controlled counterparts. Ideally, the use of an optimally designed controlled release preparation in medical treatment is characterized by a minimum of drug substance being employed to cure or control the condition in a minimum amount of time. Advantages of controlled release formulations include extended activity of the drug, reduced dosage frequency, and increased subject compliance. In addition, controlled release formulations can be used to affect the time of onset of action or other characteristics, such as blood levels of the drug, and can thus affect the occurrence of side (*e.g.*, adverse) effects.

Most controlled release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled release of an active ingredient can be stimulated by various conditions including, but not limited to, pH, temperature, enzymes, water, or other physiological conditions or compounds.

In some or any embodiments, the drug may be administered using intravenous infusion, an implantable osmotic pump, a transdermal patch, liposomes, or other modes of administration. In some or any embodiments, a pump may be used (*see,* Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used. In yet another embodiment, a controlled release system can be placed in a subject at an appropriate site determined by a practitioner of skill, *i.e.,* thus requiring only a fraction of the systemic dose (*see, e.g.,* Goodson, Medical Applications of Controlled Release, vol. 2, pp. 115-138 (1984)). Other controlled release systems are discussed in the review by Langer (Science 249:1527-1533 (1990)). The active ingredient can be dispersed in a solid inner matrix, *e.g.,* polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, *e.g.,* polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The active ingredient then diffuses through the outer polymeric membrane in a release rate controlling step. The percentage of active ingredient in such parenteral compositions is highly dependent on the specific nature thereof, as well as the needs of the subject.

### Parenteral Dosage Forms

In some or any embodiments, provided are parenteral dosage forms. Parenteral dosage forms can be administered to subjects by various routes including, but not limited to, subcutaneous, intravenous (including bolus injection), intramuscular, and intra-arterial. Because their administration typically bypasses subjects' natural defenses against contaminants, parenteral dosage forms are typically, sterile or capable of being sterilized prior to administration to a subject. In some or any embodiments, parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. In some or any embodiments, suitable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms.

### Transdermal, Topical & Mucosal Dosage Forms

Also provided are transdermal, topical, and mucosal dosage forms. Transdermal, topical, and mucosal dosage forms include, but are not limited to, ophthalmic solutions, sprays, aerosols, creams, lotions, ointments, gels, solutions, emulsions, suspensions, or other forms known to one of skill in the art. See, e.g., Remington: The Science and Practice of Pharmacy; Pharmaceutical Press; 22 edition (September 15, 2012); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels. Further, transdermal dosage forms include "reservoir type" or "matrix type" patches, which can be applied to the skin and worn for a specific period of time to permit the penetration of a desired amount of active ingredients.

Suitable excipients (*e.g.,* carriers and diluents) and other materials that can be used to provide transdermal, topical, and mucosal dosage forms encompassed herein are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane 1,3 diol, isopropyl myristate, isopropyl palmitate, mineral oil, and mixtures thereof to form lotions, tinctures, creams, emulsions, gels or ointments, which are nontoxic and pharmaceutically acceptable. Moisturizers or humectants can also be added to pharmaceutical compositions and dosage forms if desired. Examples of such additional ingredients are well known in the art. See, *e.g.,* Remington: The Science and Practice of Pharmacy; Pharmaceutical Press; 22 edition (September 15, 2012).

Depending on the specific tissue to be treated, additional components may be used prior to, in conjunction with, or subsequent to treatment with active ingredients provided. In some or any embodiments, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery enhancing or penetration enhancing agent. Different salts, hydrates or solvates of the active ingredients can be used to further adjust the properties of the resulting composition.

### Dosage and Unit Dosage Forms

In human therapeutics, the doctor will determine the posology which the doctor considers most appropriate according to a preventive or curative treatment and according to the age, weight, stage of the infection and other factors specific to the subject to be treated. In some or any embodiments, doses are from about 1 to about 1000 mg per day for an adult, or from about 5 to about 250 mg per day or from about 10 to 50 mg per day for an adult. In some or any embodiments, doses are from about 5 to about 400 mg per day or 25 to 200 mg per day per adult. In some or any embodiments, dose rates of from about 50 to about 500 mg per day are also contemplated. In some or any embodiments, doses for subcutaneous administration are from about 1 to about 50 mg per day, or from about 1 to about 25 mg per day, or from about 1 to about 10 mg per day, or from about 1 to about 20 mg per day, or from about 5 to about 25 mg per day, or from about 5 mg to about 20 mg per day, or from about 10 to about 20 mg per day. In some or any embodiments, doses for oral administration are from about 5 to about 250 mg per day, from about 5 to 200 mg per day, or from about 50 mg to about 100 mg per day, or from about 75 mg to about 1125 mg per day, or from about 10 mg to about 200 mg per day. In some embodiments, the mg/day amounts are for an adult.In further aspects, provided are methods of treating a condition associated with voltage-gated sodium channel function and/or pain in a subject by administering, to a subject in need thereof, a therapeutically or prophylactically effective amount of a compound provided herein, or a pharmaceutically acceptable salt thereof. The amount of the compound or composition which will be therapeutically or prophylactically effective in the treatment of a disorder or one or more symptoms thereof will vary with the nature and severity of the disease or condition, and the route by which the active ingredient is administered. The frequency and dosage will also vary according to factors specific for each subject depending on the specific therapy (*e.g.,* therapeutic or prophylactic agents) administered, the severity of the disorder, disease, or condition, the route of administration, as well as age, body, weight, response, and the past medical history of the subject. Effective doses may be extrapolated from dose-response curves derived from *in vitro* or animal model test systems.

In some or any embodiments, exemplary doses of a composition include milligram or microgram amounts of the active compound per kilogram of subject or sample weight (*e.g.,* about 10 micrograms per kilogram to about 50 milligrams per kilogram, about 100 micrograms per kilogram to about 25 milligrams per kilogram, or about 100 microgram per kilogram to about 10 milligrams per kilogram). For compositions provided herein, in some or any embodiments, the dosage administered to a subject is 0.01 mg/kg to 3 mg/kg of the subject's body weight, or 0.10 mg/kg to 3 mg/kg of the subject's body weight, based on weight of the active compound. In some or any embodiments, the dosage administered to a subject is between 0.20 mg/kg and 2.00 mg/kg, or between 0.30 mg/kg and 1.50 mg/kg of the subject's body weight. In some embodiments, the dosage is administered subcutaneously to a subject and is between about 0.01 mg/kg to 1 mg/kg (inclusive), or between about 0.03 mg/kg to 0.5 mg/kg (inclusive) of the subject's body weight, based on weight of the active compound. In some embodiments, the dosage is administered orally to a subject and is between about 0.10 mg/kg to 5 mg/kg (inclusive) of the subject's body weight, or between about 0.10 mg/kg to 2 mg/kg (inclusive) of the subject's body weight, based on weight of the active compound.

In some or any embodiments, the recommended daily dose range of a composition provided herein for the conditions described herein lie within the range of from about 0.1 mg to about 1000 mg per day, given as a single once-a-day dose or as divided doses throughout a day. In some or any embodiments, the daily dose is administered twice daily in equally divided doses. In some or any embodiments, the daily dose is administered thrice daily in equally divided doses. In some or any embodiments, the daily dose is administered four times daily in equally divided doses. In some or any embodiments, a daily dose range should be from about 0.01 mg to about 400 mg per day, from about 0.1 mg to about 250 mg per day, from about 10 mg to about 200 mg per day, in other embodiments, or from about 10 mg and about 150 mg per day, in further embodiments, between about 25 and about 100 mg per day. It may be necessary to use dosages of the active ingredient outside the ranges disclosed herein in some cases, as will be apparent to those of ordinary skill in the art. Furthermore, it is noted that the clinician or treating physician will know how and when to interrupt, adjust, or terminate therapy in conjunction with subject response.

Different therapeutically effective amounts may be applicable for different diseases and conditions, as will be readily known by those of ordinary skill in the art. Similarly, amounts sufficient to prevent, manage, treat or ameliorate such disorders, but insufficient to cause, or sufficient to reduce, adverse effects associated with the composition provided herein are also encompassed by the herein described dosage amounts and dose frequency schedules. Further, when a subject is administered multiple dosages of a composition provided herein, not all of the dosages need be the same. In some or any embodiments, the dosage administered to the subject may be increased to improve the prophylactic or therapeutic effect of the composition or it may be decreased to reduce one or more side effects that a particular subject is experiencing.

In some or any embodiment, the dosage of the composition provided herein, based on weight of the active compound, administered to prevent, treat, manage, or ameliorate a disorder, or one or more symptoms thereof in a subject is 0.1 mg/kg, 1 mg/kg, 2 mg/kg, 3 mg/kg, 4 mg/kg, 5 mg/kg, 6 mg/kg, 10 mg/kg, or 15 mg/kg or more of a subject's body weight. In another embodiment, the dosage of the composition or a composition provided herein administered to prevent, treat, manage, or ameliorate a disorder, or one or more symptoms thereof in a subject is a unit dose of 0.1 mg to 200 mg, 0.1 mg to 100 mg, 0.1 mg to 50 mg, 0.1 mg to 25 mg, 0.1 mg to 20 mg, 0.1 mg to 15 mg, 0.1 mg to 10 mg, 0.1 mg to 7.5 mg, 0.1 mg to 5 mg, 0.1 to 2.5 mg, 0.25 mg to 20 mg, 0.25 to 15 mg, 0.25 to 12 mg, 0.25 to 10 mg, 0.25 mg to 7.5 mg, 0.25 mg to 5 mg, 0.5 mg to 2.5 mg, 1 mg to 20 mg, 1 mg to 15 mg, 1 mg to 12 mg, 1 mg to 10 mg, 1 mg to 7.5 mg, 1 mg to 5 mg, or 1 mg to 2.5 mg.

In some or any embodiments, treatment or prevention can be initiated with one or more loading doses of a compound or composition provided herein followed by one or more maintenance doses. In such embodiments, the loading dose can be, for instance, about 6 to about 40 mg per day, or about 10 to about 20 mg per day for one day to five weeks. The loading dose can be followed by one or more maintenance doses. In some or any embodiments, each maintenance does is, independently, about from about 1 mg to about 20 mg per day, between about 2.5 mg and about 15 mg per day, or between about 2.5 and about 8 mg per day. Maintenance doses can be administered daily and can be administered as single doses, or as divided doses.

In some or any embodiments, a dose of a compound or composition provided herein can be administered to achieve a steady-state concentration of the active ingredient in blood or serum of the subject. The steady-state concentration can be determined by measurement according to techniques available to those of skill or can be based on the physical characteristics of the subject such as height, weight and age. In some or any embodiments, a sufficient amount of a compound or composition provided herein is administered to achieve a steady-state concentration in blood or serum of the subject of from about 100 to about 1000 ng/mL, from about 150 to about 800 ng/mL, or from about 300 to about 600 ng/mL. In some or any embodiments, loading doses can be administered to achieve steady-state blood or serum concentrations of about 300 to about 2000 ng/mL, or about 400 to about 800 ng/mL for one to five days. In some or any embodiments, maintenance doses can be administered to achieve a steady-state concentration in blood or serum of the subject of from about 100 to about 1000 ng/mL, from about 150 to about 800 ng/mL, or from about 300 to about 600 ng/mL. In some or any embodiments, administration of the same composition may be repeated and the administrations may be separated by at least 1 day, 2 days, 3 days, 5 days, 10 days, 15 days, 30 days, 45 days, 2 months, 75 days, 3 months, or 6 months. In other embodiments, administration of the same prophylactic or therapeutic agent may be repeated and the administration may be separated by at least at least 1 day, 2 days, 3 days, 5 days, 10 days, 15 days, 30 days, 45 days, 2 months, 75 days, 3 months, or 6 months.

In some or any embodiments, provided herein are unit dosages comprising a compound, or a pharmaceutically acceptable salt thereof, in a form suitable for administration. Such forms are described in detail herein. In some or any embodiments, the unit dosage comprises 1 to 1000 mg, 1 to 100 mg or 10 to 50 mg active ingredient. In particular embodiments, the unit dosages comprise about 1, 5, 10, 25, 50, 100, 125, 250, 500 or 1000 mg active ingredient. Such unit dosages can be prepared according to techniques familiar to those of skill in the art.

In some or any embodiments, dosages of the second agents to be used in a combination therapy are provided herein. In some or any embodiments, dosages lower than those which have been or are currently being used to treat pain are used in the combination therapies provided herein. The recommended dosages of second agents can be obtained from the knowledge of those of skill in the art. For those second agents that are approved for clinical use, recommended dosages are described in, for example, Hardman et al., eds., 1996, Goodman & Gilman's The Pharmacological Basis Of Therapeutics 9th Ed, Mc-Graw-Hill, New York; Physician's Desk Reference (PDR) 57th Ed., 2003, Medical Economics Co., Inc., Montvale, NJ; which are incorporated herein by reference in their entirety.

In various embodiments, the therapies (*e.g.,* a compound provided herein and the second agent) are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart. In various embodiments, the therapies are administered no more than 24 hours apart or no more than 48 hours apart. In some or any embodiments, two or more therapies are administered within the same patient visit. In other embodiments, the compound provided herein and the second agent are administered concurrently.

In other embodiments, the compound provided herein and the second agent are administered at about 2 to 4 days apart, at about 4 to 6 days apart, at about 1 week part, at about 1 to 2 weeks apart, or more than 2 weeks apart.

In some or any embodiments, administration of the same agent may be repeated and the administrations may be separated by at least 1 day, 2 days, 3 days, 5 days, 10 days, 15 days, 30 days, 45 days, 2 months, 75 days, 3 months, or 6 months. In other embodiments, administration of the same agent may be repeated and the administration may be separated by at least at least 1 day, 2 days, 3 days, 5 days, 10 days, 15 days, 30 days, 45 days, 2 months, 75 days, 3 months, or 6 months.

In some or any embodiments, a compound provided herein and a second agent are administered to a patient, in some or any embodiments, a mammal, such as a human, in a sequence and within a time interval such that the compound provided herein can act together with the other agent to provide an increased benefit than if they were administered otherwise. In some or any embodiments, the second active agent can be administered at the same time or sequentially in any order at different points in time; however, if not administered at the same time, they should be administered sufficiently close in time so as to provide the desired therapeutic or prophylactic effect. In some or any embodiments, the compound provided herein and the second active agent exert their effect at times which overlap. Each second active agent can be administered separately, in any appropriate form and by any suitable route. In other embodiments, the compound provided herein is administered before, concurrently or after administration of the second active agent.

In some or any embodiments, the compound provided herein and the second agent are cyclically administered to a patient. Cycling therapy involves the administration of a first agent (*e.g.,* a first prophylactic or therapeutic agent) for a period of time, followed by the administration of a second agent and/or third agent (*e.g.,* a second and/or third prophylactic or therapeutic agent) for a period of time and repeating this sequential administration. Cycling therapy can reduce the development of resistance to one or more of the therapies, avoid or reduce the side effects of one of the therapies, and/or improve the efficacy of the treatment.

In some or any embodiments, the compound provided herein and the second active agent are administered in a cycle of less than about 3 weeks, about once every two weeks, about once every 10 days or about once every week. One cycle can comprise the administration of a compound provided herein and the second agent by infusion over about 90 minutes every cycle, about 1 hour every cycle, about 45 minutes every cycle. Each cycle can comprise at least 1 week of rest, at least 2 weeks of rest, at least 3 weeks of rest. The number of cycles administered is from about 1 to about 12 cycles, more typically from about 2 to about 10 cycles, and more typically from about 2 to about 8 cycles.

In other embodiments, courses of treatment are administered concurrently to a patient, i.e., individual doses of the second agent are administered separately yet within a time interval such that the compound provided herein can work together with the second active agent. In some or any embodiments, one component can be administered once per week in combination with the other components that can be administered once every two weeks or once every three weeks. In other words, the dosing regimens are carried out concurrently even if the therapeutics are not administered simultaneously or during the same day.

The second agent can act additively or synergistically with the compound provided herein. In some or any embodiments, the compound provided herein is administered concurrently with one or more second agents in the same pharmaceutical composition. In another embodiment, a compound provided herein is administered concurrently with one or more second agents in separate pharmaceutical compositions. In still another embodiment, a compound provided herein is administered prior to or subsequent to administration of a second agent. Also contemplated are administration of a compound provided herein and a second agent by the same or different routes of administration, *e.g.,* oral and parenteral. In some or any embodiments, when the compound provided herein is administered concurrently with a second agent that potentially produces adverse side effects including, but not limited to, toxicity, the second active agent can advantageously be administered at a dose that falls below the threshold that the adverse side effect is elicited.

### Kits

Also provided are kits for use in methods of treatment of pain and/or a condition associated with voltage-gated sodium channel function or a pain-related disorder. The kits can include a compound or composition provided herein, a second agent or composition, and instructions providing information to a health care provider regarding usage for treating the pain or a pain-related disorder. Instructions may be provided in printed form or in the form of an electronic medium such as a floppy disc, CD, or DVD, or in the form of a website address where such instructions may be obtained. A unit dose of a compound or composition provided herein, or a second agent or composition, can include a dosage such that when administered to a subject, a therapeutically or prophylactically effective plasma level of the compound or composition can be maintained in the subject for at least 1 day. In some or any embodiments, a compound or composition can be included as a sterile aqueous pharmaceutical composition or dry powder (*e.g.,* lyophilized) composition.

In some or any embodiments, suitable packaging is provided. As used herein, "packaging" includes a solid matrix or material customarily used in a system and capable of holding within fixed limits a compound provided herein and/or a second agent suitable for administration to a subject. Such materials include glass and plastic (*e.g.,* polyethylene, polypropylene, and polycarbonate) bottles, vials, paper, plastic, and plastic-foil laminated envelopes and the like. If e-beam sterilization techniques are employed, the packaging should have sufficiently low density to permit sterilization of the contents.

### Methods of Use

Provided herein is a method for treating a condition associated with voltage-gated sodium channel function and/or pain in a subject, which comprises contacting the subject with a therapeutically or prophylactically effective amount of a 11,13-modified, non-hydrated ketone saxitoxin disclosed herein, *e.g.,* a 11,13-modified, non-hydrated ketone saxitoxin of Formula (I), (P-1), (P-Ia), (P-Ib), (P-Ic), or (P-Id) and 1-46, 49-52, and 53-70 including a single enantiomer, a mixture of an enantiomeric pair, an individual diastereomer, a mixture of diastereomers, an individual stereoisomer, a mixture of stereoisomers, or a tautomeric form thereof; or a pharmaceutically acceptable salt, solvate, prodrug, phosphate, or active metabolite thereof.

Provided herein is a method for the treatment of a condition associated with voltage-gated sodium channel function in a mammal, comprising the administration of a therapeutically or prophylactically effective amount of a compound of Formula (I), (P-1), (P-Ia), (P-Ib), (P-Ic), or (P-Id) described herein or a pharmaceutical composition described herein.

In some embodiments, the mammal is a human. In a group of embodiments, the condition is pain or the condition is associated with pain. In some embodiments, the condition is pain. In some embodiments, the condition is associated with pain. In some embodiments, the pain is nociceptive pain. In some embodiments, the pain is neuropathic pain. In some embodiments, the pain is inflammatory pain. In some embodiments, the pain is refractory to other forms of pain medications.

In a group of embodiments, the condition is selected from the group consisting of erythromelalgia, diabetic peripheral neuropathy, paroxysmal extreme pain disorder, complex regional pain syndrome, trigeminal neuralgia, multiple sclerosis, osteoarthritis, postherpetic neuralgia, cancer pain, cluster headache, migraine, sciatica, endometriosis, fibromyalgia, and postsurgical pain. In a group of embodiments, the condition is selected from the group consisting of epilepsy, Parkinson's disease, a mood disorder, psychosis, amyotropic lateral sclerosis, glaucoma, ischemia, a spasticity disorder, and obsessive compulsive disorder.

In some or any embodiments, provided herein are methods for treating pain and/or a condition associated with voltage-gated sodium channel function in a subject. In some or any embodiments, the methods encompass the step of administering to the subject in need thereof an amount of a compound effective for the treatment pain and/or a condition associated with voltage-gated sodium channel function in combination with a second agent effective for the treatment or prevention of pain and/or a condition associated with voltage-gated sodium channel function. The compound can be any compound as described herein, and the second agent can be any second agent described in the art or herein. In some or any embodiments, the compound is in the form of a pharmaceutical composition or dosage form, as described elsewhere herein.

In some or any embodiments, provided herein are methods for treating a condition associated with voltage-gated sodium channel function in a subject. In some or any embodiments, the methods encompass the step of administering to the subject in need thereof a therapeutically or prophylactically effective amount of a compound effective for the treatment of a condition associated with voltage-gated sodium channel function in combination with a second agent effective for the treatment of a condition associated with voltage-gated sodium channel function. The compound can be any compound as described herein, and the second agent can be any second agent described in the art or herein. In some or any embodiments, the compound is in the form of a pharmaceutical composition or dosage form, as described elsewhere herein.

In some or any embodiments, the pain to be reduced, ameliorated, treated, or prevented is associated with a condition or is a condition selected from acute pain, anal fissures, back pain, chronic pain, dental pain, joint pain, neck pain, neuropathic pain, obstetric pain, post-herpetic neuralgia, shingles, tension headaches, trigeminal blepharospasm, pain associated with cardiac arrythmia, focal dystonia, hyperhidrosis, muscle spasms, urinary bladder relaxation, visceral pain, sympathetically maintained pain, myositis pain, musculoskeletal pain, lower back pain, pain from sprains and strains, pain associated with functional bowel disorders, non-cardiac chest pain, pain associated with irritable bowel syndrome, pain associated with myocardial ischemia, toothache pain, pain from dysmenorrhea, erythromelalgia, diabetic peripheral neuropathy, paroxysmal extreme pain disorder, complex regional pain syndrome, trigeminal neuralgia, multiple sclerosis, osteoarthritis, postherpetic neuralgia, cancer, cluster headache, migraine, sciatica, endometriosis, fibromyalgia, dry eye syndrome, (acute) corneal injuries or abrasions, corneal infections, pain associated with Parkinson's disease, pain associated with ALS, and surgery (in some embodiments, post-surgery; in some embodiments, ocular surgery). In some or any embodiments, the pain to be reduced, ameliorated, treated, or prevented is pain in an acute care setting, including post-surgery. In some or any embodiments, the pain to be reduced, ameliorated, treated, or prevented is pain in an acute care setting, including post-surgery and the compound is administered intraveneously. In some or any embodiments, the pain to be reduced, ameliorated, treated, or prevented is ocular pain. In some or any embodiments, the pain to be reduced, ameliorated, treated, or prevented is ocular pain and the compound is administered topically. In some or any embodiments, the pain to be reduced, ameliorated, treated, or prevented is subacute or chronic pain. In some or any embodiments, the pain to be reduced, ameliorated, treated, or prevented is subacute or chronic pain and the compound is administered subcutaneously. In some or any embodiments, the pain to be reduced, ameliorated, treated, or prevented is subacute or chronic pain and the compound is administered orally.

In some or any embodiments, the condition associated with voltage-gated sodium channel function is selected from itch, cough, epilepsy, Parkinson's disease, a mood disorder, psychosis, amyotrophic lateral sclerosis (ALS), cardiac arrhythmia, glaucoma, ischemia, a spasticity disorder, and obsessive compulsive disorder. In some or any embodiments, the condition associated with voltage-gated sodium channel function is selected from pain, itch, cough, glaucoma, and ischemia. In some or any embodiments, the condition associated with voltage-gated sodium channel function is selected from pain, itch, and cough. In some or any embodiments, the condition associated with voltage-gated sodium channel function is pain.

In some or any embodiments, the compounds described herein are used for delaying the onset of pain, or reducing the severity or duration of pain. In some or any embodiments, the compounds described herein are used for the reduction of the severity or duration of pain associated with voltage-gated sodium channel function. In some embodiments, the compounds described herein are used for delaying or preventing onset of pain.

In some or any embodiments, the compounds described herein are used for prevention of pain or of a condition associated with voltage-gated sodium channel function.

In some or any embodiments, the compounds described herein are used for treatment of pain or of a condition associated with voltage-gated sodium channel function.

### Assay Methods

Compounds can be assayed for efficacy in treating pain and/or a condition associated with voltage-gated sodium channel function according to any assay known to those of skill in the art. Exemplary assay methods are provided elsewhere herein.

### Second Therapeutic Agents

In some or any embodiments, the compounds and compositions provided herein are useful in methods of treatment of pain and/or a condition associated with voltage-gated sodium channel function, that comprise further administration of a second agent effective for the treatment of pain and/or a pain-related disorder and/or a condition associated with voltage-gated sodium channel function. The second agent can be any agent known to those of skill in the art to be effective for the treatment of pain and/or a pain-related disorder and/or a condition associated with voltage-gated sodium channel function, including those currently approved by the United States Food and Drug Administration, or other similar body of a country foreign to the United States. In some or any embodiments, the second agent is a local anesthetic (in some or any embodiments, a steroid), an opioid, a vasoconstrictor, a glucocorticoid, adrenergic drugs (in some or any embodiments, alpha agonists or mixed central-peripheral alpha-2- agonists), vanilloids, an anti-inflammatory agent (e.g. NSAID, or an anti-inflammatory agent associated with ocular conditions, including cyclosporine and lifitegrast) or a chemical permeation enhancer. In some embodiments, the second agent is an inhibitor of Na_{V} 1.8. In some or any embodiments, chemical permeation enhancers include anionic surfactants, cationic surfactants, nonionic surfactants. In some or any embodiments, the second agent is bupivacaine, levobupivicaine, tetracaine, ropivacaine, epinephrine, phenylephrine, clonidine, sodium lauryl sulfate, sodium octyl sulfate, dodecyltrimethylammonium bromide, octyltrimethylammonium bromide, polyoxyethylene (20) sorbitan monolaurate, and/or polyoxyethylene (20) sorbitan monooleate.

In some or any embodiments, a compound provided herein is administered in combination with one second agent. In further embodiments, a compound provided herein is administered in combination with two second agents. In still further embodiments, a compound provided herein is administered in combination with two or more second agents.

As used herein, the term "in combination" includes the use of more than one therapy (e.g., one or more prophylactic and/or therapeutic agents). The use of the term "in combination" does not restrict the order in which therapies (*e.g.,* prophylactic and/or therapeutic agents) are administered to a subject with a disorder. A first therapy *(e.g.,* a prophylactic or therapeutic agent such as a compound provided herein) can be administered prior to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (*e.g.,* a prophylactic or therapeutic agent) to a subject with a disorder.

As used herein, the term "synergistic" includes a combination of a compound provided herein and another therapy (*e.g.,* a prophylactic or therapeutic agent) which has been or is currently being used to prevent, manage or treat a disorder, which is more effective than the additive effects of the therapies. A synergistic effect of a combination of therapies (*e.g.,* a combination of prophylactic or therapeutic agents) permits the use of lower dosages of one or more of the therapies and/or less frequent administration of said therapies to a subject with a disorder. The ability to utilize lower dosages of a therapy (*e.g.,* a prophylactic or therapeutic agent) and/or to administer said therapy less frequently reduces the toxicity associated with the administration of said therapy to a subject without reducing the efficacy of said therapy in the prevention or treatment of a disorder). In addition, a synergistic effect can result in improved efficacy of agents in the prevention or treatment of a disorder. Finally, a synergistic effect of a combination of therapies (*e.g.,* a combination of prophylactic or therapeutic agents) may avoid or reduce adverse or unwanted side effects associated with the use of either therapy alone.

The active compounds provided herein can be administered in combination or alternation with another therapeutic agent, in particular an agent effective in the treatment of pain and/or a pain-related disorder and/or a condition associated with voltage-gated sodium channel function. In combination therapy, effective dosages of two or more agents are administered together, whereas in alternation or sequential-step therapy, an effective dosage of each agent is administered serially or sequentially. The dosages given will depend on absorption, inactivation and excretion rates of the drug as well as other factors known to those of skill in the art. It is to be noted that dosage values will also vary with the severity of the pain or a pain-related disorder to be alleviated. It is to be further understood that for any particular subject, specific dosage regimens and schedules should be adjusted over time according to the individual need and the professional judgment of the person administering or supervising the administration of the compositions.

### EXAMPLES

As used herein, the symbols and conventions used in these processes, schemes and examples, regardless of whether a particular abbreviation is specifically defined, are consistent with those used in the contemporary scientific literature, for example, the Journal of the American Chemical Society or the Journal of Biological Chemistry. Specifically, but without limitation, the following abbreviations may be used in the examples and throughout the specification: g (grams); mg (milligrams); mL (milliliters); µL (microliters); mM (millimolar); µM (micromolar); Hz (Hertz); MHz (megahertz); mmol (millimoles); hr or hrs (hours); min (minutes); MS (mass spectrometry); ESI (electrospray ionization); Ph (phenyl); TLC (thin layer chromatography); HPLC (high pressure liquid chromatography); THF (tetrahydrofuran); CDCl₃ (deuterated chloroform); AcOH (acetic acid); DCM (dichloromethane); DMSO (dimethylsulfoxide); DMSO-*d₆* (deuterated dimethylsulfoxide); EtOAc (ethyl acetate); MeOH (methanol); Tces (2,2,2-trichloroethoxysulfonyl); -Si(*tert-*Bu)(Ph)₂ and -Si^{t}BuPh₂ (tert-butyl-diphenylsilyl); and BOC (t-butyloxycarbonyl). AZADO refers to 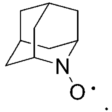 CDI refers to

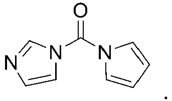

For all of the following examples, standard work-up and purification methods known to those skilled in the art can be utilized. Unless otherwise indicated, all temperatures are expressed in ° C (degrees Celsius). All reactions are conducted at room temperature unless otherwise noted. Synthetic methodologies illustrated herein are intended to exemplify the applicable chemistry through the use of specific examples and are not indicative of the scope of the disclosure.

### Synthetic Example 1

### Preparation of Non-hydrated Ketone Inhibitors of Nav1.7

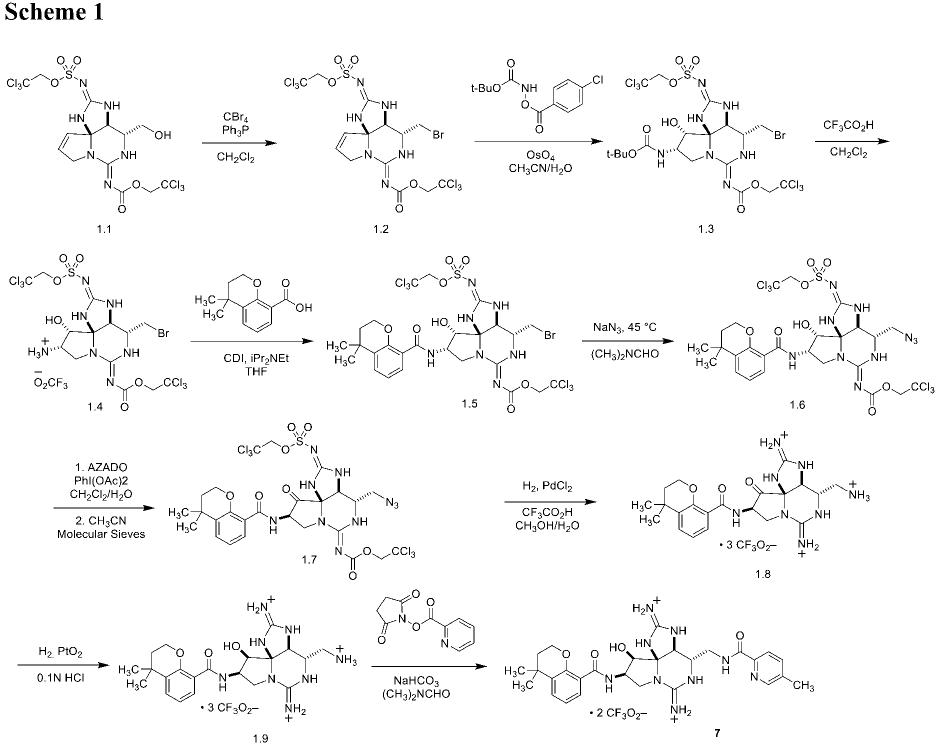

Compound 7 was prepared as shown in Scheme 1.

To a solution of alcohol **1.1** (3.36 g, 5.55 mmol, 1.0 equiv) in 170 mL of dichloromethane at room temperature was added triphenylphosphine (2.91 g, 11.1 mmol, 2.0 equiv). The resulting solution was cooled to 0 °C and carbon tetrabromide (3.68 g, 11.1 mmol, 2.0 equiv) was added in a portion wise manner. After an additional 15 minutes, the ice bath was removed, and the reaction was heated to 32 °C. After 16 hours at this temperature, the reaction was diluted with 170 mL of dichloromethane. The organic layer was washed with 2 x 150 mL of water, 100 mL of brine, dried over Na₂SO₄ and concentrated under reduced pressure. The crude material was purified by column chromatography (gradient elution: 35:65-70:30 EtOAc/hexanes) to obtain the bromide **1.2** as a white solid (2.83 g, 4.24 mmol, 77%).

To a solution of tert-butyl 4-chlorobenzoyloxycarbamate (3.5 g, 12.9 mmol, 3.0 equiv) in 50 mL of acetonitrile was added OsO₄ (1.77 mL of a 4% aqueous solution, 0.28 mmol, 0.065 equiv). After 30 minutes, a solution of **1.2** (2.88 g, 4.29 mmol, 1.0 equiv) in 30 mL of acetonitrile was added followed immediately by the addition of 8 mL of H₂O. The resulting mixture was stirred at 30 °C for 2 days and quenched by the addition of 50 mL of saturated aqueous Na₂S₂O₃ solution. The mixture was stirred for an additional 30 minutes then diluted with H₂O (100 mL) and extracted with 3 x 200 mL of EtOAc. The combined organic layers were washed with 100 mL of brine, dried with Na₂SO₄ and concentrated *in vacuo.* Purification of the residue by column chromatography on silica gel (gradient elution: 35:65→60:40 EtOAc/hexanes) afforded the carbamate **1.3** as a white solid (2.76 g, 3.45 mmol, 80%).

To a solution of **1.3** (2.76 g, 3.45 mmol) in 140 mL of dichloromethane was added trifluoroacetic acid (15 mL). The resulting solution was stirred at room temperature for 4 hours and concentrated *in vacuo* to afford the trifluoroacetate salt **1.4** that was used in the following step without further purification.

To a solution of 4,4-dimethylchromane-8-carboxylic acid (1.07 g, 5.18 mmol, 1.5 equiv) in 20 mL of THF at room temperature was added carbonyldiimidazole (0.84 g, 5.18 mmol, 1.5 equiv). After stirring of the reaction for 16 hours at room temperature, a solution of **1.4** (2.42 g, 3.45 mmol, 1.0 equiv) in 15 mL of THF was added. The reaction mixture was allowed to stir overnight. Following this time, *i*Pr₂NEt (2.41 mL, 13.8 mmol, 4.0 equiv) was added. The resulting mixture was stirred at room temperature for 16 hours and quenched by addition of 30 mL 1N HCl. The aqueous layer was extracted with 3 x 100 mL of EtOAc. The combined organic layer was dried with Na₂SO₄ and concentrated *in vacuo.* The residue was purified by a flash column (gradient elution: 45:55→75:25 EtOAc/hexanes) to obtain the desired amide **1.5** (2.22 g, 2.5 mmol, 72%) as a white solid.

To a solution of bromide **1.5** (1.81 g, 2.04 mmol, 1.0 equiv) in 20 mL of dimethylformamide at room temperature was added sodium azide (0.66 g, 10.2 mmol, 5.0 equiv) and the resulting solution was heated at 45 °C for 2 days. The reaction was cooled to room temperature and diluted with 200 mL of EtOAc. The resulting solution was washed with 3 x 100 mL of H₂O and 2 x 100 mL of brine. The organic layer was dried with Na₂SO₄, filtered, concentrated *in vacuo* and purified by column chromatography (gradient elution: 50:50→75:25 EtOAc/hexanes) to obtain the desired azide **1.6** (1.35 g, 1.58 mmol, 78%) as a pale yellow solid.

To a stirred solution of compound **1.6** (1.32 g, 1.55 mmol, 1.0 equiv) in 58 mL of dichloromethane were added sequentially water (3 mL) and iodobenzene diacetate (750 mg, 2.32 mmol, 1.5 equiv). The resulting solution was stirred for 10 minutes. To this reaction mixture was added a solution of 2-azaadamantane N-oxyl (AZADO) (71 mg, 0.46 mmol, 0.3 equiv) in dichloromethane (15 mL) dropwise over a period of 7 minutes. The biphasic mixture was stirred vigorously for an additional 40 minutes. The reaction was quenched with 10 mL of isopropyl alcohol and stirred for 45 minutes. The mixture was dried with MgSO₄, filtered and the MgSO₄ was washed with a small portion of dichloromethane. Hexane (680 mL) was gradually added to the filtrate and the contents were gently stirred at room temperature for 45 minutes. The resultant precipitate was filtered off to afford 1.2 g of an off-white solid. The material was dissolved in acetonitrile (70 mL). The solution was dried using 4 Å molecular sieves and stirred overnight at room temperature. The solution layer was filtered, concentrated *in vacuo* and the residue was purified by reversed-phase chromatography (Biotage SNAP Ultra C18 60 g, 25 µM column, eluting with gradient flow from 50:50→90:10 MeCN/H₂O with 10 mM aqueous CF₃CO₂H over 15 min, 214 nm UV detection). Fractions containing the desired product were lyophilized to afford ketone **1.7** as a white solid (790 mg).

Trifluoroacetic acid (7 mL) and PdCl₂ (140 mg) were added to a solution of intermediate **1.7** (720 mg, 0.84 mmol) in 50 mL of 4:1 MeOH/H₂O. H₂ gas was bubbled through the reaction mixture for 20 minutes, after which time bubbling was ceased and the reaction was stirred under an atmosphere of H₂ for 8 hours. The reaction mixture was filtered through a 0.2 µm PTFE syringe filter. The flask and filters were washed with 10 mL of MeOH and the filtrate was concentrated under reduced pressure without heating to afford **1.8.** The residue was dissolved in 20 mL of 0.1N HCl, filtered through a 0.2 µm PTFE syringe filter and used without any further purification in the next step.

A flask was charged with 100 mg of PtO₂ (100 mg) and 20 mL of 0.1N HCl was added. The system was sealed with a septum and flushed with H₂. After 5 minutes the mixture was observed to turn black and the pre-filtered solution of **1.8** in 20 mL of 0.1N HCl was added by syringe. An additional 4 mL of 0.1N HCl was used to rinse the syringe and added to the reaction mixture. The mixture stirred under an atmosphere of H₂ for two days. The reaction mixture was filtered through a 0.2 µm PTFE syringe filter and the filter was washed with H₂O. The filtrate was concentrated under reduced pressure, dissolved in 20 mL of aqueous 10 mM trifluoroacetic acid and purified by reversed-phase HPLC (Bonna-Agela Durashell C18, 10 µM, 21 x 250 mm column, eluting with gradient flow from 12:88→20:80% MeCN/H₂O with 10 mM trifluoroacetic acid over 30 minutes, 214 nm UV detection). At a flow rate of 20 mL/min, the tris-trifluoroacetate salt of intermediate **1.9** had a retention time of 15-19 min and was isolated as a white solid (280 mg, 0.36 mmol, 42%).

To a solution of **1.9** (30 mg, 0.038 mmol, 1.0 equiv) in 0.1 M sodium bicarbonate (1.1 mL) was added a solution of 2,5-dioxopyrrolidin-1-yl-5-methylpyridine-2-carboxylic acid (13.4 mg, 0.057 mmol, 1.5 equiv) in 0.6 mL of dimethylformamide. After stirring for 30 min, the reaction was quenched by addition of 10 mM aqueous trifluoroacetic acid (3 mL) and purified by reversed-phase HPLC (Bonna-Agela Durashell C18, 10 µM, 21x 250 mm column, eluting with gradient flow from 17:83→25:75 MeCN/H₂O with 10 mM trifluoroacetic acid over 30 minutes, 214 nm UV detection). At a flow rate of 20 mL/min, the bis-trifluoroacetate salt of Compound **7** had a retention time of 21-27 min and was isolated as a white solid (20 mg, 0.025 mmol, 66%).

¹H NMR (400 MHz, D₂O) : δ 8.14-8.06 (m, 2H), 7.91 (dd, *J =* 7.8, 1.7 Hz, 1H), 7.81 (dd, *J* = 7.8, 1.7 Hz, 1H), 7.24 (d, *J =* 7.8 Hz, 1H), 4.94 (s, 1H), 4.91 (d, *J =* 8.1 Hz, 1H), 4.73 (ddd, *J* = 10.5, 6.6, 3.5 Hz, 1H), 4.56-4.53 (m, 2H), 4.30 (dd, *J* = 11.5, 7.7 Hz, 1H), 4.02 (t, *J* = 7.3 Hz, 1H), 3.90 (dd, *J* = 11.4, 4.5 Hz, 1H), 3.82-3.80 (m, 2H), 2.58 (s, 3H), 2.08 (dd, *J =* 6.7, 4.3 Hz, 2H), 1.51 (s, 3H), 1.50 (s, 3H); MS (ES+, m/z) calcd for C₂₈H₃₆N₉O₄⁺: 562.29; found: 562.30 (MH)⁺.

The following compounds were prepared using a procedure similar to the procedure in Scheme 1 above:

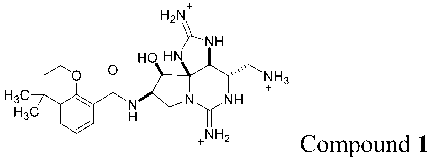

¹H-NMR (400 MHz; D₂O): δ 7.74 (ddd, *J* = 19.3, 7.8, 1.7 Hz, 2H), 7.14 (t, *J* = 7.8 Hz, 1H), 4.92 (d, *J =* 1.0 Hz, 1H), 4.68 (m, 2H), 4.45-4.42 (m, 2H), 4.32-4.27 (m, 1H), 4.09 (dd, *J* = 11.9, 3.1 Hz, 1H), 3.80 (dd, *J=* 11.3, 3.5 Hz, 1H), 3.44 (dd, *J* = 13.4, 3.7 Hz, 1H), 3.31 (dd, *J* = 13.5, 11.3 Hz, 1H), 1.99-1.96 (m, 2H), 1.40 (s, 3H), 1.39 (s, 3H); MS (ES+, m/z) calcd for C₂₁H₃₁N₈O₃⁺: 443.25; found: 443.20 (MH⁺).

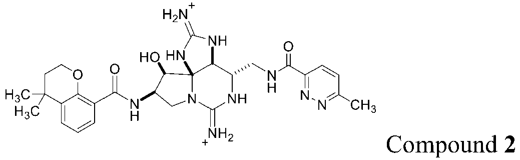

¹H NMR (400 MHz, D₂O) : δ 8.34 (d, *J =* 8.7 Hz, 1H), 8.02 (d, *J* = 8.8 Hz, 1H), 7.92 (dd, *J* = 7.8, 1.7 Hz, 1H), 7.82 (dd, *J* = 7.8, 1.7 Hz, 1H), 7.25 (t, *J* = 7.8 Hz, 1H), 4.97 (s, 1H), 4.93 (d, *J* = 8.0 Hz, 1H), 4.57-4.54 (m, 2H), 4.34 (dd, *J* = 11.4, 7.7 Hz, 1H), 4.06 (t, *J* = 7.44 Hz, 1H), 3.93 (dd, *J* = 11.4, 4.4 Hz, 1H), 3.87 (d, *J* = 7.3 Hz, 2H), 2.92 (s, 3H), 2.09 (dd, *J* = 6.8, 4.5 Hz, 2H), 2.23-2.16 (m, 2H), 1.52 (s, 3H), 1.51 (s, 3H); MS (ES+, m/z) calcd for C₂₇H₃₅N₁₀O₄⁺: 563.28; found: 563.20 (MH⁺).

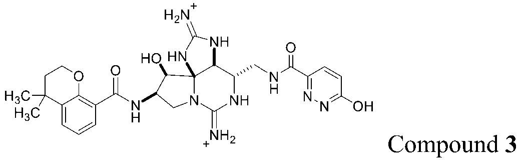

¹H NMR (400 MHz, D₂O) : δ 8.17 (d, *J =* 9.9 Hz, 1H), 7.90 (dd, *J =* 7.8, 1.7 Hz, 1H), 7.81 (dd, *J* = 7.8, 1.7 Hz, 1H), 7.32 (d, *J* = 9.9 Hz, 1H), 7.24 (t, *J* = 7.8 Hz, 1H), 4.92-4.86 (m, 2H), 4.78-4.72 (m, 1H), 4.54 (td, *J* = 5.3, 1.9 Hz, 2H), 4.34 (dd, *J* = 11.5, 7.7 Hz, 1H), 3.97 (t, *J* = 7.4 Hz, 1H), 3.91 (dd, *J* = 11.4, 4.5 Hz, 1H), 3.76 (dd, *J* = 7.4, 2.6 Hz, 2H), 2.07 (dd, *J* = 6.7, 4.5 Hz, 2H), 1.50 (s, 3H), 1.49 (s, 3H); MS (ES+, m/z) calcd for C₂₆H₃₃N₁₀O₅⁺: 565.26; found: 565.20 (MH⁺).

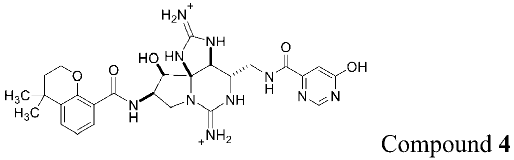

¹H NMR (400 MHz, D₂O) : δ 8.50 (s, 1H), 7.91 (dd, *J* = 7.6, 1.6 Hz, 1H), 7.81 (dd, *J =* 7.6, 1.6 Hz, 1H), 7.26-7.21 (m, 2H), 4.92 (s, 1H), 4.89 (d, *J =* 8.4 Hz, 1H), 4.79-4.72 (m, 1H), 4.55-4.52 (m, 2H), 4.33 (dd, *J =* 11.4, 7.7 Hz, 1H), 3.99 (t, *J =* 7.4 Hz, 1H), 3.91 (dd, *J* = 11.3, 4.4 Hz, 1H), 3.79-3.77 (m, 2H), 2.07 (dd, *J =* 6.6, 4.3 Hz, 2H), 1.50 (s, 3H), 1.49 (s, 3H); MS (ES+, m/z) calcd for C₂₆H₃₃N₁₀O₅⁺: 565.26; found: 565.20 (MH⁺).

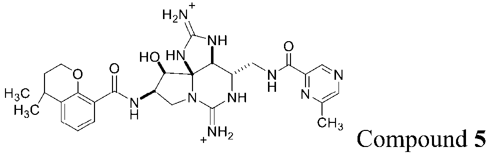

¹H NMR (400 MHz, D₂O) : δ 9.10 (s, 1H), 8.85 (s, 1H), 7.89 (dd, *J =* 7.8, 1.7 Hz, 1H), 7.80 (dd, *J* = 7.8, 1.8 Hz, 1H), 7.23 (t, *J* = 7.8 Hz, 1H), 4.94 (d, *J* = 0.8 Hz, 1H), 4.91 (d, *J* = 8.4 Hz, 1H), 4.77-4.70 (m, 1H), 4.53 (ddd, *J* = 6.2, 4.5, 2.0 Hz, 2H), 4.32 (dd, *J* = 11.5, 7.7 Hz, 1H), 4.03-4.00 (m, 1H), 3.91 (dd, *J =* 11.4, 4.5 Hz, 1H), 3.82 (d, *J =* 7.5 Hz, 2H), 2.78 (s, 3H), 2.13-2.01 (m, 2H), 1.50 (s, 3H), 1.49 (s, 3H); MS (ES+, m/z) calcd for C₂₇H₃₅N₁₀O₄⁺: 563.28; found: 563.20 (MH⁺).

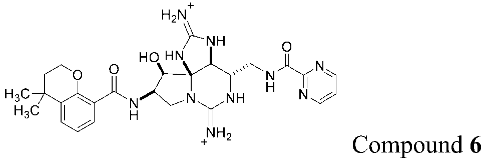

¹H NMR (400 MHz, D₂O) : δ 9.10 (d, *J* = 5.0 Hz, 2H), 7.91 (dd, *J* = 7.8, 1.6 Hz, 1H), 7.86 (t, *J* = 5.0 Hz, 1H), 7.81 (dd, *J* = 7.8, 1.6 Hz, 1H), 7.24 (t, *J* = 7.8 Hz, 1H), 4.94 (s, 1H), 4.93 (d, *J =* 7.1 Hz, 1H), 4.77-4.73 (m, 1H), 4.56-4.52 (m, 1H), 4.36 (dd, *J =* 11.5, 7.7 Hz, 1H), 4.05-4.01 (m, 1H), 3.91 (dd, *J =* 11.4, 4.4 Hz, 1H), 3.88-3.77 (m, 2H), 2.07 (dd, *J* = 6.5, 4.2 Hz, 2H), 1.50 (s, 3H), 1.49 (s, 3H); MS (ES+, m/z) calcd for C₂₆H₃₃N₁₀O₄⁺: 549.27; found: 549.30 (MH⁺).

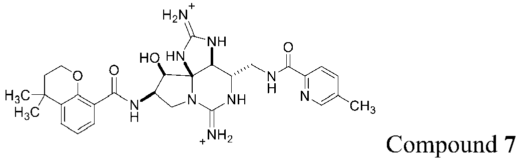

¹H NMR (400 MHz, D₂O) : δ 8.14-8.06 (m, 2H), 7.91 (dd, *J =* 7.8, 1.7 Hz, 1H), 7.81 (dd, *J =* 7.8, 1.7 Hz, 1H), 7.24 (d, *J =* 7.8 Hz, 1H), 4.94 (s, 1H), 4.91 (d, *J =* 8.1 Hz, 1H), 4.73 (ddd, *J* = 10.5, 6.6, 3.5 Hz, 1H), 4.56-4.53 (m, 2H), 4.30 (dd, *J* = 11.5, 7.7 Hz, 1H), 4.02 (t, *J =* 7.3 Hz, 1H), 3.90 (dd, *J =* 11.4, 4.5 Hz, 1H), 3.82-3.80 (m, 2H), 2.58 (s, 3H), 2.08 (dd, *J =* 6.7, 4.3 Hz, 2H), 1.51 (s, 3H), 1.50 (s, 3H); MS (ES+, m/z) calcd for C₂₈H₃₆N₉O₄⁺: 562.29; found: 562.30 (MH⁺).

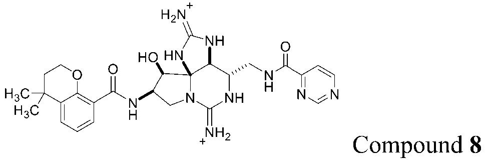

¹H NMR (400 MHz, D₂O) : δ 9.49 (d, *J* = 1.3 Hz, 1H), 9.26 (d, *J =* 5.2 Hz, 1H), 8.29 (dd, *J* = 5.2, 1.4 Hz, 1H), 7.97 (dd, *J* = 7.8, 1.7 Hz, 1H), 7.87 (dd, *J* = 7.8, 1.7 Hz, 1H), 7.30 (t, *J =* 7.8 Hz, 1H), 5.00 (s, 1H), 4.97 (d, *J =* 8.0 Hz, 1H), 4.86-4.80 (m, 1H), 4.62-4.59 (m, 2H), 4.41 (dd, *J* = 11.4, 7.8 Hz, 1H), 4.09 (t, *J =* 7.3 Hz, 1H), 3.98 (dd, *J =* 11.5, 4.4 Hz, 1H), 2.20-2.07 (m, 2H), 2.14 (dd, *J* = 6.8, 4.6 Hz, 2H), 1.57 (s, 3H), 1.56 (s, 3H); MS (ES+, m/z) calcd for C₂₆H₃₃N₁₀O₄⁺: 549.27; found: 549.30 (MH⁺).

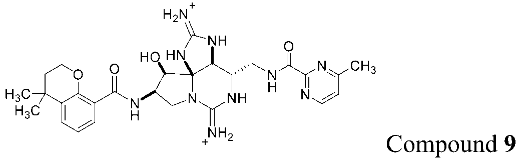

¹H NMR (400 MHz, D₂O) : δ 8.94 (d, *J* = 5.2 Hz, 1H), 7.95 (dd, *J* = 7.8, 1.7 Hz, 1H), 7.85 (dd, *J* = 7.8, 1.7 Hz, 2H), 7.77 (d, *J =* 5.3 Hz, 1H), 7.28 (t, *J =* 7.8 Hz, 1H), 5.02-4.94 (m, 2H), 4.82-4.75 (m, 1H), 4.64-4.49 (m, 2H), 4.39 (dd, *J =* 11.5, 7.8 Hz, 1H), 4.08-4.04 (m, 1H), 3.95 (dd, *J =* 11.6, 4.4 Hz, 1H), 3.91-3.78 (m, 2H), 2.80 (s, 3H), 2.10 (dd, *J =* 6.6, 4.3 Hz, 2H), 1.54 (s, 3H), 1.53 (s, 3H); MS (ES+, m/z) calcd for C₂₇H₃₅N₁₀O₄⁺: 563.28; found: 563.20 (MH⁺).

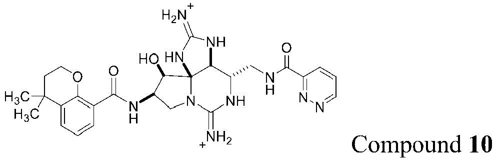

¹H NMR (400 MHz, D₂O): δ 9.49 (dd, *J =* 5.1, 1.5 Hz, 1H), 8.44 (dd, *J* = 8.5, 1.6 Hz, 1H), 8.13 (dd, *J* = 8.6, 5.0 Hz, 1H), 7.90 (dd, *J =* 7.8, 1.5 Hz, 1H), 7.80 (dd, *J* = 7.7, 1.5 Hz, 1H), 7.23 (t, *J* = 7.7 Hz, 1H), 4.96 (s, 1H), 4.91 (d, *J* = 3.9 Hz, 1H), 4.77-4.70 (m, 1H), 4.55-4.52 (m, 2H), 4.33 (dd, *J* = 11.6, 7.7 Hz, 1H), 4.05 (t, *J* = 7.4 Hz, 1H); MS (ES+, m/z) calcd for C₂₆H₃₃N₁₀O₄⁺: 549.27; found: 549.30 (MH⁺).

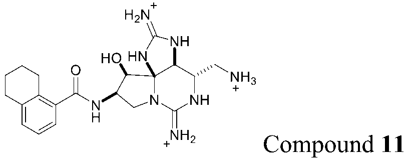

¹H NMR (400 MHz, D₂O) : δ 7.44-7.23 (m, 3H), 4.99-4.91 (m, 1H), 4.72-4.62 (m, 1H), 4.39-4.26 (m, 1H), 4.17-4.06 (m, 1H), 3.85-3.71 (m, 1H), 3.53-3.42 (m, 1H), 3.42-3.27 (m, 1H), 3.02-2.79 (m, 4H), 1.99-1.77 (m, 4H); MS (ES+, m/z) calcd for C₂₀H₂₉N₈O₂⁺: 413.24; found: 413.20 (MH⁺).

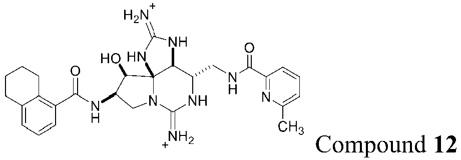

¹H NMR (400 MHz, D₂O): δ 8.14 (t, *J =* 7.8 Hz, 1H), 8.06 (t, *J* = 9.3 Hz, 1H), 7.74 (d, *J =* 7.7 Hz, 1H), 7.47-7.33 (m, 3H), 4.96 (s, 1H), 4.92-4.84 (m, 2H), 4.33-4.23 (m, 1H), 4.04 (t, *J* = 7.1 Hz, 1H), 3.87-3.83 (m, 3H), 3.04-2.89 (m, 4H), 2.78 (s, 3H), 1.94 (dt, *J* = 6.4, 3.2 Hz, 4H; MS (ES+, m/z) calcd for C₂₇H₃₄N₉O₃⁺: 532.28; found: 532.30 (MH⁺).

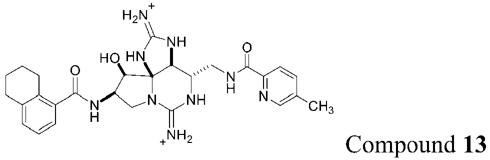

¹H NMR (400 MHz, D₂O) : δ 8.69 (s, 1H), 8.14 (s, 1H), 8.08 (t, *J* = 8.0 Hz, 1H), 7.47-7.38 (m, 3H), 4.96 (s, 1H), 4.92-4.88 (m, 2H), 4.32-4.28 (m, 1H), 4.05 (t, *J =* 7.0, 1H), 3.88-3.84 (m, 3H), 3.05-2.87 (m, 4H), 2.61 (s, 3H), 1.96 (dt, *J =* 6.3, 3.1 Hz, 4H); MS (ES+, m/z) calcd for C₂₇H₃₄N₉O₃⁺: 532.28; found: 532.30 (MH⁺).

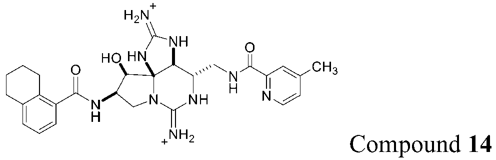

¹H NMR (400 MHz, D₂O) : δ 8.73 (d, *J* = 5.3 Hz, 1H), 8.18 (s, 1H), 7.83 (dd, *J* = 5.3, 0.7 Hz, 1H), 7.45-7.36 (m, 3H), 5.02-4.85 (m, 3H), 4.33 (dd, *J* = 11.2, 8.1 Hz, 1H), 4.04 (t, *J* = 7.1, 1H), 3.89-3.81 (m, 3H), 3.05-3.29 (m, 4H), 2.70 (s, 3H), 1.93 (t, *J =* 3.0 Hz, 4H); MS (ES+, m/z) calcd for C₂₇H₃₄N₉O₃⁺: 532.28; found: 532.30 (MH⁺).

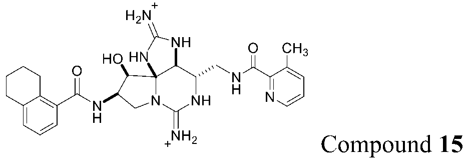

¹H NMR (400 MHz, D₂O) : δ 8.60 (dd, *J* = 4.8, 0.7 Hz, 1H), 8.05 (dt, *J* = 7.9, 0.7 Hz, 1H), 7.71 (dd, *J* = 7.9, 4.7 Hz, 1H), 7.46-7.35 (m, 4H), 4.97-4.85 (m, 3H), 4.37 (dd, *J* = 11.3, 8.4, 1H), 4.02 (dd, J = 9.1, 6.0 Hz, 1H), 3.90-3.73 (m, 3H), 2.99-2.91 (m, 4H), 2.63 (s, 3H), 1.96-1.90 (m, 4H); MS (ES+, m/z) calcd for C₂₇H₃₄N₉O₃⁺: 532.28; found: 532.30 (MH⁺).

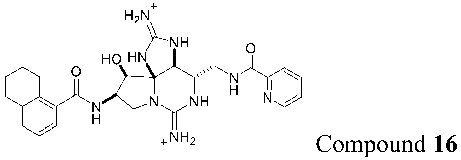

¹H NMR (400 MHz, D₂O) : δ 8.82 (dt, *J* = 4.8, 1.2 Hz, 1H), 8.22 (dd, *J* = 4.8, 1.2 Hz, 1H), 7.86-7.81 (m, 1H), 7.44-7.35 (m, 3H), 4.94-4.85 (m, 3H), 4.33-4.28 (m, 1H), 4.03 (t, *J* = 7.3, 1H), 3.88-3.81(m, 3H), 2.98-2.90 (m, 4H), 1.94-1.91 (m, 4H); MS (ES+, m/z) calcd for C₂₆H₃₂N₉O₃+: 518.26; found: 518.20 (MH⁺).

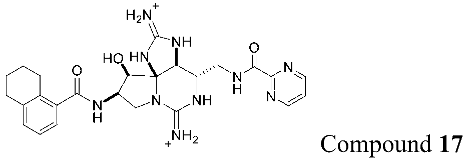

¹H NMR (400 MHz, D₂O) : δ 9.13 (d, *J* = 5.0 Hz, 2H), 7.89 (d, *J* = 5.0 Hz, 1H), 7.45-7.36 (m, 3H), 5.01-4.87 (m, 3H), 4.43-4.33 (m, 1H), 4.10-4.01 (m, 1H), 3.95-3.77 (m, 3H), 3.05-2.90 (m, 4H), 1.99-1.90 (m, 4H); MS (ES+, m/z) calcd for C₂₅H₃₁N₁₀O₃⁺: 519.26; found: 519.30 (MH⁺).

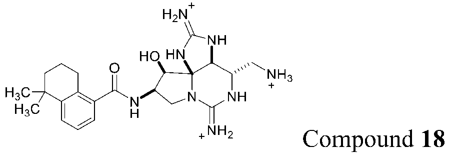

¹H NMR (400 MHz, D₂O) : δ 7.83 (dd, *J =* 7.8, 1.2 Hz, 1H), 7.54-7.46 (m, 2H), 5.03 (s, 1H), 4.95 (td, *J* = 8.6, 6.2 Hz, 1H), 4.79-4.74 (m, 1H), 4.42 (dd, *J* = 11.4, 8.7 Hz, 1H), 4.21 (dd, *J* = 11.3, 3.4 Hz, 1H), 3.89 (dd, *J* = 11.5, 6.1 Hz, 1H), 3.57 (dd, *J* = 13.5, 3.8 Hz, 1H), 3.45 (dd, *J =* 13.4, 11.3 Hz, 1H), 3.02 (dd, *J =* 7.9, 4.8 Hz, 2H), 2.09-1.98 (m, 2H), 1.97-1.88 (m, 2H), 1.53 (s, 6H); MS (ES+, m/z) calcd for C₂₂H₃₃N₈O₂⁺: 441.27; found: 441.30 (MH⁺).

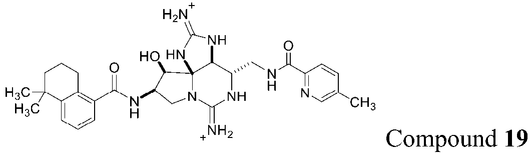

¹H NMR (400 MHz, D₂O) : δ 8.74 (s, 1H), 8.18-8.12 (m, 2H), 7.82 (dd, *J* = 7.7, 1.1 Hz, 1H), 7.53-7.45 (m, 2H), 4.99 (s, 1H), 4.95-4.90 (m, 2H), 4.36-4.31 (m, 1H), 4.11-4.07 (m, 1H), 3.93-3.87 (m, 3H), 3.02-2.99 (m, 2H), 2.66 (s, 3H), 2.05-1.98 (m, 2H), 1.92 (dt, *J* = 5.7, 2.7 Hz, 2H), 1.53 (s, 6H); MS (ES+, m/z) calcd for C₂₉H₃₈N₉O₃⁺: 560.31; found: 560.30 (MH⁺).

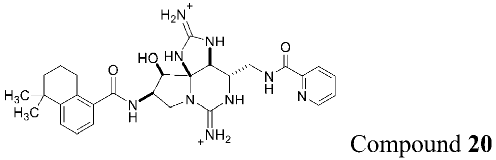

¹H NMR (400 MHz, D₂O) : δ 8.93-8.90 (m, 1H), 8.34-8.26 (m, 2H), 7.94-7.90 (m, 1H), 7.85-7.81 (m, 1H), 7.56-7.46 (m, 2H), 5.02-4.94 (m, 3H), 4.42-4.35 (m, 1H), 4.14-4.08 (m, 1H), 3.96-3.88 (m, 3H), 3.06-3.00 (m, 2H), 2.08-1.99 (m, 2H), 1.96-1.91 (m, 2H), 1.54 (s, 6H); MS (ES+, m/z) calcd for C₂₈H₃₆N₉O₃⁺: 546.29; found: 546.30 (MH⁺).

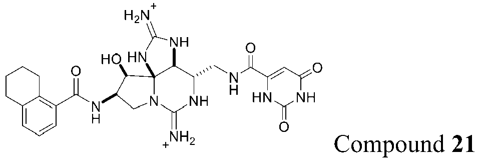

¹H NMR (400 MHz, D₂O) : δ 7.44-7.34 (m, 3H), 6.37 (s, 1H), 4.97-4.84 (m, 3H), 4.38 (dd, *J* = 11.4, 8.5, 1H), 3.97-3.93(m, 1H), 3.85 (dd, *J* = 11.4, 5.7 Hz, 1H), 3.79-3.65 (m, 2H), 2.98-2.89 (m, 4H), 1.96-1.88 (m, 4H); MS (ES+, m/z) calcd for C₂₅H₃₁N₁₀O₅⁺: 551.25; found: 551.20 (MH⁺).

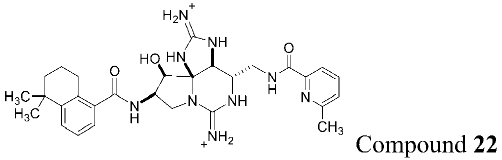

¹H NMR (400 MHz, D₂O) : δ 8.20-8.10 (m, 2H), 7.83-7.77 (m, 2H),7.54-7.45 (m, 2H), 5.00 (s, 1H), 4.96-4.90 (m, 2H), 4.37-4.32 (m, 1H), 4.10 (t, *J* = 7.0 Hz, 1H), 3.91 (dd, *J =* 12.3, 6.0 Hz, 3H), 3.02-2.99 (m, 2H), 2.84 (s, 3H), 2.04-1.99 (m, 2H), 1.93-1.91 (m, 2H), 1.53 (s, 6H); MS (ES+, m/z) calcd for C₂₉H₃₈N₉O₃⁺: 560.31; found: 560.30 (MH⁺).

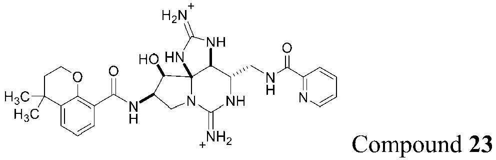

¹H NMR (400 MHz, D₂O) : δ 8.85 (dd, *J* = 4.9, 1.1 Hz, 1H), 8.29-8.24 (m, 2H), 7.94 (dd, *J* = 7.8, 1.7 Hz, 1H), 7.89-7.82 (m, 2H), 7.27 (t, *J =* 7.8 Hz, 1H), 4.97 (s, 1H), 4.94 (d, *J* = 8.0 Hz, 1H), 4.78-4.72 (m, 1H), 4.59-4.55 (m, 2H), 4.34 (dd, *J* = 11.4, 7.7 Hz, 1H), 4.05 (t, *J* = 7.3 Hz, 1H), 3.94 (dd, *J =* 11.5, 4.4 Hz, 1H), 3.85 (d, *J =* 7.0 Hz, 2H), 2.11 (dd, *J* = 6.7, 4.4 Hz, 2H), 1.54 (s, 3H), 1.53 (s, 3H); MS (ES+, m/z) calcd for C₂₇H₃₄N₉O₄⁺: 548.27; found: 548.30 (MH⁺).

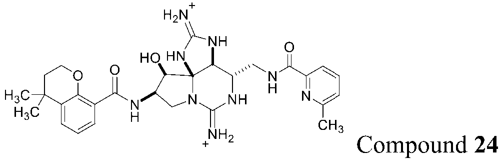

¹H NMR (400 MHz, D₂O) : δ 8.16 (t, *J* = 7.80 Hz, 1H), 8.06 (s, 1H), 7.91 (dd, *J* = 7.8, 1.7 Hz, 1H), 7.81 (dd, *J* = 7.8, 1.7 Hz, 1H), 7.76 (d, *J* = 7.8 Hz, 1H), 7.24 (t, *J =* 7.8 Hz, 1H), 4.95 (s, 1H), 4.92 (d, *J =* 8.0 Hz, 1H), 4.75-4.70 (m, 1H), 4.54 (td, *J =* 5.3, 2.1 Hz, 2H), 4.30 (dd, *J =* 11.5, 7.7 Hz, 1H), 4.03 (t, *J* = 7.2 Hz, 1H), 3.91 (dd, *J* = 11.4, 4.4 Hz, 1H), 3.82 (d, *J =* 7.3 Hz, 2H), 2.78 (s, 3H), 2.08 (dd, *J =* 6.6, 4.3 Hz, 2H), 1.51 (s, 3H), 1.50 (s, 3H); MS (ES+, m/z) calcd for C₂₈H₃₆N₉O₄⁺: 562.29; found: 562.30 (MH⁺).

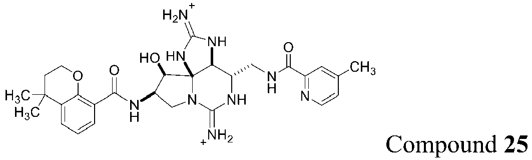

¹H NMR (400 MHz, D₂O) : δ 8.70 (d, *J* = 5.2 Hz, 1H), 8.14 (d, *J* = 0.7 Hz, 1H), 7.92 (dd, *J* = 7.8, 1.7 Hz, 1H), 7.82 (dd, *J* = 7.8, 1.7 Hz, 1H), 7.80-7.77 (m, 1H), 7.25 (t, *J* = 7.8 Hz, 1H), 4.95 (s, 1H), 4.92 (d, *J =* 8.0 Hz, 1H), 4.78-4.72 (m, 1H), 4.57-4.53 (m, 2H), 4.34 (dd, *J =* 11.4, 7.7 Hz, 1H), 4.03 (t, *J =* 7.4 Hz, 1H), 3.94-3.90 (m, 1H), 3.83 (d, *J* = 7.1 Hz, 2H), 2.68 (s, 3H), 2.09 (dd, *J* = 6.5, 4.1 Hz, 2H), 1.52 (s, 3H), 1.51 (s, 3H); MS (ES+, m/z) calcd for C₂₈H₃₆N₉O₄⁺: 562.29; found: 562.30 (MH⁺).

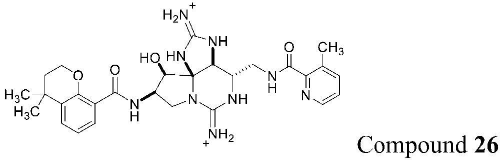

¹H NMR (400 MHz, D₂O) : δ 8.59 (d, *J =* 4.5 Hz, 1H), 8.06 (d, *J =* 7.8 Hz, 1H), 7.90 (dd, *J* = 7.8, 1.7 Hz, 1H), 7.80 (dd, *J* = 7.8, 1.7 Hz, 1H), 7.71 (dd, *J* = 7.9, 4.9 Hz, 1H), 7.23 (t, *J = 7.8* Hz, 1H), 4.94 (s, 1H), 4.90 (d, *J =* 8.0 Hz, 1H), 4.78-4.72 (m, 1H), 4.55-4.52 (m, 2H), 4.37 (dd, *J =* 11.5, 7.7 Hz, 1H), 4.01 (dd, *J* = 8.7, 6.7 Hz, 1H), 3.92 (dd, *J =* 11.5, 4.4 Hz, 1H), 3.80 (d, *J* = 8.3 Hz, 2H), 2.61 (s, 3H), 2.07 (dd, *J* = 6.4, 3.9 Hz, 2H), 1.50 (s, 3H), 1.49 (s, 3H); MS (ES+, m/z) calcd for C₂₈H₃₆N₉O₄⁺: 562.29; found: 562.30 (MH⁺).

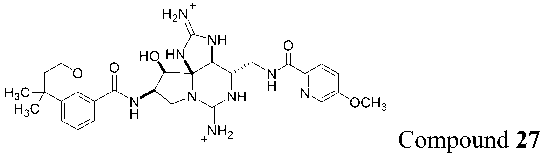

¹H NMR (400 MHz, D₂O) : δ 8.49 (d, *J* = 2.8 Hz, 1H), 8.20 (d, *J =* 8.8 Hz, 1H), 7.91 (dd, *J* = 7.8, 1.6 Hz, 1H), 7.82 (dd, *J* = 7.8, 1.7 Hz, 1H), 7.72 (dd, *J* = 8.8, 2.9 Hz, 1H), 7.25 (t, *J = 7.8* Hz, 1H), 4.95 (s, 1H), 4.90 (d, *J =* 2.3 Hz, 1H), 4.77-4.66 (m, 1H), 4.56-4.53 (m, 2H), 4.28 (dd, *J =* 11.4, 7.6 Hz, 1H), 4.11 (s, 3H), 4.02 (t, *J =* 7.1 Hz, 1H), 3.90 (dd, *J =* 11.6, 4.5 Hz, 1H), 3.82 (s, 1H), 3.80 (d, *J* = 3.1 H, 1H), 2.08 (dd, *J =* 6.8, 4.6 Hz, 2H), 1.51 (s, 3H), 1.50 (s, 3H); MS (ES+, m/z) calcd for C₂₈H₃₆N₉O₅⁺: 578.28; found: 578.30 (MH⁺).

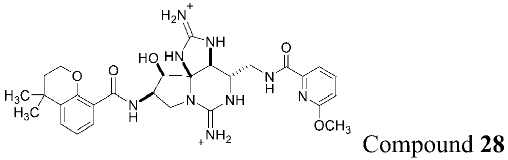

¹H NMR (400 MHz, D₂O): δ 8.23 (dd, *J* = 8.3, 7.4 Hz, 1H), 8.09 (dd, *J* = 7.8, 1.7 Hz, 1H), 8.01 (d, *J* = 6.8 Hz, 1H), 7.94 (dd, *J* = 7.8, 1.7 Hz, 1H), 7.41-7.37 (m, 2H), 5.05 (s, 1H), 5.04 (d, *J =* 8.8 Hz, 1H), 4.85-4.82 (m, 1H), 4.71-4.67 (m, 2H), 4.41 (dd, *J* = 11.4, 7.7 Hz, 1H), 4.31 (s, 3H), 4.15 (t, *J =* 7.1 Hz, 1H), 4.04 (dd, *J =* 11.6, 4.6 Hz, 1H), 3.95 (s, 1H), 3.94 (d, *J* = 7.2 Hz, 1H), 2.23 (dd, *J =* 6.8, 4.1 Hz, 2H), 1.67 (s, 3H), 1.66 (s, 3H); MS (ES+, m/z) calcd for C₂₈H₃₆N₉O₅⁺: 578.28; found: 578.30 (MH⁺).

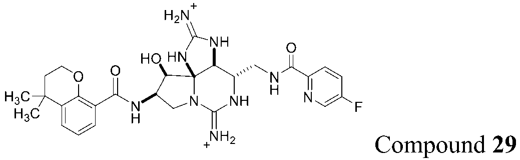

¹H NMR (400 MHz, D₂O) : δ 8.85 (d, *J* = 2.8 Hz, 1H), 8.43 (dd, *J* = 8.8, 4.4 Hz, 1H), 8.12-8.07 (m, 2H), 7.94 (dd, *J* = 7.8, 1.8 Hz, 1H), 7.38 (t, *J* = 7.8 Hz, 1H), 5.04 (d, *J* = 0.8 Hz, 1H), 5.03 (d, *J* = 8.1 Hz, 1H), 4.88-4.83 (m, 1H), 4.70-4.67 (m, 2H), 4.43 (dd, *J* = 11.5, 7.7 Hz, 1H), 4.14 (dd, *J* = 7.9, 6.2 Hz, 1H), 4.04 (dd, *J =* 11.5, 4.4 Hz, 1H), 3.98-3.88 (m, 2H), 2.22 (dd, *J* = 7.0, 4.3 Hz, 2H), 1.66 (s, 3H), 1.65 (s, 3H); MS (ES+, m/z) calcd for C₂₇H₃₃FN₉O₄⁺: 566.26; found: 566.20 (MH⁺).

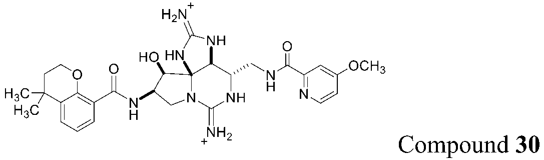

¹H NMR (400 MHz, D₂O) : δ 8.79 (d, *J* = 6.1 Hz, 1H), 8.05 (dd, *J* = 7.8, 1.7 Hz, 1H), 7.94 (d, *J* = 2.6 Hz, 1H), 7.90 (dd, *J* = 7.8, 1.7 Hz, 1H), 7.58 (dd, *J =* 6.1, 2.6 Hz, 1H), 7.34 (t, *J* = 7.8 Hz, 1H), 5.01 (s, 1H), 4.98 (d, *J =* 8.0 Hz, 1H), 4.86-4.82 (m, 1H), 4.65 (dt, *J =* 6.8, .5 Hz, 2H), 4.42 (dd, *J* = 11.4, 7.7 Hz, 1H), 4.27 (s, 3H), 4.10 (dd, *J* = 8.3, 6.6 Hz, 1H), 4.00 (dd, *J* = 11.5, 4.5 Hz, 1H), 3.95-3.85 (m, 2H), 2.18 (dd, *J* = 6.7, 4.1 Hz, 2H), 1.62 (s, 3H), 1.61 (s, 3H); MS (ES+, m/z) calcd for C₂₈H₃₆N₉O₅⁺: 578.28; found: 578.30 (MH⁺).

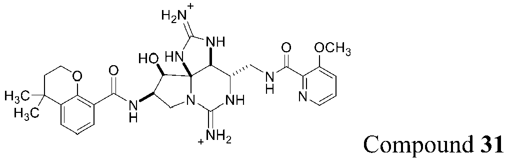

¹H NMR (400 MHz, D₂O) : δ 8.56 (dd, *J* = 4.7, 1.1 Hz, 1H), 8.10 (td, *J* = 8.8, 1.5 Hz, 2H), 8.01 (dd, *J =* 8.7, 4.7 Hz, 1H), 7.94 (dd, *J* = 7.8, 1.7 Hz, 1H), 7.38 (t, *J =* 7.8 Hz, 1H), 5.04 (s, 1H), 5.03 (d, *J =* 6.8 Hz, 1H), 4.89-4.84 (m, 1H), 4.68 (dt, *J =* 6.8, 3.6 Hz, 2H), 4.48 (dd, *J* = 11.4, 7.7 Hz, 1H), 4.28 (s, 3H), 4.13 (dd, *J* = 8.5, 6.5 Hz, 1H), 4.04 (dd, *J* = 11.5, 4.4 Hz, 1H), 3.97-3.87 (m, 2H), 2.22 (dd, *J =* 7.0, 4.3 Hz, 2H), 1.66 (s, 3H), 1.65 (s, 3H); MS (ES+, m/z) calcd for C₂₈H₃₆N₉O₅⁺: 578.28; found: 578.30 (MH⁺).

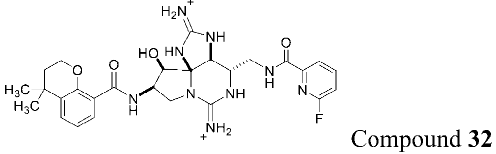

¹H NMR (400 MHz, D₂O) : δ 8.78 (d, *J* = 4.6 Hz, 1H), 8.13-8.08 (m, 2H), 8.02-7.98 (m, 1H), 7.93 (dd, *J* = 7.8, 1.7 Hz, 1H), 7.38 (t, *J* = 7.8 Hz, 1H), 5.04 (s, 1H), 5.03 (d, *J* = 8.1 Hz, 1H), 4.89-4.83 (m, 1H), 4.68 (dt, *J =* 6.6, 3.5 Hz, 2H), 4.47 (dd, *J =* 11.4, 7.7 Hz, 1H), 4.15-4.11 (m, 1H), 4.04 (dd, *J =* 11.5, 4.4 Hz, 1H), 3.93 (d, *J =* 2.2 Hz, 1H), 3.91 (d, *J* = 4.5 Hz, 1H), 2.22 (dd, *J =* 7.0, 4.2 Hz, 2H), 1.66 (s, 3H), 1.65 (s, 3H); MS (ES+, m/z) calcd for C₂₇H₃₃FN₉O₄⁺: 566.26; found: 566.30 (MH⁺).

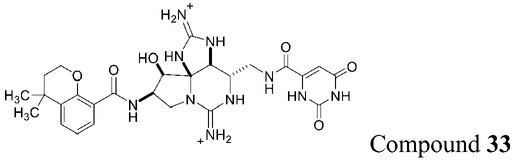

¹H NMR (400 MHz, D₂O) : δ 7.92 (dd, *J* = 7.8, 1.7 Hz, 1H), δ 7.81 (dd, *J* = 7.8, 1.7 Hz, 1H), 7.25 (t, *J* = 7.8 Hz, 1H), 6.37 (s, 1H), 4.90 (s, 1H), 4.86 (d, *J =* 8.0 Hz, 1H), 4.79-4.75 (m, 1H), 4.55 (td, *J* = 5.3, 2.2 Hz, 2H), 4.39 (dd, *J* = 11.4, 7.7 Hz, 1H), 3.98-3.90 (m, 2H), 3.73-3.71 (m, 2H), 2.08 (dd, *J* = 6.7, 4.3 Hz, 2H), 1.51 (s, 3H), 1.50 (s, 3H); MS (ES+, m/z) calcd for C₂₆H₃₃N₁₀O₆⁺: 581.26; found: 581.30 (MH⁺).

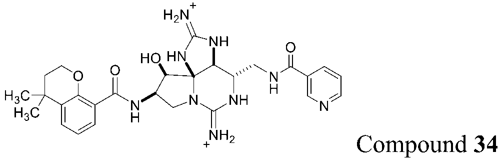

¹H-NMR (400 MHz; D₂O): δ 9.18 (d, *J* = 2.3 Hz, 1H), 8.97 (dd, *J* = 5.3, 1.4 Hz, 1H), 8.65 (dt, *J* = 8.1, 1.7 Hz, 1H), 8.00 (dd, *J* = 8.0, 5.3 Hz, 1H), 7.92 (dd, *J* = 7.9, 1.6 Hz, 1H), 7.82 (dd, *J =* 7.9, 1.7 Hz, 1H), 7.25 (t, *J =* 7.8 Hz, 1H), 4.95 (s, 1H), 4.91 (d, *J =* 4.6 Hz, 1H), 4.89 (s, 1H), 4.57-4.54 (m, 2H), 4.39 (dd, *J* = 11.4, 7.9 Hz, 1H), 4.01 (dd, *J =* 9.0, 6.7 Hz, 1H), 3.93 (dd, *J =* 11.4, 4.6 Hz, 1H), 3.85-3.76 (m, 2H), 2.09 (dd, *J =* 7.1, 4.5 Hz, 2H), 1.51 (s, 3H), 1.51 (s, 3H); MS (ES+, m/z) calcd for C₂₇H₃₃N₉O₄⁺: 548.27; found: 548.3 (MH⁺).

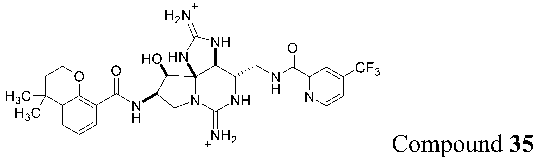

¹H-NMR (400 MHz; D₂O): δ 9.23 (d, *J =* 5.0 Hz, 1H), 8.65 (s, 1H), 8.27 (ddd, *J =* 5.0, 0.9, 0.5 Hz, 1H), 8.11 (dd, *J =* 7.8, 1.7 Hz, 1H), 7.95 (dd, *J =* 7.7, 1.7 Hz, 1H), 7.40 (t, *J* = 7.8 Hz, 1H), 5.06 (s, 1H), 5.05 (d, *J* = 8.3 Hz, 1H), 4.49-4.46 (m, 2H), 4.19-4.15 (m, 2H), 4.06 (dd, *J* = 11.5, 4.5 Hz, 1H), 3.99-3.95 (m, 2H), 2.24 (dd, *J* = 7.1, 4.4 Hz, 2H), 1.68 (s, 3H), 1.67 (s, 3H); MS (ES+, m/z) calcd for C₂₈H₃₂F₃N₉O₄⁺: 616.25; found: 616.3 (MH⁺).

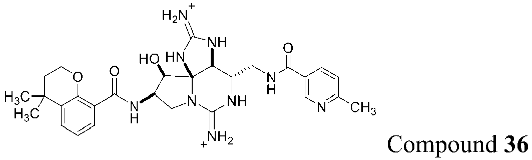

¹H-NMR (400 MHz; D₂O): δ 9.12 (d, *J =* 1.9 Hz, 1H), 8.78 (dd, *J =* 8.4, 2.1 Hz, 1H), 8.07 (s, 1H), 7.93 (dd, *J* = 7.8, 1.7 Hz, 1H), 7.82 (dd, *J* = 7.7, 1.7 Hz, 1H), 7.25 (t, *J=* 7.8 Hz, 1H), 4.94 (s, 1H), 4.89 (d, *J =* 7.8 Hz, 1H), 4.57-4.53 (m, 2H), 4.39 (dd, *J =* 11.6, 7.7 Hz, 1H), 4.01 (dd, *J =* 8.9, 6.3 Hz, 1H), 3.93 (dd, *J* = 11.5, 4.4 Hz, 1H), 3.84-3.74 (m, 2H), 2.95 (s, 3H), 2.08 (dd, *J* = 6.9, 4.5 Hz, 2H), 1.51 (s, 3H), 1.51 (s, 3H); MS (ES+, m/z) calcd for C₂₈H₃₅N₉O₄⁺: 562.28; found: 562.3 (MH⁺).

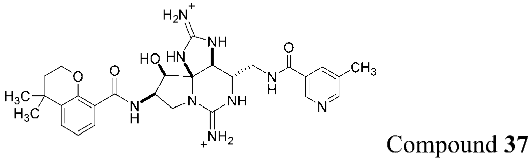

¹H-NMR (400 MHz; D₂O): δ 9.00 (s, 1H), 8.83 (s, 1H), 8.57 (s, 1H), 7.90 (dd, *J =* 7.8, 1.7 Hz, 1H), 7.80 (dd, *J* = 7.8, 1.8 Hz, 1H), 7.23 (t, *J* = 7.8 Hz, 1H), 4.93 (s, 1H), 4.90 (d, *J* = 3.5 Hz, 1H), 4.55-4.52 (m, 2H), 4.37 (dd, *J* = 11.5, 7.8 Hz, 1H), 4.01-3.97 (m, 1H), 3.91 (dd, *J =* 11.5, 4.5 Hz, 1H), 3.82-3.72 (m, 2H), 2.66 (s, 3H), 2.07 (dd, *J =* 6.7, 4.1 Hz, 2H), 1.49 (s, 3H), 1.48 (s, 3H); MS (ES+, m/z) calcd for C₂₈H₃₅N₉O₄⁺: 562.28; found: 562.3 (MH⁺).

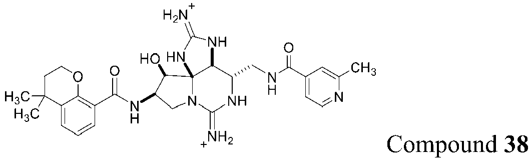

¹H-NMR (400 MHz; D₂O): δ 9.02 (d, *J* = 5.8 Hz, 1H), 8.34 (s, 1H), 8.30-8.28 (m, 1H), 8.11 (dd, *J* = 7.8, 1.7 Hz, 1H), 7.95 (dd, *J* = 7.7, 1.8 Hz, 1H), 7.40 (t, *J =* 7.7 Hz, 1H), 5.05 (s, 1H), 5.01 (d, *J =* 8.0 Hz, 1H), 4.93-4.89 (m, 1H), 4.72-4.68 (m, 2H), 4.52 (dd, *J =* 11.3, 7.6 Hz, 1H), 4.16-4.12 (m, 1H), 4.06 (dd, *J =* 11.5, 4.5 Hz, 1H), 3.93 (dd, *J* = 7.7, 4.2 Hz, 2H), 3.09 (s, 3H), 2.24 (dd, *J* = 6.9, 3.9 Hz, 2H), 1.68 (s, 3H), 1.67 (s, 3H); MS (ES+, m/z) calcd for C₂₈H₃₅N₉O₄⁺: 562.28; found: 562.3 (MH⁺).

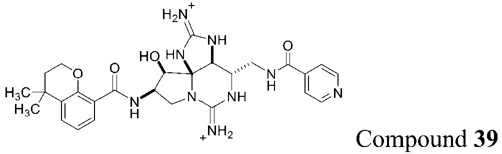

¹H-NMR (400 MHz; D₂O): δ 9.14-9.12 (m, 2H), 8.35-8.33 (m, 2H), 8.09 (dd, *J* = 7.9, 1.7 Hz, 1H), 7.94 (dd, *J =* 7.7, 1.7 Hz, 1H), 7.38 (t, *J =* 7.8 Hz, 1H), 5.04 (s, 1H), 5.00 (d, *J* = 8.1 Hz, 1H), 4.91-4.87 (m, 1H), 4.70-4.67 (m, 2H), 4.50 (dd, *J* = 11.5, 7.6 Hz, 1H), 4.15-4.11 (m, 1H), 4.05 (dd, *J* = 11.3, 4.8 Hz, 1H), 3.92 (dd, *J* = 7.7, 4.1 Hz, 2H), 2.22 (dd, *J* = 6.9, 4.1 Hz, 2H), 1.66 (s, 3H), 1.65 (s, 3H); MS (ES+, m/z) calcd for C₂₇H₃₃N₉O₄⁺: 548.27; found: 548.3 (MH⁺).

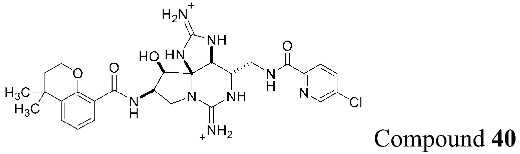

¹H-NMR (400 MHz; D₂O): δ 8.81 (d, *J =* 2.2 Hz, 1H), 8.23-8.21 (m, 1H), 8.18 (d, *J* = 8.4 Hz, 1H), 7.90 (dd, *J* = 8.0, 1.5 Hz, 1H), 7.81 (dd, *J* = 7.8, 1.7 Hz, 1H), 7.24 (t, *J* = 7.8 Hz, 1H), 4.94 (s, 1H), 4.91 (d, *J =* 7.6 Hz, 1H), 4.76-4.71 (m, 1H), 4.55-4.52 (m, 2H), 4.29 (dd, *J* = 11.5, 7.8 Hz, 1H), 4.01 (t, *J* = 7.2 Hz, 1H), 3.90 (dd, *J* = 11.4, 4.6 Hz, 1H), 3.81 (d, *J* = 7.2 Hz, 2H), 3.14-3.13 (m, 1H), 2.98 (t, *J* = 1.1 Hz, 1H), 2.07 (dd, *J* = 6.6, 4.4 Hz, 2H), 1.50 (s, 3H), 1.49 (s, 3H); MS (ES+, m/z) calcd for C₂₇H₃₂ClN₉O₄⁺: 582.23; found: 582.3 (MH⁺).

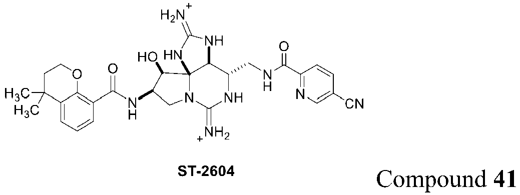

¹H-NMR (400 MHz; D₂O): δ 9.31 (dd, *J* = 2.0, 0.9 Hz, 1H), 8.73 (dd, *J* = 8.2, 2.0 Hz, 1H), 8.51 (dd, *J* = 8.2, 0.9 Hz, 1H), 8.10 (dd, *J= 7.8,* 1.7 Hz, 1H), 7.94 (dd, *J* = 7.8, 1.7 Hz, 1H), 7.39 (t, *J* = 7.8 Hz, 1H), 5.05 (s, 1H), 5.03 (d, *J =* 8.0, 1H), 4.71-4.67 (m, 2H), 4.44 (dd, *J =* 11.3, 7.7 Hz, 1H), 4.17-4.13 (m, 1H), 4.04 (dd, *J* = 11.6, 4.5 Hz, 1H), 3.97-3.94 (m, 2H), 2.23 (dd, *J* = 6.8, 4.2 Hz, 2H), 1.67 (s, 3H), 1.66 (s, 3H); MS (ES+, m/z) calcd for C₂₉H₃₃N₉O₄⁺: 572.27; found: 573.4 (MH⁺).

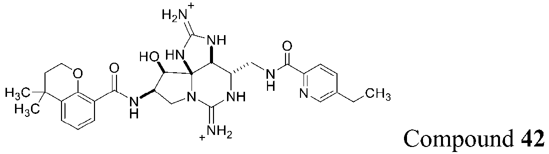

¹H-NMR (400 MHz; D₂O): δ 8.69 (d, *J =* 1.4 Hz, 1H), 8.16-8.06 (m, 2H), 7.91 (dd, *J* = 7.8, 1.7 Hz, 1H), 7.81 (dd, *J* = 7.8, 1.7 Hz, 1H), 7.24 (t, *J* = 7.8 Hz, 1H), 4.94 (s, 1H), 4.91 (d, *J* = 7.9, 1H), 4.75-4.70 (m, 1H), 4.54 (ddd, *J* = 6.3, 4.6, 2.0 Hz, 2H), 4.29 (dd, *J* = 11.4, 7.7 Hz, 1H), 4.03-4.00 (m, 1H), 3.90 (dd, *J* = 11.5, 4.4 Hz, 1H), 3.82 (s, 1H), 3.81 (d, *J* = 2.0 Hz, 1H), 2.92 (q, *J* = 7.6 Hz, 2H), 2.07 (dd*, J =* 6.7, 4.4 Hz, 1H), 1.50 (s, 3H), 1.49 (s, 3H), 1.40 (t, *J =* 7.6 Hz, 3H); MS (ES+, m/z) calcd for C₂₉H₃₈N₉O₄⁺: 576.30; found: 576.30 (MH⁺).

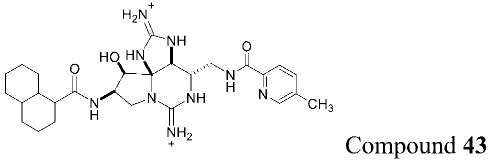

¹H-NMR (400 MHz; D₂O): δ 8.07 (s, 1H), 8.11-8.02 (m, 3H), 4.90 (s, 1H), 4.76 (d, *J* = 8.3, 1H), 4.70-4.63 (m, 1H), 4.15 (dd, *J* = 11.3, 8.5 Hz, 1H), 4.00 (t, *J* = 7.4 Hz, 1H), 3.79 (d, *J* = 7.1 Hz, 2H), 3.71 (dd, *J* = 11.3, 5.8 Hz, 1H), 2.72-2.62 (m, 1H), 2.58 (s, 3H), 2.17-2.12 (m, 1H), 2.05-1.23 (m, 15H); MS (ES+, m/z) calcd for C₂₇H₄₀N₉O₃⁺: 538.33; found: 538.5 (MH⁺).

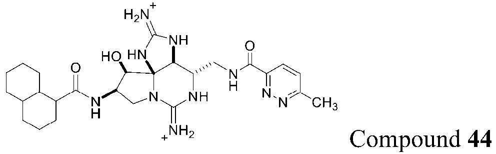

¹H-NMR (400 MHz; D₂O): δ 8.31 (d, *J* = 8.7 Hz, 1H), 8.00 (d, *J* = 8.7 Hz, 1H), 4.91 (s, 1H), 4.76 (d, *J =* 8.4, 1H), 4.70-4.64 (m, 1H), 4.20 (dd, *J =* 11.2, 8.6 Hz, 1H), 4.03 (t, *J* = 7.6 Hz, 1H), 3.85-3.82 (m, 2H), 3.73 (dd, *J* = 11.3, 5.6 Hz, 1H), 2.91 (s, 3H), 2.70-2.61 (m, 1H), 2.16-2.11 (m, 1H), 2.05-1.20 (m, 15H); MS (ES+, m/z) calcd for C₂₆H₃₉N₁₀O₃⁺: 539.32; found: 539.50 (MH⁺).

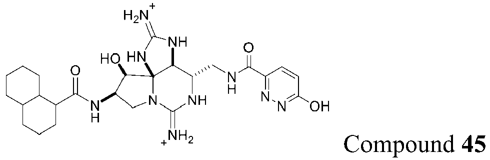

¹H-NMR (400 MHz; D₂O): δ 8.16 (d, *J* = 9.9 Hz, 1H), 7.32 (d, *J* = 9.9 Hz, 1H), 4.85 (d, *J* = 0.86 Hz, 1H), 4.77-4.63 (m, 2H), 4.19 (dd, *J =* 11.0, 8.0 Hz, 1H), 3.97-3.93 (m, 1H), 3.81-3.64 (m, 3H), 3.73 (dd, *J =* 11.3, 5.6 Hz, 1H), 2.91 (s, 3H), 2.69-2.61 (m, 1H), 2.16-2.10 (m, 1H), 2.01-1.20 (m, 15H); MS (ES+, m/z) calcd for C₂₅H₃₇N₁₀O₄⁺: 541.30; found: 541.5 (MH⁺).

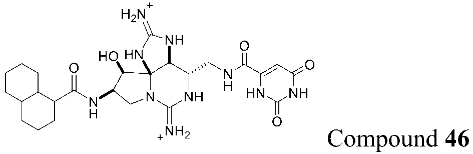

¹H-NMR (400 MHz; D₂O): δ 6.34 (s, 1H), 4.83 (s, 1H), 4.75-4.64 (m, 2H), 4.30-4.18 (m, 1H), 3.92 (dd, *J* = 8.9, 6.9 Hz, 1H), 3.77-3.56 (m, 3H), 2.68-2.60 (m, 1H), 2.14-2.10 (m, 1H), 1.99-1.21 (m, 15H); MS (ES+, m/z) calcd for C₂₅H₃₇N₁₀O₅⁺: 557.29; found: 557.30 (MH⁺).

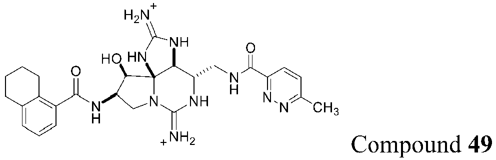

¹H NMR (400 MHz, D₂O) : δ 8.32 (d, *J* = 8.7 Hz, 1H), 8.01 (d, *J =* 8.7 Hz, 1H), 7.46-7.31 (m, 3H), 4.94-4.85 (m, 3H), 4.38-4.24 (m, 1H), 4.09-3.97 (m, 1H), 3.91-3.74 (m, 3H), 2.97-2.89 (m, 4H), 2.90 (s, 3H), 1.91 (dt, *J =* 6.4, 3.2 Hz, 4H); MS (ES+, m/z) calcd for C₂₆H₃₃N₁₀O₃⁺: 533.27; found: 533.30 (MH⁺).

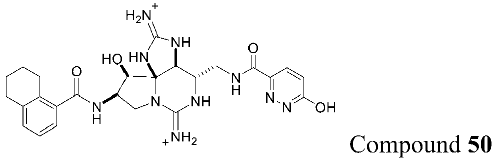

¹H NMR (400 MHz, D₂O) : δ 8.15 (d, *J* = 9.9 Hz, 1H), 7.43-7.33 (m, 3H), 7.30 (d, *J* = 9.9 Hz, 1H), 4.92-4.83 (m, 3H), 4.31 (dd, *J =* 11.3, 8.3 Hz, 1H), 3.95 (dd, *J =* 8.8, 6.2 Hz, 1H), 3.85-3.68 (m, 3H), 2.95-2.86 (m, 4H), 1.89 (dt, *J* = 6.3, 3.2 Hz, 4H); MS (ES+, m/z) calcd for C₂₅H₃₁N₁₀O⁺: 535.25; found: 535.30 (MH⁺).

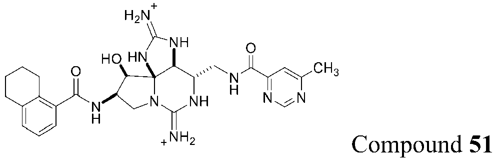

¹H NMR (400 MHz, D₂O) : δ 8.01 (s, 2H), 7.46-7.35 (m, 3H), 4.94-4.84 (m, 3H), 4.33 (dd, *J* = 11.4, 8.3 Hz, 1H), 4.03 (t, *J* = 7.4 Hz, 1H), 3.88-3.82 (m, 3H), 2.99-2.91 (m, 4H), 2.81 (s, 3H), 1.94 (dt, *J* = 5.8, 2.8 Hz, 4H); MS (ES+, m/z) calcd for C₂₅H₃₁N₁₀O₃⁺: 533.27; found: 533.30 (MH⁺).

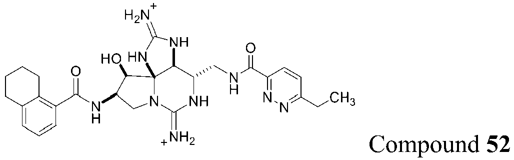

¹H NMR (400 MHz, D₂O) : δ 8.67 (d, *J =* 1.4 Hz, 1H), 8.07 (d, *J =* 2.1 Hz, 1H), 8.05 (s, 1H), 7.42-7.33 (m, 3H), 4.93-4.84 (m, 3H), 4.29-4.20 (m, 1H), 4.01 (t, *J*= 7.3 Hz, 1H), 3.84-3.77 (m, 3H), 2.96-2.86 (m, 6H), 1.90 (dt, *J* = 5.9, 2.8 Hz, 4H), 1.38 (t, *J* = 15.3 Hz, 3H); MS (ES+, m/z) calcd for C₂₈H₃₆N₉O₃⁺: 546.29; found: 546.40 (MH⁺).

### Synthetic Example 2

### Preparation of Non-hydrated Ketone Inhibitors of Nav1.7

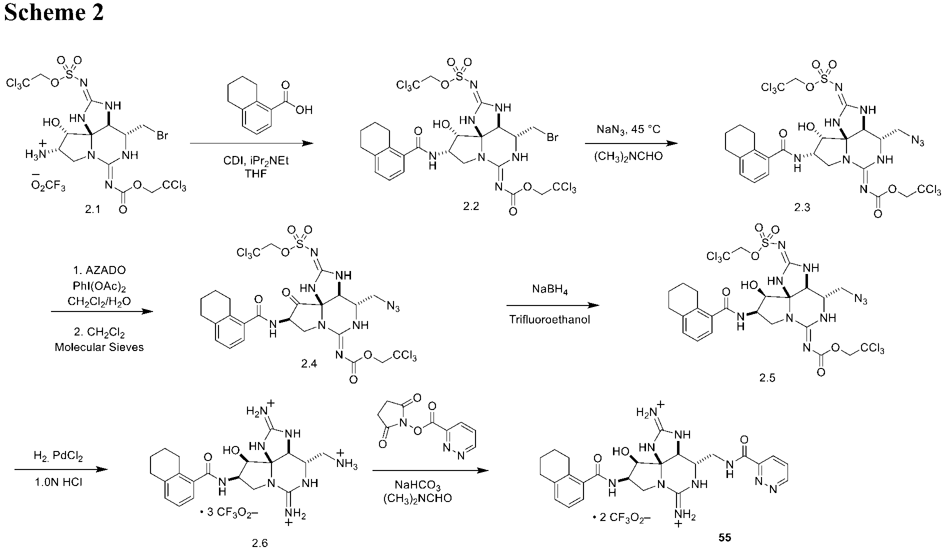

Compound 55 was prepared as shown in Scheme 2.

To a solution of 5,6,7,8-tetrahydro-1-naphthalenecarboxylic acid (0.91 g, 5.18 mmol, 1.5 equiv) in 20 mL of THF at room temperature was added carbonyldiimidazole (0.84 g, 5.18 mmol, 1.5 equiv). After stirring of the reaction for 16 hours at room temperature, a solution of **2.1** (2.42 g, 3.45 mmol, 1.0 equiv) in 15 mL of THF was added. The reaction mixture was allowed to stir overnight. Following this time, *i*Pr₂NEt (2.41 mL, 13.8 mmol, 4.0 equiv) was added. The resulting mixture was stirred at room temperature for 16 hours and quenched by addition of 30 mL 1N HCl. The aqueous layer was extracted with 3 x 100 mL of EtOAc. The combined organic layer was dried with Na₂SO₄ and concentrated *in vacuo.* The residue was purified by a flash column (gradient elution: 45:55→75:25 EtOAc/hexanes) to obtain the desired amide **2.2** (2.24 g, 2.6 mmol, 73%) as a white solid.

To a solution of bromide **2.2** (1.76 g, 2.04 mmol, 1.0 equiv) in 20 mL of dimethylformamide at room temperature was added sodium azide (0.66 g, 10.2 mmol, 5.0 equiv) and the resulting solution was heated at 45 °C for 2 days. The reaction was cooled to room temperature and diluted with 200 mL of EtOAc. The resulting solution was washed with 3 x 100 mL of H₂O and 2 x 100 mL of brine. The organic layer was dried with Na₂SO₄, filtered, concentrated *in vacuo* and purified by column chromatography (gradient elution: 50:50→75:25 EtOAc/hexanes) to obtain the desired azide **2.3** (1.34 g, 1.62 mmol, 79%) as a pale-yellow solid.

To a stirred solution of compound **2.3** (1.0 g, 1.21 mmol, 1.0 equiv) in 44 mL of CH₂Cl₂ were added sequentially water (2.3 mL) and iodobenzene diacetate (584.6 mg, 1.81 mmol, 1.5 equiv). The resulting solution was stirred for 5 minutes. To this reaction mixture then added a solution of 2-azaadamantane N-oxyl (AZADO) (55.3 mg, 0.36 mmol, 0.3 equiv) in CH₂Cl₂ (10 mL) dropwise over the period of 5 minutes. The biphasic mixture stirred vigorously for an additional 15 minutes. The reaction was then quenched with 8 mL of isopropyl alcohol and stirred for 30 minutes. MgSO₄ was added to the mixture to absorb the water. Organic layer was filtered and the MgSO₄ washed with a small portion of CH₂Cl₂. Hexanes (550 mL) was gradually added, and the mixture was gently stirred at room temperature for 45 minutes and filtered to give 1.0 g of an off-white solid. This material was used for the next step without any purification.

The crude material (1.0 g, 1.19 mmol) was dissolved in anhydrous CH₂Cl₂ (30 mL) and 4Å Molecular sieves was added. The resulting suspension stirred at room temperature overnight. The molecular sieves were removed by filtration and the filter cake was washed with additional CH₂Cl₂. The combined filtrates were evaporated under reduced pressure to dryness to give **2.4.** To this residue was added trifluoroethanol (20 mL) and resulting solution was cooled to 0°C. NaBH₄ (135 mg, 3.57 mmol, 3.0 equiv) was added and mixture stirred at same temperature for 20 minutes. Trifluoroethanol was evaporated under reduced pressure and residue was dissolved in CH₂Cl₂ and partitioned with water (2:1). The aqueous phase was extracted with CH₂Cl₂ (1x) and combined organic were washed with brine (1x), dried and evaporated under reduced pressure to afford crude **2.5.** This material was purified by column chromatography using hexanes/ethyl acetate (2:3) to afford 470 mg, 0.57 mmol (47% yield) of **2.5** (The undesired alcohol is recovered and reprocessed to yield an additional 15-20% of **2.5**).

To a stirred solution of compound **2.5** (470 mg, 0.57 mmol) in MeOH (40 mL) was added water 10 mL. Trifluoroacetic acid (5 mL) and PdCl₂ (70 mg) were added and H₂ gas was bubbled through the reaction mixture for 10 minutes, after which time bubbling was ceased and the reaction was stirred under an atmosphere of H₂ for 6 hours. The reaction mixture was sequentially filtered through a 0.2 µm PTFE syringe filter. The flask and filters were washed with 15 mL of MeOH and the filtrate concentrated under reduced pressure. The residue was dissolved in 30 mL of 1:2 MeCN/1.0 M aqueous HCl and stirred at room temperature for 2.5 days. The reaction mixture was concentrated under reduced pressure, dissolved in 15 mL aqueous 10 mM trifluoroacetic acid solution and purified by reversed-phase HPLC (Bonna-Agela Durashell C18, 10 µM, 21 x 250 mm column, eluting with gradient flow from 10-16% MeCN/ with 10 mM TFA over 30 minutes, 214 nm UV detection). At a flow rate of 20 mL/min, **2.6** had a retention time of 16-22.5 min and the corresponding Tri-TFA salt **2.6** was isolated as a white solid (240 mg, 0.31 mmol, 54%).

A solution of **2.6** (60 mg, 0.08 mmol, 1.0 equiv) in 0.1M sodium bicarbonate (2.4 mL) was cooled to 0°C. A solution of 2,5-dioxopyrrolidin-1-yl-pyridazine 3-carboxylic acid (20.3mg, 0.091mmol,1.2 equiv.) in 0.9 mL of dimethylformamide was added dropwise. The ice bath is removed, and mixture stirred at room temperature for 30 minutes. An in-process LCMS analysis sample was taken and showed reaction completion. It was quenched by addition of 10 mM TFA (3 mL) and then purified by reversed-phase HPLC (Bonna-Agela Durashell C18, 10 µM, 25 x 250 mm column, eluting with gradient flow from 16-22% MeCN/ with 10 mM TFA over 30 minutes, 214 nm UV detection). At a flow rate of 20 mL/min, Compound **55** • 2 TFA had a retention time of 12.5-18.5 min and the corresponding TFA salt was isolated as a white solid (50 mg, 0.067mmol, 83%).

Compound **55** ¹H NMR (400 MHz, D₂O) δ 9.38 (dd, *J* = 5.1, 1.6 Hz, 1H), 8.33 (dd, *J* = 8.6, 1.6 Hz, 1H), 8.01 (dd, *J =* 8.6, 5.1 Hz, 1H), 7.34 - 7.19 (m, 3H), 4.84 - 4.72 (m, 3H), 4.21 (dd, *J =* 11.4, 8.1 Hz, 1H), 3.96 - 3.89 (m, 1H), 3.80 - 3.69 (m, 3H), 2.86 - 2.77 (m, 4H), 1.79 (t, *J* = 3.0 Hz, 4H); MS (ES+, m/z) calcd for C₂₅H₃₁N₁₀O₃⁺: 519.26; found: 519.30 (MH⁺).

The following compounds were prepared using a procedure similar to the procedure in Scheme 2 above:

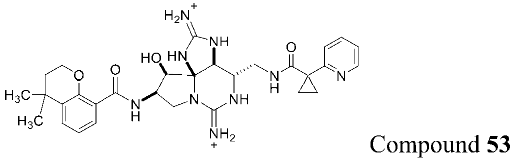

¹H NMR (400 MHz, D₂O) δ 8.79 - 8.72 (m, 1H), 8.58 (td, *J* = 8.0, 1.6 Hz, 1H), 8.10 (d, *J* = 7.9 Hz, 1H), 8.05 - 7.98 (m, 1H), 7.77 (dd, *J* = 7.8, 1.7 Hz, 1H), 7.67 (dd, *J =* 7.8, 1.7 Hz, 1H), 7.10 (t, *J* = 7.8 Hz, 1H), 4.79 - 4.70 (m, 1H), 4.62 - 4.57 (m, 1H), 4.44 - 4.36 (m, 2H), 4.16 (dd, *J =* 11.4, 7.6 Hz, 1H), 4.06 - 3.95 (m, 1H), 3.74 (dd, *J =* 11.4, 4.5 Hz, 1H), 3.69 - 3.62 (m, 1H), 3.46 - 3.32 (m, 2H), 1.97 - 1.90 (m, 2H), 1.84 - 1.76 (m, 2H), 1.63 - 1.55 (m, 2H), 1.37 (s, 3H), 1.36 (s, 3H), 1.17 (d, *J =* 6.2 Hz, 2H); MS (ES+, m/z) calcd for C₃₀H₄₀N₉O₃⁺: 574.32; found: 574.32 (MH⁺).

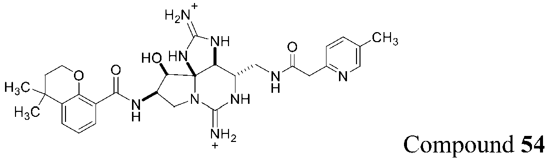

MS (ES+, m/z) calcd for C₂₉H₃₈N₉O₃⁺: 576.30; found: 576.32 (MH⁺).

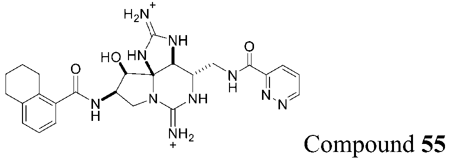

¹H NMR (400 MHz, D₂O) δ 9.38 (dd, *J* = 5.1, 1.6 Hz, 1H), 8.33 (dd, *J* = 8.6, 1.6 Hz, 1H), 8.01 (dd, *J* = 8.6, 5.1 Hz, 1H), 7.34 - 7.19 (m, 3H), 4.84 - 4.72 (m, 3H), 4.21 (dd, *J =* 11.4, 8.1 Hz, 1H), 3.96 - 3.89 (m, 1H), 3.80 - 3.69 (m, 3H), 2.86 - 2.77 (m, 4H), 1.79 (t, *J =* 3.0 Hz, 4H); MS (ES+, m/z) calcd for C₂₅H₃₁N₁₀O₃⁺: 519.26; found: 519.30 (MH⁺).

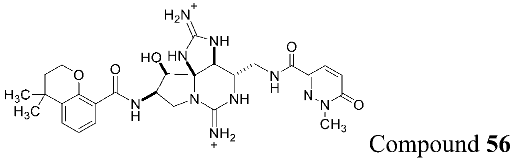

¹H NMR (400 MHz, D₂O) δ 8.15 (d, *J =* 9.6 Hz, 1H), 7.92 (dd, *J* = 7.8, 1.6 Hz, 1H), 7.81 (dd, *J =* 7.8, 1.7 Hz, 1H), 7.31 (d, *J* = 9.6 Hz, 1H), 7.24 (t, *J* = 7.8 Hz, 1H), 4.93 - 4.86 (m, 2H), 4.77 - 4.72 (m, 1H), 4.58 - 4.51 (m, 2H), 4.34 (dd, *J* = 11.4, 7.7 Hz, 1H), 4.00 (s, 3H), 3.98 - 3.88 (m, 2H), 3.80 - 3.72 (m, 2H), 2.11 - 2.05 (m, 2H), 1.51 (s, 3H), 1.50 (s, 3H); MS (ES+, m/z) calcd for C₂₇H₃₅N₁₀O₅⁺: 579.28 found: 579.30 (MH⁺).

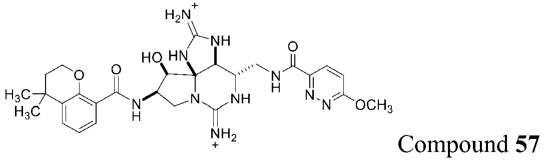

¹H NMR (400 MHz, D₂O) δ 8.17 (d, *J* = 9.3 Hz, 1H), 7.78 (dd, *J* = 7.8, 1.6 Hz, 1H), 7.68 (dd, *J* = 7.7, 1.7 Hz, 1H), 7.43 (d, *J* = 9.3 Hz, 1H), 7.15 - 7.09 (m, 1H), 4.86 - 4.73 (m, 3H), 4.45 - 4.39 (m, 2H), 4.22 - 4.14 (m, 4H), 3.90 (t, *J* = 7.3 Hz, 1H), 3.78 (dd, *J* = 11.5, 4.4 Hz, 1H), 3.74 - 3.68 (m, 2H), 1.98 - 1.92 (m, 2H), 1.38 (s, 3H), 1.37 (s, 3H); ); MS (ES+, m/z) calcd for C₂₇H₃₅N₁₀O₅⁺: 579.28 found: 579.30 (MH⁺).

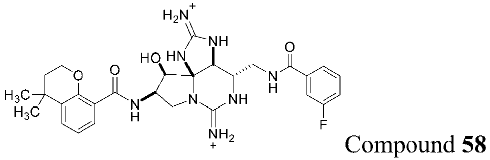

¹H NMR (400 MHz, D₂O) δ 7.94 (s, 1H), 7.82 (dd, *J* = 7.8, 1.7 Hz, 1H), 7.70 - 7.64 (m, 2H), 7.62 - 7.56 (m, 2H), 7.45 - 7.39 (m, 1H), 7.12 (t, *J* = 7.8 Hz, 1H), 4.79 - 4.72 (m, 2H), 4.45 - 4.39 (m, 2H), 4.26 - 4.18 (m, 2H), 3.87 - 3.82 (m, 1H), 3.80 - 3.75 (m, 1H), 3.62 (d, *J* = 7.6 Hz, 2H), 1.99 - 1.93 (m, 2H), 1.39 (s, 3H), 1.38 (s, 3H); ); MS (ES+, m/z) calcd for C₂₈H₃₄FN₈O₄⁺: 565.27 found: 565.30 (MH⁺).

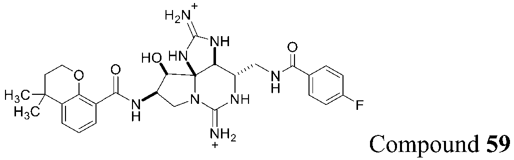

¹H NMR (400 MHz, D₂O) δ 7.93 - 7.86 (m, 2H), 7.82 (dd, *J* = 7.8, 1.7 Hz, 1H), 7.68 (dd, *J* = 7.7, 1.7 Hz, 1H), 7.31 (t, *J* = 8.9 Hz, 2H), 7.12 (t, *J* = 7.8 Hz, 1H), 4.78 - 4.73 (m, 2H), 4.44 - 4.40 (m, 2H), 4.27 - 4.17 (m, 2H), 3.87 - 3.82 (m, 1H), 3.78 (dd, *J* = 11.4, 4.5 Hz, 1H), 3.61 (d, *J* = 7.6 Hz, 2H), 1.99 - 1.93 (m, 2H), 1.39 (s, 3H), 1.38 (s, 3H); MS (ES+, m/z) calcd for C₂₈H₃₄FN₈O₄⁺: 565.27 found: 565.30 (MH⁺).

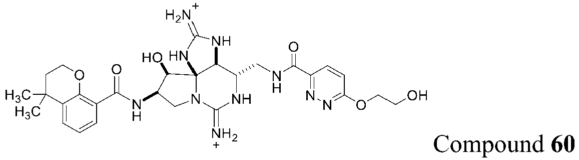

¹H NMR (400 MHz, D₂O) δ 8.02 (d, *J* = 9.7 Hz, 1H), 7.80 (dd, *J* = 7.8, 1.7 Hz, 1H), 7.68 (dd, *J =* 7.7, 1.7 Hz, 1H), 7.19 (d, *J =* 9.7 Hz, 1H), 7.11 (t, *J =* 7.8 Hz, 1H), 4.79 - 4.69 (m, 3H), 4.46 - 4.38 (m, 4H), 4.25 - 4.18 (m, 1H), 4.02 (t, *J* = 5.3 Hz, 2H), 3.86 - 3.75 (m, 2H), 3.68 - 3.59 (m, 2H), 1.99 - 1.92 (m, 2H), 1.38 (s, 3H), 1.38 (s, 3H); ); MS (ES+, m/z) calcd for C₂₈H₃₇N₁₀O₆⁺: 609.29 found: 609.30(MH⁺).

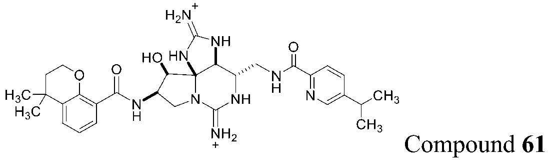

¹H NMR (400 MHz, D₂O) δ 8.62 (s, 1H), 8.10 - 8.05 (m, 2H), 7.80 (dd, *J* = 7.8, 1.7 Hz, 1H), 7.68 (dd, *J* = 7.8, 1.7 Hz, 1H), 7.11 (t, *J =* 7.8 Hz, 1H), 4.85 - 4.73 (m, 3H), 4.46 - 4.38 (m, 2H), 4.19 - 4.13 (m, 1H), 3.93 - 3.85 (m, 1H), 3.77 (dd, *J=* 11.4, 4.5 Hz, 1H), 3.73 - 3.64 (m, 2H), 3.16 - 3.08 (m, 1H), 1.99 - 1.92 (m, 2H), 1.38 (d, *J* = 3.2 Hz, 6H), 1.32 (s, 3H), 1.31 (s, 3H); MS (ES+, m/z) calcd for C₃₀H₄₀N₉O₄⁺: 590.32 found: 590.40(MH⁺).

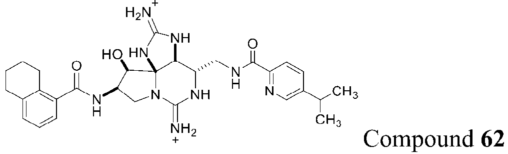

¹H NMR (400 MHz, D₂O) δ 8.67 - 8.61 (m, 1H), 8.16 - 8.07 (m, 2H), 7.35 - 7.20 (m, 3H), 4.83 - 4.71 (m, 3H), 4.19 - 4.11 (m, 1H), 3.89 (t, *J =* 7.3 Hz, 1H), 3.75 - 3.65 (m, 3H), 3.20 - 3.09 (m, 1H), 2.86 - 2.76 (m, 4H), 1.84 - 1.74 (m, 4H), 1.33 (s, 3H), 1.31 (s, 3H). ); MS (ES+, m/z) calcd for C₂₉H₃₈N₉O₃⁺: 560.31 found: 560.40(MH⁺).

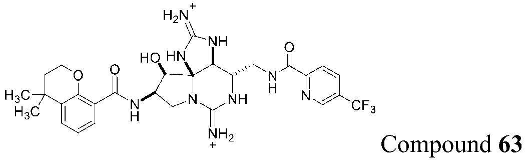

¹H NMR (400 MHz, D₂O) δ 9.03 (s, 1H), 8.40 (dd, *J* = 8.3, 1.7 Hz, 1H), 8.25 (d, *J* = 8.2 Hz, 1H), 7.80 (dd, *J* = 7.8, 1.7 Hz, 1H), 7.68 (dd, *J* = 7.8, 1.7 Hz, 1H), 7.11 (t, *J =* 7.8 Hz, 1H), 4.85 - 4.71 (m, 3H), 4.45 - 4.38 (m, 2H), 4.18 (dd, *J* = 11.4, 7.7 Hz, 1H), 3.90 (t, *J =* 7.4 Hz, 1H), 3.78 (dd, *J =* 11.5, 4.5 Hz, 1H), 3.74 - 3.67 (m, 2H), 1.98 - 1.92 (m, 2H), 1.39 (s, 3H), 1.38 (s, 3H); MS (ES+, m/z) calcd for C₂₈H₃₃F₃N₉O₄⁺: 616.26 found: 616.30 (MH⁺).

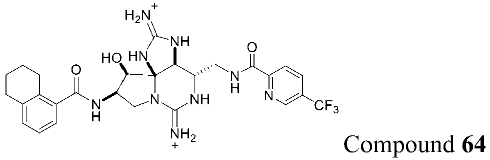

¹H NMR (400 MHz, D₂O) δ 9.03 (s, 1H), 8.40 (dd, *J* = 8.3, 2.1 Hz, 1H), 8.25 (d, *J* = 8.2 Hz, 1H), 7.33 - 7.19 (m, 3H), 4.83 - 4.73 (m, 3H), 4.16 (dd, *J* = 11.3, 8.0 Hz, 1H), 3.90 (t, *J* = 7.0 Hz, 1H), 3.76 - 3.66 (m, 3H), 2.85 - 2.74 (m, 4H), 1.86 - 1.71 (m, 4H); MS (ES+, m/z) calcd for C₂₇H₃₁F₃N₉O₃⁺: 586.25 found: 586.30 (MH⁺).

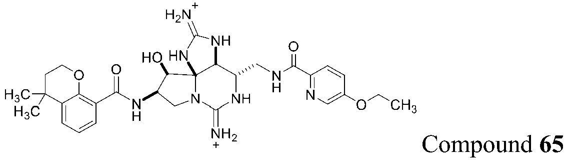

¹H NMR (400 MHz, D₂O) δ 8.35 (d, *J* = 2.7 Hz, 1H), 8.06 (d, *J* = 8.8 Hz, 1H), 7.79 (dd, *J* = 7.8, 1.7 Hz, 1H), 7.68 (dd, *J* = 7.8, 1.7 Hz, 1H), 7.58 (dd, *J* = 8.8, 2.9 Hz, 1H), 7.11 (t, *J* = 7.8 Hz, 1H), 4.82 - 4.74 (m, 2H), 4.60 - 4.54 (m, 1H), 4.48 - 4.36 (m, 2H), 4.26 (q, *J* = 7.0 Hz, 2H), 4.14 (dd, *J* = 11.4, 7.7 Hz, 1H), 3.91 - 3.84 (m, 1H), 3.76 (dd, *J* = 11.4, 4.5 Hz, 1H), 3.72 - 3.61 (m, 2H), 2.00 - 1.91 (m, 2H), 1.44 (t, *J* = 7.0 Hz, 3H), 1.38 (s, 3H), 1.37 (s, 3H); MS (ES+, m/z) calcd for C₂₉H₃₈N₉O₅⁺: 592.30 found: 592.30 (MH⁺).

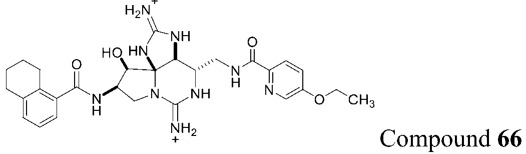

¹H NMR (400 MHz, D₂O) δ 8.34 (d, *J* = 2.8 Hz, 1H), 8.06 (d, *J* = 8.8 Hz, 1H), 7.57 (dd, *J* = 8.8, 2.8 Hz, 1H), 7.33 - 7.21 (m, 3H), 4.80 - 4.72 (m, 3H), 4.26 (q, *J* = 7.0 Hz, 2H), 4.14 - 4.06 (m, 1H), 3.91 - 3.84 (m, 1H), 3.74 - 3.64 (m, 3H), 2.85 - 2.74 (m, 4H), 1.86 - 1.73 (m, 4H), 1.44 (t, *J =* 7.0 Hz, 3H); MS (ES+, m/z) calcd for C₂₈H₃₆N₉O₄⁺: 562.29 found: 562.30 (MH⁺).

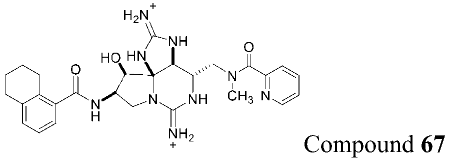

MS (ES+, m/z) calcd for C₂₇H₃₄N₉O₃⁺: 532.28 found: 532.30 (MH⁺).

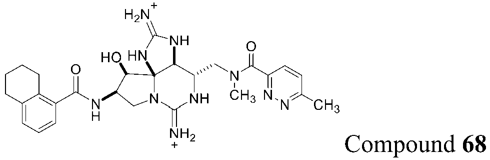

¹H NMR (400 MHz, D₂O) δ 7.98 - 7.87 (m, 2H), 7.35 - 7.18 (m, 3H), 4.89 - 4.77 (m, 3H), 4.39 (s, 1H), 4.29 - 4.18 (m, 2H), 4.13 - 4.06 (m, 1H), 3.79 - 3.72 (m, 1H), 3.54 - 3.47 (m, 1H), 3.21 (s, 1H), 3.05 (s, 2H), 2.77 (s, 2H), 2.71 (s, 1H), 1.78 (s, 4H); MS (ES+, m/z) calcd for C₂₇H₃₅N₁₀O₃⁺: 547.29 found: 547.30 (MH⁺).

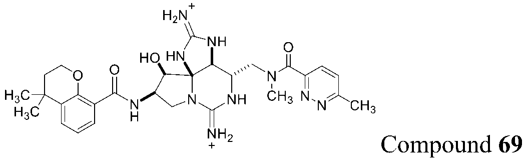

MS (ES+, m/z) calcd for C₂₈H₃₇N₁₀O₄⁺: 577.30 found: 577.30 (MH⁺).

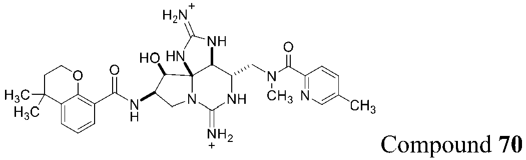

MS (ES+, m/z) calcd for C₂₉H₃₈N₉O₄⁺: 576.30 found: 576.30 (MH⁺).

### Example 2

### Nav Inhibition Assay

Electrophysiology experiments were performed on Human Embryonic Kidney 293 cells (HEK) or Chinese hamster ovary cells (CHO) transfected with the full-length cDNA coding for the appropriate human Na_{V} sodium channel α-subunit, including Na_{V} 1.7, and Nav 1.4.

Sodium currents were measured using the patch-clamp technique in the whole-cell configuration with a HEKA EPC 9 amplifier (HEKA Elektronik Dr. Schulze GmbH, Germany) or can be measured using an IonFlux 16 automated patch clamp system (Fluxion Biosciences, South San Francisco, USA) as previously described by Moran. *See,* Moran O, Picollo A, Conti F (2003) Tonic and phasic guanidinium toxin-block of skeletal muscle Na channels expressed in Mammalian cells, Biophys J 84(5):2999-3006. For manual patch-clamp experiments, borosilicate glass micropipettes (Sutter Instruments, Novato, CA) were pulled to a tip diameter yielding a resistance of 1.0-2.0 MΩ in the working solutions. The composition of intracellular solution was (in mM): CsF 125, EDTA 10, HEPES 10, NaCl 10, and the pH was adjusted to 7.2 with CsOH. The composition of extracellular solution was (in mM): NaCl 135, KC14.5, CaCl₂ 2, MgCl₂ 1, HEPES 10, and the pH was adjusted to 7.4 with NaOH. Peak currents were generally between 0.5-20 nA.

Lyophilized stock of each of the toxin derivatives were stored at -20 °C and dissolved in the external solution prior to recording. (+)-Saxitoxin and (+)-gonyautoxin-III were synthesized according to routes previously published. (Fleming JJ, McReynolds MD, Du Bois J. (+)-saxitoxin: a first and second generation stereoselective synthesis. J Am Chem Soc. 2007;129(32):9964-9975; Mulcahy JV, Du Bois J. A stereoselective synthesis of (+)-gonyautoxin 3. J Am Chem Soc. 2008;130:12630-12631). Current measurements were recorded under continuous perfusion, controlled manually by syringe addition.

The output of the EPC 9 patch-clamp amplifier was filtered with a built-in low-pass, four-pole Bessel filter having a cutoff frequency of 10 kHz and sampled at 20-50 kHz. For both manual and automated recordings, the membrane was kept at a holding potential of between -120 and -90 mV. Pulse stimulation and data acquisition were controlled with the Pulse software (HEKA Elektronik Dr. Schulze GmbH, Germany) or the IonFlux software (Fluxion Biosciences, South San Francisco, USA). All measurements were performed at room temperature (about 20-22 °C). Recordings were made at least 5 min after establishing the whole-cell and voltage-clamp configuration to allow for stabilization of the voltage-dependent properties of the channels. Currents were elicited by 10 ms step depolarizations from a holding potential to a value between -40 and +10 mV. Peak currents after channel activation were recorded. These data were baseline-normalized, plotted against toxin concentration and analyzed in Microsoft Excel software. Data were fit to a four-parameter logistic equation with the Hill slope set to 1 to determine IC₅₀ values and expressed as mean.

### Table 1- Nav Isoform Potency and Selectivity

For Table 1 and Table 2, all data were measured in HEK cells. Column 1 provides IC₅₀ data for Na_{V} 1.7 as measured using using the patch-clamp technique in the whole-cell configuration with a HEKA EPC 9 amplifier. Column 2 provides IC₅₀ data for Nav 1.4 as measured using the patch-clamp technique in the whole-cell configuration with a HEKA EPC 9 amplifier. Column 3 provides selectivity data for column 1 over column 2. ND means not detectable. NT means not tested.

For Table 3, select compounds were tested for activity in NaV 1.5. All data were also measured in HEK cells. Column 1 repeats the IC₅₀ data for NaV 1.7 shown in Table 1 and Table 2. Column 2 provides IC₅₀ data for NaV 1.5 as measured using the patch-clamp technique in the whole-cell configuration with a HEKA EPC 9 amplifier. Column 3 provides selectivity data for column 1 over column 2.

For Table 4, select compounds were tested for activity in NaV 1.6. All data were also measured in HEK cells. Column 1 repeats the IC₅₀ data for NaV 1.7 shown in Table 1 and Table 2. Column 2 provides IC₅₀ data for NaV 1.6 as measured using the patch-clamp technique in the whole-cell configuration with a HEKA EPC 9 amplifier. Column 3 provides selectivity data for column 1 over column 2.

**Table 1. Compounds**

| | **Compound** | **1 Nav 1.7** | **2 Nav 1.4** | **3** |
|---|---|---|---|---|
| **1** | 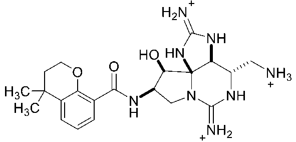 | 1.5 µM | 35 µM | 23 |
| **2** | 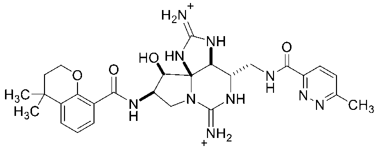 | 21 nM | 260 µM | 12000 |
| **3** | 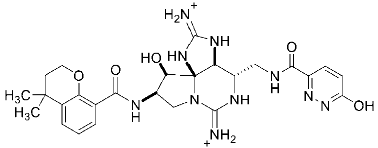 | 100 nM | NT | -- |
| **4** | 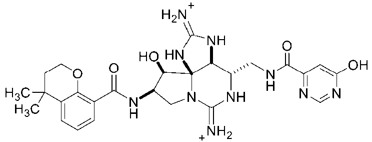 | 78 nM | NT | -- |
| **5** | 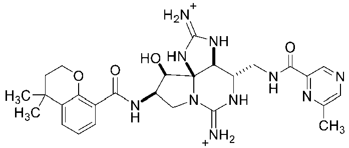 | 40 nM | NT | -- |
| **6** | 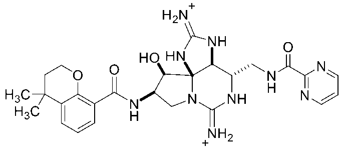 | 18 nM | 50 µM | 2700 |
| **7** | 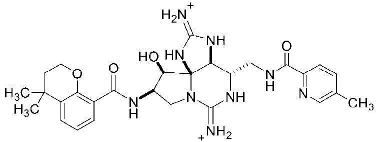 | 13 nM | 190 µM | 14500 |
| 8 | 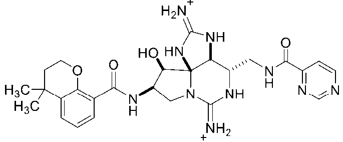 | 23 nM | 72 µM | 3000 |
| 9 | 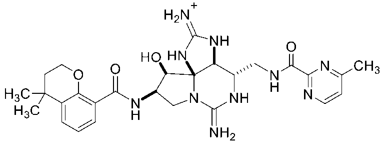 | 29 nM | NT | -- |
| 10 | 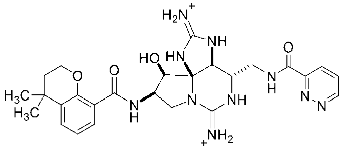 | 44 nM | NT | -- |
| 11 | 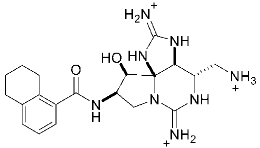 | 388 nM | 33.7 µM | 85 |
| **12** | 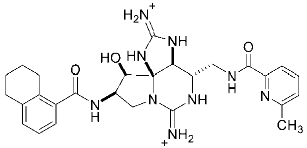 | 6.5 nM | 8.8 µM | 1350 |
| **13** | 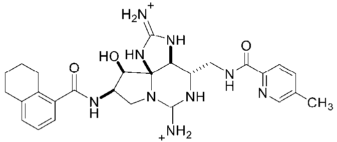 | 7 nM | 29.4 µM | 4200 |
| **14** | 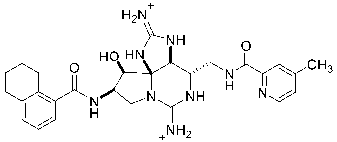 | 34 nM | 61.8 µM | 1800 |
| **15** | 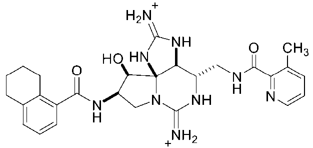 | 2.5 µM | NT | -- |
| **16** | 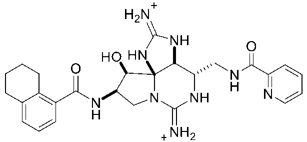 | 5 nM | 13.6 µM | 2700 |
| **17** | 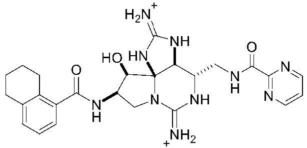 | 5 nM | 1.6 µM | 320 |
| **18** | 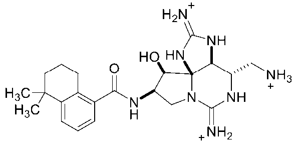 | 500 nM | NT | -- |
| **19** | 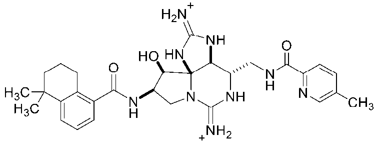 | 21 nM | NT | -- |
| **20** | 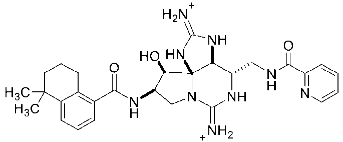 | 10 nM | NT | -- |
| **21** | 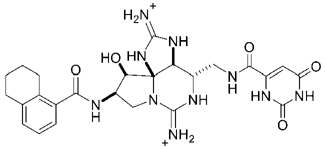 | 17 nM | 250 µM | 14700 |
| **22** | 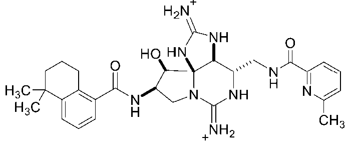 | 15 nM | 5.1 µM | 340 |
| **23** | 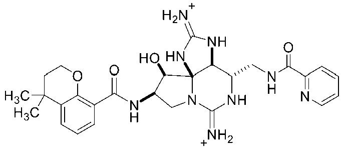 | 25 nM | 275 µM | 11000 |
| **24** | 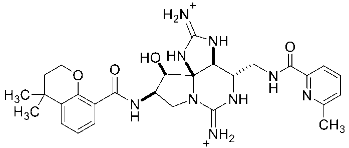 | 19 nM | 150 µM | 7890 |
| **25** | 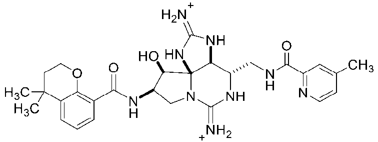 | 36.5 nM | 550 µM | 15000 |
| **26** | 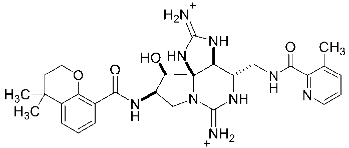 | 7.1 µM | NT | -- |
| **27** | 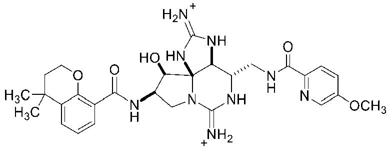 | 19 nM | 520 µM | 27000 |
| **28** | 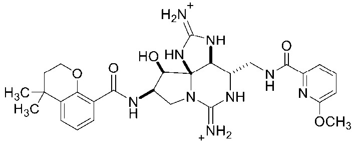 | 70 nM | NT | -- |
| **29** | 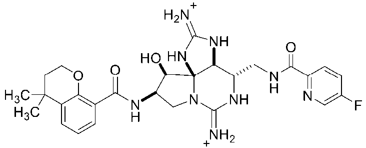 | 34 nM | 143 µM | 4200 |
| **30** | 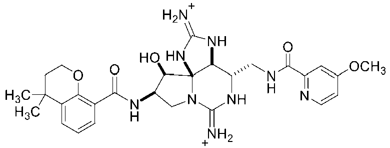 | 77 nM | NT | -- |
| **31** | 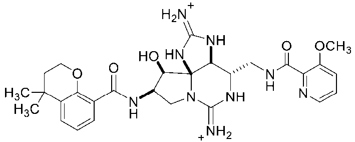 | 520 nM | NT | -- |
| **32** | 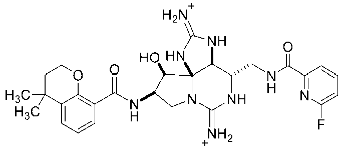 | 60 nM | NT | -- |
| **33** | 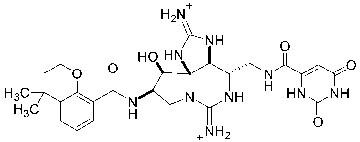 | 27 nM | 240 µM | 8800 |
| **34** | 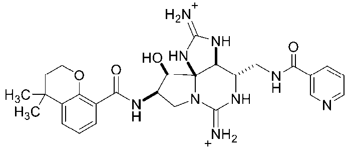 | 51 nM | NT | -- |
| **35** | 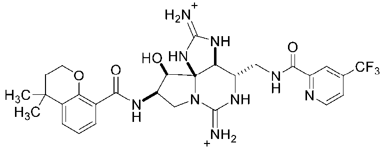 | 500 nM | NT | -- |
| **36** | 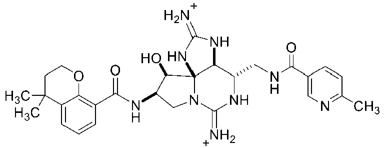 | 63 nM | NT | -- |
| **37** | 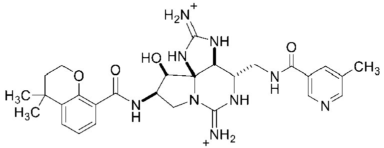 | 176 nM | NT | -- |
| **38** | 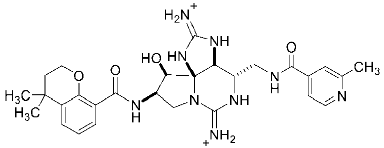 | 114 nM | NT | -- |
| **39** | 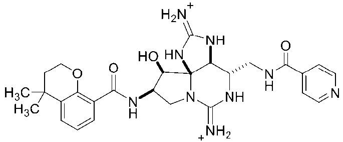 | 72 nM | NT | -- |
| **40** | 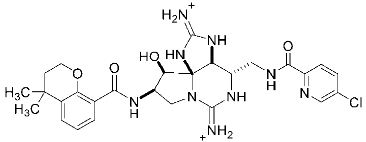 | 25 nM | NT | -- |
| **41** | 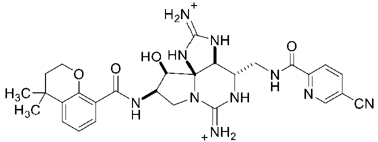 | 35 nM | NT | -- |
| **42** | 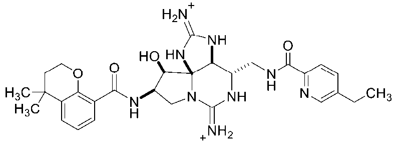 | 24 nM | NT | -- |
| **43** | 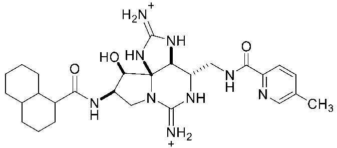 | 13 nM | 8 µM | 600 |
| **44** | 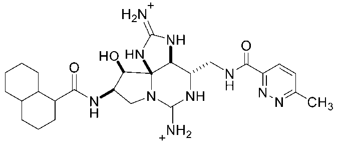 | 40 nM | 65 µM | 1600 |
| **45** | 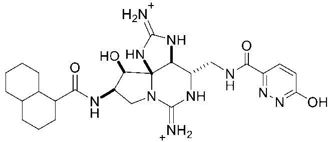 | 11 nM | 53 µM | 4800 |
| **46** | 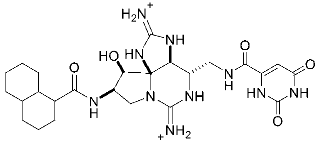 | 30 nM | 300 µM | 10000 |
| **49** | 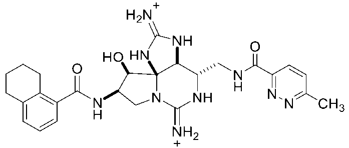 | 7 nM | 36 µM | |
| **50** | 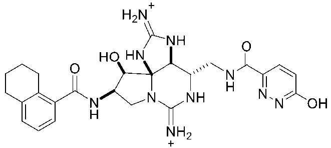 | 24 nM | 64 µM | |
| **51** | 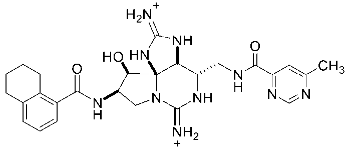 | 96 nM | NT | -- |
| **52** | 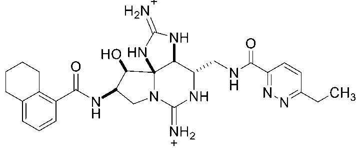 | 7 nM | 29 µM | |

**Table 2. Compounds**

| | **Compound** | **1 Nav 1.7** | **2 Nav 1.4** | **3** |
|---|---|---|---|---|
| **53** | 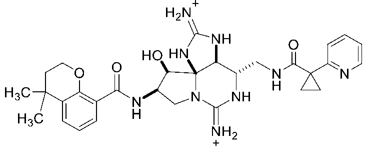 | 230 nM | NT | == |
| **54** | 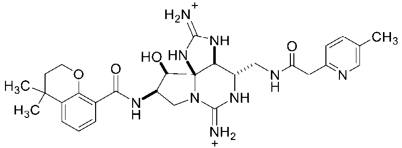 | 4.7 µM | NT | == |
| **55** | 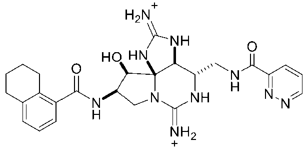 | 9.5 nM | 9.4 µM | |
| **56** | 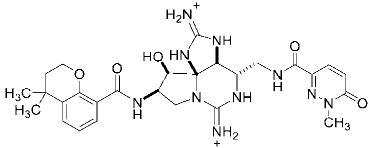 | 146 nM | NT | == |
| **57** | 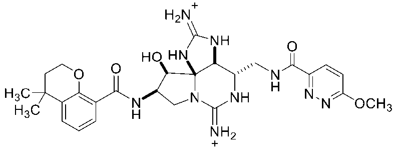 | 30 nM | NT | == |
| **58** | 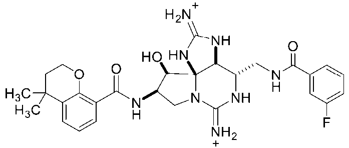 | 130 nM | NT | == |
| **59** | 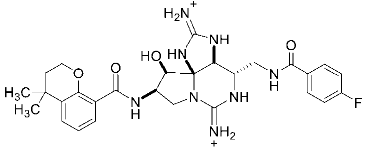 | 140 nM | NT | == |
| **60** | 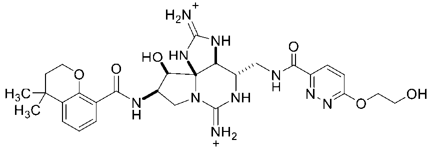 | 127 nM | 80 µM | |
| **61** | 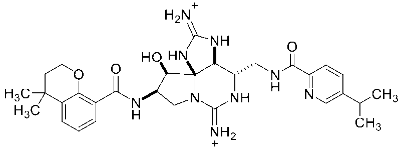 | 60 nM | 22.3 µM | |
| **62** | 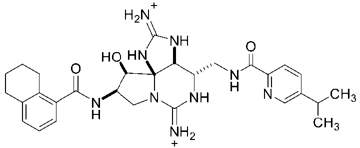 | 14 nM | 22.3 µM | |
| **63** | 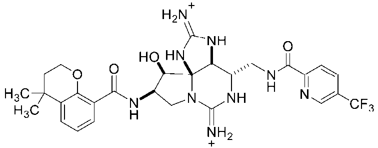 | 40 nM | 73.8 µM | |
| **64** | 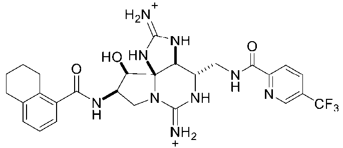 | 25 nM | 210 µM | |
| **65** | 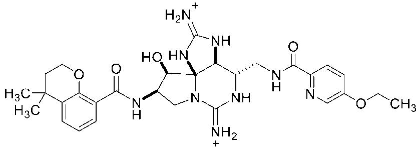 | 21 nM | 270 µM | |
| **66** | 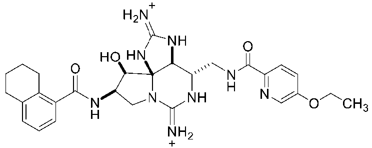 | 37.5 nM | 147 µM | |
| **67** | 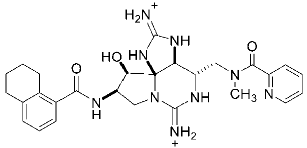 | 43 µM | NT | == |
| **68** | 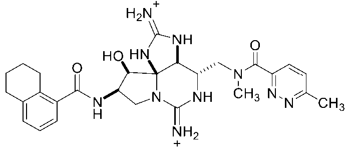 | 3 µM | NT | == |
| **69** | 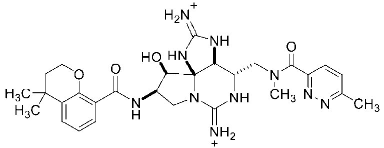 | 12 µM | NT | == |
| **70** | 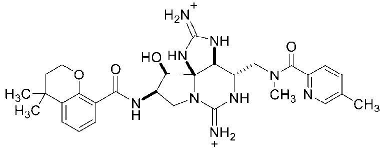 | 20 µM | NT | == |

**Table 3. Compounds**

| | **Compound** | **1 Nav 1.7** | **2 Nav 1.5** | **3** |
|---|---|---|---|---|
| **6** | 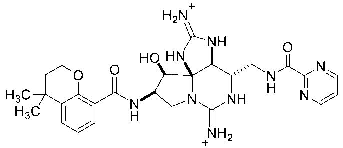 | 18 nM | 65 µM | 3600 |
| **7** | 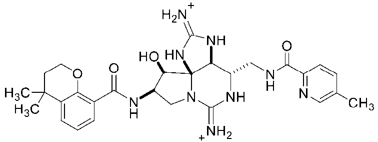 | 13 nM | 114 µM | 8700 |
| **8** | 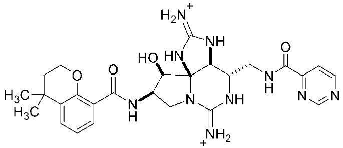 | 23 nM | 240 µM | 10400 |
| **12** | 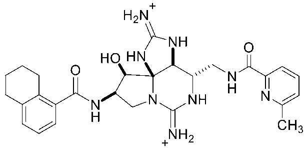 | 6.5 nM | 7.3 µM | 1100 |
| **13** | 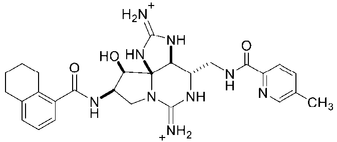 | 7 nM | 44.6 µM | 6350 |
| **16** | 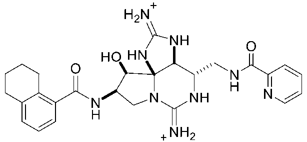 | 5 nM | 24.3 µM | 4800 |
| **49** | 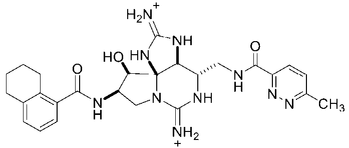 | 7 nM | 15 µM | |
| **50** | 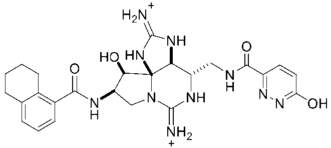 | 24 nM | 39 µM | |
| **61** | 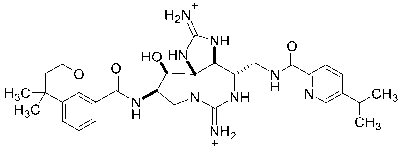 | 60 nM | 27.1 µM | |
| **62** | 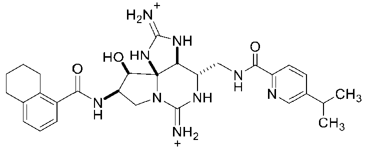 | 14 nM | 15.7 µM | |
| **63** | 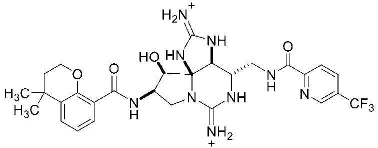 | 40 nM | 24.7 µM | |
| **64** | 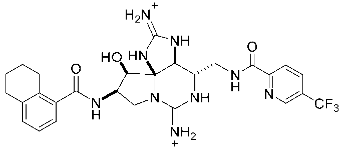 | 25 nM | 25 µM | |
| **65** | 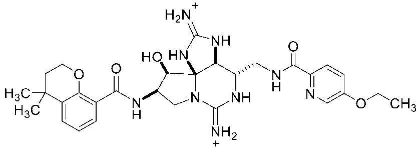 | 21 nM | 34 µM | |
| **66** | 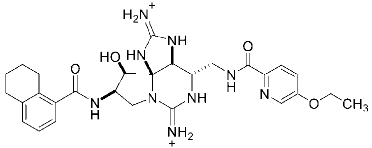 | 37.5 nM | 116.7 µM | |

**Table 4. Compounds**

| | **Compound** | **1 Nav 1.7** | **2 Nav 1.6** | **3** |
|---|---|---|---|---|
| **2** | 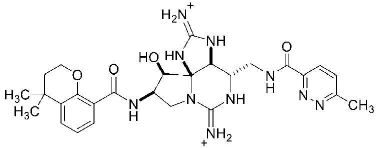 | 21 nM | 96 µM | 4500.0 |
| **5** | 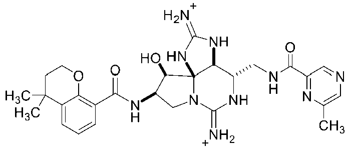 | 40 nM | 170 µM | 4000.0 |
| **6** | 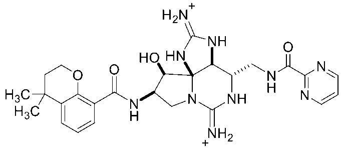 | 18 nM | >25 µM | 1380.0 |
| **7** | 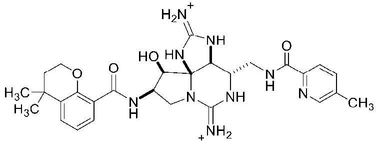 | 13 nM | 100 µM | 7500.0 |
| **8** | 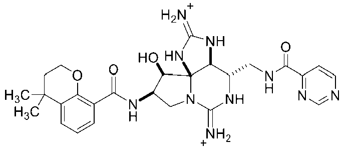 | 23 nM | 140 µM | 6000.0 |
| **9** | 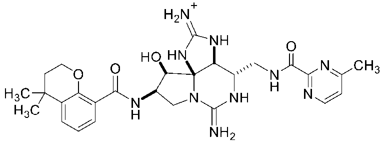 | 29 nM | 10 µM | 340.0 |
| **10** | 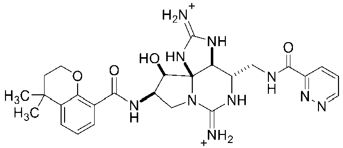 | 44 nM | 92 µM | 2000.0 |
| **12** | 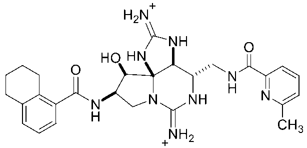 | 6.5 nM | 2.9 µM | 445.0 |
| **13** | 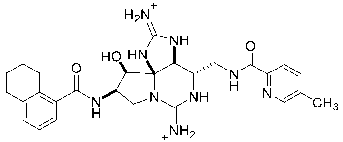 | 7 nM | 6 µM | 850.0 |
| **14** | 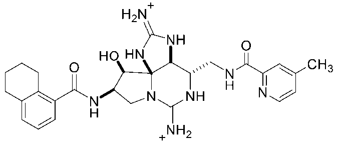 | 34 nM | 7.2 µM | 200.0 |
| **16** | 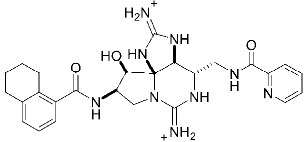 | 5 nM | 1.8 µM | 360.0 |
| **17** | 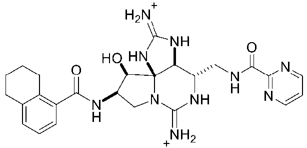 | 5 nM | 121 nM | 24.0 |
| **19** | 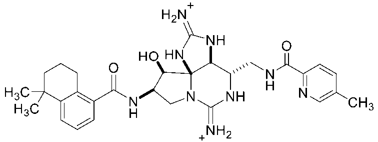 | 21 nM | 7.3 µM | 360.0 |
| **20** | 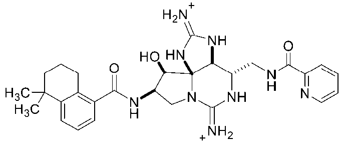 | 10 nM | 7.5 µM | 750.0 |
| **21** | 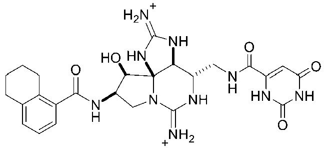 | 17 nM | 26 µM | 1500.0 |
| **22** | 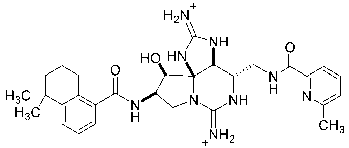 | 15 nM | 8.8 µM | 580.0 |
| **23** | 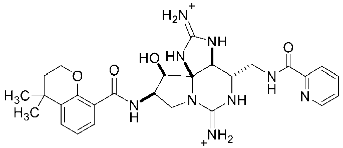 | 25 nM | 77 µM | 3000 |
| **24** | 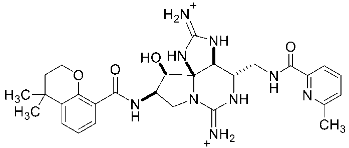 | 19 nM | 77 µM | 4000 |
| **25** | 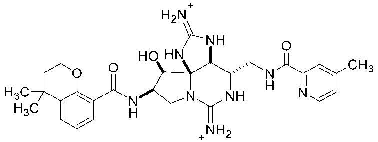 | 36.5 nM | 160 µM | 4300 |
| **27** | 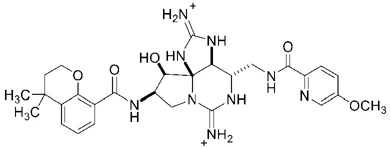 | 19 nM | 100 µM | 5200 |
| **28** | 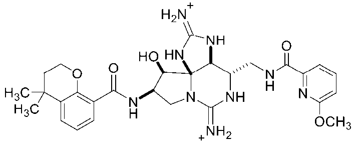 | 70 nM | 87 µM | 1200 |
| **29** | 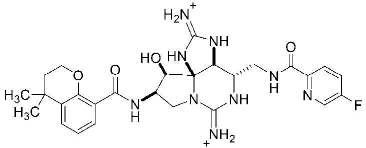 | 34 nM | 87 µM | 2500 |
| **30** | 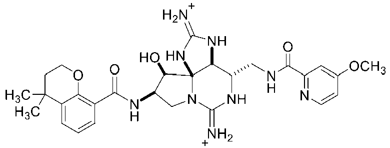 | 77 nM | 135 µM | 1700 |
| **32** | 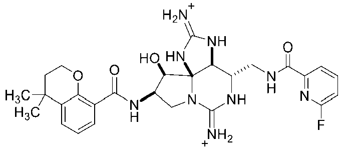 | 60 nM | 31 µM | 500 |
| **33** | 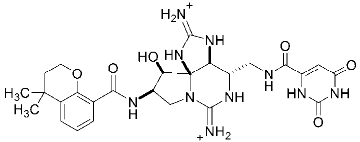 | 27 nM | 115 µM | 4200 |
| **34** | 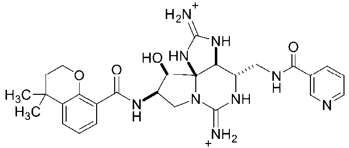 | 51 nM | 150 µM | 2900 |
| **36** | 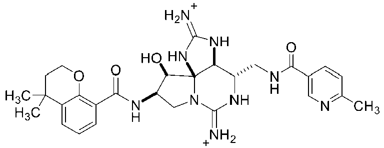 | 63 nM | 108 µM | 1700 |
| **39** | 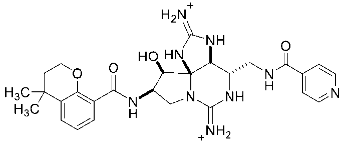 | 72 nM | 108 µM | 1500 |
| **40** | 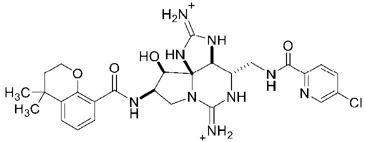 | 25 nM | 108 µM | 4300 |
| **41** | 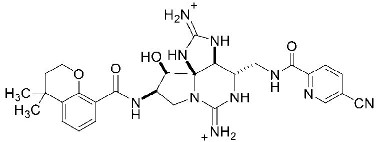 | 35 nM | 44 µM | 1200 |
| **43** | 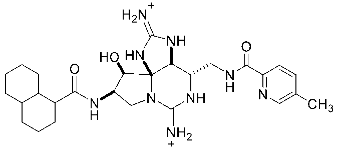 | 13 nM | 19.2 µM | 1470 |
| **44** | 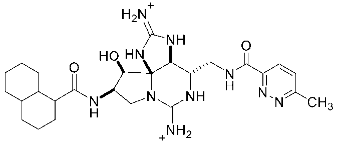 | 40 nM | 107 µM | 2650 |
| **45** | 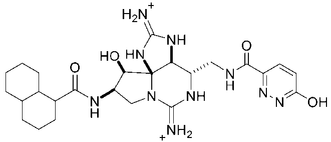 | 11 nM | 27 µM | 2450 |
| **46** | 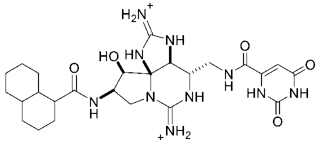 | 30 nM | 93 µM | 3100 |
| **49** | 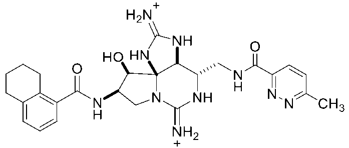 | 7 nM | 3.9 µM | 560 |
| **50** | 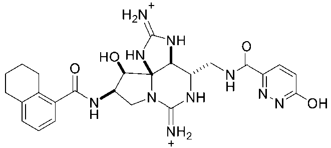 | 24 nM | 7.5 µM | 310 |
| **51** | 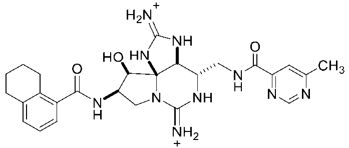 | 96 nM | 11 µM | 110 |
| **52** | 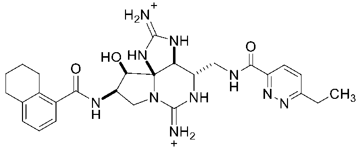 | 7 nM | 12 µM | 1710 |

### Example 3

### Degradation Assays

Compound **A** • 2AcOH, a Compound of Formula (I), was subjected to certain degradation conditions (acid hydrolysis, base hydrolysis, oxidation, heat, and photolysis) in order to demonstrate its stability.

The following UHPLC conditions were developed for the degradation studies:
Column: Acquity UPLC CSH C18, 2.1 x 150 mm, 1.7µm. P/N 186005298
Column Temperature: 30 °C
Sample Temperature: 5 °C
Detection Wavelength: 242 nm
Mobile Phase A: 0.1% trifluoroacetic acid in water
Mobile Phase B: 0.1% trifluoroacetic acid in acetonitrile
Flow Rate: 0.4 mL/min
Injection Volume: 2.0 µL
Data Collection Time: 31 minutes
Re-equilibration Time: 7 minutes
Needle Wash: 50/50 acetonitrile/water
Gradient:

| **Time (min)** | **%A** | **%B** |
|---|---|---|
| 0.0 | 95 | 5 |
| 20.0 | 75 | 25 |
| 30.0 | 5 | 95 |
| 31.0 | 5 | 95 |
| 31.1 | 95 | 5 |
| 38.0 | 95 | 5 |

Various oxidative conditions were explored. It was observed that both 3% and 0.9% H₂O₂ caused a major degradant to form, which was well resolved and identifiable. Compound **A** was not confirmed as spectrally pure, and a small peak (~0.04%) was observed to be co-eluting. Compound **A** • 2AcOH was next subjected to azonitrile-based radical initiators azobisisobutyronitrile (AIBN) and 4,4'-azobis(4-cyanovaleric acid) (ACVA) at 40 °C. A control solution of Compound **A** • 2 AcOH stored at 40 °C was included to ensure any bias due to solution temperature was negated. After approximately 7 days, the solutions were analyzed and it was observed that the 40 °C AIBN condition promoted the targeted 5% degradation while ACVA produced virtually no degradation. The control solution of Compound **A** • 2AcOH stored in method diluent at 40 °C showed no degradation. A summary of all oxidation results is presented in Table 5. In the peak purity column, N refers to co-elution of a substance. The weight % degradation was calculated using the formula: 100 - % control recovery (compared to unstressed Compound **A** • 2AcOH).

**Table 5. Oxidative Conditions and Targeted Degradation of Compound A •2AcOH**

| **Condition** | **Area %** | **Weight % Degradation** | **Peak Purity?** | **USP Resolution** |
|---|---|---|---|---|
| Unstressed | 97.8% | N/A | Y | 1.7 |
| 3% H₂O₂, 24 hrs | 91.2% | 7.2% | N | N/A |
| 0.9% H₂O₂, 24 hrs | 96.2% | 1.8% | N | N/A |
| 40 °C Control Standard 7 Days | 97.9% | 0.0% | Y | 1.7 |
| 5mM AIBN 40 °C 7 Days | 94.6% | 4.7% | Y | 1.5 |
| 5mM ACVA 40 °C 7 Days | 96.8% | <1% | Y | 1.5 |

As shown in Table 5, all peak purity and USP resolution criteria was met for the 5 mM AIBN condition at 40 °C for 7 days. Additionally, an overlay of an unstressed Compound **A** • 2 AcOH solution with that of a solution exposed to 5mM AIBN at 40 °C for 7 days showed that impurities present in Compound **A** appear to grow in intensity, which is evidence that the degradants formed are representative of the Compound **A** • 2 AcOH material and not secondary degradants. Therefore, 5mM AIBN (40 °C 7 days) condition represents a potential oxidation pathway of Compound **A** in a stability study. Compound **A** • 2AcOH was also subjected to acid and base hydrolysis, photolysis, and thermal stress. As shown in Table 6, there was virtually no degradation of Compound **A** under thermal, photolytic, or acidic stress, and about 5% degradation for base hydrolysis after 24 hours at ambient condition. In all cases, USP Resolution met ≥ 1.5 and the Compound A peak was confirmed as spectrally pure. Peak purity was measured by photodiode array (PDA), a type of UV/Vis detector that measures the absorbance of all wavelengths simultaneously.

**Table 6. Summary of Forced Degradation Experiments for Compound A • 2AcOH**

| **Condition** | Weight % Degradation | Area % | Peak Purity by PDA? | USP |
|---|---|---|---|---|
| Unstressed | N/A | 97.8 | Y | 1.7 |
| Acid Hydrolysis: (0.1N HCl in 90/10 Water/ACN, RT, 24 hrs) | N/A | 97.9 | Y | 1.7 |
| Base Hydrolysis: | 5.1 | 94.1 | Y | 1.6 |
| (0.1N NaOH in 90/10 Water/ACN, RT, 24 hrs) | | | | |
| Oxidation: (5 mM AIBN in 90/10 Water/ACN, 40 °C, 7 days) | 4.7 | 94.6 | Y | 1.7 |
| Heat:(105 °C, 7 days) | 1.5 | 97.1 | Y | 1.5 |
| Photolysis: UV at 350 nm: 200 W·hrs/m2; | 1.5 | 97.8 | Y | 1.7 |
| Visible@575 nm: 1.2 million lux·hrs | | | | |

### Example 4

### Stability in Rat and Monkey K₂EDTA Plasma

The stability of Compound **A** and a potential metabolite of Compound **A** (**M2**), formed from amide hydrolysis, was studied in rat or monkey K₂EDTA plasma. The stability of both compounds was measured using HPLC-MSMS. Stock solutions of Compound **A** and **M2** (1mg/mL) were prepared in DMSO from neat drug. The 1 mg/mL stock solutions were used in preparation of standard curve and QC working solutions. Dynamic range for Compound **A** was established from 2.00 - 2000 ng/mL. Dynamic range for **M2** was established from 4.00 - 4000 ng/mL. For both analytes a deuterated analog of Compound A (Compound A-d6) was used as an internal standardwas used as an internal standard (IS).

The procedure for sample/curve QC preparation is below:
1. Prepare curve and QCs by adding 25 µL of working solutions into 475 µL of matrix; vortex.
2. Aliquot 50 µL of each standard, QC, or sample into a 96-well plate.
3. Add 25 µL of 500 mg/mL internal standard in ammonium buffer or ammonium acetate buffer without IS for blanks, centrifuge then vortex briefly.
4. Precipitate proteins by addition of 250 µL of acetronitrile, vortex, and centrifuge briefly.
5. Transfer approximately 200 µL of each supernatant to a clean 96 well plate.
6. Add 100 µL of H₂O to each well, vortex, and centrifuge briefly.
7. Inject onto an HPLC/MS/MS/ instrument. HPLC conditions for the stability study are provided below:

### HPLC Conditions (Shimadzu LC-20AD pumps):

| | | | |
|---|---|---|---|
| Mobile Phase A: H₂O with 0.1% Formic acid | | Injection Volume: 1-40 µL | |
| Mobile Phase B: ACN with 0.1% Formic acid | | Column Heater Temperature: 50°C | |
| Rinse Solvent: DMF (v/v) | | Autosampler Temperature: 10°C | |
| Flow Rate: 0.7 mL/min | | HPLC Column: Supelco Ascentis RP-Amide, 100x4.6mm, 3µm OR Supelco Discovery HS-C18, 2.1x50mm, 3µm | |
| Pump B Starting Concentration: 5% Gradient: | | | |

| Time | Module | Events | Parameter |
|---|---|---|---|
| 0.50 | Pumps | Pump B | 15 |
| 2.00 | Pumps | Pump B | 50 |
| 2.10 | Pumps | Pump B | 95 |
| 2.15 | Pumps | Total Flow | 0.7 |
| 2.16 | Pumps | Total Flow | 1.0 |
| 2.80 | Pumps | Pump B | 95 |
| 3.00 | Pumps | Pump B | 35 |
| 3.50 | Pumps | Pump B | 35 |
| 3.75 | Pumps | Pump B | 95 |
| 4.25 | Pumps | Pump B | 95 |
| 4.50 | Pumps | Pump B | 35 |
| 5.00 | Pumps | Pump B | 35 |
| 5.25 | Pumps | Pump B | 5 |
| 6.01 | Pumps | Total Flow | 1.0 |
| 6.02 | Pumps | Total Flow | 0.7 |
| 6.50 | System | Stop | |

MS conditions for the stability study are provided below:

### MS Conditions (API-4000):

| | |
|---|---|
| CAD gas | 10 |
| CUR gas | 10 |
| GAS 1 | 50 |
| GAS 2 | 60 |
| ESI Voltage, V | 3000 |
| ESI Temperature, °C | 700 |
| Interface heater (IHE) | ON |
| DP (declustering potential) | 80 |
| EP (entrance potential) | 10 |
| CE (collision energy) | 50 |
| CXP (collision cell exit) | 12 |
| Analyte Transition, m/z (Compound A) | 563.5→ 189.1 |
| Analyte Transition, m/z (M2) | 444.3 → 190.2 |
| IS (Internal standard) Transition, m/z | 588.4 → 511.4 |
| Dwell Time, ms | 100 |

For Compound **A**, bench top stability was observed for up to 6 hours under ambient conditions. Free-thaw (F/T) stability was observed for at least n=4 F/T cycles. Long-term stability experiments confirmed approximately 34 days of storage stability at -70 ° for rat and monkey. All results are shown in Table 7 below. LQC refers to lower quality control; MQC refers to midle quality control; and, HQC refers to higher quality control.

**Table 7. Stability of Compound A in Rat and Monkey K₂EDTA plasma**

| **Rat 6-hour Bench Top Stability** | | | |
|---|---|---|---|
| | Nominal Concentration (ng/mL) | 6-hour Bench Top Concentration (ng/mL) | 6-hour Bench Top % Accuracy |
| LQC | 6.00 | 5.16 | 86.0 |
| MQC | 100 | 87.6 | 87.6 |
| HQC | 1600 | 1453 | 90.8 |

| **Monkey 6-hour Bench Top Stability** | | | |
|---|---|---|---|
| | Nominal Concentration (ng/mL) | 6-hour Bench Top Concentration (ng/mL) | 6-hour Bench Top % Accuracy |
| LQC | 6.00 | 6.82 | 114 |
| MQC | 100 | 113 | 113 |
| HQC | 1600 | 1820 | 114 |

| **Rat Freeze-Thaw Stability (n=4 cycles)** | | | |
|---|---|---|---|
| | Nominal Concentration (ng/mL) | Concentration after n=4 Freeze-Thaw Cycles (ng/mL) | % Accuracy after n=4 Freeze-Thaw Cycles |
| LQC | 6.00 | 5.90 | 98.3 |
| MQC | 100 | 112 | 112 |
| HQC | 1600 | 1607 | 100 |

| **Monkey Freeze-Thaw Stability (n=4 cycles)** | | | |
|---|---|---|---|
| | Nominal Concentration (ng/mL) | Concentration after n=4 Freeze-Thaw Cycles (ng/mL) | % Accuracy after n=4 Freeze-Thaw Cycles |
| LQC | 6.00 | 6.43 | 107 |
| MQC | 100 | 102 | 102 |
| HQC | 1600 | 1597 | 99.7 |

| **Rat 34-dav LTS at -70°C** | | | |
|---|---|---|---|
| | Nominal Concentration (ng/mL) | 34 Day Concentration (ng/mL) | % Accuracy at 34 days |
| LQC | 6.00 | 5.70 | 95.5 |
| MQC | 100 | 106 | 105 |
| HQC | 1600 | 1670 | 103 |

| **Monkey 34-day LTS at -70°C** | | | |
|---|---|---|---|
| | Nominal Concentration (ng/mL) | 34 Day Concentration (ng/mL) | % Accuracy at 34 days |
| LQC | 6.00 | 6.33 | 105 |
| MQC | 100 | 107 | 107 |
| HQC | 1600 | 1534 | 95.8 |

For Compound **M2**, bench top stability was observed for up to 2 hours under ambient conditions in rat plasma and up to 6 hours in ambient conditions in monkey plasma. Free-thaw (F/T) stability was observed for at least n=4 F/T cycles. Long-term stability experiments confirmed approximately 34 days of storage stability at -70 ° for rat and monkey. All results are shown in Table 8 below.

**Table 8. Stability of Compound M2 in Rat and Monkey K₂EDTA plasma**

| **Rat 2-hour Bench Top Stability** | | | |
|---|---|---|---|
| | Nominal Concentration (ng/mL) | 2-hour Bench Top Concentration (ng/mL) | 2-hour Bench Top % Accuracy |
| LQC | 12.0 | 12.7 | 106 |
| MQC | 200 | 221 | 111 |
| HQC | 3200 | 3550 | 111 |

| **Monkey 6-hour Bench Top Stability** | | | |
|---|---|---|---|
| | Nominal Concentration (ng/mL) | 6-hour Bench Top Concentration (ng/mL) | 6-hour Bench Top % Accuracy |
| LQC | 12.0 | 13.2 | 110 |
| MQC | 200 | 190 | 95.0 |
| HQC | 3200 | 2710 | 84.6 |

| **Rat Freeze-Thaw Stability (n=4 cycles)** | | | |
|---|---|---|---|
| | Nominal Concentration (ng/mL) | Concentration after n=4 Freeze-Thaw Cycles (ng/mL) | % Accuracy after n=4 Freeze-Thaw Cycles |
| LQC | 12.0 | 11.3 | 94.3 |
| MQC | 200 | 204 | 102 |
| HQC | 3200 | 3270 | 102 |

| **Monkey Freeze-Thaw Stability (n=4 cycles)** | | | |
|---|---|---|---|
| | Nominal Concentration (ng/mL) | Concentration after n=4 Freeze-Thaw Cycles (ng/mL) | % Accuracy after n=4 Freeze-Thaw Cycles |
| LQC | 12.0 | 11.9 | 98.9 |
| MQC | 200 | 210 | 105 |
| HQC | 3200 | 3087 | 96.4 |

| **Rat 34-day LTS at -70°C** | | | |
|---|---|---|---|
| | Nominal Concentration (ng/mL) | 34 Day Concentration (ng/mL) | % Accuracy at 34 days |
| LQC | 12 | 10.76 | 89.5 |
| MQC | 200 | 200.25 | 100.1 |
| HQC | 3200 | 2905 | 90.8 |

| **Monkey 34-day LTS at -70°C** | | | |
|---|---|---|---|
| | Nominal Concentration (ng/mL) | 34 Day Concentration (ng/mL) | % Accuracy at 34 days |
| LQC | 12 | 11.5 | 97.3 |
| MQC | 200 | 195.9 | 97.9 |
| HQC | 3200 | 2756.3 | 86.1 |

### Example 5

### Stability in Male Cynomolgus Monkey Plasma

The stability of Compound **A** and Compound **B** in male Cynomolgus monkey plasma was determined at room temperature and at 4 °C. To prepare the solutions, a 10 µL solution (containing 2 µM of Compound **A** or Compound **B** in ACN/water (v:v 70:30)) was spiked into 190 µL blank fresh matrix and vortex-mixed, then each aliquot of 30 µL was transferred separately into polyethylene vials for stability samples. The samples were then stored at room temperature or 4 °C. After 2 hours, the stability samples were protein precipitated with 3000 µL IS solution (internal standard solution, 100 ng/mL dexamethasone, 100 ng/mL tolbutamide, 100 ng/mL glyburide, and 100 ng/mL verapamil in ACN), vortex-mixed for 1 minute and centrifuged at 13000 rpm for 15 minutes at 4 °C. This procedure was repeated at the 1 hour, 30 minute, and 10 minute timepoint. The stability at 0 hours was determined by immediately protein precipitating the stability sample. At each timepoint, the stability of the compounds were tested three times. The mean area ratio and the mean percentage of analyte remaining for each timepoint is shown in Table 9 and Table 10 for Compound **A** and Compound **B**, respectively. Both Compound A and Compound **B** were stable up to 2 hours at room temperature and at 4 °C in the monkey plasma.

**Table 9. Stability of Compound A in male Cynomolgus monkey plasma for 2 hours at room temperature and 4 °C**

| | **Room temperature** | | **4 °C** | |
|---|---|---|---|---|
| **Timepoint** | **Mean Area Ratio** | **Analyte Remaining (%)** | **Mean Area Ratio** | **Analyte Remaining (%)** |
| 0 | 1.89E-02 | 100 | 2.01E-02 | 100 |
| 10 minutes | 2.24E-02 | 119 | 2.05E-02 | 102 |
| 30 minutes | 1.77E-02 | 93.5 | 1.79E-02 | 89.1 |
| 1 hour | 1.74E-02 | 92.1 | 2.01E-02 | 99.8 |
| 2 hour | 1.71E-02 | 90.7 | 1.71E-02 | 84.9 |

**Table 10. Stability of Compound B in male Cynomolgus monkey plasma for 2 hours at room temperature and 4 °C**

| | **Room temperature** | | **4 °C** | |
|---|---|---|---|---|
| **Timepoint** | **Mean Area Ratio** | **Analyte Remaining (%)** | **Mean Area Ratio** | **Analyte Remaining (%)** |
| 0 | 1.91E-02 | 100 | 1.92E-02 | 100 |
| 10 minutes | 1.71E-02 | 89.7 | 1.82E-02 | 94.8 |
| 30 minutes | 1.61E-02 | 84.5 | 1.62E-02 | 84.3 |
| 1 hour | 1.63E-02 | 85.2 | 1.65E-02 | 85.9 |
| 2 hour | 1.86E-02 | 97.6 | 1.53E-02 | 80.0 |

### Example 6

### Hepatocyte Stability Assay

Metabolic stability of compounds can be evaluated using human, rat, mouse, or other animal hepatocytes to predict intrinsic clearance. Human LiverPoolTM 20-donor and animal cryopreserved hepatocytes are obtained from BioreclamationIVT. Cryopreserved hepatocytes are removed from the liquid nitrogen tank and thawed in a 37 °C water bath. As soon as the cells pull away from the vial wall, they are decanted into 48 mL of warm HT (hypoxanthine-thymidine) medium. Cells are centrifuged for four minutes at 420 rpm (50 g). After removing the supernatant, pellet is re-suspended in warm DMEM medium (Dulbecco's Modified Eagle Medium). Cell density is counted by a hemacytometer.

The assay is carried out in 96-well microtiter plates. Compounds are incubated for 0, 60, 120, and 180 minutes at 37 °C with hepatocytes. Reaction mixtures (50 µL) contain a final concentration of 1 µM test compound, 0.5 million cells/mL hepatocytes in the DMEM medium. At each of the time points (for example, 0, 1, 2, and 3 hours), 150 µL of quench solution (100% acetonitrile with 0.1% formic acid) with internal standard is transferred to each well. Midazolam is included as a positive control to verify assay performance. Plates are sealed and centrifuged at 4°C for 15 minutes at 4000 rpm. The supernatant is transferred to fresh plates for LC/MS/MS analysis.

All samples are analyzed on LC/MS/MS using an AB Sciex API 4000 instrument, coupled to a Shimadzu LC-20AD LC Pump system. Analytical samples are separated using a Waters Atlantis T3 dC18 reverse phase HPLC column (20 mm x 2.1 mm) at a flow rate of 0.5 mL/min. The mobile phase consists of 0.1% formic acid in water (solvent A) and 0.1% formic acid in 100% acetonitrile (solvent B). Elution conditions are detailed in below.

| **Time (min)** | **Flow (µL/min)** | **%A** | **%B** |
|---|---|---|---|
| 0 | 500 | 98 | 5 |
| 0.30 | 500 | 98 | 5 |
| 1.30 | 500 | 2 | 98 |
| 1.70 | 500 | 2 | 98 |
| 1.71 | 500 | 98 | 5 |
| 2.50 | 500 | 98 | 5 |

The extent of metabolism is calculated as the disappearance of the test compound, compared to the 0-min control reaction incubations. Initial rates are calculated for the compound concentration and used to determine t_{1/2} values and subsequently, the intrinsic clearance, CLint = (0.693)(1/ t_{1/2} (min))(mL incubation/million cells).

This method of determination of the intrinsic clearance makes the assumption that the testing concentration is far below the Michaelis-Menten constant of the compound to its metabolizing enzymes.

### Example 7

### Metabolic Stability in Human Plasma

Human plasma (K₂EDTA) is obtained from Bioreclamation IVT and the assay is carried out in 96-well microtiter plates. Reaction mixtures (50 µL) contain a final concentration of 1 µM test compound. The extent of metabolism is calculated as the disappearance of the test compound compared to the 0-min control reaction incubations. Propantheline is included as a positive control to verify assay performance.

At each of the four time points, 250 µL of quench solution (50% acetonitrile and 50% methanol) with internal standard (bucetin for positive ESI mode) is transferred to each well. Plates are sealed, vortexed, and centrifuged at 4 °C for 15 minutes at 4000 rpm. The supernatant is transferred to fresh plates for LC/MS/MS analysis.

All samples are analyzed on LC/MS/MS using an AB Sciex API 4000 instrument, coupled to a Shimadzu LC-20AD LC Pump system. Analytical samples are separated using a Waters Atlantis T3 dC18 reverse phase HPLC column (10 mm x 2.1 mm) at a flow rate of 0.5 mL/min. The mobile phase consists of 0.1% formic acid in water (solvent A) and 0.1% formic acid in 100% acetonitrile (solvent B). Elution conditions are detailed below.

| **Time (min)** | **Flow (µL/min)** | **%A** | **%B** |
|---|---|---|---|
| 0 | 500 | 98 | 2 |
| 0.30 | 500 | 98 | 2 |
| 1.40 | 500 | 2 | 98 |
| 2.00 | 500 | 2 | 98 |
| 2.01 | 500 | 98 | 2 |
| 2.50 | 500 | 98 | 2 |

Initial rates of the clearance of test compounds are calculated using linear regression of semi-log plot of % remaining of compounds versus time. The elimination rate constant (equals to -slope) of the linear regression is then used to determine t_{1/2} values.

### Example 8

### Ratio of Hydrated Ketone 11S and 11R Diastereomers for Comparative Compound in Rat, Monkey, and Human Blood:Buffer

The ratio of diastereomers (S-C11 and R-C11) for a select hydrated ketone ("Comparative Compound") upon addition to human, monkey, and rat 1:1 K₂EDTA whole blood:buffer (9.2 mM ammonium acetate, pH 6.8) was determined. Stock solutions of Comparative Compound were prepared at 1 mg/mL in DMSO from two separated lots of Comparative Compound. Each lot was composed of a different ratio of 11S to 11R. The first lot was high in 11S as compared to 11R ("High 11S Material"). The second lot was high in 11R compared to 11S ("High 11R Material").

Stock solutions of Comparative Compound (1 mg/mL) were used in the preparation of standard curve and QC Working Solutions. The following experimental sequence was repeated for both Working Solutions originating from High 11S Material and those originating from High 11R Material. Whole blood was incubated at 37 °C for approximately 30 minutes prior to being spiked with the Working Solutions at the LQC (lower quality control), MQC (middle quality control), and HQC (higher quality control) levels. These were then extracted via acetonitrile precipitation.

The procedure for sample/curve QC preparation is below:
1. Prepare curve and QCs by adding 25 µL of Working Solutions into 475 µL of matrix; vortex.
2. Aliquot 50 µL of each standard, QC, or sample into a 96-well plate.
3. Add 25 µL of 4000 mg/mL internal standard in ammonium buffer or ammonium acetate buffer without IS (internal standard) for blanks, centrifuge then vortex briefly.
4. Precipitate proteins by addition of 250 µL of acetronitrile, vortex, and centrifuge briefly.
5. Transfer approximately 100 µL of each supernatant to a clean 96 well plate.
6. Add 100 µL of H₂O to each well, vortex, and centrifuge briefly.
7. Inject onto an HPLC/MS/MS/ instrument.

HPLC conditions for the study are provided below:

### HPLC Conditions (Shimadzu LC-20AD pumps):

| | | | |
|---|---|---|---|
| Mobile Phase A: H₂O with 0.1% formic acid | | Injection Volume: 1-20 µL | |
| Mobile Phase B: ACN with 0.1% formic acid | | Column Heater Temperature: 50 °C | |
| Rinse Solvent: 1:1 MeOH:H₂O (v/v) | | Autosampler Temperature: 10 °C | |
| Flow Rate: 0.7 mL/min | | HPLC Column: Supelco Discovery HS-C18, 2.1x50mm, 3µm | |
| Gradient: | | | |
| 0.50 | Pumps | Pump B Conc. | 25 |
| 1.98 | Pumps | Total Flow | 0.7 |
| 1.99 | Pumps | Total Flow | 1.4 |
| 2.00 | Pumps | Pump B | 35 |
| 2.25 | Pumps | Pump B | 95 |
| 3.00 | Pumps | Pump B | 95 |
| 3.25 | Pumps | Pump B | 40 |
| 4.00 | Pumps | Pump B | 40 |
| 4.25 | Pumps | Pump B | 95 |
| 4.90 | Pumps | Total Flow | 1.4 |
| 4.95 | Pumps | Total Flow | 0.7 |
| 5.00 | Pumps | Pump B | 95 |
| 5.50 | Pumps | Pump B | 15 |
| 6.00 | System | Stop | |

MS conditions for the stability study are provided below:

### MS Conditions (API-4000):

| | |
|---|---|
| CAD gas | 12 |
| CUR gas | 10 |
| GAS 1 | 50 |
| GAS 2 | 60 |
| ESI Voltage, V | 5000 |
| ESI Temperature, °C | 700 |
| Interface heater (IHE) | ON |
| DP (declustering potential) | 80 |
| EP (entrance potential) | 10 |
| CE collision energy) (hydrated ketone internal standard (IS)) | 60, 40 |
| CXP (collision cell exit) | 20 |
| Analyte Transition, m/z | 582.2 - 189.2 |
| ISTransition, m/z | 588.4 - 511.6 |
| Dwell Time, ms | 100 |

Upon addition to whole blood, the diastereomers of the Comparative Compound epimerized and came to a relatively constant ratio in monkey, rat, and human whole blood buffer. The C11-R and C11-S equilibrium of Comparative Compound favored the C11-S for both the high 11R standard and the high 11S standard. This result was consistent across human, rat, and monkey species. This is shown in FIGS. 1A, 1B, 2A, 2B, 3A, and 3B. In FIG. 1A, the 11S and 11R isomer percentage of total peak area from the high 11S standard is shown for the high, medium, and low quality control in monkey blood. The percentage of the 11S and 11R diastereomers in the neat high 11S material is also shown. FIG. 1B is the isomer percentage of total peak area from the high 11R standard for the high, medium, and low quality control in monkey blood. The percentage of the 11S and 11R diastereomers in the neat high 11R material is also shown. As shown in FIG. 1A and FIG. 1B, the ratio of diasteromers in both the high 11S and high 11R standards come to a realtively constant ratio upon addition to blood and this equilibrium favors the C11-S diastereomer. This same effect is shown in FIG. 2A, FIG. 2B, FIG. 3A, and FIG. 3B. FIG. 2A is the isomer percentage of the 11S and 11R diasteomers from the high 11S standard in human blood compared to the neat high 11S material. FIG. 2B is the isomer percentage of the 11S and 11R diasteomers from the high 11R standard in human blood compared to the neat high 11R material. FIG. 3A and FIG. 3B show this effect in rat blood. FIG. 3A shows the isomer percentage of the high 11S standard compared to the high 11S neat material and FIG. 3B shows the isomer percentage of high 11R standard compared to the high 11R material.

All publications, patents, and patent applications cited in this specification are herein incorporated by reference as if each individual publication, patent, or patent application were specifically and individually indicated to be incorporated by reference. While the claimed subject matter has been described in terms of various embodiments, the skilled artisan will appreciate that various modifications, substitutions, omissions, and changes may be made without departing from the spirit thereof. Accordingly, it is intended that the scope of the claimed subject matter is limited solely by the scope of the following claims, including equivalents thereof.

Embodiments of the invention are set out in the following clauses.
1. A compound of Formula (I): 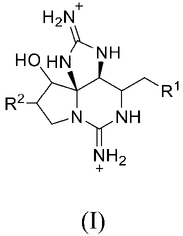 wherein
R¹ is -NR^{3a}R³, -NR⁴C(O)R^{4a}, -CH₂NR⁴C(O)R^{4a}, heteroaryl, -CH₂NH₂, -CH₂CH₂NH₂, -CH₂NH₃⁺, -CH₂CH₂NH₃⁺, -NR⁵S(O)₂R^{5a}, -CH₂NR⁵S(O)₂R^{5a}, -NR⁶C(O)NR^{6a}R^{6b}, -CH₂NR⁶C(O)NR^{6a}R^{6b}, -NR⁸C(O)OR^{8a}, or -CH₂NR⁸C(O)OR^{8a};
R² is -NR⁷C(O)R^{7a}, -NR⁷S(O)₂R^{7a}, or -NR⁷C(O)OR^{7a};
R³ is hydrogen or C₁₋₆alkyl;
R^{3a} is hydrogen or C₁₋₆alkyl;
R⁴ is hydrogen or C₁₋₆alkyl;
R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
one pair of two R^{4b} groups are present on one carbon, the second pair of two R^{4b} are present on another carbon, and each pair of R^{4b} are taken together to form an oxo group;
R⁵ is hydrogen or C₁₋₆alkyl;
R^{5a} is heterocycloalkyl, amino, C₁₋₆alkylamino, or di- C₁₋₆alkylamino;
R⁶ is hydrogen or C₁₋₆alkyl;
R^{6a} is hydrogen, or C₁₋₆alkyl;
R^{6b} is hydrogen, C₁₋₆alkyl, C₁₋₆alkoxy or cycloalkyl;
R⁸ is hydrogen or C₁₋₆alkyl;
R^{8a} is hydrogen, C₁₋₆alkyl, cycloalkyl, or phenyl;
R⁷ is hydrogen or C₁₋₆alkyl;
R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; or
a pharmaceutically acceptable salt, hydrate, solvate, stereoisomer, tautomer, mixture, or combination thereof.
2. The Compound of clause 1
wherein
R¹ is -NR^{3a}R³, -NR⁴C(O)R^{4a}, -CH₂NR⁴C(O)R^{4a}, heteroaryl, -CH₂NH₂, -CH₂CH₂NH₂, -CH₂NH₃⁺, -CH₂CH₂NH₃⁺, -NR₅S(O)₂R^{5a}, -CH₂NR⁵S(O)₂R^{5a}, -NR⁶C(O)NR^{6a}R^{6b}, -CH₂NR⁶C(O)NR^{6a}R^{6b}, or -NR⁸C(O)OR^{8a}, or -CH₂NR⁸C(O)OR^{8a};
R² is -NR⁷C(O)R^{7a}, -NR⁷S(O)₂R^{7a}, or -NR⁷C(O)OR^{7a};
R³ is hydrogen or C₁₋₆alkyl;
R^{3a} is hydrogen or C₁₋₆alkyl;
R⁴ is hydrogen or C₁₋₆alkyl;
R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl; cycloalkylalkyl; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
each R^{4b}, when present, is independently C₁₋₆alkyl, hydroxy, halo, halo-C₁₋₆alkyl, C₁₋₆alkoxy, cyano, or phenyl;
R⁵ is hydrogen or C₁₋₆alkyl;
R^{5a} is heterocycloalkyl, amino, C₁₋₆alkylamino, or di- C₁₋₆alkylamino;
R⁶ is hydrogen or C₁₋₆alkyl;
R^{6a} is hydrogen, or C₁₋₆alkyl;
R^{6b} is hydrogen, C₁₋₆alkyl, C₁₋₆alkoxy or cycloalkyl;
R⁸ is hydrogen or C₁₋₆alkyl;
R^{8a} is hydrogen, C₁₋₆alkyl, cycloalkyl or phenyl;
R⁷ is hydrogen or C₁₋₆alkyl;
R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl; or
a pharmaceutically acceptable salt, hydrate, solvate, stereoisomer, tautomer, mixture, or combination thereof.
3. The Compound of clause 1 or 2, wherein the compound is according to Formula (P-Ia): 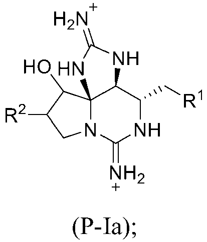 or a pharmaceutically acceptable salt, hydrate, solvate, stereoisomer, tautomer, mixture, or combination thereof.
4. The Compound of any one of clauses 1-3, wherein the compound is according to Formula (P-Ib-1) or (P-Ib-2): 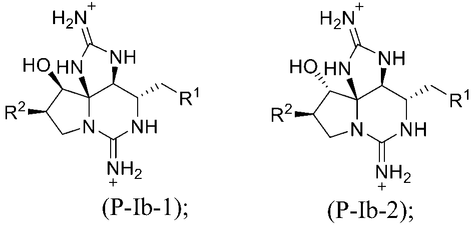 or a pharmaceutically acceptable salt, hydrate, solvate, stereoisomer, tautomer, mixture, or combination thereof.
5. The Compound of any one of clauses 1-4, wherein R¹ is -NR^{3a}R³ or -NR⁴C(O)R^{4a}.
6. The Compound of any one of clauses 1-4, wherein R¹ is -NR⁴C(O)R^{4a},
7. The Compound of any one of clauses 1-6, wherein R⁴ is hydrogen.
8. The Compound of any one of clauses 1-7, wherein R^{4a} is aryl optionally substituted with 1, 2, 3, or 4 R^{4b} groups; cycloalkyl optionally substituted with 1, 2, 3, or 4 R^{4b} groups; heteroaryl optionally substituted with 1, 2, 3, or 4 R^{4b} groups; heterocylic optionally substituted with 1, 2, 3, or 4 R^{4b} groups; or heteroarylalkyl optionally substituted with 1, 2, 3, or 4 R^{4b} groups.
9. The Compound of any one of clauses 1-8, wherein R^{4a} is heteroaryl optionally substituted with 1, 2, 3, or 4 R^{4b} groups.
10. The Compound of any one of clauses 1-9, wherein R^{4a} is pyridinyl, pyrazinyl, pyrimidinyl, or prazinyl, where R^{4a} is optionally substituted with 1, 2, 3, or 4 R^{4b} groups.
11. The Compound of any one of clauses 1-10, wherein R^{4a} is unsubstituted heteroaryl.
12. The Compound of any one of clauses 1-8, wherein R^{4a} is aryl optionally substituted with 1, 2, 3 or 4 R^{4b} groups.
13. The Compound of any one of clauses 1-7, wherein R^{4a} is hydrogen.
14. The Compound of any one of clauses 1-7, wherein R^{4a} is C₁₋₆alkyl.
15. The Compound of any one of clauses 1-7, wherein R^{4a} halo-C₁₋₆alkyl.
16. The Compound of any one of clauses 1-7, wherein R^{4a} is amino-C₁₋₆alkyl; C₁₋₆alkylaminoC₁₋₆alkyl; or di-C₁₋₆alkylaminoC₁₋₆alkyl.
17. The Compound of any one of clauses 1-7, wherein R^{4a} is C₁₋₆alkoxyC₁₋₆alkyl.
18. The Compound of any one of clauses 1-7, wherein R^{4a} is cycloalkyl optionally substituted with 1 or 2 R^{4b} groups.
19. The Compound of clause 18, wherein one R^{4b} is present and is heteroaryl.
20. The Compound of any one of clauses 1-7, wherein R^{4a} is unsubstituted cycloalkylalkyl.
21. The Compound of any one of clauses 1-7, wherein R^{4a} is aralkyl where the aryl portion is optionally substituted with 1, 2, 3, or 4 R^{4b}.
22. The Compound of any one of clauses 1-8, wherein R^{4a} is heteroarylalkyl where the heteroaryl portion is optionally substituted with 1, 2, 3, or 4 R^{4b} groups.
23. The Compound of any one of clauses 1-10, 12, 18, and 21-22, wherein each R^{4b}, when present, is independently C₁₋₆alkyl, hydroxy, hydroxyC₁₋₆alkyloxy, halo, halo-C₁₋₆alkyl, or C₁₋₆alkoxy.
24. The Compound of any one of clauses 1-10, 12, 18, and 21-22, wherein each R^{4b}, when present, is independently C₁₋₆alkyl or hydroxy.
25. The Compound of any one of clauses 1-10, 12, 18, and 21-24, wherein one R^{4b} is present.
26. The Compound of any one of clauses 1-10, 12, 18, and 21-24, wherein two R^{4b} are present.
27. The Compound of any one of clauses 8-10 and 22, wherein each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl.
28. The Compound of any one of clauses 8-10 and 22, wherein one pair of two R^{4b} groups are present on one carbon, the second pair of two R^{4b} are present on another carbon, and each pair of R^{4b} are taken together to form an oxo group.
29. The Compound of any one of clauses 1-4, wherein R¹ is -NR^{3a}R³.
30. The Compound of any one of clauses 1-4, and 29, wherein R^{3a} and R³ are both C₁₋₆alkyl; or R^{3a} and R³ are both hydrogen.
31. The Compound of any one of clauses 1-4, wherein R¹ is -NR⁵S(O)₂R^{5a}.
32. The Compound of any one of clauses 1-4, and 31, wherein R⁵ is hydrogen.
33. The Compound of any one of clauses 1-4, wherein R¹ is -NR⁶C(O)NR^{6a}R^{6b}.
34. The Compound of any one of clauses 1-4 and 33 wherein R⁶ is hydrogen.
35. The Compound of any one of clauses 1-4, wherein R¹ is -NR⁸C(O)OR^{8a}.
36. The Compound of any one of clauses 1-4, and 35, wherein R⁸ is hydrogen.
37. The Compound of any one of Clauses 1-36, wherein R⁷ is hydrogen.
38. The Compound of any one of Clauses 1-37, wherein R^{7a} is heterocyclic optionally substituted with 1, 2, 3, or 4 R^{7b}.
39. The Compound of any one of Clauses 1-38, wherein the heterocyclic in R^{7a} is benzo-1,4-dioxanyl, benzodioxolyl, 2,3-dihydrobenzofuranyl, chromanyl, 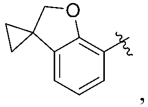 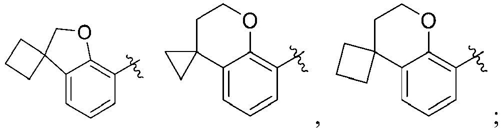 each of which is optionally substituted with 1, 2, or 3 groups independently selected from halo, C₁₋₆alkyl, halo-C₁₋₆alkyl, hydroxy, C₁₋₆alkoxy, halo-C₁₋₆alkoxy, and phenyl.
40. The Compound of any one of Clauses 1-38, wherein R^{7a} is unsubstituted 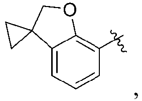 unsubstituted 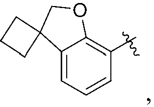 unsubstituted 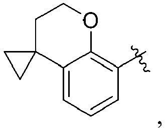 or unsubstituted 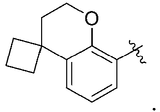 41. The Compound of any one of Clauses 1-38, wherein the heterocyclic in R^{7a} comprises one heteroatom which is oxygen and wherein the heterocyclic is optionally substituted with 1, 2, or 3 groups independently selected from halo, C₁₋₆alkyl, halo-C₁₋₆alkyl, hydroxy, C₁₋₆alkoxy, halo-C₁₋₆alkoxy, and phenyl.
42. The Compound of any one of Clauses 1-38, wherein the heterocyclic in R^{7a} comprises one heteroatom which is oxygen and wherein the heterocyclic is optionally substituted with one gem-di-C₁₋₃alkyl or one gem-dihalo.
43. The Compound of any one of Clauses 1-38, wherein the heterocyclic in R^{7a} is benzo-1,4-dioxanyl optionally substituted with one gem-di-C₁₋₃alkyl, cyclopropyl, or one gem-dihalo.
44. The Compound of any one of Clauses 1-38, wherein R^{7a} is 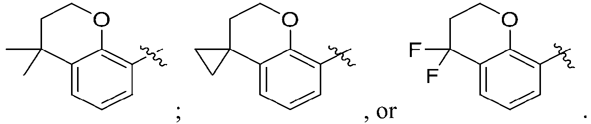 45. The Compound of any one of Clauses 1-37, wherein R^{7a} is aryl optionally substituted with 1, 2, 3, or 4 R^{7b}.
46. The Compound of any one of Clauses 1-37, and 45, wherein the aryl in R^{7a} is phenyl, naphthyl, tetrahydronaphthyl, fluorenyl, 6,7,8,9-tetrahydro-5*H*-benzo[7]annulenyl, 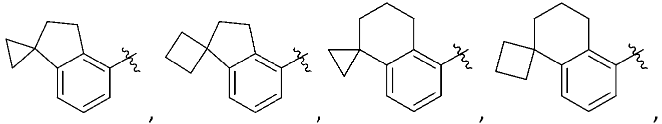 or indanyl; each of which is optionally substituted with 1, 2, or 3 groups independently selected from halo, C₁₋₆alkyl, halo-C₁₋₆alkyl, hydroxy, C₁₋₆alkoxy, halo-C₁₋₆alkoxy, C₁₋₆alkylthio, C₁₋₆alkylsulfonyl, and amino.
47. The Compound of any one of Clauses 1-37, and 45, wherein the aryl in R^{7a} is tetrahydronaphthyl, 6,7,8,9-tetrahydro-5*H*-benzo[7]annulenyl, or indanyl; each of which is optionally substituted with one gem-di-C₁₋₃alkyl or one gem-di-halo.
48. The Compound of any one of Clauses 1-37, and 45, wherein R^{7a} is unsubstituted 6,7,8,9-tetrahydro-5*H*-benzo[7]annulenyl, unsubstituted 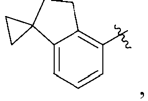 unsubstituted 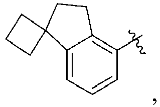 unsubstituted 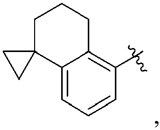 or unsubstituted 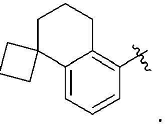 49. The Compound of any one of Clauses 1-37, wherein R^{7a} is 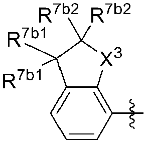 where X³ is -O-, -O-C(R^{7b3})(R^{7b3})-, -C(R^{7b3})(R^{7b3})-O-, -C(R^{7b3})(R^{7b3})-, -C(R^{7b3})(R^{7b3})-C(R^{7b4})(R^{7b4})-, or
-C(R^{7b3})(R^{7b3})-C(R^{7b4})(R^{7b4})-C(R^{7b5})(R^{7b5})- and each R^{7b1}, R^{7b2}, R^{7b3}, R^{7b4}, and R^{7b5} is independently hydrogen, halo, or C₁₋₃-alkyl.
50. The Compound of Clause 49, wherein a) one R^{7b1} is methyl or ethyl and the other R^{7b1} is hydrogen, or b) the two R^{7b1} are both hydrogen, or c) the two R^{7b1} are both methyl; and each R^{7b2}, R^{7b3}, R^{7b4}, and R^{7b5} are hydrogen.
51. The Compound of Clause 49 or 50, wherein X³ is -C(R^{7b3})(R^{7b3})-O-.
52. The Compound of Clause 49 or 50, wherein X³ is -C(R^{7b3})(R^{7b3})-C(R7b⁴)(R^{7b4})-.
53. The Compound of any one of clauses 1-37, where R^{7a} is 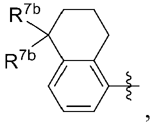 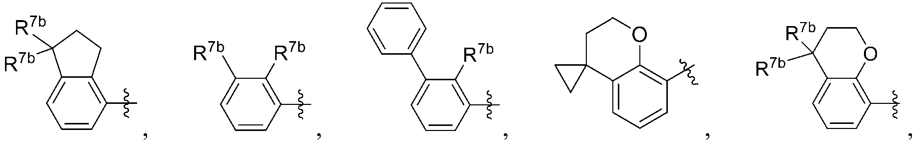 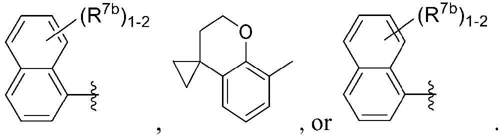 54. The Compound of Clause 53, wherein R^{7a} is 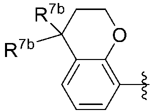 or tetrahydronaphthyl.
55. The Compound of clause 53 or 54, wherein a) one R^{7b} is methyl or ethyl and the other R^{7b} is hydrogen, b) the two R^{7b} are both hydrogen, or c) the two R^{7b} are both methyl.
56. The Compound of clause 1, or a pharmaceutically acceptable salt, hydrate, solvate, stereoisomer, tautomer, mixture, or combination thereof, selected from the group consisting of:

| | |
|---|---|
| **1** | 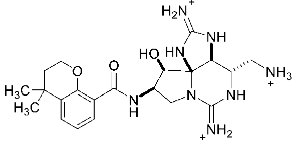 |
| **2** | 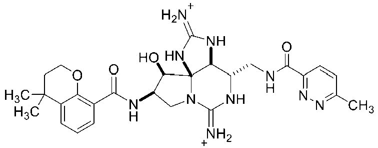 |
| **3** | 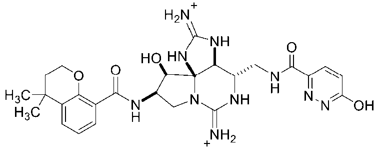 |
| **4** | 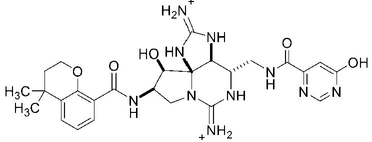 |
| **5** | 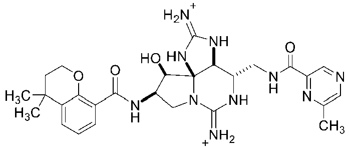 |
| **6** | 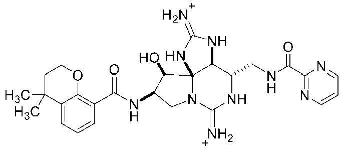 |
| **7** | 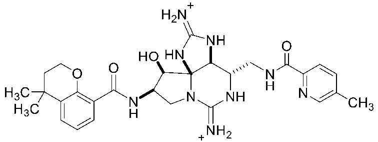 |
| **8** | 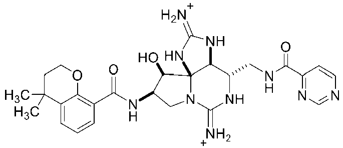 |
| **9** | 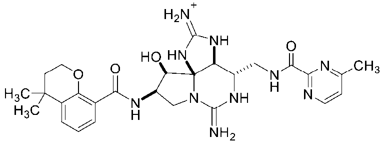 |
| **10** | 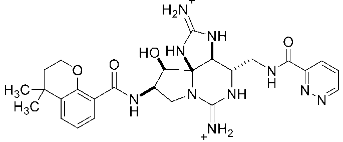 |
| **11** | 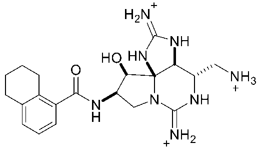 |
| **12** | 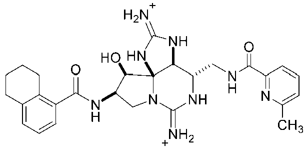 |
| **13** | 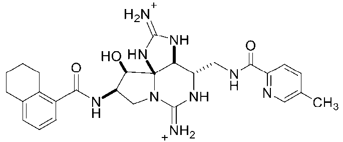 |
| **14** | 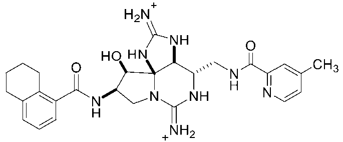 |
| **15** | 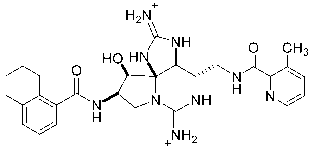 |
| **16** | 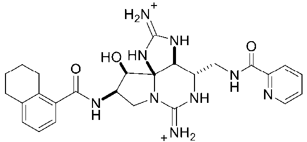 |
| **17** | 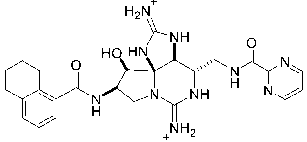 |
| **18** | 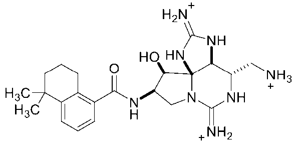 |
| **19** | 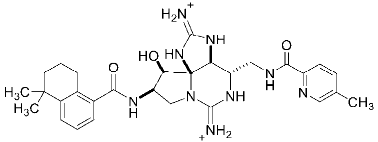 |
| **20** | 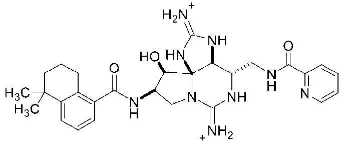 |
| **21** | 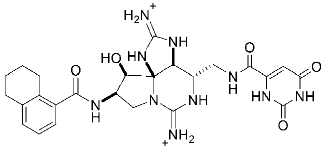 |
| **22** | 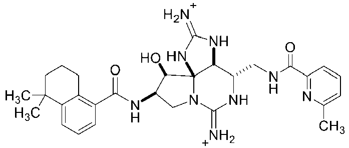 |
| **23** | 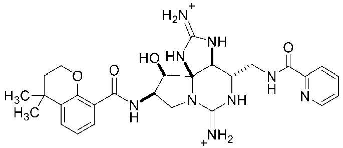 |
| **24** | 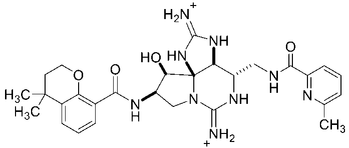 |
| **25** | 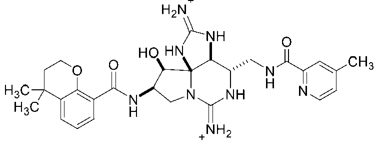 |
| **26** | 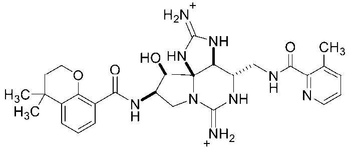 |
| **27** | 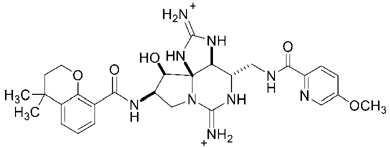 |
| **28** | 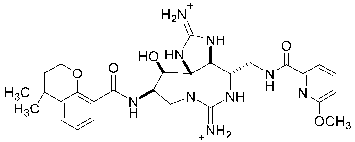 |
| **29** | 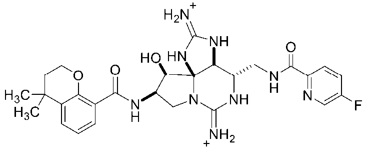 |
| **30** | 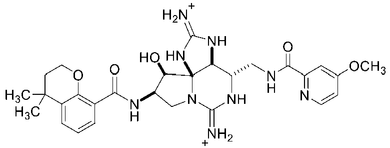 |
| **31** | 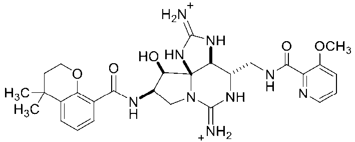 |
| **32** | 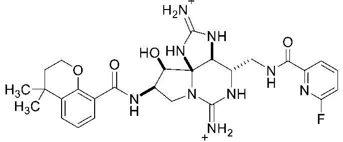 |
| **33** | 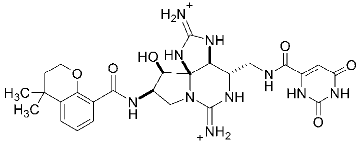 |
| **34** | 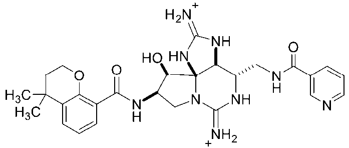 |
| **35** | 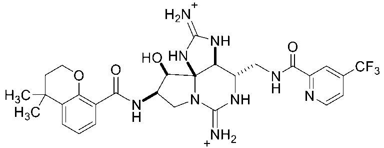 |
| **36** | 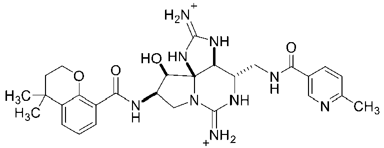 |
| **37** | 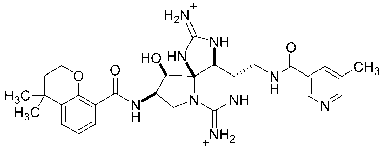 |
| **38** | 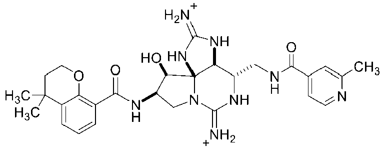 |
| **39** | 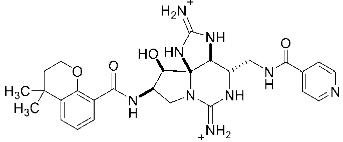 |
| **40** | 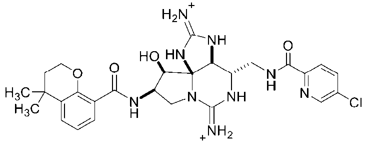 |
| **41** | 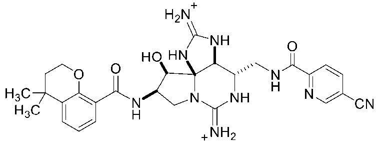 |
| **42** | 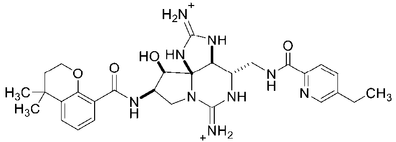 |
| **43** | 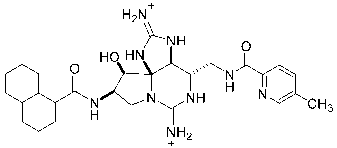 |
| **44** | 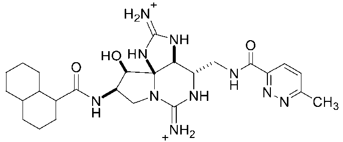 |
| **45** | 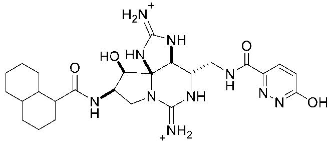 |
| **46** | 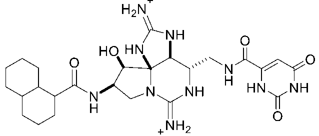 |
| **49** | 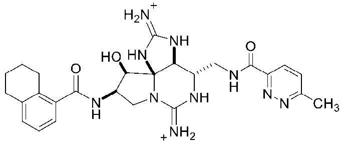 |
| **50** | 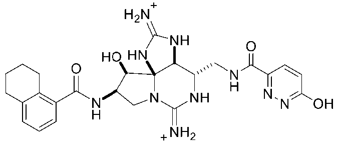 |
| **51** | 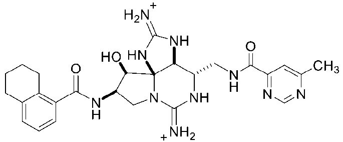 |
| **52** | 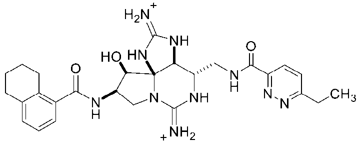 |
| **53** | 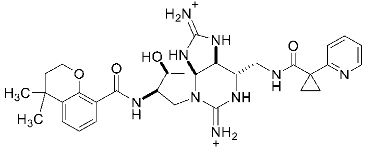 |
| **54** | 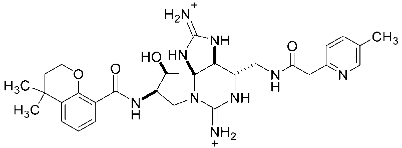 |
| **55** | 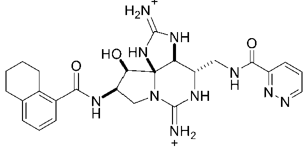 |
| **56** | 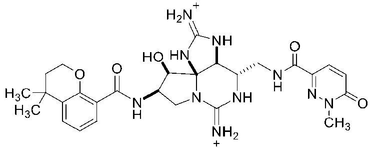 |
| **57** | 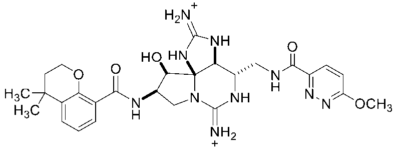 |
| **58** | 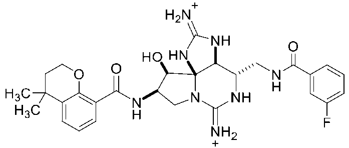 |
| **59** | 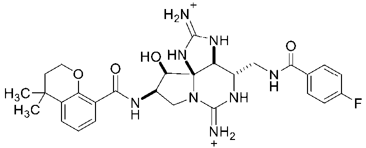 |
| **60** | 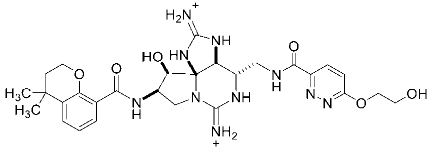 |
| **61** | 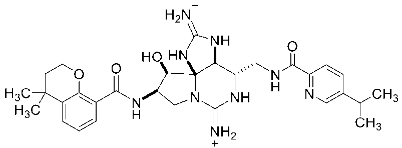 |
| **62** | 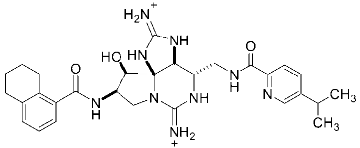 |
| **63** | 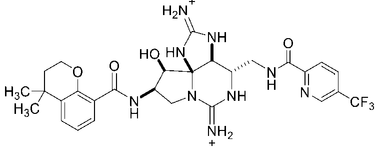 |
| **64** | 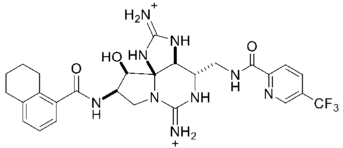 |
| **65** | 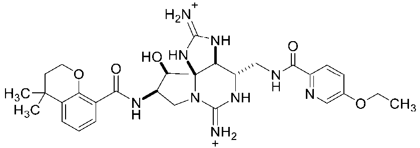 |
| **66** | 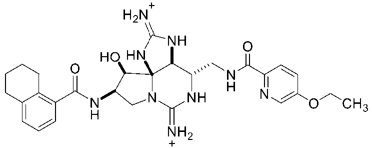 |
| **67** | 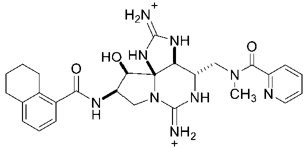 |
| **68** | 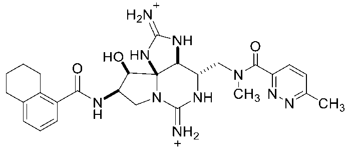 |
| **69** | 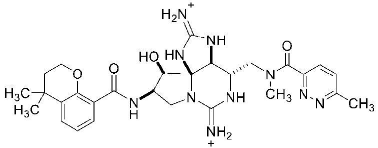 and |
| **70** | 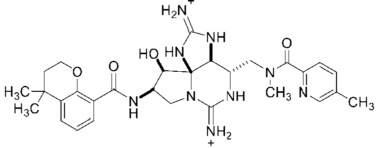 |

57. A pharmaceutical composition comprising the compound of any one of clauses 1-56 and a pharmaceutically acceptable carrier.
58. The pharmaceutical composition of clause 57, wherein the composition is an oral or injectable composition.
59. The pharmaceutical composition of clause 58, wherein the injectable composition is a subcutaneously injectable composition.
60. A method for the treatment of a condition associated with voltage-gated sodium channel function in a mammal, comprising the administration of a therapeutically or prophylactically effective amount of the compound of any one of clauses 1-56 or the pharmaceutical composition of any one of clauses 57-59.
61. The method of clause 60, wherein the mammal is a human.
62. The method of clause 60 or 61, wherein the condition is pain or wherein the condition is associated with pain.
63. The method of clause 60 or 61, wherein the condition is pain.
64. The method of clause 60 or 61, wherein the condition is associated with pain.
65. The method of any one of clauses 60-62, wherein the condition is selected from the group consisting of erythromelalgia, diabetic peripheral neuropathy, paroxysmal extreme pain disorder, complex regional pain syndrome, trigeminal neuralgia, multiple sclerosis, arthritis, osteoarthritis, postherpetic neuralgia, cancer pain, cluster headache, migraine, sciatica, endometriosis, fibromyalgia, postsurgical pain, subacute pain, chronic pain, pain and/or discomfort associated with dry eye syndrome, pain associated with (acute) corneal injuries or abrasions, acute ocular pain, chronic ocular pain, pain associated with corneal infections, pain associated with Parkinson's disease, pain associated with ALS, pain associated with ocular surgery, epilepsy, Parkinson's disease, a mood disorder, psychosis, amyotropic lateral sclerosis, glaucoma, ischemia, a spasticity disorder, and obsessive compulsive disorder.
66. The method of clause 60 or 61, wherein the condition is selected from the group consisting of acute pain, subacute pain, post-surgical pain, and ocular pain.
67. A compound of Formula (X) 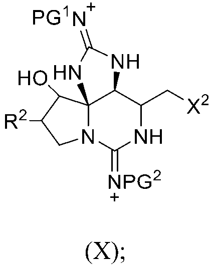 or a salt thereof,
wherein
PG¹ is a nitrogen-protecting group;
PG² is a nitrogen-protecting group;
X² is halo, CN, or N₃;
R² is -NR⁷C(O)R^{7a}, -NR⁷S(O)₂R^{7a}, or -NR⁷C(O)OR^{7a};
R⁷ is hydrogen or C₁₋₆alkyl;
R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl.
68. A method of preparing a compound of Formula (I) comprising
a) in a single step, deprotecting and reducing a compound of Formula (X-1) 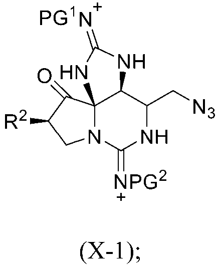 to obtain a compound of Formula (X-2): 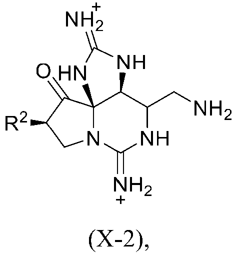 or a salt thereof;
b) reducing the ketone of Formula (X-2) to obtain a compound of Formula (X-3): 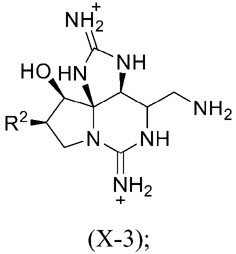 or a salt thereof;
c) coupling the compound of Formula (X-3) with a compound of Formula X⁴-LG to obtain a compound of Formula I; wherein
   PG¹ is a nitrogen-protecting group;
   PG² is a nitrogen-protecting group;
   X⁴ is -C(=O)R^{4a}, -S(O)₂R^{5a}, -C(O)NR^{6a}R^{6b}, or -C(O)OR^{8a};
   LG is a leaving group selected from the group consisting of halo, 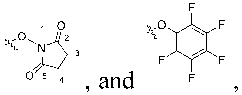 where 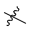 represents the point of attachment of LG to X⁴;
   R² is -NR⁷C(O)R^{7a}, -NR⁷S(O)₂R^{7a}, or -NR⁷C(O)OR^{7a};
   R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
   each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   one pair of two R^{4b} groups are present on one carbon, the second pair of two R^{4b} are present on another carbon, and each pair of R^{4b} are taken together to form an oxo group;
   R^{5a} is heterocycloalkyl, amino, C₁₋₆alkylamino, or di- C₁₋₆alkylamino;
   R^{6a} is hydrogen, or C₁₋₆alkyl;
   R^{6b} is hydrogen, C₁₋₆alkyl, C₁₋₆alkoxy or cycloalkyl;
   R^{8a} is hydrogen, C₁₋₆alkyl, cycloalkyl or phenyl;
   R⁷ is hydrogen or C₁₋₆alkyl;
   R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
   each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl;
      to yield a compound of Formula I; and
d) optionally isolating the compound of Formula I.
69. A method of preparing a compound of Formula (I) comprising
a) reducing the ketone in a compound of Formula (X-1) 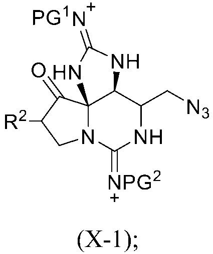 to obtain a compound of Formula (X-2B): 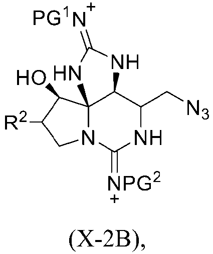 or a salt thereof;
b) in a single step, deprotecting and reducing the compound of Formula (X-2B) to obtain a compound of Formula (X-3) 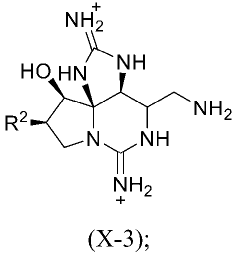 or a salt thereof;
c) coupling the compound of Formula (X-3) with a compound of Formula X⁴-LG to obtain a compound of Formula I; wherein
   PG¹ is a nitrogen-protecting group;
   PG² is a nitrogen-protecting group;
   X⁴ is -C(O)R^{4a}, -S(O)₂R^{5a}, -C(O)NR^{6a}R^{6b}, or -C(O)OR^{8a};
   LG is a leaving group selected from the group consisting of halo, 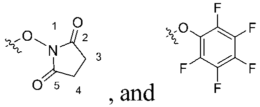 , where 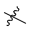 represents the point of attachment of LG to X⁴;
   R² is -NR⁷C(O)R^{7a}, -NR⁷S(O)₂R^{7a}, or -NR⁷C(O)OR^{7a};
   R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
   each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
   one pair of two R^{4b} groups are present on one carbon, the second pair of two R^{4b} are present on another carbon, and each pair of R^{4b} are taken together to form an oxo group;
   R^{5a} is heterocycloalkyl, amino, C₁₋₆alkylamino, or di- C₁₋₆alkylamino;
   R^{6a} is hydrogen, or C₁₋₆alkyl;
   R^{6b} is hydrogen, C₁₋₆alkyl, C₁₋₆alkoxy or cycloalkyl;
   R^{8a} is hydrogen, C₁₋₆alkyl, cycloalkyl or phenyl;
   R⁷ is hydrogen or C₁₋₆alkyl;
   R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
   each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl;
      to yield a compound of Formula I; and
d) optionally isolating the compound of Formula I.

## Claims

1. A compound of Formula (X-4) 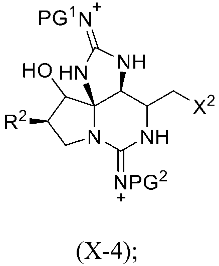 or a salt thereof,
wherein
PG¹ is a nitrogen-protecting group;
PG² is a nitrogen-protecting group;
X² is halo, CN, or N₃;
R² is -NR⁷C(O)R^{7a}, -NR⁷S(O)₂R^{7a}, or -NR⁷C(O)OR^{7a};
R⁷ is hydrogen or C₁₋₆alkyl;
R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl.

2. The compound of claim 1, wherein X² is N₃.

3. The compound of claim 1, wherein the compound of (X-4) is

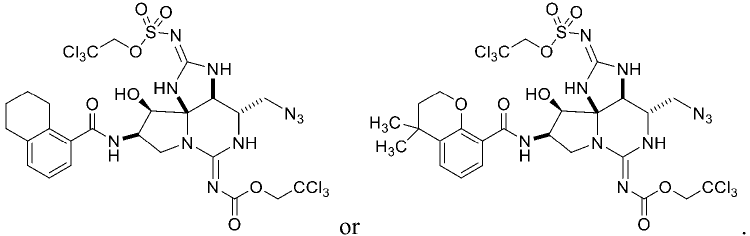

4. A method of preparing a compound of Formula (I) comprising
a) in a single step, deprotecting and reducing a compound of Formula (X-1) 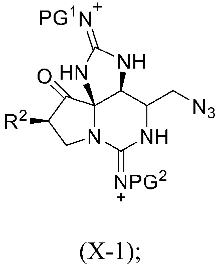 to obtain a compound of Formula (X-2): 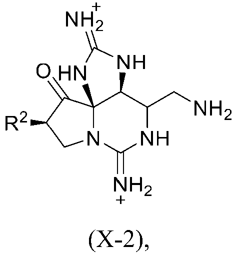 or a salt thereof;
b) reducing the ketone of Formula (X-2) to obtain a compound of Formula (X-3): 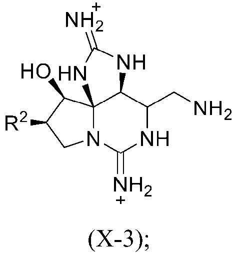 or a salt thereof;
c) coupling the compound of Formula (X-3) with a compound of Formula X⁴-LG to obtain a compound of Formula I; 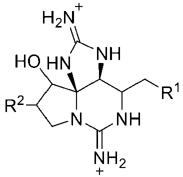 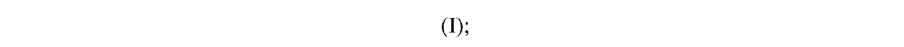 wherein
PG¹ is a nitrogen-protecting group;
PG² is a nitrogen-protecting group;
X⁴ is -C(=O)R^{4a}, -S(O)₂R^{5a}, -C(O)NR^{6a}R^{6b}, or -C(O)OR^{8a};
LG is a leaving group selected from the group consisting of halo, 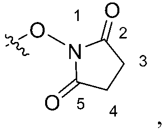
and 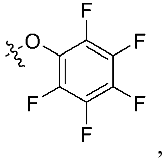
where 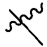
represents the point of attachment of LG to X⁴;
R¹ is -NR⁴C(O)R^{4a}, -NR^{3a}R³, -CH₂NR⁴C(O)R^{4a}, heteroaryl, -CH₂NH₂, -CH₂CH₂NH₂, -CH₂NH₃⁺, -CH₂CH₂NH₃⁺, -NR⁵S(O)₂R^{5a}, -CH₂NR⁵S(O)₂R^{5a}, -NR⁶C(O)NR^{6a}R^{6b}, -CH₂NR⁶C(O)NR^{6a}R^{6b}, -NR⁸C(O)OR^{8a}, or -CH₂NR⁸C(O)OR^{8a};
R² is -NR⁷C(O)R^{7a}, -NR⁷S(O)₂R^{7a}, or -NR⁷C(O)OR^{7a};
R⁴ is hydrogen;
R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
one pair of two R^{4b} groups are present on one carbon, the second pair of two R^{4b} are present on another carbon, and each pair of R^{4b} are taken together to form an oxo group;
R⁵ is hydrogen;
R^{5a} is heterocycloalkyl, amino, C₁₋₆alkylamino, or di- C₁₋₆alkylamino;
R⁶ is hydrogen;
R^{6a} is hydrogen, or C₁₋₆alkyl;
R^{6b} is hydrogen, C₁₋₆alkyl, C₁₋₆alkoxy or cycloalkyl;
R⁸ is hydrogen or C₁₋₆alkyl;
R^{8a} is hydrogen, C₁₋₆alkyl, cycloalkyl or phenyl;
R⁷ is hydrogen or C₁₋₆alkyl;
R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl;
to yield a compound of Formula I; and
d) optionally isolating the compound of Formula I.

5. A method of preparing a compound of Formula (I) comprising
a) reducing the ketone in a compound of Formula (X-1) 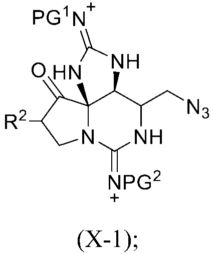 to obtain a compound of Formula (X-2B): 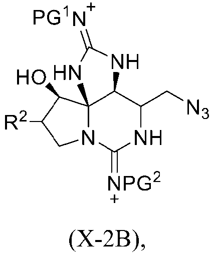 or a salt thereof;
b) in a single step, deprotecting and reducing the compound of Formula (X-2B) to obtain a compound of Formula (X-3) 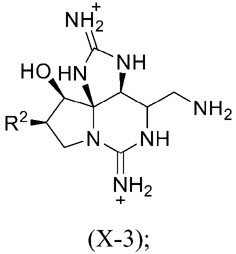 or a salt thereof;
c) coupling the compound of Formula (X-3) with a compound of Formula X⁴-LG to obtain a compound of Formula I; 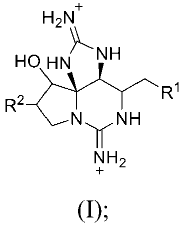 wherein
PG¹ is a nitrogen-protecting group;
PG² is a nitrogen-protecting group;
X⁴ is -C(O)R^{4a}, -S(O)₂R^{5a}, -C(O)NR^{6a}R^{6b}, or -C(O)OR^{8a};
LG is a leaving group selected from the group consisting of halo, 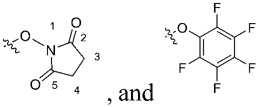 , where 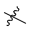 represents the point of attachment of LG to X⁴;
R¹ is -NR⁴C(O)R^{4a}, -NR^{3a}R³, -CH₂NR⁴C(O)R^{4a}, heteroaryl, -CH₂NH₂, -CH₂CH₂NH₂, -CH₂NH₃⁺, -CH₂CH₂NH₃⁺, -NR⁵S(O)₂R^{5a}, -CH₂NR⁵S(O)₂R^{5a}, -NR⁶C(O)NR^{6a}R^{6b}, -CH₂NR⁶C(O)NR^{6a}R^{6b}, -NR⁸C(O)OR^{8a}, or -CH₂NR⁸C(O)OR^{8a};
R² is -NR⁷C(O)R^{7a}, -NR⁷S(O)₂R^{7a}, or -NR⁷C(O)OR^{7a};
R⁴ is hydrogen
R^{4a} is hydrogen; C₁₋₆alkyl; halo-C₁₋₆alkyl; amino-C₁₋₆alkyl; C₁₋₆alkylaminoC₁₋₆alkyl; di-C₁₋₆alkylaminoC₁₋₆alkyl; C₁₋₆alkoxyC₁₋₆alkyl; cycloalkyl optionally substituted with 1, 2, 3, or 4 R^{4b} groups; cycloalkylalkyl optionally substituted with 1, 2, 3, or 4 R^{4b} groups; aryl optionally substituted with 1, 2, 3, or 4 R^{4b} groups; aralkyl where the aryl portion is optionally substituted with 1, 2, 3, or 4 R^{4b} groups; heterocycloalkyl optionally substituted with 1, 2, 3, or 4 R^{4b} groups; heteroaryl optionally substituted with 1, 2, 3, or 4 R^{4b} groups; heteroaralkyl where the heteroaryl portion is optionally substituted with 1, 2, 3, or 4 R^{4b} groups; heterocyclic optionally substituted with 1, 2, 3, or 4 R^{4b} groups; or heterocyclicalkyl where the heterocyclic portion is optionally substituted with 1, 2, 3, or 4 R^{4b} groups;
each of the 1, 2, 3, or 4 R^{4b}, when present, is independently C₁₋₆alkyl, hydroxyC₁₋₆alkyloxy, hydroxy, halo, halo-C₁₋₆alkyl, C₁₋₆alkoxy, cyano, phenyl, or heteroaryl; or
2 R^{4b} groups, when on the same carbon atom, are taken together to form an oxo group, and the other 2 R^{4b}, when present, are independently C₁₋₆alkyl, hydroxyC₁₋₆alkyloxy, hydroxy, halo, halo-C₁₋₆alkyl, C₁₋₆alkoxy, cyano, phenyl, or heteroaryl; or
one pair of two R^{4b} groups are present on one carbon, the second pair of two R^{4b} are present on another carbon, and each pair of R^{4b} are taken together to form an oxo group;
R⁵ is hydrogen;
R^{5a} is heterocycloalkyl, amino, C₁₋₆alkylamino, or di- C₁₋₆alkylamino;
R⁶ is hydrogen;
R^{6a} is hydrogen, or C₁₋₆alkyl;
R^{6b} is hydrogen, C₁₋₆alkyl, C₁₋₆alkoxy or cycloalkyl;
R⁸ is hydrogen or C₁₋₆alkyl;
R^{8a} is hydrogen, C₁₋₆alkyl, cycloalkyl or phenyl;
R⁷ is hydrogen or C₁₋₆alkyl;
R^{7a} is aryl optionally substituted with 1, 2, 3, or 4 R^{7b}; heterocyclic optionally substituted with 1, 2, 3, or 4 R^{7b}; or biphenyl optionally substituted with 1, 2, 3, or 4 R^{7b}; and
each R^{7b}, when present, is independently halo, C₁₋₆alkyl, halo-C₁₋₆alkyl, aryl-C₁₋₆alkyl, hydroxy, C₁₋₆alkoxy, halo-C₁₋₆alkoxy, aryloxy, nitro, C₁₋₆alkylthio, halo-C₁₋₆alkylthio, C₁₋₆alkylsulfinyl, halo-C₁₋₆alkylsulfinyl, C₁₋₆alkylsulfonyl, halo-C₁₋₆alkylsulfonyl, amino, C₁₋₆alkylamino, di-C₁₋₆alkylamino, -C(O)(heterocycloalkyl), phenyl, or cyano; where the aryl in aryloxy and aryl-C₁₋₆alkyl are optionally substituted with 1, 2, or 3 groups independently selected from C₁₋₆alkyl, halo, and halo-C₁₋₆alkyl;
to yield a compound of Formula I; and
d) optionally isolating the compound of Formula I.

6. The compound of claim 1, 4, or 5, wherein PG¹ and PG² are independently selected from 9-fluorenylmethyloxycarbonyl (Fmoc), tert-butoxycarbonyl (Boc), benzyloxycarbonyl (CBz), acetyl, trichloroacetyl, trifluoroacetyl, -C(O)OCH₂CCl₃ (Troc), p-methoxyphenyl, benzyl, p-methoxybenzyl, p-methoxybenzylcarbonyl, triphenylmethyl, benzylidenyl, 2,2,2-trichloroethoxysulfonyl (Tces), p-methoxybenzenesulfonyl (Mbs) and p-toluenesulfonyl (tosyl).

7. The compound of claim 1, 4, or 5, wherein PG¹ is 2,2,2-trichloroethoxysulfonyl (Tces).

8. The compound of claim 1, 4, or 5, wherein PG² is -C(O)OCH₂CCl₃ (Troc).

9. The compound of claim 1, 4, or 5, wherein R² is -NR⁷C(O)R^{7a}.

10. The compound of claim 1, 4, or 5, wherein R⁷ is hydrogen.

11. The compound of claim 1, 4, or 5, wherein R^{7a} is aryl optionally substituted with 1, 2, 3, or 4 R^{7b}.

12. The compound of claim 1, 4, or 5, wherein R^{7a} is heterocyclic optionally substituted with 1, 2, 3, or 4 R^{7b}.

13. The compound of claim 1, 4, or 5, wherein each R^{7b}, when present, is independently C₁₋₆alkyl.

14. The compound of claim 4 or 5, wherein R¹ is -NHC(O)R^{4a}.

15. The compound of claim 4 or 5, wherein X⁴ is -C(=O)R^{4a}.

16. A compound selected from 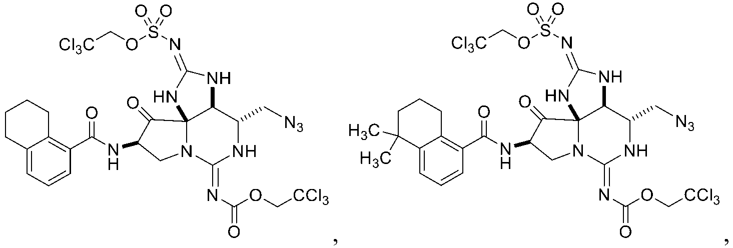 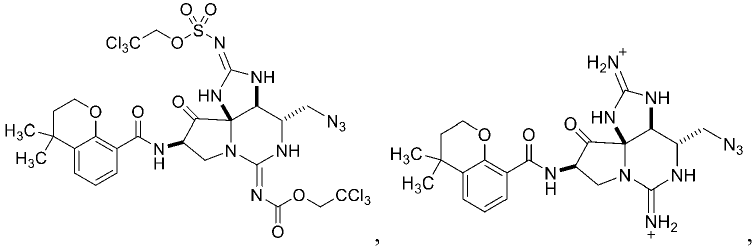 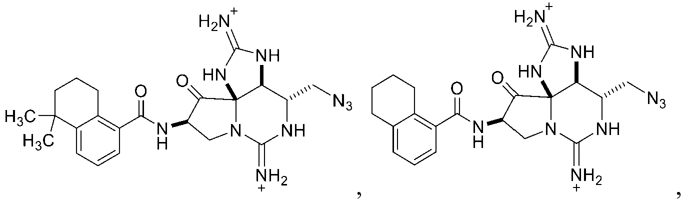 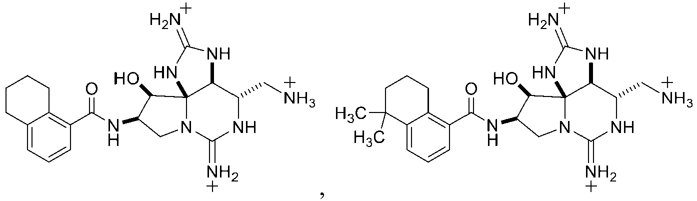 and 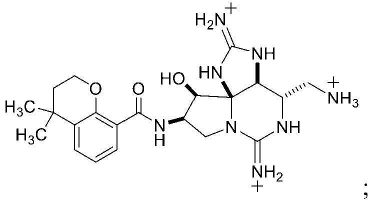 or a salt thereof.

17. The compound of claim 16 of the formula:
